# EUROPÄISCHE PATENTSCHRIFT

(11) **EP 0 599 906 B1**
(45) Veröffentlichungstag und Bekanntmachung des Hinweises auf die Patenterteilung: **15.01.1997**
(21) Anmeldenummer: 92917128.8
(22) Anmeldetag: 07.08.1992
(51) Int. Cl.: C07D 333/68, C07D 333/70, C07D 275/04, C07D 335/02, C07D 309/06, C07D 261/08, A01N 25/32, C07D 275/02

(54) **HERBIZIDE MITTEL, ENTHALTEND 3-AMINOBENZO B]THIOPHENE ALS ANTIDOTS**
HERBICIDES CONTAINING 3-AMINOBENZO B]THIOPHENES AS AN ANTIDOTE
HERBICIDES CONTENANT DES 3-AMINOBENZO B]THIOPHENE COMME ANTIDOTES

(30) Priorität: 16.08.1991 DE 4126999
(43) Veröffentlichungstag der Anmeldung: 08.06.1994
(73) Patentinhaber: BASF Aktiengesellschaft, 67063 Ludwigshafen (DE)
(72) Erfinder: HAGEN, Helmut, D-6710 Frankenthal (DE); NILZ, Gerhard, D-6701 Dannstadt (DE); ROETSCH, Thomas, D-6700 Ludwigshafen (DE); WALTER, Helmut, D-6719 Obrigheim (DE); LANDES, Andreas, D-6703 Limburgerhof (DE)
(86) Internationale Anmeldenummer: EP9201798
(87) Internationale Veröffentlichungsnummer: WO9304057

(56) Entgegenhaltungen:
- EP-A- 0 187 487
- EP-A- 0 378 508
- DE-A- 3 212 135
- DE-A- 4 039 734
- CHEMICAL AND PHARMACEUTICAL BULLETIN. Bd. 30, Nr. 3, 1982, TOKYO JP Seiten 851
- - 858 Y. TOMIOKA ET AL. 'Studies on Aromatic Nitro Compounds. IV. Reactions of 2-Nitronaphthlenes with Active Methylene Compounds in the Presence of Bases' in der Anmeldung erw hnt
- CHEMICAL ABSTRACTS, vol. 95, no. 9, 31. August 1981, Columbus, Ohio, US; abstract no. 80621c, K. GEWALD ET AL. 'Syntheses of 3-amino-2,4- dicyanothiopphenols using derivatives of 2-amino-3-cyanothiopyran-6 ylidenecyanoacetic acid' Seite 753 ;Spalte 1 ; in der Anmeldung erw hnt

## Beschreibung

Die vorliegende Erfindung betrifft herbizide Mittel enthaltend mindestens ein antagonistisch wirksames 3-Aminobenzo[b]thiophen der allgemeinen Formel I 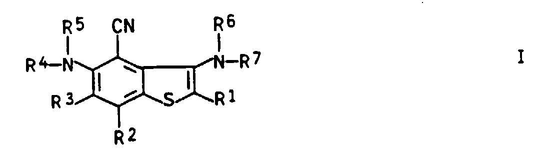 in der die Variablen folgende Bedeutung haben:
R¹ eine Gruppe -COX oder -COOX, wobei
X Wasserstoff, Halogen, die Aminogruppe, die unsubstituiert sein oder ein bis drei Substituenten tragen kann, ausgewählt aus einer Gruppe bestehend aus C₁-C₄-Alkyl-, Hydroxy-C₁-C₄-alkyl-, Phenyl-, und Benzylsubstituenten,
   eine C₁-C₁₀-Alkylgruppe, eine C₂-C₈-Alkenylgruppe, eine C₃-C₁₀-Cycloalkylgruppe oder eine C₃-C₁₀-Cycloalkenylgruppe, wobei die letztgenannten vier Gruppen unsubstituiert oder partiell oder vollständig halogeniert sein können und wobei die letztgenannten vier Gruppen auch eine oder zwei weitere Carboxyloder Carbonylgruppen enthalten können,
   einen 5- oder 6-gliedrigen aromatischen Heterocyclus mit einem Sauerstoff-und einem Schwefelatom oder mit 1 bis 3 Heteroatomen, ausgewählt aus einer Gruppe bestehend aus 1 bis 3 Stickstoffatomen und einem Sauerstoff- oder Schwefelatom, wobei nicht drei Heteroatome gleichzeitig benachbart sein können,
   die Phenyl- oder die Naphthylgruppe, wobei diese Gruppen einen bis drei Substituenten tragen können, ausgewählt aus einer Gruppe bestehend aus Halogen, -COY oder -COOY, und wobei diese Gruppen noch zusätzlich so viele Halogenatome tragen können, wie substituierbare C-Atome vorhanden sind;
Y steht für C₁-C₆-Alkyl, C₃-C₆-Cycloalkyl oder Phenyl;
R², R³ Wasserstoff, Cyano, Nitro, Halogen, Mercapto, die Aminogruppe, deren Stickstoffatom ein bis drei Substituenten tragen kann, ausgewählt aus einer Gruppe bestehend aus C₁-C₄-Alkyl-, Hydroxy-C₁-C₄-alkyl-, Phenyl-, und Benzylsubstituenten, eine C₁-C₁₀-Alkylgruppe, eine C₁-C₆-Alkoxygruppe, eine C₂-C₈-Alkenylgruppe, eine C₃-C₁₀-Cycloalkylgruppe oder eine C₃-C₁₀-Cycloalkenylgruppe, wobei die letztgenannten vier Gruppen partiell oder vollständig halogeniert sein können, die Phenyl- oder die Naphthylgruppe,
   einen 5- oder 6-gliedrigen aromatischen Heterocyclus mit einem Sauerstoff- und einem Schwefelatom oder mit einem bis drei Heteroatomen, ausgewählt aus einer Gruppe bestehend aus drei Stickstoffatomen und einem Sauerstoff- oder Schwefelatom, wobei im Falle von drei Heteroatomen nicht alle gleichzeitig benachbart sein können;
R⁴ bis R⁷ Wasserstoff, die Phenylgruppe, die Naphthylgruppe oder einen 5- oder 6-gliedrigen aromatischen Heterocyclus mit einem Sauerstoff- und einem Schwefelatom oder mit einem bis drei Heteroatomen, ausgewählt aus einer Gruppe bestehend aus drei Stickstoffatomen und einem Sauerstoff- oder Schwefelatom, wobei im Falle von drei Heteroatomen nicht alle gleichzeitig benachbart sein können; wobei die aromatischen und heteroaromatischen Gruppen noch einen bis drei Substituenten tragen können, ausgewählt aus einer Gruppe bestehend aus Halogen, -COY oder -COOY, und wobei diese Gruppen noch zusätzlich so viele Halogenatome tragen können, wie substituierbare C-Atome vorhanden sind, eine C₁-C₁₀-Alkylgruppe, eine C₂-C₆-Alkenylgruppe, eine C₃-C₁₀-Cycloalkylgruppe, eine C₃-C₁₀-Cycloalkenylgruppe;
oder R⁴ und R⁵ und/oder R⁶ und R⁷ bilden zusammen mit dem Stickstoffatom, an das sie gebunden sind, einen 5- bis 7-gliedrigen Ring;
sowie die basischen Salze derjenigen Verbindung I, die mindestens eine Carboxyl-, Hydroxythiocarbonyl- oder Sulfonsäuregruppe tragen und die sauren Salze derjenigen Verbindungen I, die ein basisches Stickstoffatom enthalten,
und mindestens einen herbiziden Wirkstoff aus
   A) der Gruppe der Cyclohexenon-Derivate der allgemeinen Formel II 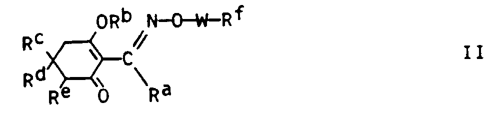 in der die Substituenten folgende Bedeutung haben:
      R^{a}
         eine C₁-C₆-Alkylgruppe;
      R^{b}
         Wasserstoff, das Äquivalent eines landwirtschaftlich brauchbaren Kations, eine C₂-C₈-Alkylcarbonyloxygruppe, eine C₁-C₁₀-Alkylsulfonylgruppe, eine C₁-C₁₀-Alkylphosphonylgruppe oder die Benzoyl-, Benzolsulfonyl- oder Benzolphosphonylgruppe, wobei die drei letztgenannten Gruppen noch jeweils 1 bis 5 Halogenatome tragen können;
      R^{c}
         Wasserstoff, die Cyanogruppe, die Formylgruppe, eine C₁-C₆-Alkylgruppe, eine C₁-C₄-Alkoxy-C₁-C₆-alkyl- oder C₁-C₄-Alkylthio-C₁-C₆-alkylgruppe, eine Phenoxy-C₁-C₆-alkyl-, Phenylthio-C₁-C₆-alkyl-, Pyridyloxy-C₁-C₆-alkyl- oder Pyridylthio-C₁-C₆-alkylgruppe, wobei die Phenyl- und Pyridylringe jeweils noch einen bis drei Reste tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Alkenyl, C₃-C₆-Alkenyloxy, C₃-C₆-Alkinyl, C₃-C₆-Alkinyloxy und -NR^{g}R^{h}, wobei
         - R^{g}: Wasserstoff, C₁-C₄-Alkyl, C₃-C₆-Alkenyl, C₃-C₆-Alkinyl, C₁-C₆-Acyl oder Benzoyl, das einen bis drei Reste tragen kann, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy und C₁-C₄-Alkylthio und
         - R^{h}: Wasserstoff, C₁-C₄-Alkyl, C₃-C₆-Alkenyl oder C₃-C₆-Alkinyl
         bedeuten;
      eine C₃-C₇-Cycloalkyl- oder eine C₅-C₇-Cycloalkenylgruppe, wobei diese Gruppen noch einen bis drei Reste tragen können, ausgewählt aus einer Gruppe bestehend aus Hydroxyl, Halogen, C₁-C₄-Alkyl, partiell oder vollständig halogeniertem C₁-C₄-Alkyl, C₁-C₄-Alkoxy, C₁-C₄-Alkylthio, Benzylthio, C₁-C₄-Alkylsulfonyl, C₁-C₄-Alkylsulfenyl und C₁-C₄-Alkylsulfinyl,
      ein 5-gliedriger gesättigter Heterocyclus, der ein oder zwei Sauerstoff- oder Schwefelatome oder ein Sauerstoff- und ein Schwefelatom als Heteroatome enthält, und der noch einen bis drei Reste tragen kann, ausgewählt aus einer Gruppe bestehend aus C₁-C₄-Alkyl, partiell oder vollständig halogeniertem C₁-C₄-Alkyl, C₁-C₄-Alkoxy und C₁-C₄-Alkylthio,
      ein 6- oder 7-gliedriger gesättigter oder ein- oder zweifach ungesättigter Heterocyclus, der ein oder zwei Sauerstoff- oder Schwefelatome oder ein Sauerstoff und ein Schwefelatom als Heteroatome enthält, und der noch einen bis drei Reste tragen kann, ausgewählt aus einer Gruppe bestehend aus Hydroxyl, Halogen, C₁-C₄-Alkyl, partiell oder vollständig halogeniertem C₁-C₄-Alkyl, C₁-C₄-Alkoxy, C₁-C₄-Alkylthio,
      ein 5-gliedriger Heteroaromat, enthaltend ein bis drei Heteroatome, ausgewählt aus einer Gruppe bestehend aus ein oder zwei Stickstoffatomen und einem Sauerstoff- oder Schwefelatom, wobei der Heteroaromat noch einen bis drei Reste tragen kann, ausgewählt aus einer Gruppe bestehend aus Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₂-C₆-Alkenyl, C₂-C₆-Alkenyloxy, C₃-C₆-Alkinyloxy und C₁-C₄-Alkoxy-C₁-C₄-alkyl,
      eine Phenyl- oder Pyridylgruppe, die jeweils noch einen bis drei Reste tragen kann, ausgewählt aus einer Gruppe bestehend aus Nitro, Cyano, Formyl, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Alkenyl, C₃-C₆-Alkenyloxy, C₃-C₆-Alkinyl, C₃-C₆-Alkinyloxy und -NR^{g}R^{h}, wobei R^{g} und R^{h} die oben genannte Bedeutung haben;
      R^{d}
         Wasserstoff, die Hydroxylgruppe oder, wenn R^{c} für eine C₁-C₆-Alkylgruppe steht, eine C₁-C₆-Alkylgruppe;
      R^{e}
         Wasserstoff, Halogen, die Cyanogruppe, eine C₁-C₄-Alkoxycarbonylgruppe oder eine C₁-C₄-Alkylketoximgruppe;
      W
         eine C₁-C₆-Alkylen-, C₃-C₆-Alkenylen- oder C₃-C₆-Alkinylenkette, die jeweils noch ein bis drei Reste tragen können, ausgewählt aus einer Gruppe bestehend aus drei C₁-C₃-Alkylsubstituenten, drei Halogenatomen und einem Methylensubstituenten;
      eine C₃-C₆-Alkylen- oder C₄-C₆-Alkenylenkette, die beide noch eine bis drei C₁-C₃-Alkylreste tragen können, wobei jeweils eine Methylengruppe der Ketten durch ein Sauerstoff- oder Schwefelatom, eine Sulfoxid- oder Sulfongruppe oder eine Gruppe -N(Rⁱ)-substituiert sein kann, wobei
         - Rⁱ: für Wasserstoff, C₁-C₄-Alkyl, C₃-C₆-Alkenyl oder C₃-C₆-Alkinyl steht;
         - R^{f}: Wasserstoff; die Vinylgruppe;
         eine Gruppe -CH=CH-Z, wobei Z für Cyano, Halogen, einen C₁-C₄-Alkylrest, einen partiell oder vollständig halogenierten C₁-C₄-Alkylrest, einen C₃-C₆-Cycloalkylrest, der gewünschtenfalls seinerseits noch einen bis drei Substituenten tragen kann, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy; den Carboxylrest, einen C₁-C₈-Alkoxycarbonylrest, den Benzyloxycarbonylrest, den Phenyl-, Thienyl- oder Pyridylrest, wobei diese drei aromatischen Reste unsubstituiert sein oder einen bis drei Substituenten tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio sowie C₃-C₆-Cycloalkyl, wobei der Cycloalkylsubstituent unsubstituiert sein oder seinerseits noch einen bis drei Reste tragen kann, ausgewählt aus einer Gruppe bestehend aus Halogen, C₁-C₄-Alkyl, partiell oder vollständig halogeniertem C₁-C₄-Alkyl und C₁-C₄-Alkoxy, steht;
      die Ethinylgruppe, die einen der folgenden Reste tragen kann:
         einen C₁-C₄-Alkylrest, einen C₃-C₆-Cycloalkylrest, der gewünschtenfalls noch einen bis drei Substituenten tragen kann, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy, oder einen Phenyl-, Thienyl- oder Pyridylrest, wobei diese aromatischen Reste unsubstituiert sein oder jeweils noch einen bis drei Substituenten tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy und C₁-C₄-Alkylthio;
      die Phenylgruppe, eine Halogenphenylgruppe, eine Dihalogenphenylgruppe, eine 5-gliedrige heteroaromatische Gruppe mit ein bis drei Heteroatomen, ausgewählt aus einer Gruppe bestehend aus ein bis drei Stickstoffatomen und einem Sauerstoff- oder Schwefelatom, oder eine 6-gliedrige heteroaro-. matische Gruppe mit ein bis vier Stickstoffatomen, die nicht alle gleichzeitig benachbart sein können,
         wobei die Phenyl- und Heteroarylgruppen gewünschtenfalls noch einen bis drei Reste tragen können, ausgewählt aus einer Gruppe bestehend aus Nitro, einen C₁-C₄-Alkoxyrest, einen C₁-C₄-Alkylthiorest, einen partiell oder vollständig halogenierten C₁-C₄-Alkoxyrest, Resten Z,
         oder einem Rest -NR^{k}R^{l}, wobei
         - R^{k}: Wasserstoff, C₁-C₄-Alkyl, C₃-C₆-Alkenyl oder C₃-C₆-Alkinyl und
         - R^{l}: Wasserstoff, C₁-C₄-Alkyl, C₃-C₆-Alkenyl, C₃-C₆-Alkinyl, C₁-C₆-Acyl oder Benzoyl, das gewünschtenfalls noch einen bis drei Substituenten tragen kann, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy und C₁-C₄-Alkylthio,
         bedeuten,
      oder
   B) der Gruppe der 2-(4-Heteroaryloxy)- oder 2-(4-Aryloxy)-phenoxyessigsäurederivate der Formel III 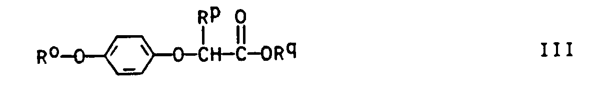 in der die Substituenten folgende Bedeutung haben:
      R^{o}
         die Phenylgruppe, die Pyridylgruppe, eine Benzoxazylgruppe, eine Benzthiazylgruppe oder eine Benzpyrazinylgruppe, wobei diese aromatischen Ringsysteme unsubstituiert sein oder ein bis zwei der folgenden Reste tragen können: Halogen, Nitro, C₁-C₄-Alkyl, partiell oder vollständig halogeniertes C₁-C₄-Alkyl und/oder partiell oder vollständig halogeniertes C₁-C₄-Alkoxy;
      R^{p}
         Wasserstoff oder die Methylgruppe und
      R^{q}
         Wasserstoff, eine C₁-C₄-Alkylgruppe, eine C₃-C₄-Alkenylgruppe, eine C₃-C₄-Alkinylgruppe, eine C₁-C₄-Alkoxy-C₁-C₄-alkylgruppe, eine C₃-C₄-Alkylideniminooxy-C₂-C₃-alkylgruppe, eine Tetrahydrofuranylmethylgruppe, eine Isoxazolidingruppe oder das Äquivalent eines pflanzenverträglichen Kations.

Außerdem betrifft die Erfindung ein Verfahren zur selektiven Bekämpfung von unerwünschtem Pflanzenwuchs auf Anbauflächen von Kulturpflanzen mit diesen herbiziden Mitteln sowie neue 3-Aminobenzo[b]thiophenderivate I'.

3-Aminobenzo[b]thiophenderivate vom Typ der Verbindung I sind aus folgenden Druckschriften bekannt:
- R.J.Beck, J. org. Chem. 37(21), 3224-3226 (1972);
- H.Schaefer et al., J. Prakt. Chem. 327(2), 328-332 (1985);
- K.Gewald et al., Z. Chem. 21(5), 183-184 (1981);
- DE-A-37 40 984;
- EP-A-323 590;
- DE-A-38 27 253;
- EP-A-201 165;
- EP-A-187 487;
- DE-A-34 29 830;
- EP-A-378 508 und
- aus der älteren Anmeldung DE-A 40 39 734.

Die in den genannten Druckschriften beschriebenen Verbindungen unterscheiden sich jedoch von den 3-Aminobenzo[b]thiophenen I in den Positionen 4 (Cyanogruppe) und 5 (Aminogruppe). Ferner ist eine antagonistische Wirkung der bekannten Verbindungen in Kombination mit herbiziden Wirkstoffen der genannten Literatur nicht zu entnehmen.

Gemäß der EP-A-378 508 können 3-Aminothiophene der Formel 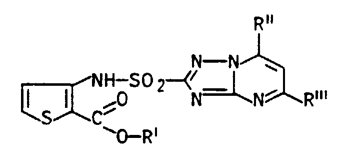 (R' = Alkyl oder Cycloalkyl; R", R"' = Wasserstoff, Alkyl, Trifluormethyl oder Cyclopropyl)

als antagonistisch wirksame Verbindungen in herbiziden Formulierungen verwendet werden. Dieser Druckschrift ist jedoch kein Hinweis zu entnehmen, ob (annellierte) Substituenten in 4- und 5-Position des Thiophenringes möglich sind.

Schließlich werden in der DE-A 40 39 734 2-Aminothiophene mit ebenfalls antagonistischer Wirkung auf Herbizide beschrieben.

Herbizide Mischungen, die aus der EP-A 378 508 und der DE-A 40 39 734 bekannte Antagonisten enthalten, können allerdings auch eine ertragsmindernde Nebenwirkung haben.

Aufgabe der vorliegenden Erfindung war es deshalb, herbizide Mittel bereitzustellen, die eine gute Bekämpfung unerwünschter Pflanzen gewährleisten, ohne jedoch die Nutzpflanzen nennenswert zu schädigen oder deren Ernteertrag wesentlich herabzusetzen.

Gemäß dieser Aufgabe wurden die Eingangs definierten herbiziden Mittel gefunden.

Des weiteren wurden Verfahren zur Behandlung von Pflanzenkulturen mit den antagonistisch wirksamen Verbindungen I und den Herbiziden II oder den Herbiziden III gefunden, wobei es unerheblich ist, ob die Verbindungen I und II oder I und III gemeinsam oder getrennt formuliert und ausgebracht werden und in welcher Reihenfolge die Applikation bei getrennter Ausbringung erfolgt.

Die herbiziden Mittel enthalten mindestens eine antagonistisch wirksame Verbindung I und mindestens ein Herbizid II oder ein Herbizid III.

Es können jedoch noch weitere antagonistisch oder herbizid wirksame Verbindungen in den erfindungsgemäßen herbiziden Mitteln enthalten sein.

Derivate I mit sauren Endgruppen oder mit basischen Stickstoffatomen können in Form ihrer landwirtschaftlich brauchbaren Salze vorliegen.

Als landwirtschaftlich brauchbare Salze kommen im allgemeinen die Salze von solchen Säuren oder Basen in Betracht, welche die antagonistische Wirkung von I nicht beeinträchtigen.

Als Säureadditionssalze eignen sich beispielsweise die Hydrochloride und -bromide, Sulfate, Nitrate, Phosphate, Oxalate oder die Dodecylbenzolsulfonate.

Als basische Salze eignen sich beispielsweise diejenigen der Alkalimetalle, insbesondere die Natrium- und Kaliumsalze, die der Erdalkalimetalle, insbesondere Calcium-, Magnesium- und Bariumsalze und die der übergangsmetalle, insbesondere Mangan-, Kupfer-, Zink- und Eisensalze sowie die Ammoniumsalze, die gewünschtenfalls ein bis drei C₁-C₄-Alkyl-, Hydroxy-C₁-C₄-alkylsubstituenten und/oder einen Phenyl- oder Benzylsubstituenten tragen können, insbesondere Diisopropylammonium-, Tetramethylammonium-, Tetrabutylammonium-, Trimethylbenzylammonium- und Trimethyl-(2-hydroxyethyl)-ammoniumsalze, die Phosphoniumsalze, die Sulfoniumsalze, insbesondere Tri-(C₁-C₄-)alkylsufoniumsalze und die Sulfoxoniumsalze, insbesondere Tri-(C₁-C₄-)alkylsulfoxoniumsalze.

Die Substituenten der 3-Aminobenzo[b]thiophene I haben im einzelnen die folgende Bedeutung:
R¹
   eine Gruppe -COX oder -COOX, wobei
X
   - Wasserstoff, Halogen wie Fluor, Chlor, Brom und Jod, insbesondere Fluor und Chlor,
   - die Aminogruppe, die gewünschtenfalls ein bis drei Substituenten tragen kann, ausgewählt aus einer Gruppe bestehend aus C₁-C₄-Alkylsubstituenten wie Methyl, Ethyl, n-Propyl, 1-Methylethyl, n-Butyl, 1-Methylpropyl, 2-Methylpropyl und 1,1-Dimethylethyl, vorzugsweise Methyl oder Ethyl, Hydroxy-C₁-C₄-alkylsubstituenten wie Hydroxymethyl, 1-Hydroxyeth-1-yl, 2-Hydroxyeth-1-yl, 1-Hydroxy-prop-1-yl, 2-Hydroxy-prop-1-yl, 3-Hydroxy-prop-1-yl, 1-Hydroxy-prop-2-yl, 2-Hydroxy-prop-2-yl, 1-Hydroxybut-1-yl, 2-Hydroxy-but-1-yl, 3-Hydroxy-but-1-yl, 4-Hydroxy-but-1-yl, 1-Hydroxy-but-2-yl, 2-Hydroxybut-2-yl, 1-Hydroxy-but-3-yl, 2-Hydroxy-but-3-yl, 1-Hydroxy-2-methyl-prop-3-yl, 2-Hydroxy-2-methyl-prop-3-yl, 3-Hydroxy-2-methyl-prop-3-yl, und 2-Hydroxymethyl-prop-2-yl, vorzugsweise 1-Hydroxyeth-1-yl, Phenyl- und Benzylsubstituenten,
   - eine C₁-C₁₀-Alkylgruppe, insbesondere eine C₁-C₆-Alkylgruppe
      wie Methyl, Ethyl, n-Propyl, 1-Methylethyl, n-Butyl, 1-Methyl-propyl, 2-Methylpropyl, 1,1-Dimethylethyl, n-Pentyl, 2-Methylbutyl, 3-Methylbutyl, 2,2-Di-methylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl, Isopropyl oder tert.-Butyl, wobei die Alkylgruppe jeweils unsubstituiert sein oder einen oder zwei Reste tragen kann, ausgewählt aus einer Gruppe bestehend aus Halogen wie vorstehend genannt, insbesondere Fluor und Chlor, Carboxyl und Carbonyl, und wobei die Alkylgruppe jeweils noch zusätzlich so viele Halogenatome wie vorstehend genannt, insbesondere Fluor und Chlor, tragen kann, wie substituierbare H-Atome vorhanden sind,
   - eine C₂-C₈-Alkenylgruppe, insbesondere eine C₂-C₆-Alkenylgruppe wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl,. 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Di-methyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, vorzugsweise Ethenyl oder Prop-1-enyl, wobei die Alkenylgruppe jeweils unsubstituiert sein oder einen oder zwei Reste tragen kann, ausgewählt aus einer Gruppe bestehend aus Halogen wie vorstehend genannt, insbesondere Fluor und Chlor, Carboxyl und Carbonyl, und wobei jede Alkenylgruppe gewünschtenfalls noch zusätzlich so viele Halogenatome wie vorstehend genannt, insbesondere Fluor und Chlor, tragen kann, wie substituierbare H-Atome vorhanden sind,
   - eine C₃-C₁₀-Cycloalkyl- oder C₃-C₁₀-Cycloalkenylgruppe, insbesondere eine C₃-C₈-Cycloalkyl- oder C₅-C₈-Cycloalkenylgruppe wie Cyclopropyl, Cyclobutyl, Cyclopentyl, Cyclohexyl, Cycloheptyl, Cyclooctyl, Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl, vorzugsweise Cyclopentyl oder Cyclohexyl, wobei der Carbocyclus jeweils unsubstituiert sein oder einen oder zwei Reste tragen kann, ausgewählt aus einer Gruppe bestehend aus Halogen wie vorstehend genannt, insbesondere Fluor und Chlor, Carboxyl und Carbonyl, und wobei jeder Carbocyclus gewünschtenfalls noch zusätzlich so viele Halogenatome wie vorstehend genannt, insbesondere Fluor und Chlor, tragen kann, wie substituierbare H-Atome vorhanden sind,
   - einen 5- oder 6-gliedrigen aromatischen Heterocyclus mit einem Sauerstoff und einem Schwefelatom oder mit 1 bis 3 Heteroatomen, ausgewählt aus einer Gruppe bestehend aus 3 Stickstoffatomen und einem Sauerstoffoder Schwefelatom, wobei nicht alle drei Heteroatome gleichzeitig benachbart sein können, beispielsweise 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, vorzugsweise Pyridin-2-yl;
   - die Phenyl- oder die Naphthylgruppe, wobei diese Gruppen unsubstituiert sein oder einen bis drei Substituenten tragen können, ausgewählt aus einer Gruppe bestehend aus Halogen wie vorstehend genannt, insbesondere Fluor und Chlor, -COY oder -COOY, und wobei diese Gruppen noch zusätzlich so viele Halogenatome wie vorstehend genannt, insbesondere Fluor und Chlor, tragen können, wie substituierbare H-Atome vorhanden sind;
      Y steht für C₁-C₆-Alkyl wie vorstehend genannt, insbesondere für Methyl oder Ethyl, C₃-C₆-Cycloalkyl wie vorstehend genannt, vorzugsweise Cyclopentyl oder Cyclohexyl, oder Phenyl;
Ganz besonders bevorzugt bedeutet R¹:
- Wasserstoff
- Acyl
- Methoxycarbonyl, n-Butoxycarbonyl, tert.-Butoxycarbonyl, 2-Heptyloxycarbonyl, 1-Propen-3-oxycarbonyl oder Benzyloxycarbonyl
- Carbonsäureamid
- Carboxyessigsäureesthylester
- Oxalylsäureethylester
- Benzoyl

R², R³ unabhängig voneinander
   - Wasserstoff, Cyano, Nitro, Mercapto,
   - Halogen wie Fluor, Chlor, Brom und Jod, insbesondere Fluor und Chlor,
   - die Aminogruppe, die unsubstituiert sein oder ein bis drei Substituenten tragen kann, ausgewählt aus einer Gruppe bestehend aus C₁-C₄-Alkylsubstituenten wie vorstehend genannt, vorzugsweise Methyl, Hydroxy-C₁-C₄-alkylsubstituenten wie vorstehend genannt, Phenyl- und Benzylsubstituenten,
   - eine C₁-C₁₀-Alkylgruppe, insbesondere eine C₁-C₆-Alkylgruppe wie vorstehend genannt, vorzugsweise Methyl oder Ethyl, wobei die Alkylgruppe jeweils unsubstituiert sein oder einen oder zwei Reste tragen kann, ausgewählt aus einer Gruppe bestehend aus Halogen wie vorstehend genannt, insbesondere Fluor und Chlor, Carboxyl und Carbonyl, und wobei jede Alkylgruppe gewünschtenfalls noch zusätzlich so viele Halogenatome wie vorstehend genannt, insbesondere Fluor und Chlor, tragen kann, wie substituierbare H-Atome vorhanden sind,
   - eine C₁-C₆-Alkoxygruppe wie Methoxy, Ethoxy, n-Propoxy, 1-Methylethoxy, n-Butoxy, 1-Methyl-propoxy, 2-Methylpropoxy, 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy, vorzugsweise Methoxy oder Ethoxy,
   - eine C₂-C₈-Alkenylgruppe, insbesondere eine C₂-C₆-Alkenylgruppe wie vorstehend genannt, vorzugsweise Ethenyl, wobei die Alkenylgruppe jeweils unsubstituiert sein oder einen oder zwei Reste tragen kann, ausgewählt aus einer Gruppe bestehend aus Halogen wie vorstehend genannt, insbesondere Fluor und Chlor, Carboxyl und Carbonyl, und wobei jede Alkenylgruppe gewünschtenfalls noch zusätzlich so viele Halogenatome wie vorstehend genannt, insbesondere Fluor und Chlor, tragen kann, wie substituierbare H-Atome vorhanden sind,
   - eine C₃-C₁₀-Cycloalkyl- oder C₃-C₁₀-Cycloalkenylgruppe, insbesondere eine C₃-C₈-Cycloalkyl- oder C₅-C₈-Cycloalkenylgruppe wie vorstehend genannt, vorzugsweise Cyclopentyl oder Cyclohexyl, wobei die Carbocyclen unsubstituiert sein oder einen oder zwei Reste tragen können, ausgewählt aus einer Gruppe bestehend aus Halogen wie vorstehend genannt, insbesondere Fluor und Chlor, Carboxyl und Carbonyl, und wobei jeder Carbocyclus gewünschtenfalls noch zusätzlich so viele Halogenatome wie vorstehend genannt, insbesondere Fluor und Chlor, tragen kann, wie substituierbare H-Atome vorhanden sind,
   - die Phenyl- oder die Naphthylgruppe, die jeweils unsubstituiert sein oder einen bis drei Substituenten tragen kann, ausgewählt aus einer Gruppe bestehend aus Halogen wie vorstehend genannt, insbesondere Fluor und Chlor, -COY oder -COOY wie vorstehend genannt, und wobei die Phenyl- und die Naphthylgruppe gewünschtenfalls zusätzlich noch so viele Halogenatome tragen können, wie substituierbare H-Atome vorhanden sind,
   - einen 5- oder 6-gliedrigen aromatischen Heterocyclus mit einem Sauerstoff- und einem Schwefelatom oder mit 1 bis 3 Heteroatomen, ausgewählt aus einer Gruppe bestehend aus 1 bis 3 Stickstoffatomen und einem Sauerstoff- oder Schwefelatom, wobei nicht alle drei Heteroatome gleichzeitig benachbart sein können, beispielsweise 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, vorzugsweise Pyridin-2-yl, Pyridin-3-yl oder Pyridin-4-yl.
Ganz besonders bevorzugt bedeuten R² und R³ Wasserstoff;

R⁴ bis R⁷ unabhängig voneinander
- Wasserstoff,
- eine C₁-C₁₀-Alkylgruppe, insbesondere eine C₁-C₆-Alkylgruppe wie vorstehend genannt, vorzugsweise Methyl oder Ethyl, wobei die Alkylgruppe jeweils unsubstituiert sein oder einen oder zwei Reste tragen kann, ausgewählt aus einer Gruppe bestehend aus Halogen wie vorstehend genannt, insbesondere Fluor und Chlor, Carboxyl und Carbonyl, und wobei jede Alkylgruppe gewünschtenfalls noch zusätzlich so viele Halogenatome wie vorstehend genannt, insbesondere Fluor und Chlor, tragen kann, wie substituierbare H-Atome vorhanden sind,
- eine C₂-C₈-Alkenylgruppe, insbesondere eine C₂-C₆-Alkenylgruppe wie vorstehend genannt, vorzugsweise Ethenyl, wobei die Alkenylgruppe jeweils unsubstituiert sein oder einen oder zwei Reste tragen kann, ausgewählt aus einer Gruppe bestehend aus Halogen wie vorstehend genannt, insbesondere Fluor und Chlor, Carboxyl und Carbonyl, und wobei jede Alkenylgruppe gewünschtenfalls noch zusätzlich so viele Halogenatome wie vorstehend genannt, insbesondere Fluor und Chlor, tragen kann, wie substituierbare H-Atome vorhanden sind,
- eine C₃-C₁₀-Cycloalkyl- oder C₃-C₁₀-Cycloalkenylgruppe, insbesondere eine C₃-C₈-Cycloalkyl- oder C₅-C₈-Cycloalkenylgruppe wie vorstehend genannt, vorzugsweise Cyclopentyl oder Cyclohexyl, wobei der Carbocyclus jeweils unsubstituiert sein oder einen oder zwei Reste tragen kann, ausgewählt aus einer Gruppe bestehend aus Halogen wie vorstehend genannt,. insbesondere Fluor und Chlor, Carboxyl und Carbonyl, und wobei jeder Carbocyclus gewünschtenfalls noch zusätzlich so viele Halogenatome wie vorstehend genannt, insbesondere Fluor und Chlor, tragen kann, wie substituierbare H-Atome vorhanden sind,
- die Phenyl- oder die Naphthylgruppe, die unsubstituiert sein oder jeweils einen bis drei Substituenten tragen können, ausgewählt aus einer Gruppe bestehend aus Halogen wie vorstehend genannt, insbesondere Fluor und Chlor, -COY oder -COOY wie vorstehend genannt, und wobei die Phenyl- und die Naphthylgruppe gewünschtenfalls zusätzlich noch so viele Halogenatome tragen können, wie substituierbare H-Atome vorhanden sind,
- einen 5- oder 6-gliedrigen aromatischen Heterocyclus mit einem Sauerstoff- und einem Schwefelatom oder mit 1 bis 3 Heteroatomen, ausgewählt aus einer Gruppe bestehend aus 3 Stickstoffatomen und einem Sauerstoff- oder Schwefelatom, wobei nicht alle drei Heteroatome gleichzeitig benachbart sein können, beispielsweise 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, vorzugsweise Pyridin-2-yl, Pyridin-3-yl oder Pyridin-4-yl, wobei jeder Heterocyclus unsubstituiert sein oder einen oder zwei Substituenten tragen kann, ausgewählt aus einer Gruppe bestehend aus Halogen wie vorstehend genannt, insbesondere Fluor und Chlor, -COY oder -COOY wie vorstehend genannt, und wobei jeder Heterocyclus gewünschtenfalls zusätzlich noch so viele Halogenatome tragen können, wie substituierbare H-Atome vorhanden sind;
oder R⁴ und R⁵, R⁶ und R⁷ bilden zusammen mit dem jeweiligen Stickstoffatom, an das sie gebunden sind, einen 5-, 6- oder 7-gliedrigen Heterocyclus wie Pyrrolidinyl, Piperidinyl, Morpholinyl und Azepinyl;
ganz besonders bevorzugt bedeutet R⁴ bis R⁷ Wasserstoff.
3-Aminobenzo[b]thiophene der Formel I' 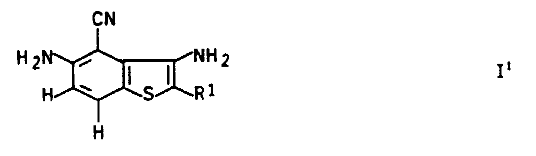 sind neu.

Die 3-Aminobenzo[b]thiophene der Formeln I und I' sind auf verschiedene Weise erhältlich; vorzugsweise nach einem der folgenden Verfahren:

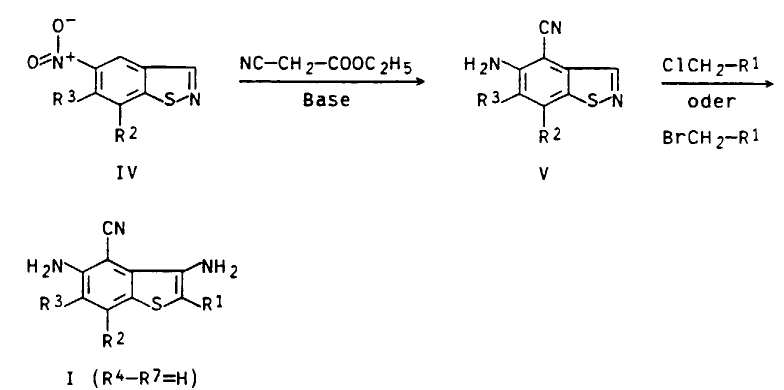

Die Einführung der Cyanogruppe in das Nitro-benzthiazol IV bei gleichzeitiger Reduktion der Nitrogruppe erfolgt vorteilhaft mittels Cyanessigester in Gegenwart einer Base nach literaturbekannter Methode [vgl. Chem. Pharm. Bull. 30, 851 (1982)].

Als Base kommen beispielsweise Alkalimetallhydroxide wie Natriumhydroxid und Kaliumhydroxid, Erdalkalimetallalkoholate wie Natriummethanolat und Kalium-tert.-butylat in Betracht, wobei Kalium-tert.-butylat besonders bevorzugt ist.

Vorzugsweise arbeitet man in einem inerten Lösungsmittel, insbesondere in einem polaren aprotischen Lösungsmittel wie Dimethylformamid und N-Methylpyrrolidon.

Die Lösungsmittelmenge ist nicht kritisch; sie wird in der Regel so gewählt, daß die Reaktionsmischung leicht rührbar bleibt; vorzugsweise liegt sie zwischen der 3- und 6-fachen molaren Menge, bezogen auf die Menge an IV.

In der Regel arbeitet man mit einem überschuß an Cyanessigester, beispielsweise mit der 2- bis 6-fachen molaren Menge, bevorzugt der 3-fachen molaren Menge, bezogen auf die Menge an Nitro-benzthiazol IV.

Die Menge an Base ist nicht kritisch. Für einen hohen Umsatz empfiehlt sich die Verwendung der halben bis doppelten molare Menge an Base, bezogen auf die Menge an Cyanessigester.

Bevorzugt setzt man Base und Cyanessigester in etwa stöchiometrischem Verhältnis ein.

Normalerweise sind Reaktionstemperaturen zwischen etwa 0 und 100°C, insbesondere zwischen 20 und 80°C, bevorzugt zwischen 40 und 60°C, ausreichend.

Im allgemeinen führt man die Reaktion in einem Druckbereich zwischen 0,5 und 5 bar, insbesondere zwischen 0,8 und 3 bar, vorzugsweise bei Atmosphärendruck, durch.

Das Cyano-benzthiazol V wird zweckmäßigerweise in einem polaren Lösungsmittel wie Sulfolan oder Methoxypropanal, z.B. mit Chlor- oder Bromessigsäurederivaten, in die Verbindungen I überführt.

Vorteilhaft arbeitet man hierbei in Gegenwart einer starken Base, beispielsweise einem Alkalimetallhydroxid wie Natriumhydroxid und Kaliumhydroxid.

Vorzugsweise werden alle Reaktionspartner in stöchiometrischen Mengen eingesetzt, jedoch kann auch ein Überschuß der einen oder anderen Komponenten, bis etwa 5 mol-%, vorteilhaft sein.

Die Reaktionstemperatur liegt normalerweise zwischen 20 und 200°C, vorzugsweise zwischen 80 und 200°C, insbesondere zwischen 160°C und der Siedetemperatur des jeweiligen Verdünnungsmittels.

Bezüglich des Druckes gelten die vorstehend für die überführung der Nitro-benzthiazole IV in die Cyano-benzthiazole V gemachten Angaben.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Cyano-benzthiazole V sind neu. Weitere 3-Aminobenzo[b]thiophene I, bei denen einer oder mehrere der Substituenten R⁴ bis R⁷ nicht Wasserstoff bedeuten, sind auf an sich bekannte Weise herstellbar, beispielsweise durch Reaktion der Aminogruppen mit Hal-R⁴, Hal-R⁵, Hal-R⁶ oder Hal-R⁷, wobei Hal Halogen wie Chlor und Brom bedeutet. In der Regel arbeitet man hierbei in Gegenwart einer Base, wobei beispielsweise Alkalimetallhydroxide wie Natrium- und Kaliumhydroxid, Erdalkalimetallhydroxide wie Calciumhydroxid, Alkalimetallalkoholate wie Natriummethanolat, Natrium- und Kaliumethanolat und Kalium-tert.-butylat, Erdalkalimetallalkoholate wie Calciumalkoholat, Alkalimetallhydride wie Natrium- und Kaliumhydrid, Erdalkalimetallhydride wie Calciumhydrid, aliphatische Amine wie Dimethylamin, Triethylamin, Diisopropylamin, Dimethylanilin, Dimethylbenzylamin und Piperidin sowie heteroaromatische Amine wie Pyridin und 4-Dimethylaminopyridin in Betracht kommen. Als inerte Lösungs- oder Verdünnungsmittel eignen sich z.B. aliphatische Kohlenwasserstoffe wie n-Hexan, Benzin und Petrolether, aromatische Kohlenwasserstoffe wie Benzol, Toluol, Xylol, chlorierte Kohlenwasserstoffe wie Dichlormethan, Chloroform, Tetrachlormethan, 1,2-Dichlorethan und Chlorbenzol, stickstoffhaltige Heteroaromaten wie Pyridin und Chinolin, cyclische Ether wie Tetrahydrofuran und Dioxan, Nitrile wie Acetonitril und Propionitril, sowie Dimethylformamid, Dimethylsulfoxid und N-Methylpyrrolidon oder eine Mischung der genannten Solventien. Bei Anwesenheit eines Phasentransferkatalysators wie Trioctylpropylammoniumchlorid und Cetyltrimethylammoniumchlorid kann die Reaktion auch in einem 2-Phasensystem aus Wasser und einem Kohlenwasserstoff, z.B. Tetrachlorkohlenstoff, durchgeführt werden. Zweckmäßigerweise werden alle Reaktionspartner in stöchiometrischen Mengen eingesetzt, jedoch kann auch ein Überschuß der einen oder anderen Komponente, bis etwa 10 mol-%, verwendet werden. Im allgemeinen liegt die Reaktionstemperatur zwischen 0 und 200°C, vorzugsweise zwischen 20 und 140°C, insbesondere bei der Siedetemperatur des jeweiligen Lösungsmittels.

Weitere Synthesemethoden zur Herstellung der 3-Aminobenzo[b]thiophene I können der folgenden Literatur entnommen werden:
- R.J.Beck, J. org. Chem. 37(21), 3224-3226 (1972);
- H.Schaefer et al., J. Prakt. Chem. 327(2), 328-332 (1985);
- K.Gewald et al., Z. Chem. 21(5), 183-184 (1981);
- DE-A-37 40 984;
- EP-A-323 590;
- DE-A-38 27 253;
- EP-A-201 165;
- EP-A-187 487;
- DE-A-34 29 830;
- EP-A-378 508 und
- DE-A-40 39 734

Die 3-Aminobenzo[b]thiophene I bzw. I' eigenen sich als Antidots, um herbizide Wirkstoffe für Kulturpflanzen wie Kulturhirse, Reis, Mais, Getreidearten (Weizen, Roggen, Gerste, Hafer), Baumwolle, Zuckerrüben, Zuckerrohr und Soja verträglicher zu machen. Sie wirken antagonistisch auf Herbizide der verschiedensten Stoffklassen wie Triazine, Phenylharnstoffderivate, Carbamate, Thiolcarbamate, Halogenacetanilide, Benzoesäurederivte sowie insbesondere Halogenphenoxyessigsäureester, substituierte Phenoxyphenoxyessigsäureester, Phenoxyphenoxypropionsäureester und insbesondere Cyclohexenon-Derivate.

Herbizid wirksame Cyclohexenon-Derivate II sind beispielsweise aus EP-A 228 598, EP-A 230 235, EP-A 238 021, EP-A 368 227, US-A 4 432 786, DE-A 24 39 104 und DE-A 38 38 309 bekannt. Sie dienen vorwiegend zur Bekämpfung unerwünschter Gräser in dicotylen Kulturen und in Gräsern, die nicht zur Familie der Gramineen zählen. In Abhängigkeit der Substituenten und der Dosierung der Verbindungen des Typs II bei ihrer Anwendung können diese Cyclohexenone auch zur selektiven Bekämpfung von unerwünschten Gräsern in Gramineen-Kulturen wie Weizen und Reis eingesetzt werden.

Weitere Cyclohexenon-Derivate II lassen sich in an sich bekannter Weise nach literaturbekannten Syntheseverfahren (vgl. z.B. EP-A 169 521) darstellen, beispielsweise durch Umsetzung von Triketonen VI (bekannt z.B. aus EP-A 80 301, EP-A 125 094, EP-A 142 741, US-A 4,249,937, EP-A 137 174 und EP-A 177 913) mit Hydroxylaminen VII (bekannt z.B. aus Houben-Weyl, Methoden der Organischen Chemie, Band 10/1, Seite 1181 ff): 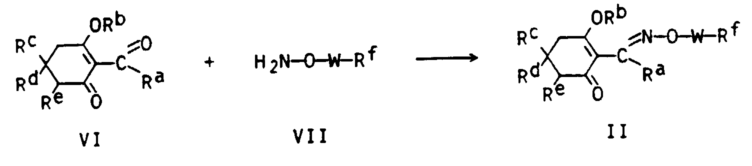 Zweckmäßig führt man die Umsetzung in heterogener Phase in einem Lösungsmittel, bevorzugt in Gegenwart einer Base, durch, wobei das Hydroxylamin vorzugsweise als Ammoniumsalz eingesetzt wird.

Als Basen eigenen sich beispielsweise die Carbonate, Hydrogencarbonate, Acetate, Alkoholate und Oxide von Alkalimetallen und Erdalkalimetallen wie Natriumhydroxid, Kaliumhydroxid, Magnesiumoxid und Calciumoxid, des weiteren organische Basen wie Pyridin und tertiäre Amine wie Triethylamin.

Triketon und Hydroxylamin werden vorzugsweise in etwa stöchiometrischen Mengen eingesetzt. Die Menge an Base ist nicht kritisch, beträgt normalerweise aber ca. 0,5 bis 2 mol-Äquivalent, bezogen auf die Menge an VI.

Im allgemeinen liegt die Reaktionstemperatur zwischen 0 und 80°C.

Als Lösungsmittel eignen sich beispielsweise Dimethylsulfoxid, Alkohole wie Methanol, Ethanol und Isopropanol, Kohlenwasserstoffe wie Hexan und Cyclohexan, aromatische Kohlenwasserstoffe wie Benzol und Toluol, Ester wie Essigsäureethylester und Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Das Produkt II kann z.B. durch Einengen der Mischung, Verteilen des Rückstandes in Methylenchlorid/Wasser und Abdestillieren des Lösungsmittels unter vermindertem Druck isoliert werden.

Man kann für diese Umsetzung aber auch unmittelbar die freie Hydroxylaminbase, z. B. in Form einer wäßrigen Lösung, verwenden; je nach verwendetem Lösungsmittel für das Hydroxylamin VII erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol, aliphatische und aromatische, gegebenenfalls chlorierte Kohlenwasserstoffe wie Hexan, Cyclohexan, Methylenchlorid, Toluol und Dichlorethan, Ester wie Essigsäureethylester, Nitrile wie Acetonitril und cyclische Ether wie Dioxan und Tetrahydrofuran.

Besondere Bedingungen bezüglich des Druckes sind nicht erforderlich; normalerweise nimmt man die Umsetzung daher bei Atmosphärendruck vor.

Alkalimetallsalze der Verbindungen II können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sufoxoniumhydroxiden.

Die Verbindung des Typs VI können z.B. aus den entsprechenden Cyclohexan-1,3-dionen der Formel VIII 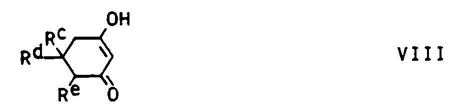 nach bekannten Methoden (Tetrahedron Lett., 2491 (1975)) hergestellt werden.

Es ist auch möglich, die Verbindungen der Formel VI über die Zwischenstufe der Enolester herzustellen, die bei der Umsetzung von Verbindungen der Formel VIII mit Säurechloriden in Gegenwart von Basen anfallen und anschließend mit bestimmten Imidazol- oder Pyridinderivaten umgelagert werden (JP-OS 79/063 052). 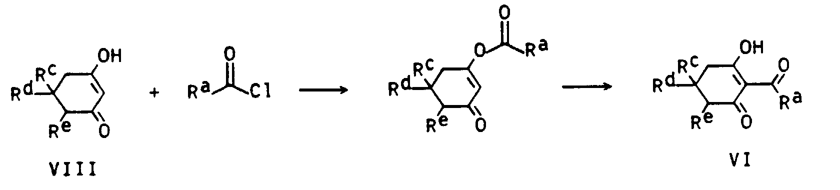 Zu den Hydroxylaminen der Formel VII gelangt man in der Regel über eine Reihe bekannter Verfahrensschritte ausgehend von bekannten Vorprodukten: 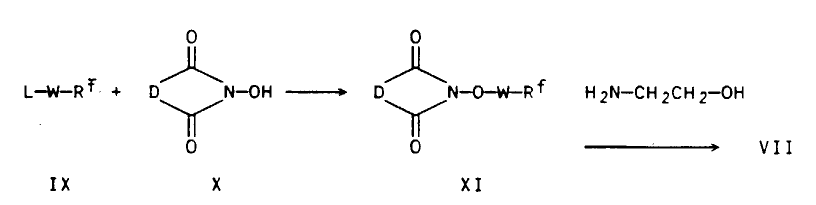 L = die Hydroxylgruppe oder eine Abgangsgruppe, z.B. Halogen wie Chlor, Brom und Jod oder CH₃SO₂-O-.

Die zur Synthese der Hydroxylamine VII benötigten Alkylierungsmittel sind literaturbekannt oder lassen sich nach bekannten Methoden darstellen.

Synthesen von Derivaten in denen W eine aliphatische oder olfinische Kette bedeutet, die gegebenenfalls durch Heteroatome unterbrochen sein kann, sind den folgenden Druckschriften zu entnehmen:

DE-A 34 37 919; Tetrahetron Lett. 28, 2639 (1979); Org. Synth. Coll. vol. 1, 436 (1944); DE-A 26 54 646; DE-A 27 14 561; J. Org. Chem. 52, 3587 (1987); DE-A 94 8871; DE-A 94 8872; J. Med. Chem. 26, 1570 (1983); Synthesis, 675 (1983); J. Org. Chem. 48, 4970 (1983); Org. Synth. Coll. Vol. V, 249; EP-A 48 911; EP-A 143 952; US 4,686,735.

Zur Herstellung von Verbindungen II, in denen W eine aliphatische oder olefinische Kette und R^{f} einen Heterocyclus bedeutet, sei auf folgende Literatur verwiesen:
J. Heterocycl. Chem. 14, 525 (1976); JP 55 051 004; JP 55 047 601;
Houben Weyl: Methoden der organischen Chemie Band 4/3, 4. Auflage 1971, S. 424 ff; DE-A-28 21 409; Chem. Ber. 114, 3667 und 3674 (1981).

Herstellungsmethoden, die von geeigneten Carbinolen IX (L=OH) ausgehen, sind beispielsweise bekannt aus:

Tetrahedron 35, 329 (1979); Chem. Lett., 423 (1977); Houben/Weyl: Methoden der organischen Chemie, Band 13/9B, 4. Auflage 1984, S. 964 ff; dto. Band 5/3, 4. Auflage 1962, S. 862 und 899 ff; dto. Band 5/4, 4. Auflage 1960, S. 361 ff.

Die Darstellung von Alkylierungsmitteln in denen W eine substituierte oder unsubstituierte C₃-C₆-Alkinylgruppe bedeutet, kann nach klassischen Methoden [vgl. J. Med Chem. 29, 1389 (1986); dto. 24, 678 (1981); EP-A 131 302; J. Chem. Ecol. 10, 1201 (1982)] oder durch Kupplung von 1-Alkinylderivaten mit Aryl- oder Hetarylhalogeniden in Gegenwart von Palladiumkatalysatoren [vgl. z.B. Tetrahedron Lett. 50, 4467 (1975)] erfolgen.

IX wird mit einem cyclischen Hydroxylimid X gekoppelt und das erhaltene geschützte Hydroxylaminderivat XI zum freien Hydroxylamin VII gespalten, bevorzugt mit 2-Aminoethanol.

Bei der Verwendung von HO-W-R^{f} empfiehlt sich das Arbeiten nach der Mitsunobu-Variante [vgl. Synthesis, 1 (1981) und J. Med. Chem. 33, 187 (1990)].

In den cyclischen Hydroxyimiden X steht D z.B. für C₂-C₃-Alkylen, C₂-Alkenylen oder einen mit bis zu drei Doppelbindungen und gegebenenfalls ein Stickstoffatom enthaltenden 5- oder 6-gliedrigen Ring, z.B. für Phenylen, Pyridinylen, Cyclopentylen, Cyclohexylen oder Cyclohexenylen. Beispielsweise kommen folgende Substanzen in Betracht: 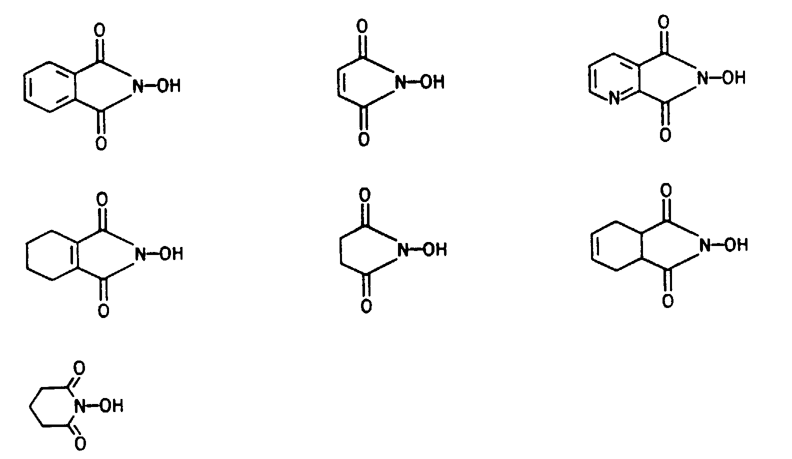 Die Umsetzung der Verbindungen IX mit den Hydroxyimiden X wird zweckmäßigerweise in Gegenwart einer Base ausgeführt. Geeignet sind prinzipiell alle Basen, die in der Lage sind, die Hydroxyimide X zu deprotonieren ohne das Imidsystem anzugreifen.

Dies sind insbesondere die sogenannten nicht nucleophilen Basen. Beispielsweise genannt seien Mineralbase wie Alkalimetall- und Erdalkalimetallcarbonate, Alkalimetall- und Erdalkalimetallhydrogencarbonate, organische Basen wie aliphatische, cycloaliphatische und aromatische tertiäre Amine. Es können auch Gemische dieser Basen verwendet werden.

Als Einzelverbindungen seien folgende Basen beispielhaft aufgeführt: Natriumcarbonat, Kaliumcarbonat, Magnesiumcarbonat, Calciumcarbonat, Bariumcarbonat, die Hydrogencarbonate dieser Metalle, Trimethylamin, Triethylamin, Tributylamin, Ethyldiisopropylamin, N,N-Dimethylanilin, 4-N,N-Dimethylaminopyridin, Diazabicyclooctan, Diazabicycloundecan, N-Methyl-piperidin, 1,4-Dimethylpiperazin, Pyridin, Chinolin, Bipyridin, Phenanthrolin. Bevorzugt sind die preiswerten Basen Natrium- und Kaliumcarbonat.

Die Base wird im allgemeinen in äquivalenten Mengen bis zu einem überschuß von 5 Äquivalenten, bezogen auf das Hydroxyimid, zugegeben. Ein höherer überschuß ist möglich, entbehrt aber zusätzliche Vorteile. Die Verwendung einer geringen Basenmenge ist ebenfalls möglich. Bevorzugt wird jedoch eine Basenmenge von 1 bis 3, insbesondere von 1 bis 2 Äquivalenten, bezogen auf das Hydroxyimid X eingesetzt.

Die Verwendung von nucleophilen Basen, z.B. Alkalimetall- und Erdalkalimetallhydroxiden, insbesondere Natrium- und Kaliumhydroxid, ist ebenfalls möglich. In diesem Falle ist es vorteilhaft, die Base in äquivalenten Mengen bezüglich des Hydroxyimids X einzusetzen, um einem nucleophile Angriff der Hydroxylionen auf die Carbonylfunktion der Imdigruppierung vorzugbeugen.

Zweckmäßigerweise setzt man die Ausgangsverbindungen IX mit den Hydroxyimiden X in einem Lösungsmittel um, das sich unter den Reaktionsbedingungen inert verhält. Vorteilhafte Lösungsmittel sind z.B. polare aprotische Lösungsmittel wie Dimethylformamid, N-Methylpyrrolidon, Dimethylsulfoxid, Sulfolan und cyclische Harnstoffe. Die Lösungsmittelmenge ist im allgemeinen nicht kritisch.

Die Umsetzung der Ausgangsverbindungen IX mit den Hydroxyimiden X kann auch unter Anwendung der Phasentransfer-Katalyse ausgeführt werden. In diesem Falle werden mit Wasser zwei Phasen bildende Lösungsmittel, bevorzugt Chlorkohlenwasserstoffe, eingesetzt. Als Phasentransferkatalystoren eignen sich die üblicherweise zu solchen Zwecken verwendeten quartären Ammonium- und Phosphoniumsalze, Polyethylenglykole, Polyethylenglykolether und Kronenether, wie sie z.B. in Dehmlow et al., Phase Transfer Catalysis, S. 37 - 45 und S. 86 - 93, Verlag Chemie, Weinheim 1980, beschrieben sind. Die Phasentransferkatalystoren werden zweckmäpigerweise in Mengen von 1 bis 10 Vol%, bevorzugt in Mengen von 3 bis 5 Vol%, bezogen auf das Volumen der Reaktionsmischung, eingesetzt.

Die Umsetzung der Ausgangsverbindungen IX mit den Hydroxyimiden X wird im allgemeinen im Temperaturbereich zwischen 0 und 140°C, bevorzugt zwischen 20 und 100°C, insbesondere zwischen 40 und 80°C, durchgeführt. Zweckmäßigerweise wird dabei so vorgegangen, daß man das Hydroxyimid X zusammen mit der Base im Lösungsmittel vorlegt und das Ausgangsmaterial IX zu dieser Lösung dosiert. Dabei kann es sich als günstig erweisen, wenn das Hydroxyimid bei einer tieferen Temperatur, beispielsweise bei 0 bis 50°C, zugegeben und die Reaktionsmischung erst nach dieser Zugabe auf die eigentliche Reaktionstemperatur erhitzt wird.

In der Regel arbeitet man bei Normaldruck oder unter dem Eigendruck des Lösungsmittels.

Nach beendeter Reaktion wird die abgekühlte Reaktionsmischung zweckmäßigerweise mit Wasser versetzt, wobei sich die gebildeten Hydroxylaminderivate XI als kristalline Festkörper oder als Öle abscheiden. Die auf diese Weise erhaltenen Hydroxylaminderivate können, falls gewünscht, durch Umkristallisation oder durch Extraktion weiter gereinigt werden.

Die Hydroxylaminderivate XI können zwischengelagert werden oder sogleich in die Hydroxylaminderivate VII mit freier Aminogruppe umgewandelt werden. Diese Umwandlung kann nach an sich bekannten Verfahren durchgeführt werden, wie sie beispielsweise in DE-A 36 15 973 und den darin zitierten Schriften beschrieben sind. Bevorzugt wird das Verfahren gemäß DE-A 36 15 973 angewandt, nach dem die Hydroxylaminderivate VII mittels Etanolamin freigesetzt werden. Die Feisetzung der Hydroxylaminderivate VII mit Hilfe anderer Basen wie wäßrigen Mineralbasen, mit Aminen, Hydrazinen, Hydroxylaminen oder mittels wäßriger Säuren ist ebenfalls möglich.

Aus den nach diesen Verfahren erhaltenen Reaktionsgemischen können die Hydroxylaminderivate VII mittels üblicher Aufarbeitungsmethoden isoliert werden, beispielsweise durch Extration oder durch Kristallisation. Zur Erhöhung der Krisallisationstendenz dieser Hydroxylaminderivate kann es oftmals förderlich sein, diese in ihre Salze mit Mineralsäuren oder organischen Säuren überzuführen. Dazu werden im allgemeinen verdünnte Lösungen dieser Säuren mit den Hydroxylaminderivaten VII umgesetzt, und zwar zweckmäßigerweise in äquivalenten Mengen. Die erhaltenen Hydroxylammoniumsalze können wie die Hydroxylaminderivate VII mit freier Aminogruppe direkt zu den Herbiziden der Formel II weiterverarbeitet werden oder auch, falls gewünscht, gelagert werden.

Die Cyclohexenon-Derivate II können bei der Herstellung als Isomerengemische anfallen, wobei sowohl E-/Z-Isomerengemische als auch Enantiomeren- oder Diastereoisomerengemische möglich sind. Die Isomerengemische können gewünschtenfalls nach den hierfür üblichen Methoden, z.B. durch Chromatographie oder durch Kristallisation, getrennt werden.

Die Cyclohexenon-Derivate II können in mehreren tautomeren Formen geschrieben werden, die alle von der Erfindung umfaßt werden.

### Herstellungsbeispiele (Cyclohexenon-Derivate)

### Beispiel 1

### 2-[1-(3-(4-Bromphenyl)-prop-2-enyloximino)-propyl]-3-hydroxy-5-(3-tetrahydrothiopyranyl)-cyclohex-2-en-1-on

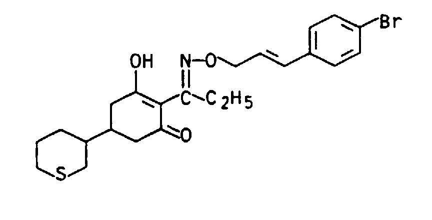

3,0 g (0,011 mol) 2-Propionyl-5-(3-tetrahydrothiopyranyl)-cyclohexan-1,3-dion und 3,0 g (0,013 mol) 3-(4-Bromphenyl)-prop-2-enyloxyamin wurden in 100 ml Methanol bei 20°C 16 Stunden gerührt. Das dabei ausgefallene Reaktionsprodukt wurde bei 0°C abgetrennt und mit eiskaltem Methanol und Petrolether nachgewaschen und getrocknet. Ausbeute: 68,4 %; Fp.: 97-99°C.

### Vorstufe 1.1

### N-[3-(4-Bromphenyl)-prop-2-enyloxy]-phthalimid

In 350 ml trockenes N-Methylpyrrolidon gab man nacheinander 18,5 g (0,11 mol) N-Hydroxyphthalimid und 31,4 g (0,11 mol) 1-Brom-[3-(4-Brom-phenyl)]-prop-2-en und tropfte anschließend bei Raumtemperatur 12,1 g (0,12 mol) Triethylamin zu. Nach viertägigem Rühren bei 20°C wurde die Reaktionsmischung auf 1,5 l Eiswasser gegossen, abfiltriert und mit Wasser und Isopropanol nachgewaschen. Ausbeute: 86,8 %; Fp.: 161-162°C.

### Vorstufe 1.2

### 3-(4-Bromphenyl)-prop-2-enyloxyamin

33,4 g (0,093 mol) N-[3-(4-Bromphenyl)-prop-2-enyloxy]-phthalimid wurden portionsweise in 50 ml Ethanolamin eingetragen; die Temperatur stieg dabei bis auf 30°C an. Nach zweistündigem Rühren bei 60°C ließ man abkühlen und versetzte die Mischung mit 200 ml Dichlormethan. Es wurde mit Eiswasser ausgeschüttelt. Nach Trocknen und Einengen der organischen Phase wurde der Rückstand aus Petrolether umkristallisiert. Ausbeute: 95,3 %; Fp.: 35-38°C.

### Beispiel 2

### 2-[1-(4-(4-Fluorphenyl)-but-3-inyloximino)-butyl-3-hydroxy-5-tetr ahydro-pyran-4-yl-cyclohex-2-enon

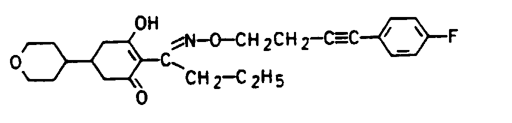

Zu einer Lösung von 4 g (15 mMol) 2-butyryl-3-hydroxy-5-tetrahydropyran-4-yl-cyclohex-2-enon in 60 ml trockenem Methanol wurden 2,7 g (15 mMol) 4-(4-Fluorphenyl)-but-3-inoxyamin gegeben. Nach 16 h Rühren bei Raumtemperatur wurde das Methanol im Wasserstrahlvakuum entfernt. Das Rohprodukt reinigte man mittels Chromatographie an Kieselgel (Laufmittel: Methylenchlorid). Ausbeute: 81,2 %.

### Vorstufe 2.1

### 4-(4-Fluorphenyl)-3-butinol

Eine Lösung von 100 g 4-Bromfluorbenzol in 350 ml Triethylamin wurde nacheinander mit 1 g Bis(triphenylphosphin)-palladium-(II)-chlorid, 3,8 g Kupfer-(I)-jodid und 8,7 g Triphenylphosphin versetzt. Diese Mischung wurde auf Rückflußtemperatur erwärmt, wonach man bei dieser Temperatur (ca. 100°C) 43,4 g 3-Butinol innerhalb 20 min zutropfte. Es wurde noch 5 h bei dieser Temperatur gerührt. Nach dem Abkühlen wurde das Triethylamin abdestilliert. Der Rückstand wurde in Methyl-tert.-butylether und Wasser aufgenommen. Die wäßrige Phase wurde noch zweimal mit Methyltert.-butyl-ether extrahiert, die vereinigten organischen Extrakte wurden nacheinander mit 1 N Salzsäure und mit 10 gew.-%iger Natriumbicarbonatlösung gewaschen und über Natriumsulfat getrocknet. Nach Entfernen des Lösungsmittels wurde das Rohprodukt im Hochvakuum destilliert. Ausbeute: 86 %.

### Vorstufe 2.2

### N-(5-(4-Fluorphenyl)-4-pentinyloxy)phthalimid

Zu einer Lösung von 33,1 g (0,186 mol) 5-Hydroxy-1-(4-fluorphenyl)-1-pentin in 430 ml trockenem Tetrahydrofuran wurden 33,4 g (0,205 mol) N-Hydroxyphthalimid und 53,8 g (0,205 mol) Triphenylphosphin gegeben. Innerhalb von 2,5 h tropfte man dann unter Temperaturkontrolle (max. 40°C) 35,7 g (0,205 mol) Diethylazodicarboxylat zu. Man rührte über Nacht bei Raumtemperatur, engte die Mischung im Vakuum ein und nahm mit 300 ml Dichlormethan auf. Es wurde zweimal mit Natriumcarbonatlösung und einmal mit gesättigter Natriumchlorid-Lösung gewaschen. Nach Trocknen und Einengen wurde das Rohprodukt an Kieselgel chromatographisch gereinigt. Als Eluens wurde zunächst Dichlormethan/n-Hexan benutzt, später dann reines Dichlormethan. Ausbeute: 82 %; Fp.: 85-88°C.
250-MHz-¹H-NMR (in DMSO-d₆):
δ [ppm] = 1,9 - 2,1 (m, 2H); 2,68 (t, 2H); 4,342 (t, 2H); 7,18 (t, 2H); 7,4 - 7,6 (m, 2H); 7,85 (s, 4H).

### vorstufe 2.3

### 5-Aminooxy-1-(4-Fluorphenyl)-1-pentin

Zu einer Mischung aus 68 ml Ethanolamin und 40 ml Dichlormethan wurden portionsweise 47,7 g (0,148 mol) des oben dargestellten Phthalimidethers gegeben. Nach 2 h Rühren bei Raumtemperatur war eine klare Lösung entstanden. Diese wurde in 300 ml eiskalte, gesättigte Natriumchlorid-Lösung gegeben. Man extrahierte die Mischung dreimal mit 100 ml Dichlormethan, wusch die vereinigten organischen Phasen einmal mit Natriumchlorid-Lösung, trocknete und engte ein. Ausbeute: 95 % (Öl).
250-MHz-¹H-NMR (in CDCl₃):
δ [ppm] = 1,8-2,0 (m, 2H); 2,47 (6, 2H); 3,8 (t, 2H); 5,4 (breites s, 2H); 6,9-7,1 (m, 2H); 7,3-7,45 (m, 2H).

### Beispiel 3

### 2-[1-[[(E)-4-(2-Thienyl)-3-butenyloxy]imino]-butyl]-3-hydroxy-5-(2H-tetra-hydropyran-4-yl)-cyclohex-2-en-1-on

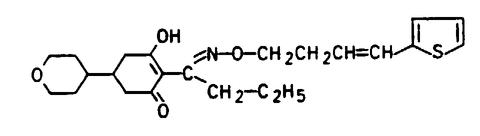

Eine Mischung aus 35 g (0,13 mol) 2-Butyryl-3-hydroxy-5-(2H-tetrahydro-pyran-4-yl)-2-cyclohexen-1-on und 24 g (0,14 mol) O-[(E)-4-(2-Thienyl)-3-butenyl]hydroxylamin in 300 ml Methanol wurde 16 h gerührt. Man engte im Vakuum ein und nahm in 1000 ml 10 gew.-%iger Natronlauge auf. Man extrahierte dreimal mit je 200 ml Methylenchlorid und stellte die wäßrige Phase unter Eiskühlung mit konz. Salzsäure auf pH 1 ein. Die wäßrige Phase wurde anschliepend dreimal mit je 200 ml Ether extrahiet, über Magensiumsulfat getrocknet und im Vakuum eingeengt. Das Rohprodukt wurde chromatographisch gereinigt. (100 g Kieselgel; Säule 30 x 15 cm; Laufmittel:Essigester) Ausbeute: 85 %.
200 MHz-¹H-NMR (in CDCl₃): δ [ppm] = 0,95 (t, 3H), 1,17-1,96 (m, 9H), 2,13 (m, 1H), 2,36 (m, 1H), 2,43-2,70 (m, 3H), 2,88 (m, 2H), 3,36 (t, 2H), 4,02 (d, 2H), 4,15 (t, 2H), 6,00 (dt, 1H), 6,60 (d, 1H), 6,80-7,20 (m, 3H), 14,75 (s, 1H).

### Vorstufe 3.1

### (E)-4-Brom-1-(2-thienyl)-1-buten

Bei 5 bis 10°C tropfte man innerhalb 1 h 225 g (1,46 mol) Cyclopropyl-2-thienylcarbinol zu 972 ml 48 %iger Bromwasserstoffsäure. Nach 2 h bei Raumtemperatur wurde die organische Phase abgetrennt und die wäßrige Lösung mit Dichlormethan extrahiert. Die vereinigten organischen Phasen wurden mit verd. Natronlauge und Wasser neutral gewaschen, getrocknet und im Vakuum eingeengt. Ausbeute: 322 g (94 % korrigert) rohes Bromid (GC: 92 %).
250 MHz-¹H-NMR (in CDCl₃): δ [ppm] = 2,65-2,80 (m, 2H), 3,46 (t, 2H), 5,90-6,10 (m, 1H), 6,61 (d, 1H), 6,80-7,00 (m, 2H), 7,14 (d, 1H).

### Vorstufe 3.2

### N-[(E)-4-(2-Thienyl)-3-butenyloxy]phthalimid

Bei 20 bis 25°C tropfte man innerhalb 2,5 h 190 ml (1,37 mol) Triethylamin zu einer Mischung aus 283 g (1,30 mol) des oben hergestellten Bromids, 1300 ml N-Methyl-2-pyrrolidinon, 10 g Kaliumiodid und 212 g (1,30 mol) N-Hydroxyphthalimid. Nach 4 h bei 20 bis 25°C goß man in 4000 ml Eiswasser und ergänzte portionsweise 5000 ml 10 gew.-%ige Natronlauge. Man extrahierte anschließend mit Essigester. Die vereinigten Essigester-Phasen wurden neutral gewaschen, getrocknet und im Vakuum eingeengt. Das Rohprodukt wurde chromatographisch gereinigt. (1000 g Kieselgel; Säule 30 x 15 cm; Laufmittel: n-Hexan/Dichlormethan 7:3) Ausbeute: 29 %; Fp.: 69-71°C (Isopropanol).
250 MHz-¹H-NMR (in d₆-DMSO): δ [ppm] = 2,55-2,70 (m, 2H), 4,28 (t, 2H),
6,00-6,20 (m, 1H), 6,77 (d, 1H), 7,00 (m, 2H), 7,35 (m, 1H), 7,87 (s, 4H).

### vorstufe 3.3

### O-[(E)-4-(2-Thienyl)-3-butenyl]hydroxylamin

Eine Mischung aus 90,2 g (0,30 mol) des oben hergestellten Phthalimidethers und 136 ml Ethanolamin wurden 3 h bei 60°C gerührt. Die kalte Reaktionsmischung goß man in 200 ml Eiswasser. Man ergänzte 200 ml ges. Natriumchlorid-Lösung und extrahierte das Hydrolysat mit Dichlormethan. Die vereinigten organischen Phasen wurden darauf dreimal mit je 100 ml gesättiger Natriumchlorid-Lösung gewaschen, über Magnesiumsulfat getrocknet und im Vakuum eingeengt. Ausbeute: 89 %.
250 MHz-¹H-NMR (in CDCl₃): δ [ppm] = 2,40-2,55 (m, 2H), 3,78 (t, 2H), 5,40 (bs, 2H), 5,95-6,20 (m, 1H), 6,57 (d, 1H), 6,80-7,15 (m, 3H).

### Beispiel 4

### 2-[1-[[2-(2-Fluorbenzyloxy)ethoxy]imino]butyl]-2-hydroxy-5-(2H-tetrahydropyran-4-yl)-2-cyclohexen-1-on

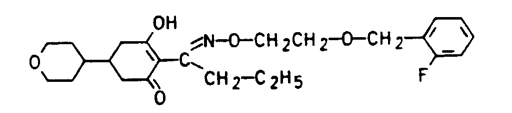

Eine Mischung aus 4,0 g (10 mmol) 2-Butyryl-3-hydroxy-5-(2H-tetrahydropyran-4-yl)-2-cyclohexen-1-on und 2,6 g (14 mmol) O-[2-(2-Fluorbenzyloxy)-ethyl]hydroxylamin in 100 ml Methanol wurde 24 h gerührt. Man engte das Reaktionsgemisch unter reduziertem Druck ein und chromtographierte das Rohprodukt an 100 g Kieselgel (Säule 30 x 4 cm; Laufmittel:Ether). Ausbeute: 54 %
300 MHz-¹H-NMR (in CDCl₃): δ [ppm] = 0,93 (t, 3H), 1,20-1,77 (m, 7H), 1,90 (m, 1H), 2,23 (m, 2H), 2,58 (m, 2H), 2,92 (m, 2H), 3,38 (t, 2H), 3,80 (m, 2H), 4,03 (m, 2H), 4,25 (m, 2H), 4,68 (s, 2H), 6,93-7,50 (m, 4H), 14,30 (s, 1H).

### Vorstufe 4.1

### N-[2-(2-Fluorbenzyloxy)ethoxy]phthalimid

Zu einer Mischung aus 165 g (0,71 mol) 1-Brom-2-(2-fluorbenzyloxy)ethan, 116 g (0,7 mol) N-Hydroxyphthalimid und 710 ml N-Methyl-2-pyrrolidinon tropfte man bei 20 bis 25°C innerhalb 1 h 108 ml Triethylamin. Nach 5 h bei 60°C goß man die kalte Reaktionsmischung in 200 ml Eiswasser, saugte den Niederschlag ab, wusch mit Wasser und Iopropanol und trocknete bei reduziertem Druck über Phosphorpentoxid. Ausbeute: 82 %. Fp.: 62-64°C.
250 MHz-¹H-NMR (in d₆-DMSO): δ [ppm] = 3,85 (m, 2H), 4,35 (m, 1H), 4,54 (s, 2H), 7,10-7,40 (m, 4H), 7,88 (s, 4H).

### Vorstufe 4.2

### O-[2-(2-Fluorbenzyloxy)ethyl]hydroxylamin

184 g (0,58 mol) des oben hergestellten Phtalimidethers wurden portionsweise in 270 ml Ethanolamin eingetragen. Nach 3 h bei 60°C goß man die kalte Reaktionsmischung in 1000 ml Eiswasser. Das Hydrolysat wurde dreimal mit je 800 ml Dichlormethan extrahiert. Die vereinigten organischen Phasen wurden mit 200 ml gesättigter Natriumchlorid-Lösung gewaschen, über Magnesiumsulfat getrocknet und bei reduziertem Druck eingeengt.
Ausbeute: 91 %.
1H-NMR (250 MHz, CDCl₃): δ [ppm] = 3,70 (dd, 2H), 3,85 (dd, 2H), 4,54 (s, 2H), 5,50 (bs, 2H), 7,00-7,50 (m, 4H).

Im Hinblick auf die bestimmungsgemäße verwendung der erfindungsgemäßen herbiziden Mittel kommen Cyclohexenonderivate der Formel II in Betracht, wobei die Substituenten im einzelnen folgende Bedeutung haben:
R^{a}
   eine unverzweigte oder verzweigte C₁-C₆-Alkylgruppe wie Methyl, Ethyl, n-Propyl, Isopropyl, n-Butyl, sec.-Butyl, i-Butyl, tert.-Butyl, n-Pentyl oder n-Hexyl, insbesondere Ethyl und Propyl;
R^{b}
   Wasserstoff;
das Äquivalent eines landwirtschaftlich brauchbaren Kations, beispielweise ein Alkalimetallkation wie Natrium oder Kalium, ein Äquivalent eines Erdalkalimetallkations wie Calzium, Magnesium und Barium, Mangan-, Kupfer-, Zink- oder Eisenkationen, Ammoniumkationen mit gewünschtenfalls einem bis drei Substituenten, ausgewählt aus einer Gruppe bestehend aus ein bis drei C₁-C₄-Alkylresten, ein bis drei Hydroxy-C₁-C₄-alkylresten und einem Phenyl- oder Benzylrest, z.B. Tetraalkyl- oder Benzyltrialkylammoniumkationen, Phosphoniumkationen, Sulfoniumkationen wie Trialkylsulfoniumkationen oder Sulfoxoniumkationen;
eine C₂-C₈-Alkylcarbonyloxygruppe wie Methylcarbonyloxy, Ethylcarbonyloxy, n-Propylcarbonyloxy, 1-Methylethylcarbonyloxy, n-Butylcarbonyloxy, 1-Methylpropylcarbonyloxy, 2-Methylpropylcarbonyloxy, 1,1-Dimethylethylcarbonyloxy, n-Pentylcarbonyloxy, 1-Methylbutylcarbonyloxy, 2-Methylbutylcarbonyloxy, 3-Methylbutylcarbonyloxy, 1,1-Dimethylpropylcarbonyloxy, 1,2-Dimethylpropylcarbonyloxy, 2,2-Dimethylpropylcarbonyloxy, 1-Ethylpropylcarbonyloxy, n-Hexylcarbonyloxy, 1-Methylpentylcarbonyloxy, 2-Methylpentylcarbonyloxy, 3-Methylpentylcarbonyloxy, 4-Methylpentylcarbonyloxy, 1,1-Dimethylbutylcarbonyloxy, 1,2-Dimethylbutylcarbonyloxy, 1,3-Dimethylbutylcarbonyloxy, 2,2-Dimethylbutylcarbonyloxy, 2,3-Dimethylbutylcarbonyloxy, 3,3-Dimethylbutylcarbonyloxy, 1-Ethylbutylcarbonyloxy, 2-Ethylbutylcarbonyloxy, 1,1,2-Trimethylpropylcarbonyloxy, 1,2,2-Trimethylpropylcarbonyloxy, 1-Ethyl-1-methylpropylcarbonyloxy und 1-Ethyl-2-methylpropylcarbonyloxy;
die Benzoylgruppe oder ein am Phenylkern durch ein bis fünf Halogenatome substituiertes Derivat;
eine unverzweigte oder verzweigte C₁-C₁₀-Alkylsulfonylgruppe, insbesondere eine C₁-C₆-Alkylsulfonylgruppe wie Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl, 1,1-Dimethylethylsulfonyl, n-Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, n-Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methyIpropylsulfonyl und 1-Ethyl-2-methylpropylsulfonyl;
die Benzolsulfonylgruppe und ein am Phenylkern durch ein bis fünf Halogenatome substituiertes Derivat;
eine unverzweigte oder verzweigte C₁-C₁₀-Alkylphosphonylgruppe, insbesondere eine C₁-C₆-Alkylphosphonylgruppe wie Methylphosphonyl, Ethylphosphonyl, n-Propylphosphonyl, 1-Methylethylphosphonyl, n-Butylphosphonyl, 1-Methylpropylphosphonyl, 2-Methylpropylphosphonyl, 1,1-Dimethylethylphosphonyl, n-Pentylphosphonyl, 1-Methylbutylphosphonyl, 2-Methylbutylphosphonyl, 3-Methylbutylphosphonyl, 1,1-Dimethylpropylphosphonyl, 1,2-Dimethylpropylphosphonyl, 2,2-Dimethylpropylphosphonyl, 1-Ethylpropylphosphonyl, Hexylphosphonyl, 1-Methylpentylphosphonyl, 2-Methylpentylphosphonyl, 3-Methylpentylphosphonyl, 4-Methylpentylphosphonyl, 1,1-Dimethylbutylphosphonyl, 1,2-Dimethylbutylphosphonyl, 1,3-Dimethylbutylphosphonyl, 2,2-Dimethylbutylphosphonyl, 2,3-Dimethylbutylphosphonyl, 3,3-Dimethylbutylphosphonyl, 1-Ethylbutylphosphonyl, 2-Ethylbutylphosphonyl, 1,1,2-Trimethylpropylphosphonyl, 1,2,2-Trimethylpropylphosphonyl, 1-Ethyl-1-methylpropylphosphonyl und 1-Ethyl-2-methylpropylphosphonyl;
die Benzolphosphonylgruppe oder ein am Phenylkern durch ein bis fünf Halogenatome substituiertes Derivat;
R^{c}
   Wasserstoff; die Cyanogruppe; die Formylgruppe;
   eine unverzweigte oder verzweigte C₁-C₆-Alkylgruppe wie vorstehend genannt, insbesondere Ethyl, n-Propyl und Isopropyl;
eine C₁-C₄-Alkoxy-C₁-C₄-alkylgruppe wie Methoxymethyl, Ethoxymethyl, n-Propoxymethyl, 1-Methoxyethyl, 2-Methoxyethyl, 1-Ethoxyethyl, 2-Ethoxyethyl, 2-Propoxyethyl, 1-Methoxypropyl, 2-Methoxypropyl, 3-Methoxypropyl, 1-Ethoxypropyl, 2-Ethoxypropyl, 3-Ethoxypropyl, 1-Methoxybutyl, 2-Methoxybutyl, 3-Methoxybutyl, 4-Methoxybutyl, 1-Ethoxybutyl, 2-Ethoxybutyl, 3-Ethoxybutyl, 4-Ethoxybutyl, 1,3-Dimethoxypropyl, vorzugsweise Methoxymethyl und 2-Ethoxy-ethyl;
eine C₁-C₄-Alkylthio-C₁-C₄-alkylgruppe wie Methylthiomethyl, Ethylthiomethyl, 1-Methylthioethyl, 2-Methylthioethyl, 1-Ethylthioethyl, 2-Ethylthioethyl, 1-Methylthiopropyl, 2-Methylthiopropyl, 3-Methylthiopropyl, 1-Ethylthiopropyl, 2-Ethylthiopropyl, 3-Ethylthiopropyl, 1-Methylthiobutyl, 2-Methylthiobutyl, 3-Methylthiobutyl, 4-Methylthiobutyl, 1-Ethylthiobutyl, 2-Ethylthiobutyl, 3-Ethylthiobutyl, 4-Ethylthiobutyl, vorzugsweise 2-Ethylthiopropyl;
eine Phenoxy-C₁-C₆-alkyl-, Phenylthio-C₁-C₆-alkyl-, Pyridyloxy-C₁-C₆-alkyl- oder Pyridylthio-C₁-C₆-alkylgruppe, wobei die Phenyl- und Pyridylringe unsubstituiert sein oder ein bis drei Reste tragen können, ausgewählt aus einer Gruppe, bestehend aus Nitro, Cyano, Halogen wie Fluor, Chlor, Brom und Jod, insbesondere Fluor und Chlor,
   unverzweigtem oder verzweigtem C₁-C₄-Alkyl wie Methyl, Ethyl, n-Propyl, Isopropyl, n-Butyl und tert.-Butyl,
   partiell oder vollständig halogeniertem C₁-C₄-Alkyl, insbesondere C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl,
   C₁-C₄-Alkoxy wie Methoxy, Ethoxy, n-Propoxy, Isopropoxy, n-Butoxy und tert.-Butoxy, vorzugsweise Methoxy und Ethoxy,
   partiell oder vollständig halogeniertem C₁-C₄-Alkoxy, insbesondere C₁-C₂-Halogenalkoxy wie Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy und Pentafluorethyloxy, insbesondere Trifluormethoxy,
   C₁-C₄-Alkylthio wie Methylthio, Ethylthio, n-Propylthio, Isopropylthio, n-Butylthio und tert.-Butylthio, vorzugsweise Methylthio und Ethylthio,
   C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1, 3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, vorzugsweise Prop-2-enyl und But-3-enyl,
   C₃-C₆-Alkenyloxy wie Prop-2-en-1-yloxy, n-Buten-4-yloxy, n-Buten-3-yloxy, 1-Methyl-prop-2-en-1-yloxy, 2-Methyl-prop-2-en-1-yloxy, n-Penten-3-yloxy, n-Penten-4-yloxy, n-Penten-5-yloxy, 1-Methyl-but-2-en-1- yloxy, 2-Methyl-but-2-en-1-yloxy, 3-Methyl-but-2-en-1-yloxy, 1-Methyl-but-3-en-1-yloxy, 2-Methylbut-3-en-1-yloxy, 3-Methyl-but-3-en-1-yloxy, 1,1-Dimethyl-prop-2-en-1-yloxy, 1,2-Dimethyl-prop-2-en-1-yloxy, 1-Ethyl-prop-2-en-1-yloxy, n-Hex-2-en-1-yloxy, n-Hex-3-en-1-yloxy, n-Hex-4-en-1-yloxy, n-Hex-5-en-1-yloxy, 1-Methyl-pent-2-en-1-yloxy, 2-Methylpent-2-en-1-yloxy, 3-Methyl-pent-2-en-1-yloxy, 4-Methyl-pent-2-en-1-yloxy, 1-Methyl-pent-3-en-1-yloxy, 2-Methyl-pent-3-en-1-yloxy, 3-Methyl-pent-3-en-1-yloxy, 4-Methyl-pent-3-en-1-yloxy, 1-Methyl-pent-4-en-1-yloxy, 2-Methyl-pent-4-en-1-yloxy, 3-Methylpent-4-en-1-yloxy, 4-Methyl-pent-4-en-1-yloxy, 1,1-Dimethyl-but-2-en-1-yloxy, 1,1-Dimethyl-but-3-en-1-yloxy, 1,2-Dimethyl-but-2-en-1-yloxy, 1,2-Dimethyl-but-3-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-2-en-1-yloxy, 2,3-Dimethyl-but-3-en-1-yloxy, 3,3-Dimethyl-but-2-en-1-yloxy, 1-Ethyl-but-2-en-1-yloxy, 1-Ethyl-but-3-en-1-yloxy, 2-Ethyl-but-2-en-1-yloxy, 2-Ethyl-but-3-en-1-yloxy, 1,1,2-Trimethylprop-2-en-1-yloxy, 1-Ethyl-1-methyl-prop-2-en-1-yloxy und 1-Ethyl-2-methyl-prop-2-en-1-yloxy, vorzugsweise Prop-2-en-1-yloxy und But-2-en-1-yloxy, C3-C6-Alkinyl wie Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pentin-5-yl, Pent-2-in-1-yl, Pent-2-in-4-yl, Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methylpent-1-in-1-yl, 4-Methyl-pent-2-in-4-yl und 4-Methyl-pent-2-in-5-yl, vorzugsweise Prop-2-inyl,
   C₃-C₆-Alkinyloxy wie Prop-2-in-3-yloxy, n-But-1-in-4-yloxy, n-But-2-in-1-yloxy, n-Pent-1-in-3-yloxy, n-Pent-1-in-4-yloxy, n-Pent-1-in-5-yloxy, Pent-2-in-1-yloxy, Pent-2-in-4-yloxy, Pent-2-in-5-yloxy, 3-Methyl-but-1-in-3-yloxy, 3-Methyl-but-1-in-4-yloxy, n-Hex-1-in-3-yloxy, n-Hex-1-in-4-yloxy, n-Hex-1-in-5-yl-oxy, n-Hex-1-in 6-yloxy, n-Hex-2-in-1-yloxy, n-Hex-2-in-4- yloxy, n-Hex-2-in-5-yloxy, n-Hex-2-in-6-yloxy, n-Hex-3-in-1-yloxy, n-Hex-3-in-2-yloxy, 3-Methyl-pent-1-in-3-yloxy, 3-Methyl-pent-1-in-4-yloxy, 3-Methylpent-1-in-5-yloxy, 4-Methyl-pent-2-in-4-yloxy und 4-Methyl-pent-2-in-5-yloxy, vorzugsweise Prop-2-inyloxy,
oder eine Gruppe NR^{g}R^{h}, wobei
R^{h}
   Wasserstoff, unverzweigtem oder verzweigtes C₁-C₄-Alkyl wie vorstehend genannt, C₃-C₆-Alkenyl wie vorstehend genannt, oder C₃-C₆-Alkinyl wie vorstehend genannt, vorzugsweise Wasserstoff,
   und
R^{g}
   Wasserstoff, unverzweigte oder verzweigtes C₁-C₄-Alkyl wie vorstehend genannt,
C₃-C₆-Alkenyl wie vorstehend genannt,
C₃-C₆-Alkinyl wie vorstehend genannt, C₁-C₆-Acyl wie Methylcarbonyl, Ethylcarbonyl, n-Propylcarbonyl, 1-Methylethyl-carbonyl, n-Butylcarbonyl, 1-Methylpropylcarbonyl, 2-Methylpropylcarbonyl, 1,1-Dimethylethylcarbonyl, n-Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, n-Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, insbesondere Methylcarbonyl und 1,1-Dimethylethylcarbonyl ;
   oder
   Benzoyl, das unsubstituiert sein oder einen bis drei Substituenten tragen kann, ausgewählt aus einer Gruppe bestehend aus Nitro, Cyano, Halogen wie vorstehend genannt, insbesondere Fluor und Chlor, unverzweigte oder verzweigtes C₁-C₄-Alkyl wie vorstehend genannt,
   partiell oder vollständig halogeniertem C₁-C₄-Alkyl, insbesondere C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl;
   C₁-C₄-Alkoxy wie vorstehend genannt und C₁-C₄-Alkylthio wie vorstehend genannt,
bedeuten;
besonders bevorzugte Phenoxy-C₁-C₆-alkyl-, Phenylthio-C₁-C₆-alkyl-, Pyridyloxy-C₁-C₆-alkyl- oder Pyridylthio-C₁-C₆-alkylgruppen sind Phenoxymethyl, Phenoxyethyl, Phenoxypropyl, Phenoxybutyl, 4-Fluorphenoxy-ethyl, 2-(4-Fluorphenoxy)-propyl, 4-Trifluormethylphenoxy-ethyl, 2-(4-Trifluormethylphenoxy)-propyl, Phenylthiomethyl, Phenylthioethyl, Phenylthiopropyl, Phenylthiobutyl, 4-Fluorphenylthio-ethyl, 2-(4-Fluorphenylthio)-propyl, 4-Trifluormethylphenylthio-ethyl, 2-(4-Trifluormethylphenylthio)-propyl, insbesondere 4-Fluorphenylthio-ethyl und 4-Trifluormethylphenylthio-ethyl;
eine C₃-C₇-Cycloalkyl- oder C₅-C₇-Cycloalkenylgruppe wie Cyclopropyl, Cyclobutyl, Cyclopentyl, Cyclohexyl, Cycloheptyl, Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl und Cyclohept-4-enyl, wobei die Carbocyclen jeweils unsubstituiert sein oder einen bis drei Reste tragen können, ausgewählt aus einer Gruppe bestehend aus Hydroxyl, Halogen wie vorstehend genannt, insbesondere Fluor und Chlor, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, C₁-C₄-Alkylthio wie vorstehend genannt, insbesondere Methylthio, Ethylthio, Benzylthio, C₁-C₄-Alkylsulfonyl wie Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl und 1,1-Dimethylethylsulfonyl, vorzugsweise Methylsulfonyl,
   sowie C₁-C₄-Alkylsulfinyl wie Methylsulfinyl, Ethylsulfinyl, n-Propylsulfinyl, 1-Methylethyl-sulfinyl, n-Butylsulfinyl, 1-Methylpropylsulfinyl, 2-Methylpropylsulfinyl und 1,1-Dimethylethylsulfinyl, vorzugsweise Methylsulfinyl,
unter den substituierten C₃-C₇-Cycloalkyl- und C₅-C₇-Cycloalkenylgruppen sind 1-Methylthiocyclopropyl, 1-Ethylthiocyclopropyl, 4-Methylcyclohexyl,
   4-Methylcyclohex-3-enyl,
   3-Ethylthio-k-hydroxy-4-methylcyclohexyl, 3,4-Dihydroxycyclohexyl, insbesondere 1-Methylthiocyclopropyl, 1-Ethylthiocyclopropyl und 3,4-Dihydroxycyclohexyl, besonders bevorzugt;
5-gliedriges Heterocycloalkyl wie Tetrahydrofuran-2-yl, Tetrahydrofuran-3-yl, Tetrahydrothien-2-yl, Tetrahydrothien-3-yl, Pyrrolidin-2-yl, Pyrrolidin-3-yl, Isoxazolidin-3-yl, Isoxazolidin-4-yl, Isoxazolidin-5-yl, Isothiazolidin-3-yl, Isothiazolidin-4-yl, Isothiazolidin-5-yl, Pyrazolidin-3-yl, Pyrazolidin-4-yl, Pyrazolidin-5-yl, Oxazolidin-2-yl, Oxazolidin-4-yl, Oxazolidin-5-yl, Thiazolidin-2-yl, Thiazolidin-4-yl, Thiazolidin-5-yl, Imidazolidin-2-yl, Imidazolidin-4-yl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, Dioxolanyl, Dithiolanyl, insbesondere Tetrahydrofuran-2-yl, Tetrahydrofuran-3-yl und Dioxolanyl, wobei die Heterocyclen jeweils unsubstituiert sein oder einen bis drei Reste tragen können, ausgewählt aus einer Gruppe bestehend aus unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, und C₁-C₄-Alkylthio wie vorstehend genannt, insbesondere Methylthio;
ein 6- oder 7-gliedriger Heterocyclus wie Tetrahydrothiopyran-2-yl, Tetrahydrothiopyran-3-yl, Tetrahydrothiopyran-4-yl, Tetrahydropyran-2-yl, Tetrahydropyran-3-yl, Tetrahydropyran-4-yl, 5,6-Dihydro-2H-thiopyran-2-yl, 5,6-Dihydro-2H-thiopyran-3-yl, 5,6-Dihydro-2H-thiopyran-4-yl, 5,6-Dihydro-2H-thiopyran-5-yl, 5,6-Dihydro-2H-thiopyran-6-yl, 5,6-Dihydro-2H-pyran-2-yl, 5,6-Dihydro-2H-pyran-3-yl, 5,6-Dihydro-2H-pyran-4-yl, 5,6-Dihydro-2H-pyran-5-yl, 5,6-Dihydro-2H-pyran-6-yl und Dioxepan-5-yl, insbesondere Tetrahydrothiopyran-3-yl, Tetrahydropyran-3-yl und Tetrahydropyran-4-yl, wobei die Heterocyclen jeweils unsubstituiert sein oder einen bis drei Reste tragen können, ausgewählt aus einer Gruppe bestehend aus Hydroxyl, Halogen wie vorstehend genannt, insbesondere Chlor und Brom, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, und C₁-C₄-Alkylthio wie vorstehend genannt, insbesondere Methylthio und Ethylthio;
ein 5-gliedriger Heteroaromat, enthaltend ein bis drei Heteroatome, ausgewählt aus einer Gruppe bestehend aus ein oder zwei Stickstoffatomen und einem Sauerstoff- oder Schwefelatom, beispielsweise 2-Furyl, 3-Furyl, 2-Thienyl, 3-Thienyl, 2-Pyrrolyl,. 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, k-Oxazolyl, 5-Oxazolyl, 2-Thiazolyl, 4-Thiazolyl, 5-Thiazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, insbesondere Isoxazolyl und Pyrazolyl, wobei der Heteroaromat unsubstituiert sein oder einen bis drei Reste tragen kann, ausgewählt aus einer Gruppe bestehend aus Cyano, Halogen wie vorstehend genannt, insbesondere Fluor und Chlor, unverzweigtem oder verzweigtem C₁-C₄-Alkyl wie vorstehend genannt, insbesondere Methyl, Ethyl und Isopropyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, partiell oder vollständig halogeniertem C₁-C₄-Alkoxy wie vorstehend genannt, insbesondere Trifluormethoxy, C₁-C₄-Alkylthio wie vorstehend genannt, insbesondere Methylthio, C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1, 3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere 1-Methylethenyl, C₂-C₆-Alkenyloxy wie Ethenyloxy, Prop-2-en-1-yloxy, n-Buten-4-yloxy, n-Buten-3-yloxy, 1-Methyl-prop-2-en-1-yloxy, 2-Methyl-prop-2-en-1-yloxy, n-Penten-3-yloxy, n-Penten-4-yloxy, n-Penten-5-yloxy, 1-Methyl-but-2-en-1-yloxy, 2-Methyl-but-2-en-1-yloxy, 3-Methyl-but-2-en-1-yloxy, 1-Methyl-but-3-en-1-yloxy, 2-Methylbut-3-en-1-yloxy, 3-Methyl-but-3-en-1-yloxy, 1,1-Dimethyl-prop-2-en-1-yloxy, 1,2-Dimethyl-prop-2-en-1-yloxy, 1-Ethyl-prop-2-en-1-yloxy, n-Hex-2-en-1-yloxy, n-Hex-3-en-1-yloxy, n-Hex-4-en-1-yloxy, n-Hex-5-en-1-yloxy, 1-Methyl-pent-2-en-1-yloxy, 2-Methylpent-2-en-1-yloxy, 3-Methyl-pent-2-en-1-yloxy, 4-Methyl-pent-2-en-1-yloxy, 1-Methyl-pent-3-en-1-yloxy, 2-Methyl-pent-3-en-1-yloxy, 3-Methyl-pent-3-en-1-yloxy, 4-Methyl-pent-3-en-1-yloxy, 1-Methyl-pent-4-en-1-yloxy, 2-Methyl-pent-4-en-1-yloxy, 3-Methylpent-4-en-1-yloxy, 4-Methyl-pent-4-en-1-yloxy, 1,1-Dimethyl-but-2-en-1-yloxy, 1,1-Dimethyl-but-3-en-1-yloxy, 1,2-Dimethyl-but-2-en-1-yloxy, 1,2-Dimethyl-but-3-en-1-yloxy, 1,3-Dimethyl-but-2-en-1-yloxy, 1,3-Dimethyl-but- 3-en-1-yloxy, 2,2-Dimethyl-but-3-en-1-yloxy, 2,3-Dimethyl-but-2-en-1-yloxy, 2,3-Dimethyl-but-3-en-1-yloxy, 3,3-Dimethyl-but-2-en-1-yloxy, 1-Ethyl-but-2-en-1-yloxy, 1-Ethyl-but-3-en-1-yloxy, 2-Ethyl-but-2-en-1-yloxy, 2-Ethyl-but-3-en-1-yloxy, 1,1,2-Trimethylprop-2-en-1-yloxy, 1-Ethyl-1-methyl-prop-2-en-1-yloxy und 1-Ethyl-2-methyl-prop-2-en-1-yloxy, vorzugsweise Prop-2-en-1-yloxy, C₃-C₆-Alkinyloxy wie Prop-2-in-3-yloxy, n-But-1-in-4-yloxy, n-But-2-in-1-yloxy, n-Pent-1-in-3-yloxy, n-Pent-1-in-4-yloxy, n-Pent-1-in-5-yloxy, Pent-2-in-1-yloxy, Pent-2-in-4-yloxy, Pent-2-in-5-yloxy, 3-Methyl-but-1-in-3-yloxy, 3-Methyl-but-1-in-4-yloxy, n-Hex-1-in-3-yloxy, n-Hex-1-in-4-yloxy, n-Hex-1-in-5-yloxy, n-Hex-1-in 6-yloxy, n-Hex-2-in-1-yloxy, n-Hex-2-in-4-yloxy, n-Hex-2-in-5-yloxy, n-Hex-2-in-6-yloxy, n-Hex-3-in-1-yloxy, n-Hex-3-in-2-yloxy, 3-Methyl-pent-1-in-3-yloxy, 3-Methyl-pent-1-in-4-yloxy, 3-Methylpent-1-in- 5-yloxy, 4-Methyl-pent-2-in-4-yloxy und 4-Methyl-pent-2-in-5-yloxy, vorzugsweise Prop-2-inyloxy, und C₁-C₄-Alkoxy-C₁-C₄-alkyl wie Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)-methyl, Methoxyethyl, Ethoxyethyl, n-Propoxyethyl, (1-Methylethoxy)ethyl, n-Butoxyethyl, (1-Methylpropoxy)ethyl, (2-Methylpropoxy)ethyl, (1,1-Dimethylethoxy)ethyl, 3-(Methoxy)propyl, 2-(Methoxy)propyl und 2-(Ethoxy)propyl, vorzugsweise Methoxymethyl und Ethoxyethyl,
eine Phenyl- oder Pyridylgruppe, die beide unsubstituiert sein oder ein bis drei Reste tragen können, ausgewählt aus einer Gruppe bestehend aus Nitro, Cyano, Formyl, Halogen wie vorstehend genannt, insbesondere Fluor und Chlor, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, partiell oder vollständig halogeniertem C₁-C₄-Alkoxy wie vorstehend genannt, insbesondere Trifluormethoxy, C₁-C₄-Alkylthio wie vorstehend genannt, insbesondere Methylthio, C₃-C₆-Alkenyl wie vorstehend genannt, insbesondere C₃-C₄-Alkenyl, vorzugsweise Prop-2-enyl, C₃-C₆-Alkenyloxy wie vorstehend genannt, insbesondere C₃-C₄-Alkenyloxy, vorzugsweise Prop-2-enyloxy, C₃-C₆-Alkinyl wie vorstehend genannt, insbesondere C₃-C₄-Alkinyl, vorzugsweise Prop-2-inyl, C₃-C₆-Alkinyloxy wie vorstehend genannt, insbesondere C₃-C₄-Alkinyloxy, vorzugsweise Prop-2-inyloxy und NR^{g}R^{h}, wobei R^{g} und R^{h} die oben genannte Bedeutung haben, vorzugsweise Wasserstoff, Acetyl und Benzoyl;
besondere bevorzugte Phenyl- und Pyridylgruppen sind Phenyl, 4-Ethylphenyl, 4-Propargyloxyphenyl, 2,4,6-Trimethylphenyl, 4-Benzoylamino-3-fluorphenyl, 4-Formylphenyl und Pyridyl;
W
   eine C₁-C₆-Alkylen-, eine C₃-C₆-Alkenylen- oder eine C₃-C₆-Alkinylenkette wie Methylen, Ethylen, Propylen, Butylen, Pentylen, Hexylen, Propenylen, Prop-2-enylen, Butenylen, But-2-enylen, But-3-enylen, Pentenylen, Pent-2-enylen, Pent-3-enylen, Pent-4-enylen, Hex-1-enylen, Hex-2-enylen, Hex-3-enylen, Hex-4-enylen, Hex-5-enylen, Prop-2-inylen, But-2-inylen, But-3-inylen, Pent-2-inylen, Pent-3-inylen, Pent-4-inylen, Hex-2-inylen, Hex-3-inylen, Hex-4-inylen, Hex-5-inylen, wobei diese Ketten gewünschtenfalls noch ein bis drei Reste tragen können, ausgewählt aus einer Gruppe bestehend aus ein bis drei Halogenatomen wie vorstehend genannt, insbesondere Fluor und Chlor, ein bis drei C₁-C₃-Alkylsubstituenten wie Methyl, Ethyl, n-Propyl und Isopropyl, insbesondere Methyl und Ethyl, und einem Methylensubstituenten. Bei den ungesättigten Ketten können sowohl die cis-als auch die trans-Form auftreten. Besonders bevorzugt sind Propylen, Butylen, Prop-2-enylen, But-2-enylen, But-3-enylen und But-3-inylen;
eine C₃-C₆-Alkylen- oder C₄-C₆-Alkenylengruppe, die beide unsubstituiert sein oder ein bis drei C₁-C₄-Alkylreste tragen könen, wobei jeweils eine Methylengruppe durch ein Sauerstoffoder Schwefelatom, eine Sulfoxid-, eine Sulfongruppe oder eine Gruppe -N(Rⁱ)-, mit Rⁱ Wasserstoff, unverzweigtes oder verzweigtes C₁-C₄-Alkyl wie vorstehend genannt, insbesondere Methyl und Ethyl, C₃-C₆-Alkenyl wie vorstehend genannt, insbesondere Prop-2-enyl und But-2-enyl, oder C₃-C₆-Alkinyl wie vorstehend genannt, insbesondere Prop-2-inyl und But-2-inyl, ersetzt sein kann, beispielsweise 3-Oxapropylen, 3-Azapropylen, 3-Thiapropylen, 3-Thiapropylen-3-oxid, 3-Thiapropylen-3,3-dioxid, 3-Oxabutylen, 3-Azabutylen, 3-Thiabutylen, 3-Thiabutylen-3-oxid, 3-Thiabutylen-3,3-dioxid, 4-Oxabutylen, 4-Azabutylen, 4-Thiabutylen, 4-Thiabutylen-4-oxid, 4-Thiabutylen-4,4-dioxid, 4-oxabut-2-enylen, 4-Azabut-2-enylen, 4-Thiabut-2-enylen, 3-Oxapentylen, 3-Azapentylen, 3-Thiapentylen, 3-Thiapentylen-3-oxid, 3-Thiapentylen-3,3-dioxid, 4-Oxapentylen, 4-Azapentylen, 4-Thiapentylen, 4-Thiapentylen-4-oxid, 4-Thiapentylen-4,4-dioxid, 5-Oxapentylen, 5-Azapentylen, 5-Thiapentylen, 5-Thiapentylen-5-oxid, 5-Thiapentylen-5,5-dioxid, 5-Oxapent-3-enylen, 5-Azapent-3-enylen, 5-Thiapent-3-enylen, 3-Oxahexylen, 3-Azahexylen, 3-Thiahexylen, 3-Thiahexylen-3-oxid, 3-Thiahexylen-3,3-dioxid, 4-Oxahexylen, 4-Azahexylen, 4-Thiahexylen, 4-Thiahexylen-4-oxid, 4-Thiahexylen-4,4-dioxid, 5-Oxahexylen, 5-Azahexylen, 5-Thiahexylen, 5-Thiahexylen-5-oxid, 5-Thiahexylen-5,5-dioxid, 6-Oxahexylen, 6-Azahexylen, 6-Thiahexylen, 6-Thiahexylen-6-oxid, 6-Thiahexylen-6,6-dioxid, 6-Oxahex-4-enylen, 6-Azahex-4-enylen, 6-Thiahex-4-enylen.

Bei den ungesättigten Ketten können die Doppelbindungen sowohl cis- als auch die trans-Konfiguration aufweisen.

Besonders bevorzugt sind 3-Oxapropylen, 2-Methyl-3-oxapropylen, 3-Oxabutylen und 4-Oxabutylen;
R^{f}
   Wasserstoff; die Vinylgruppe;
eine Gruppe-CH=CH-Z, wobei Z für Cyano;
   Halogen wie vorstehend genannt, insbesondere Fluor und Chlor; einen unverzweigten oder verzweigten C₁-C₄-Alkylrest wie vorstehend genannt, insbesondere Methyl und 1,1-Dimethylethyl ; einen partiell oder vollständig halogenierten C₁-C₄-Alkylrest wie vorstehend genannt, insbesondere Difluormethyl, Trifluormethyl, 2,2,2-Trifluorethyl und Pentafluorethyl;
einen C₃-C₆-Cycloalkyirest wie Cyclopropyl, Cyclobutyl, Cyclopentyl und Cyclohexyl, der unsubstituiert sein oder ein bis drei Substituenten tragen kann, ausgewählt aus einer Gruppe bestehend aus Hydroxyl, Halogen wie vorstehend genannt, insbesondere Fluor und Chlor, unverzweigtem oder verzweigtem C₁-C₄-Alkyl wie vorstehend genannt, insbesondere Methyl und Isopropyl, partiell oder vollständig halogeniertem C₁-C₄-Alkyl wie vorstehend genannt, insbesondere Trifluormethyl, und C₁-C₄-Alkoxy wie vorstehend genannt, insbesondere Methoxy;
den Carboxylrest;
einen C₁-C₈-Alkoxycarbonylrest wie Methoxycarbonyl, Ethoxycarbonyl, n-Propyloxycarbonyl, 1-Methyl-ethoxycarbonyl, n-Butoxycarbonyl, 1-Methylpropyloxycarbonyl, 2-Methylpropyloxycarbonyl, 1,1-Dimethylethoxycarbonyl, n-Pentoxycarbonyl, 1-Methylbutyloxycarbonyl, 2-Methylbutyloxycarbonyl, 3-Ethylbutyloxycarbonyl, 2,2-Dimethylpropyloxycarbonyl, 1-Ethylpropyloxycarbonyl, n-Hexoxycarbonyl, 1,1-Dimethyl-propoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropoxycarbonyl, 1-Ethyl-1-methylpropyloxycarbonyl und 1-Ethyl-2-methylpropyloxycarbonyl, insbesondere Methoxycarbonyl;
den Benzyloxycarbonylrest;
den Phenyl-, Thienyl- oder Pyridylrest, wobei diese Reste jeweils unsubstituiert sein oder ein bis drei Substituenten tragen können, ausgewählt aus einer Gruppe bestehend aus Nitro, Cyano, Halogen wie vorstehend genannt, insbesondere Fluor und Chlor, unverzweigtem oder verzweigtem C₁-C₄-Alkyl wie vorstehend genannt, insbesondere Methyl, Ethyl und 1-Methylethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy und Ethoxy, partiell oder vollständig halogeniertem C₁-C₄-Alkoxy wie vorstehend genannt, insbesondere Difluormethoxy und Trifluormethoxy, C₁-C₄-Alkylthio wie vorstehend genannt, insbesondere Methylthio, C₃-C₆-Cycloalkyl wie vorstehend genannt, wobei der Cycloalkylsubstituent unsubstituiert sein oder seinerseits ein bis drei Reste tragen kann, ausgewählt aus einer Gruppe bestehend aus Halogen wie vorstehend genannt, insbesondere Fluor und Chlor, unverzweigtem oder verzweigtem C₁-C₄-Alkyl wie vorstehend genannt, insbesondere Methyl, partiell oder vollständig halogeniertem C₁-C₄-Alkyl wie vorstehend genannt, insbesondere Trifluormethyl, und C₁-C₄-Alkoxy wie vorstehend genannt, insbesondere Methoxy;
steht;
die Ethinylgruppe, die einen der folgenden Reste tragen kann: unverzweigtes oder verzweigtes C₁-C₄-Alkyl wie vorstehend genannt, insbesondere Methyl und Ethyl,
C₃-C₆-Cycloalkyl wie Cyclopropyl, Cyclobutyl, Cyclopentyl und Cyclohexyl, wobei der Cycloalkylrest unsubstituiert sein oder seinerseits einen bis drei Substituenten tragen kann, ausgewählt aus einer Gruppe bestehend aus Hydroxyl, Halogen wie vorstehend genannt, insbesondere Fluor und Chlor, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, und C₁-C₄-Alkoxy wie vorstehend genannt, insbesondere Methoxy, den Phenyl-, Thienyl- oder Pyridylrest, wobei jeder dieser drei aromatischen Reste unsubstituiert sein oder einen bis drei Substituenten tragen kann, ausgewählt aus einer Gruppe bestehend aus Nitro, Cyano, Halogen wie vorstehend genannt, insbesondere Fluor und Chlor, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, partiell oder vollständig halogeniertem C₁-C₄-Alkoxy wie vorstehend genannt, insbesondere Trifluormethoxy und C₁-C₄-Alkylthio wie vorstehend genannt, insbesondere Methylthio;
sowie die Phenylgruppe; eine Halogenphenylgruppe; eine Dihalogenphenylgruppe;
eine 5-gliedrige heteroaromatische Gruppe mit ein bis drei Heteroatomen, ausgewählt aus einer Gruppe bestehend aus ein bis drei Stickstoffatomen und einem Sauerstoff- oder Schwefelatom wie Furanyl, Thienyl, Pyrrolyl, Isoxazolyl, Isothiazolyl, Pyrazolyl, Oxazolyl, Thiazolyl, Imidazolyl, 1,2,4-Oxadiazolyl, 1,2,4-Thiadiazolyl, 1,2,4-Triazolyl, 1,3,4-Oxadiazolyl, 1,3,4-Thiadiazolyl und 1,3,4-Triazolyl, insbesondere Furanyl und Thienyl;
eine 6-gliedrige heteroaromatische Gruppe mit ein bis vier Stickstoffatomen als Heteroatomen wie Pyridyl, Pyrimidyl, Pyrazyl, Pyridazinyl, Triazyl, Tetrazyl, insbesondere Pyridyl und Pyrimidyl;
   wobei die Phenyl- und Heteroarylgruppen unsubstituiert sein oder ein bis drei der folgenden Reste tragen können, im Falle der Heteroarylreste jedoch höchstens so viele, wie substituierbare C-Atome vorhanden sind:
Nitro;
   C₁-C₄-Alkoxyreste wie vorstehend genannt, insbesondere Methoxy; C₁-C₄-Alkylthioreste wie vorstehend genannt, insbesondere Methylthio;
   partiell oder vollständig halogenierte C₁-C₄-Alkoxyreste, insbesondere C₁-C₂-Halogenalkoxyreste wie vorstehend genannt, vorzugsweise Trifluormethoxy;
   Resten Z und einem Rest -NR^{k}R^{l} mit
   - R^{k}: Wasserstoff, unverzweigtem oder verzweigtem C₁-C₄-Alkyl wie vorstehend genannt, insbesondere Methyl und Ethyl, C₃-C₆-Alkenyl wie vorstehend genannt, insbesondere Prop-2-en-1-yl oder C₃-C₆-Alkinyl wie vorstehend genannt, insbesondere Prop-2-in-1-yl, und
   - R^{l}: Wasserstoff, unverzweigtem oder verzweigtem C₁-C₄-Alkyl wie vorstehend genannt, insbesondere Methyl und Ethyl, C₃-C₆-Alkenyl wie vorstehend genannt, insbesondere Prop-2-en-1-yl, C₃-C₆-Alkinyl wie vorstehend genannt, insbesondere Prop-2-in-1-yl, unverzweigtem oder verzweigtem C₁-C₆-Acyl wie Acetyl, Propionyl und Butyryl, oder Benzoyl, das gewünschtenfalls seinerseits noch einen bis drei Substituenten tragen kann, ausgewählt aus einer Gruppe bestehend aus Nitro, Cyano, Halogen wie vorstehend genannt, insbesondere Fluor und Chlor, unverzweigtem oder verzweigtem C₁-C₄-Alkyl wie vorstehend genannt, insbesondere Methyl, partiell oder vollständig halogeniertem C₁-C₄-Alkyl wie vorstehend genannt, insbesondere Trifluormethyl, C₁-C₄-Alkoxy wie vor- . stehend genannt, insbesondere Methoxy und Ethoxy, und C₁-C₄-Alkylthio wie vorstehend genannt, insbesondere Methylthio;

Bei mehreren Resten Z können die Substituenten gleich oder verschieden sein.

Ganz besonders bevorzugte Cyclohexenon-Derivate der Formel II, deren Kulturpflanzenverträglichkeit durch substituierte 3-Aminobenzo[b]thiophene I verbessert werden kann, sind den folgenden Tabellen 1 bis 8 zu entnehmen:

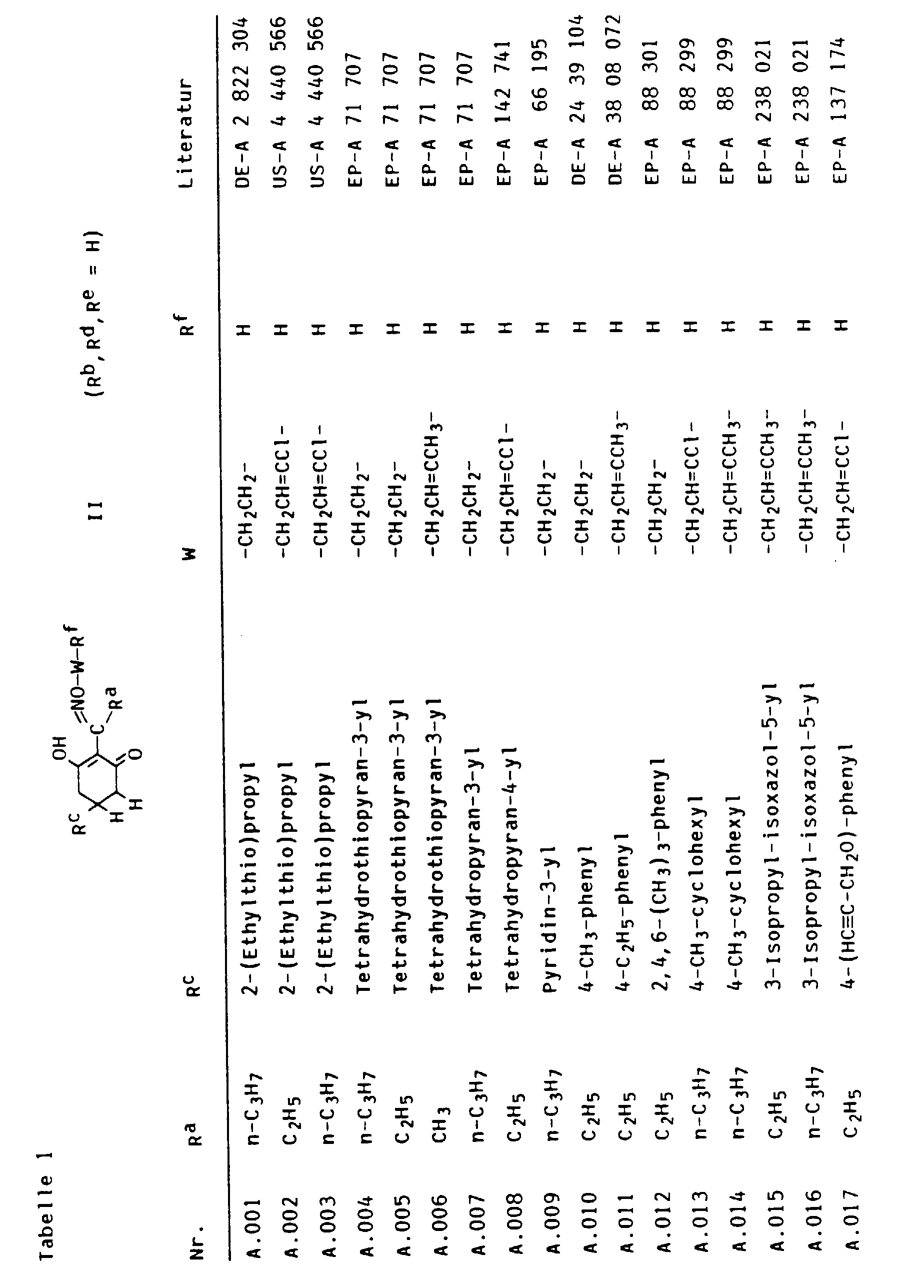

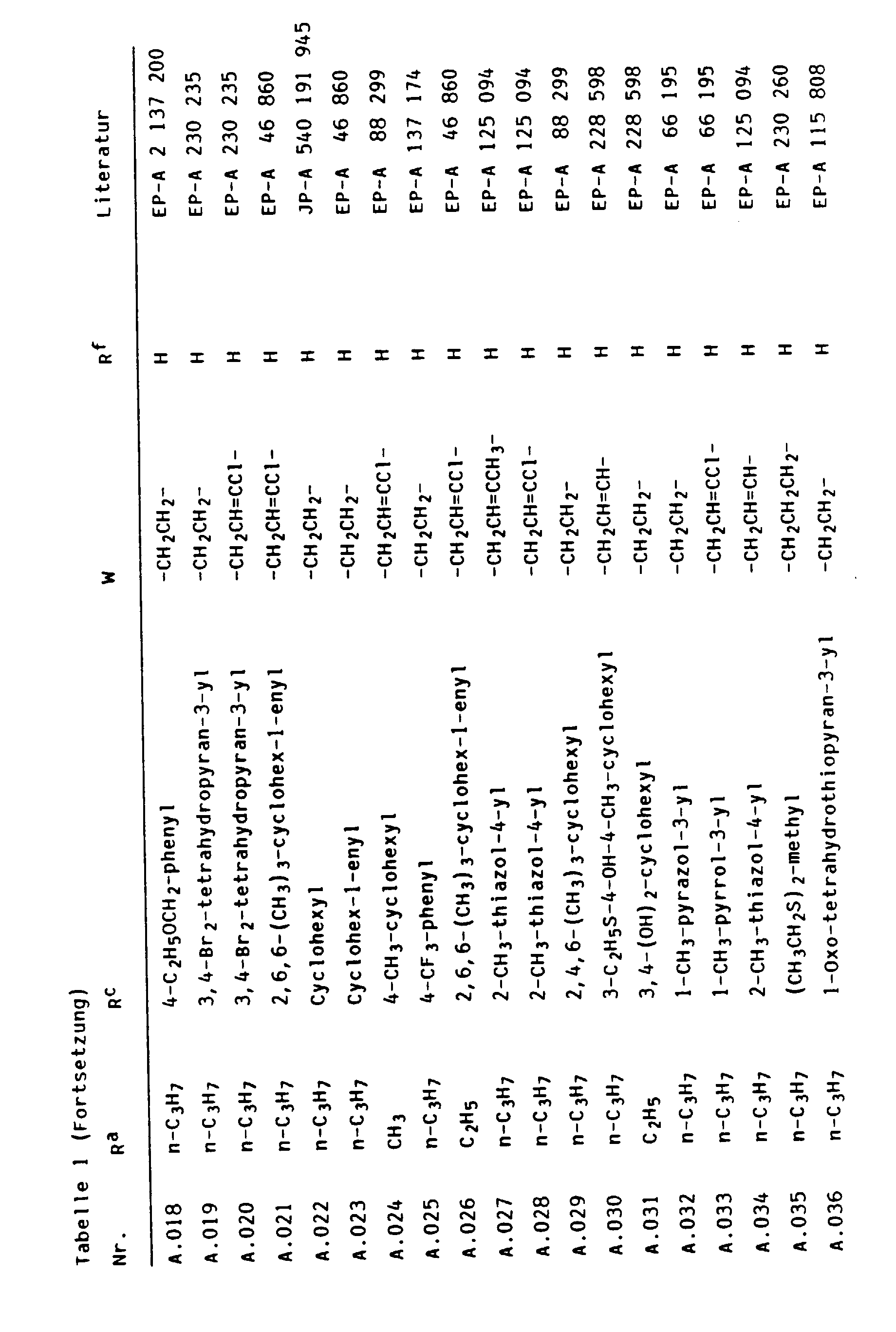

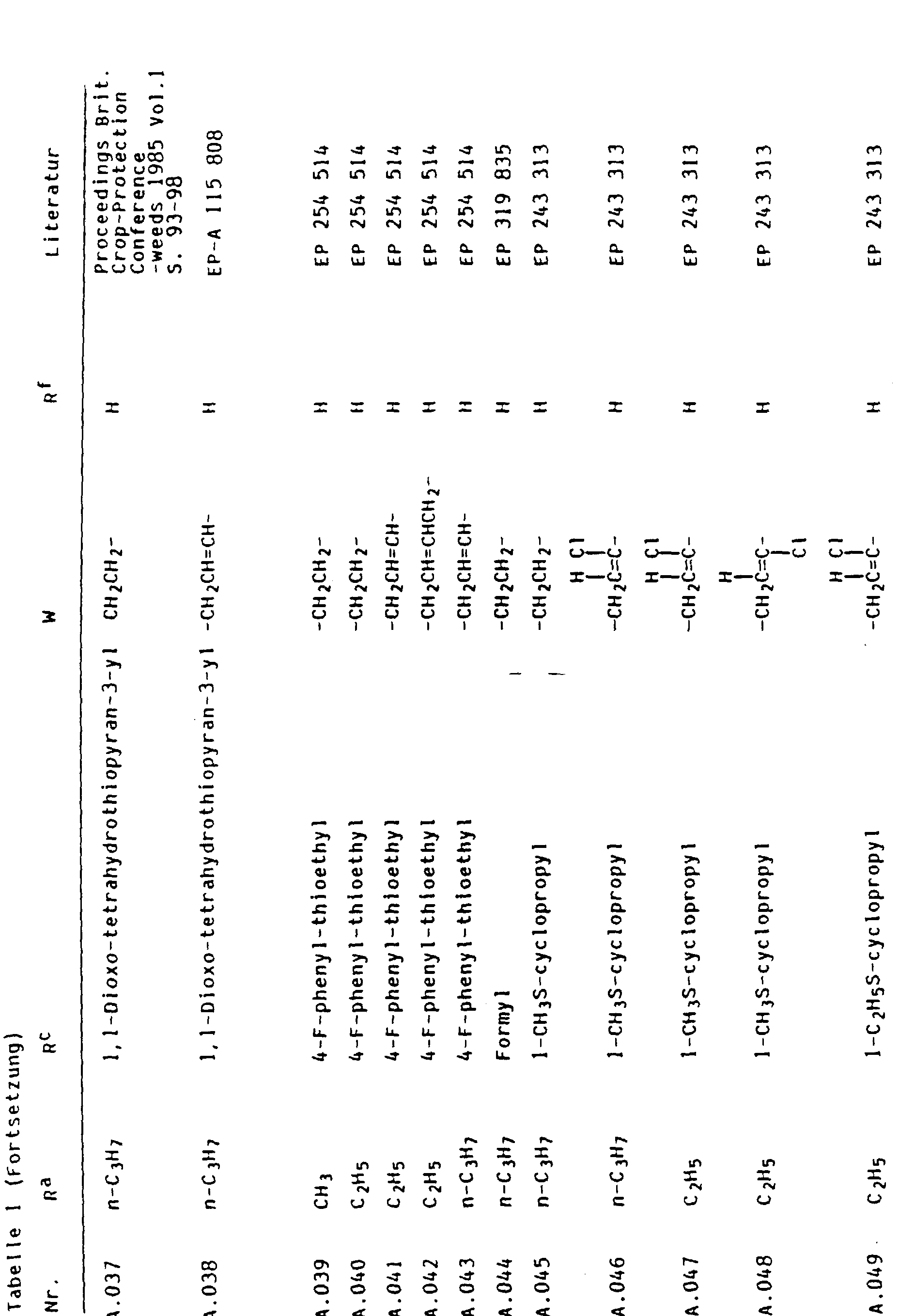

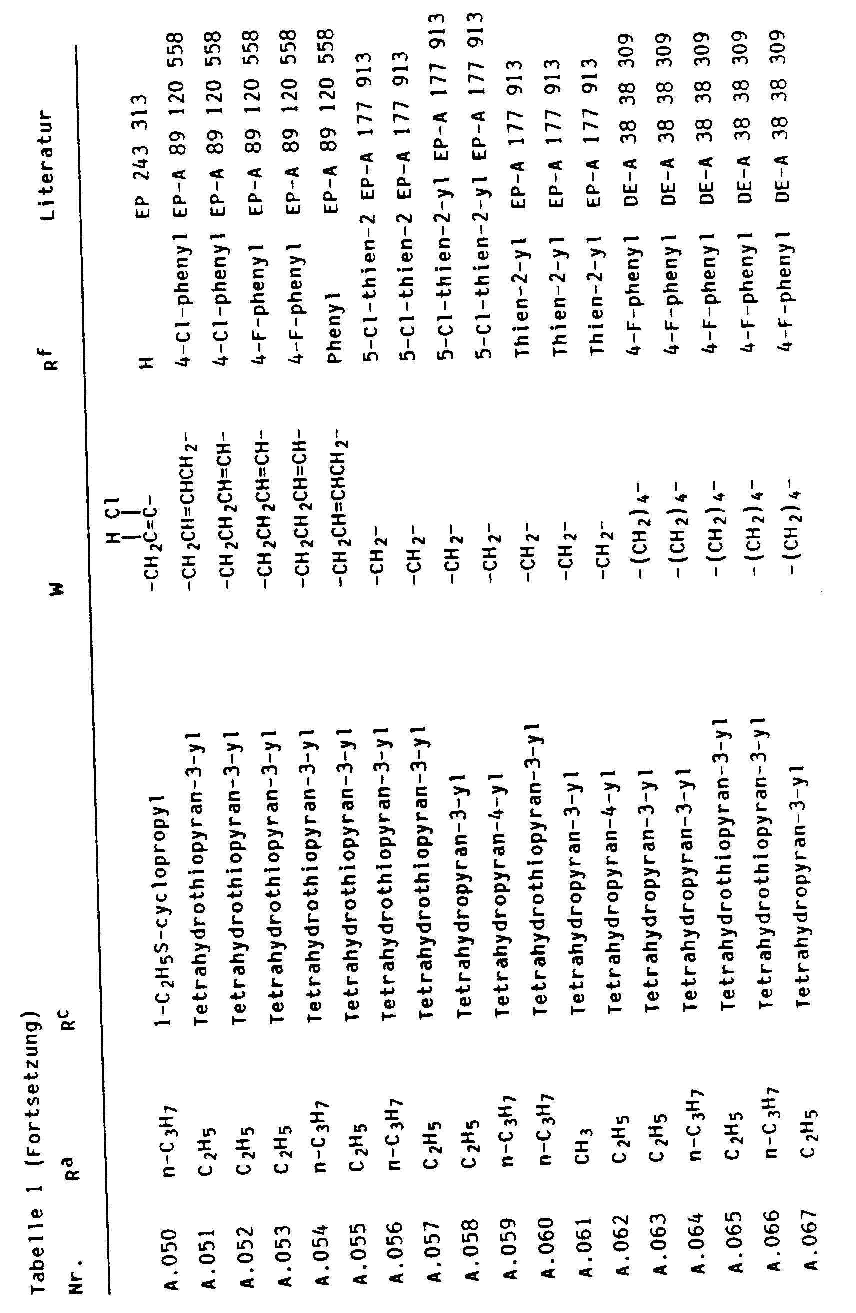

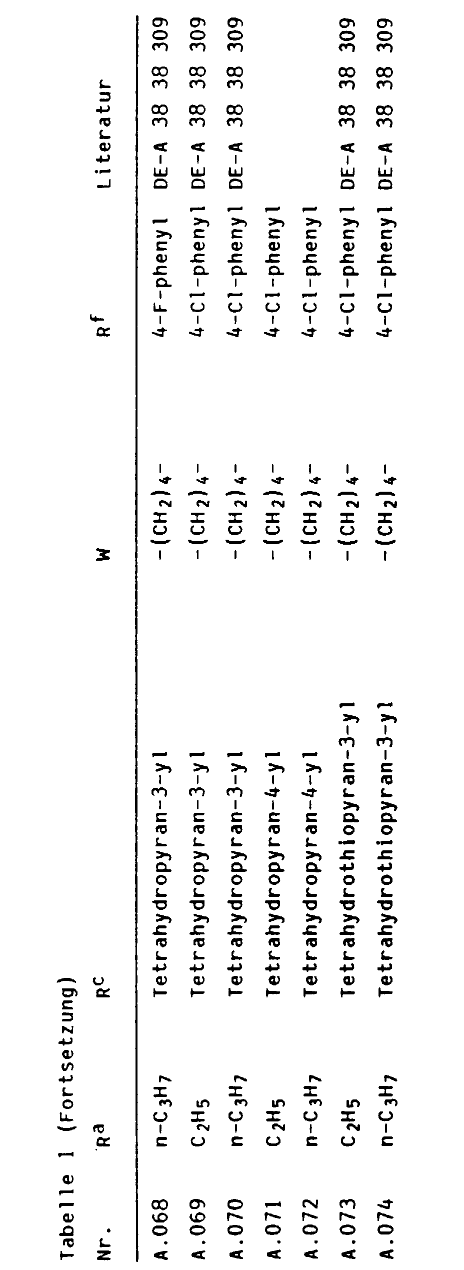

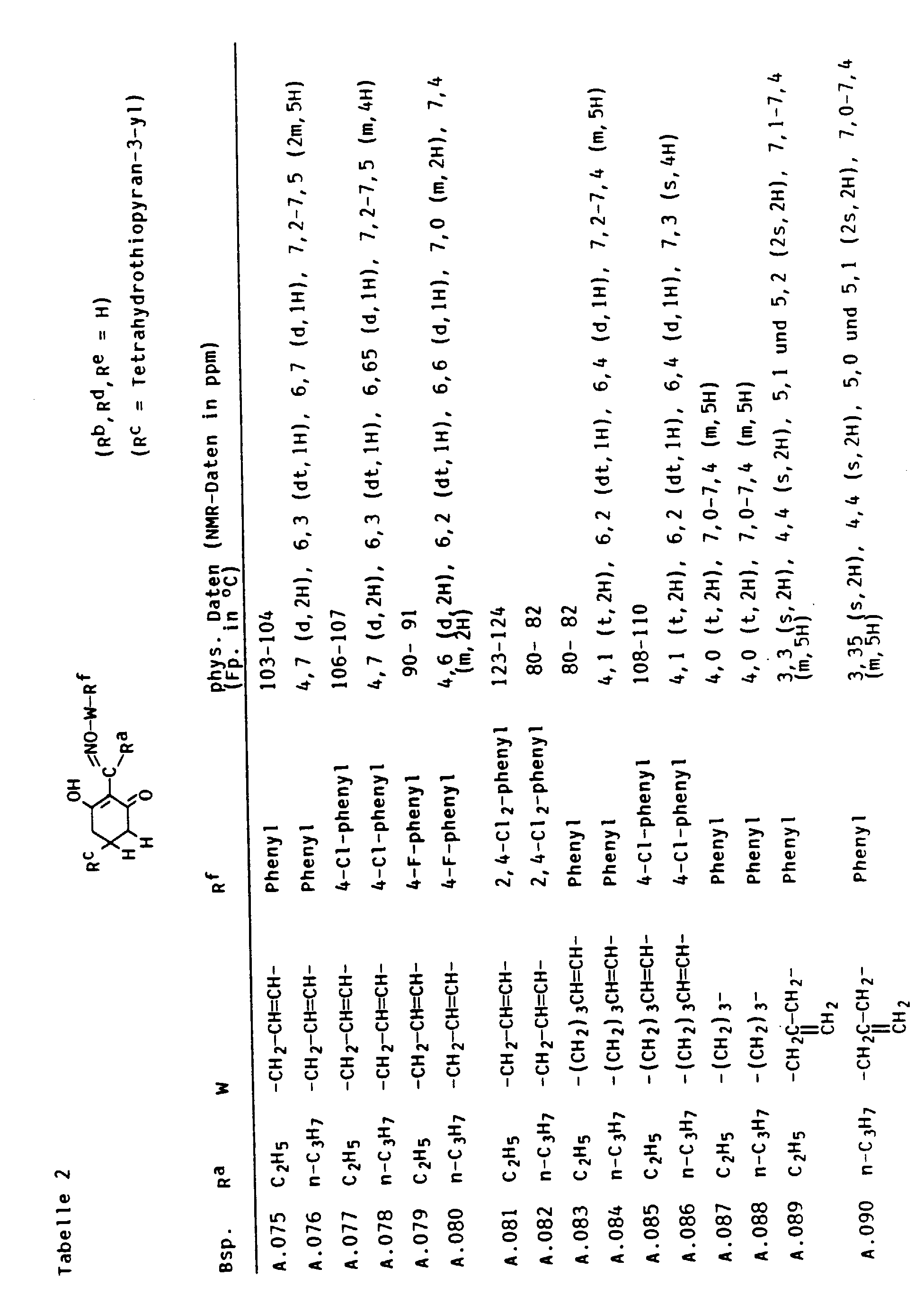

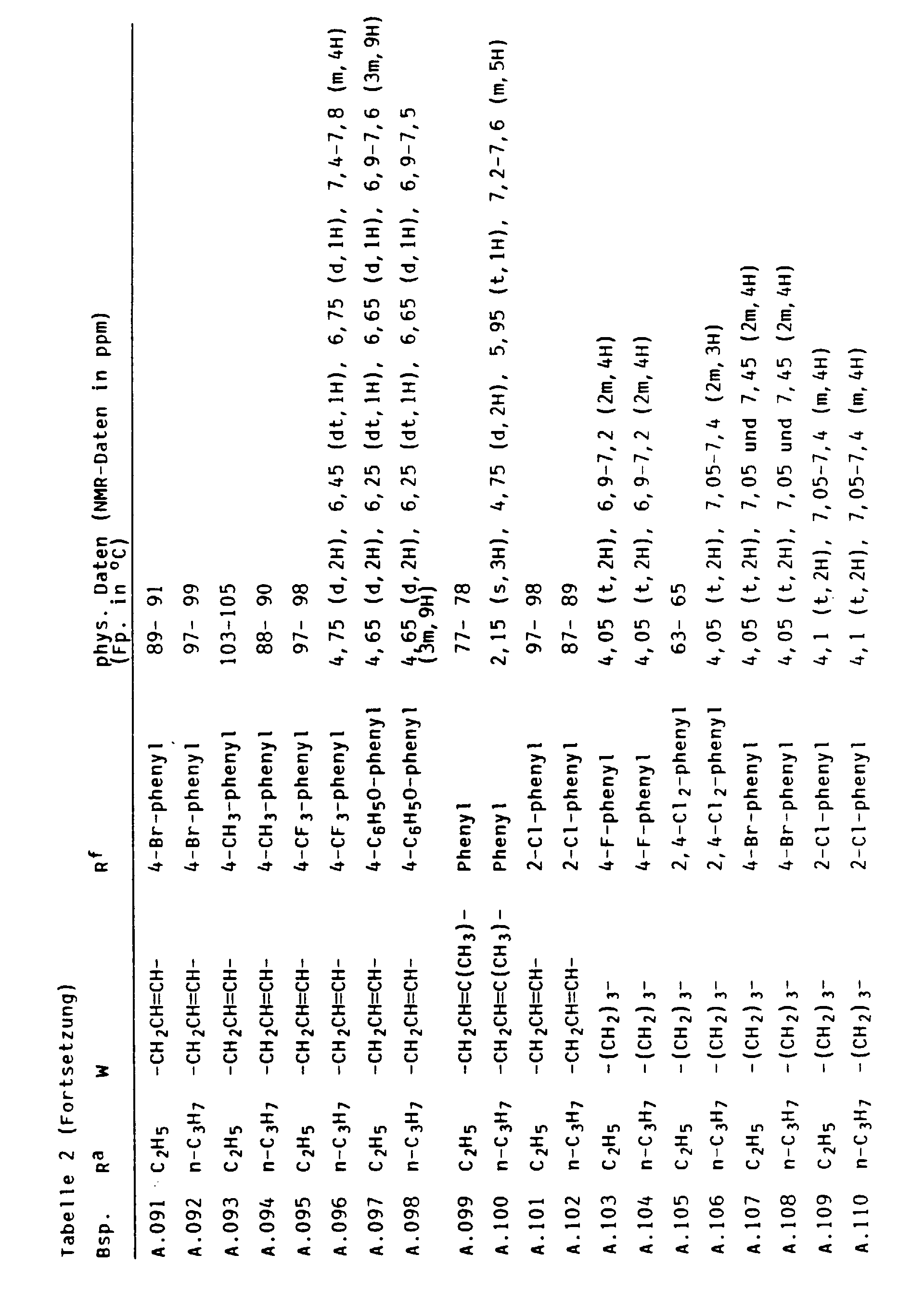

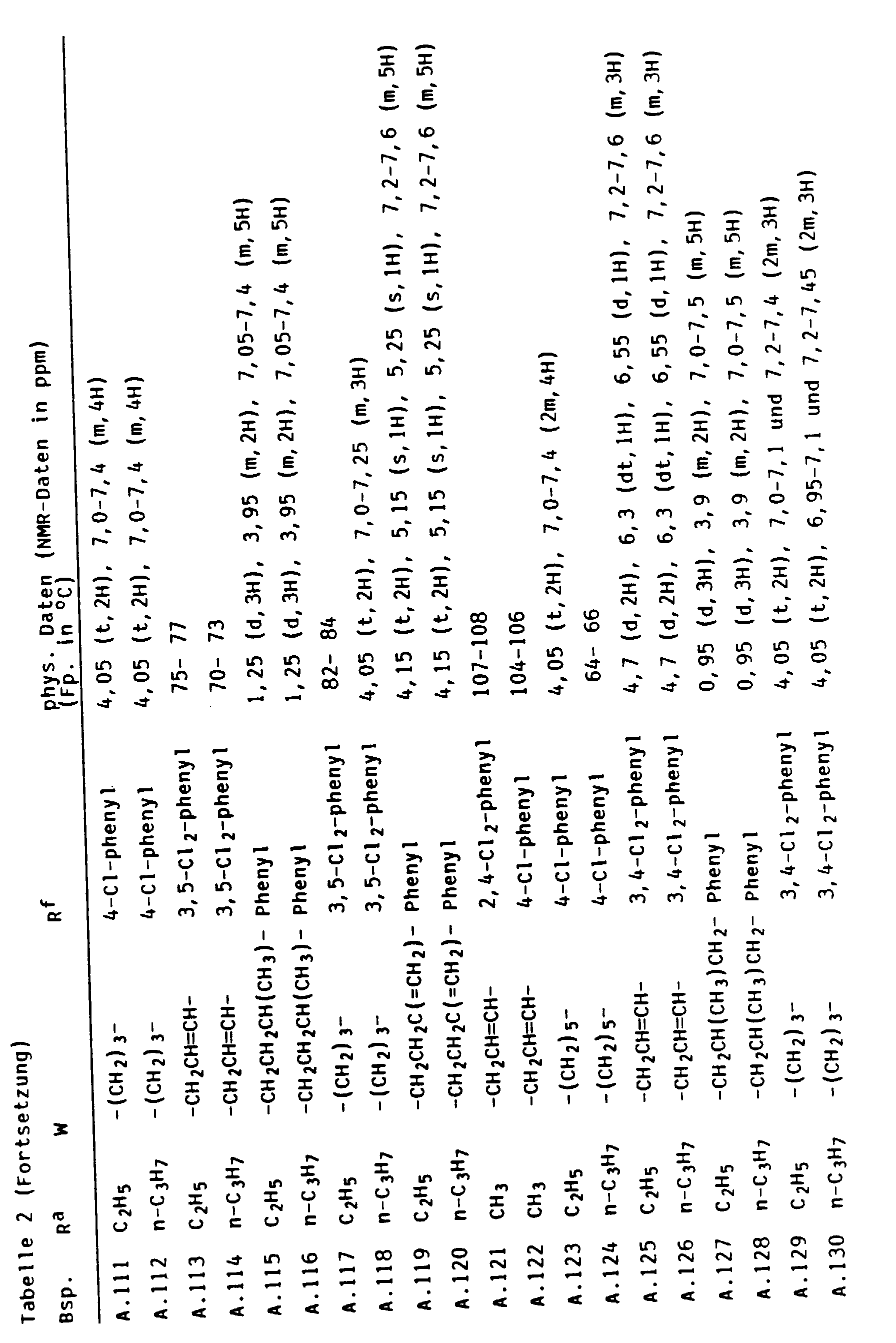

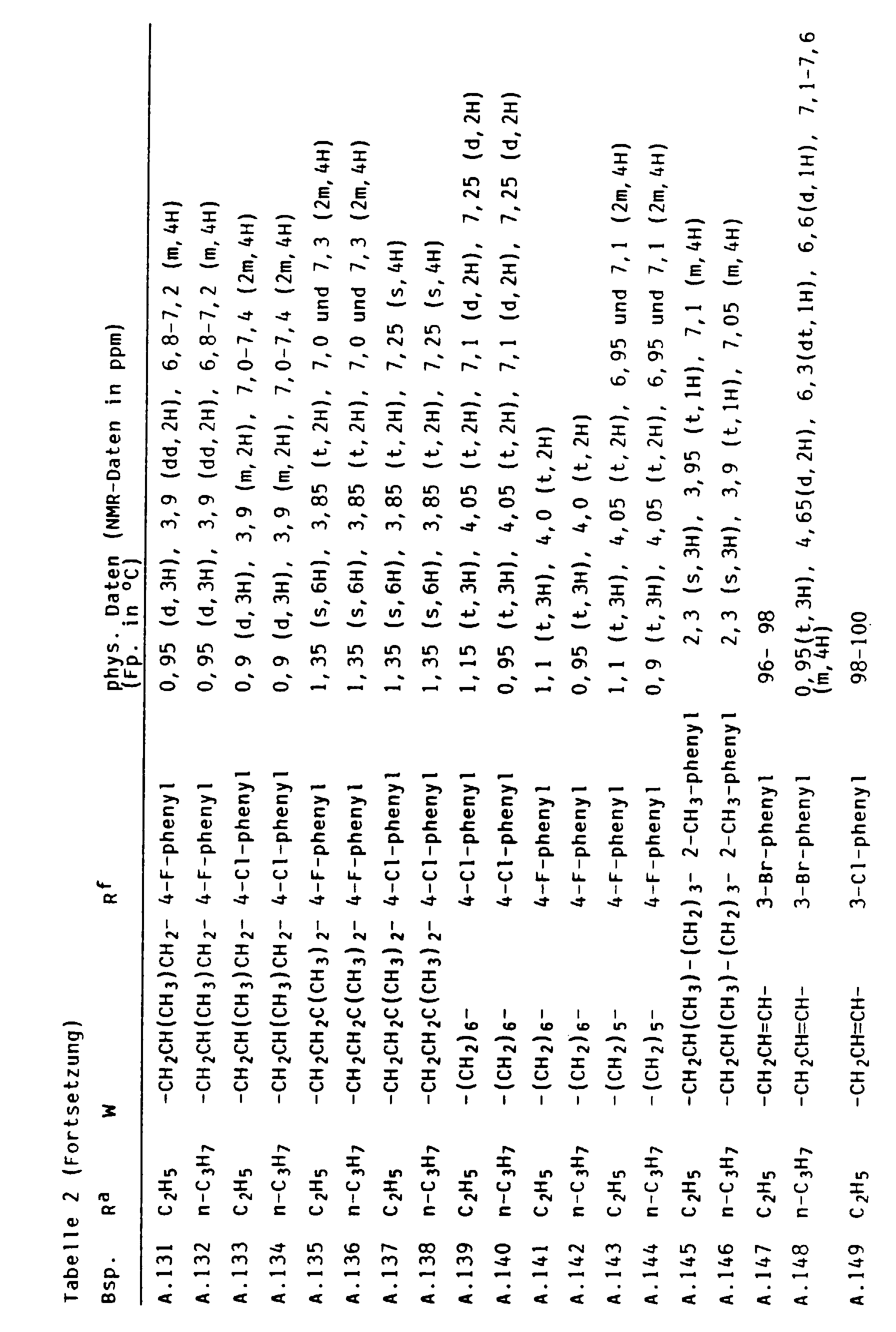

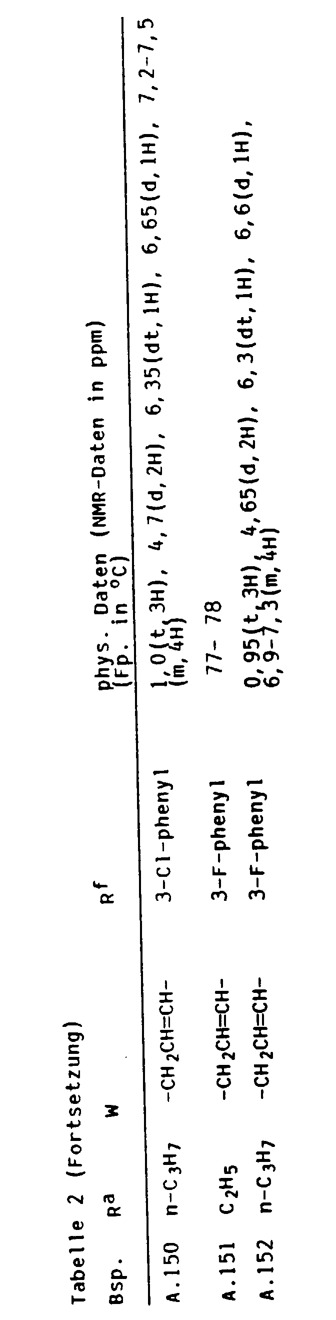

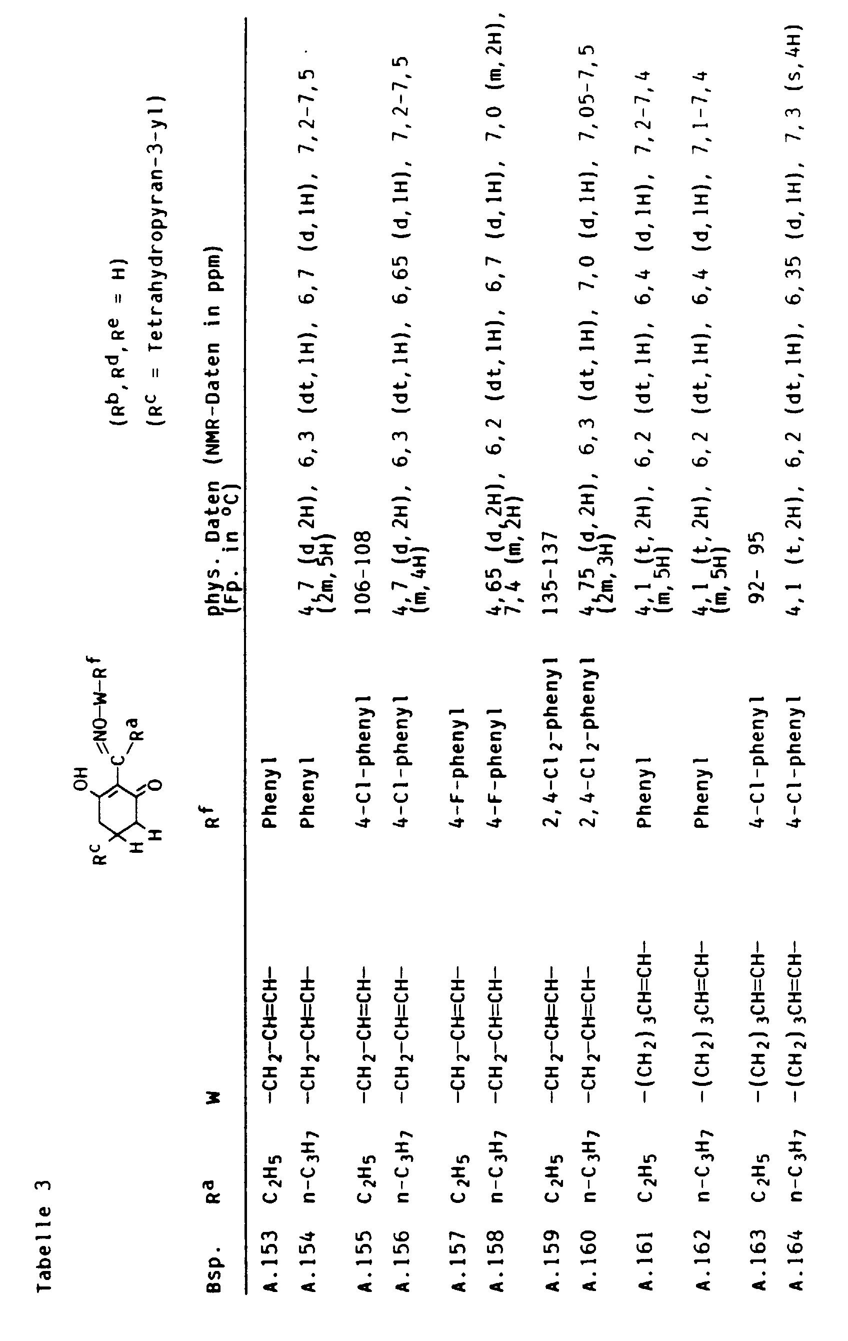

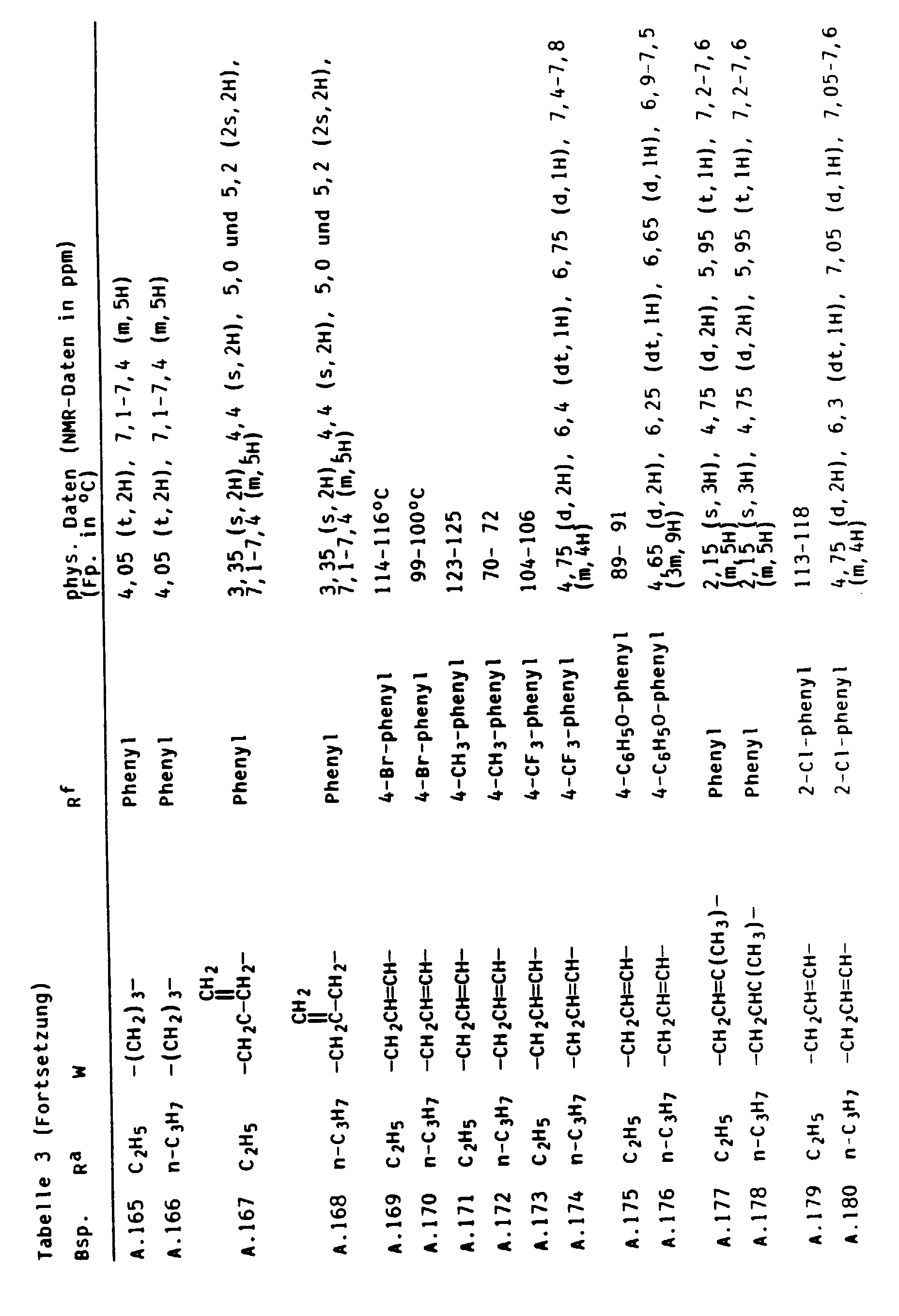

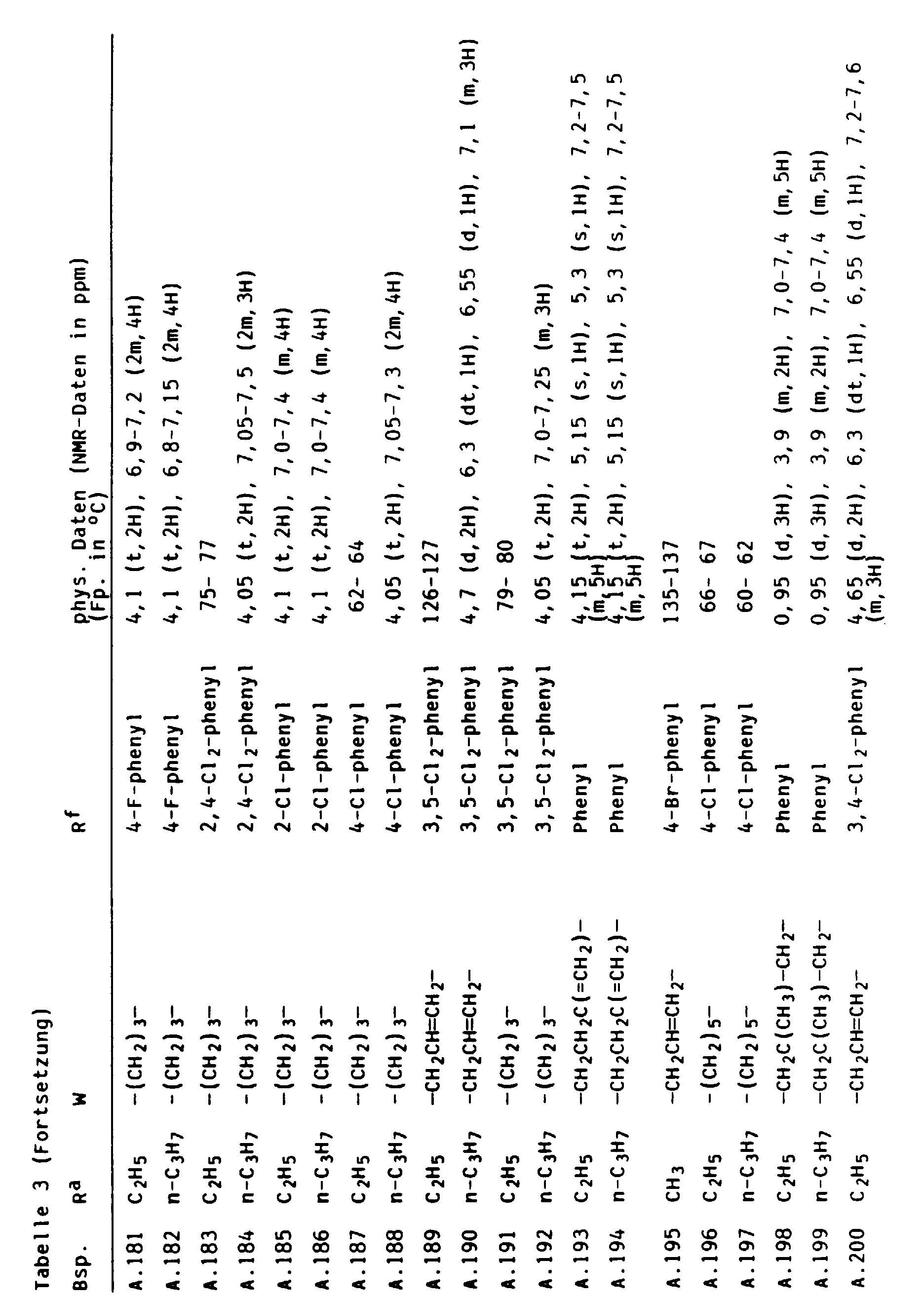

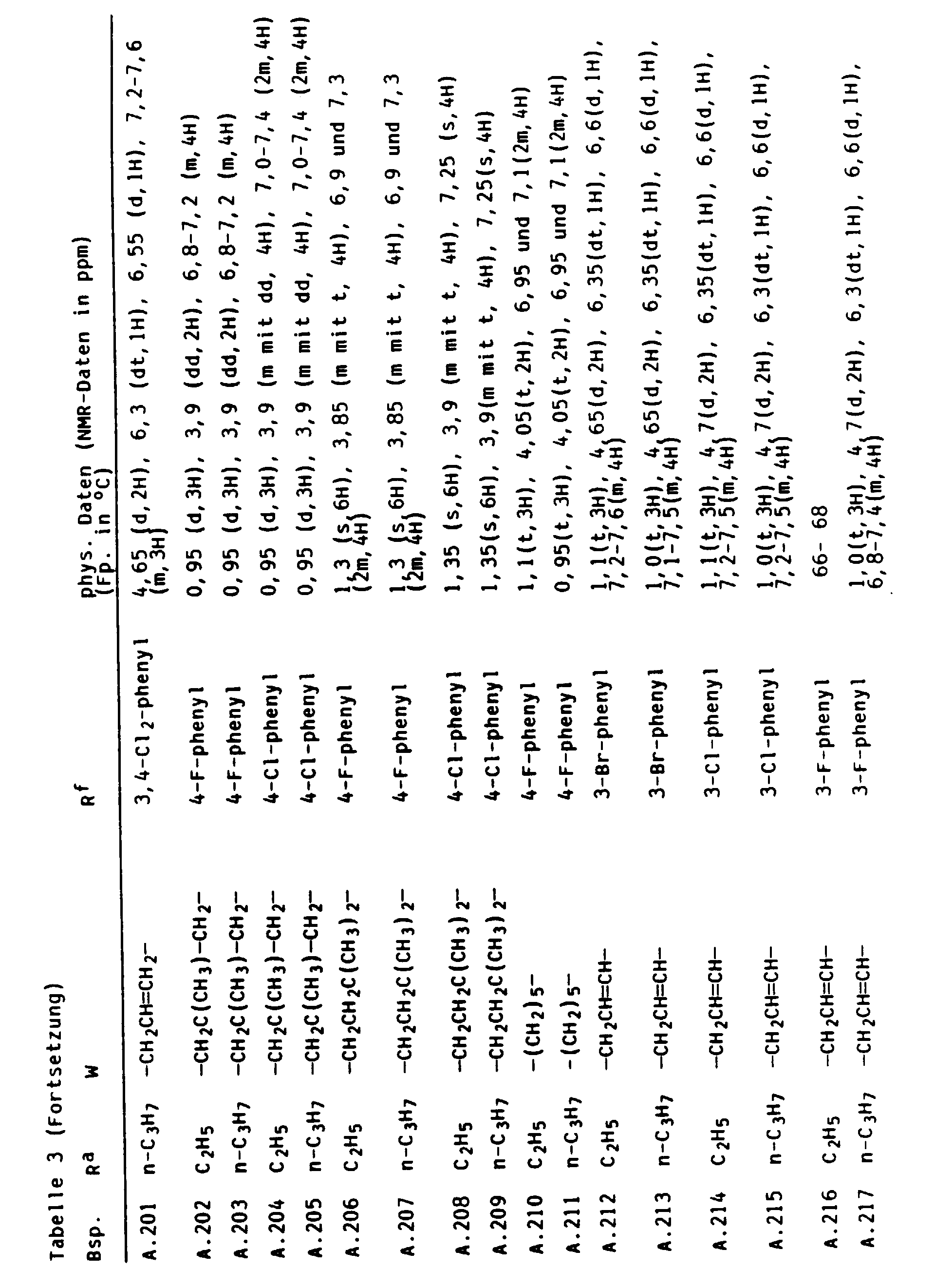

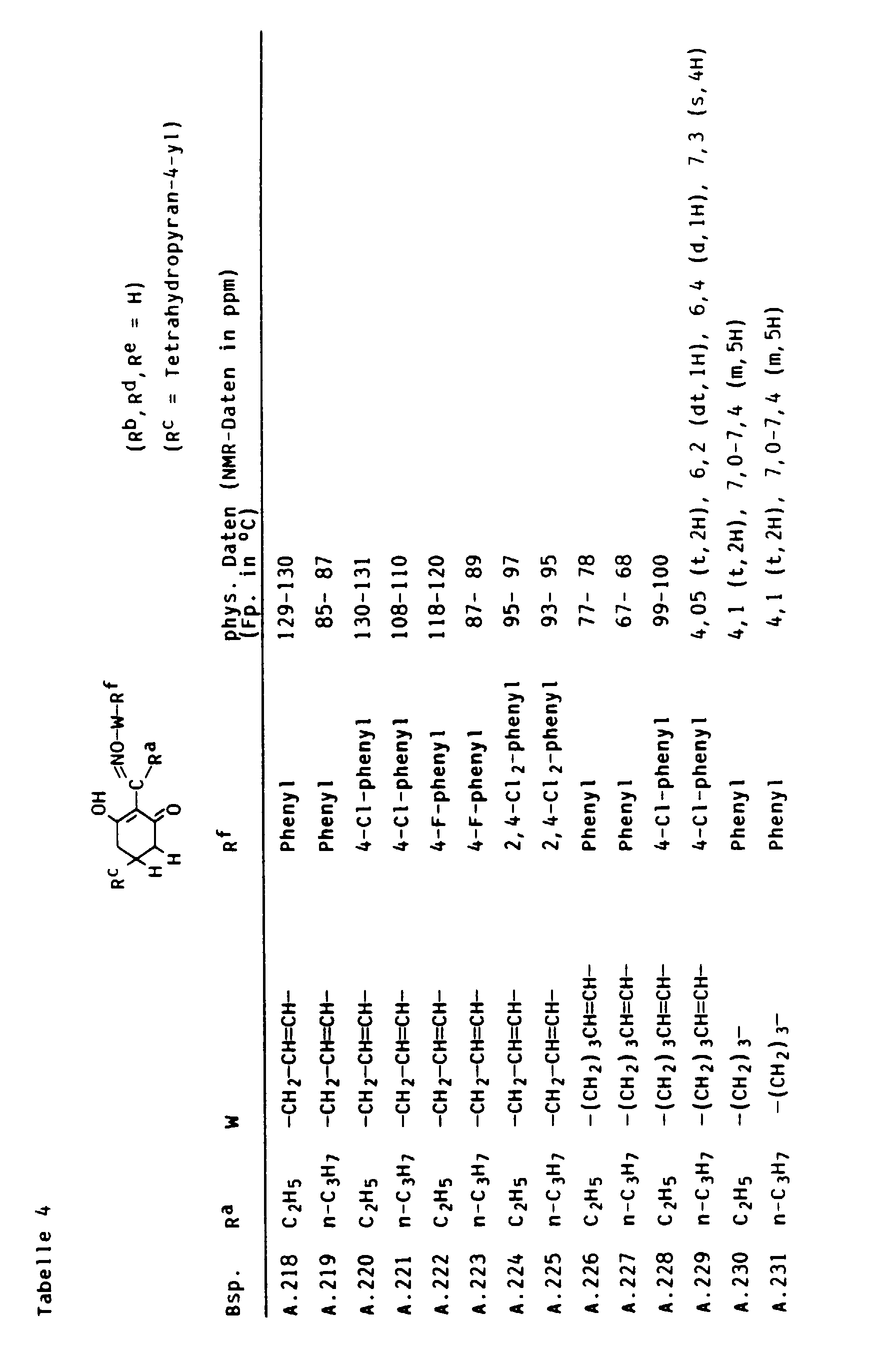

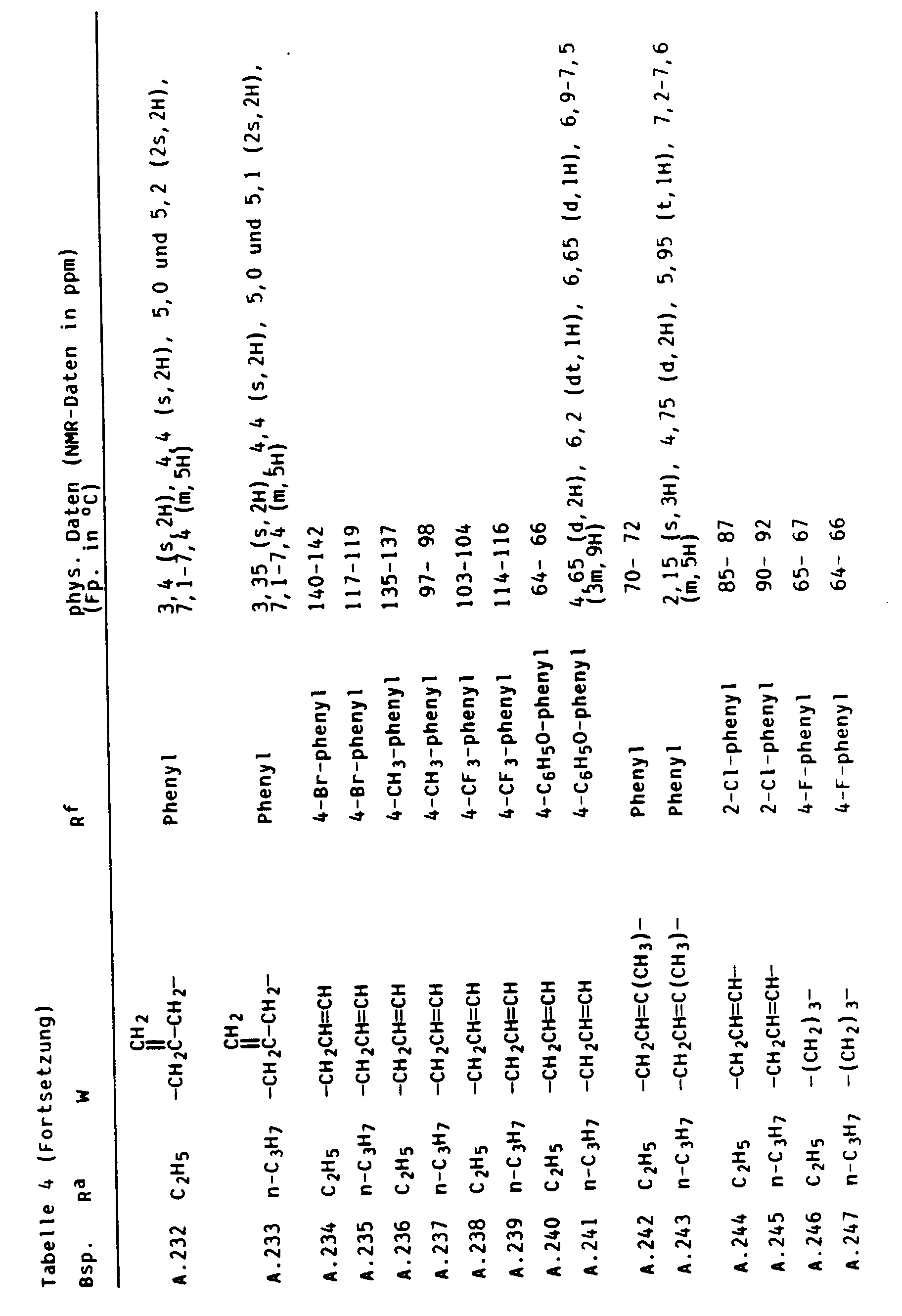

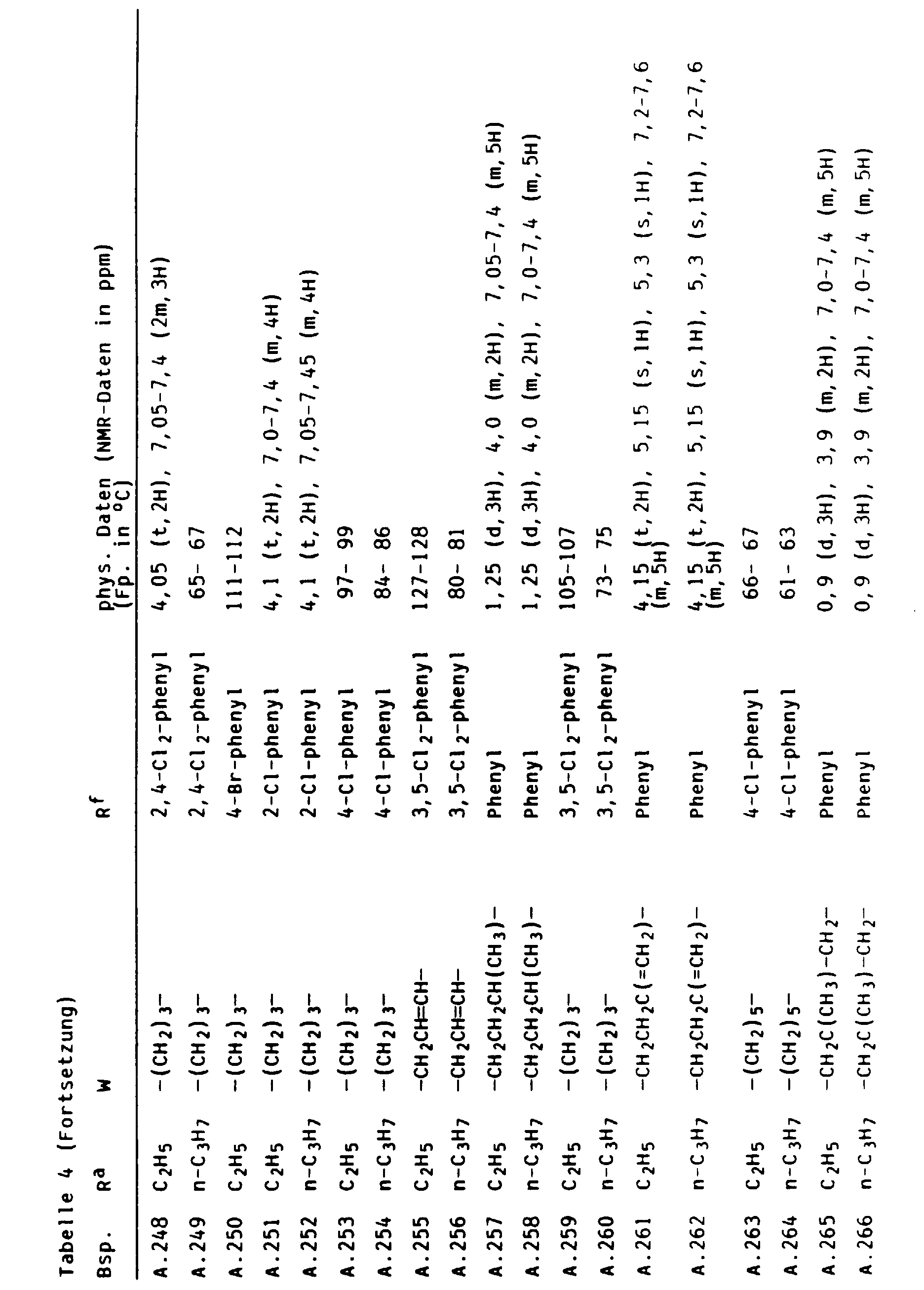

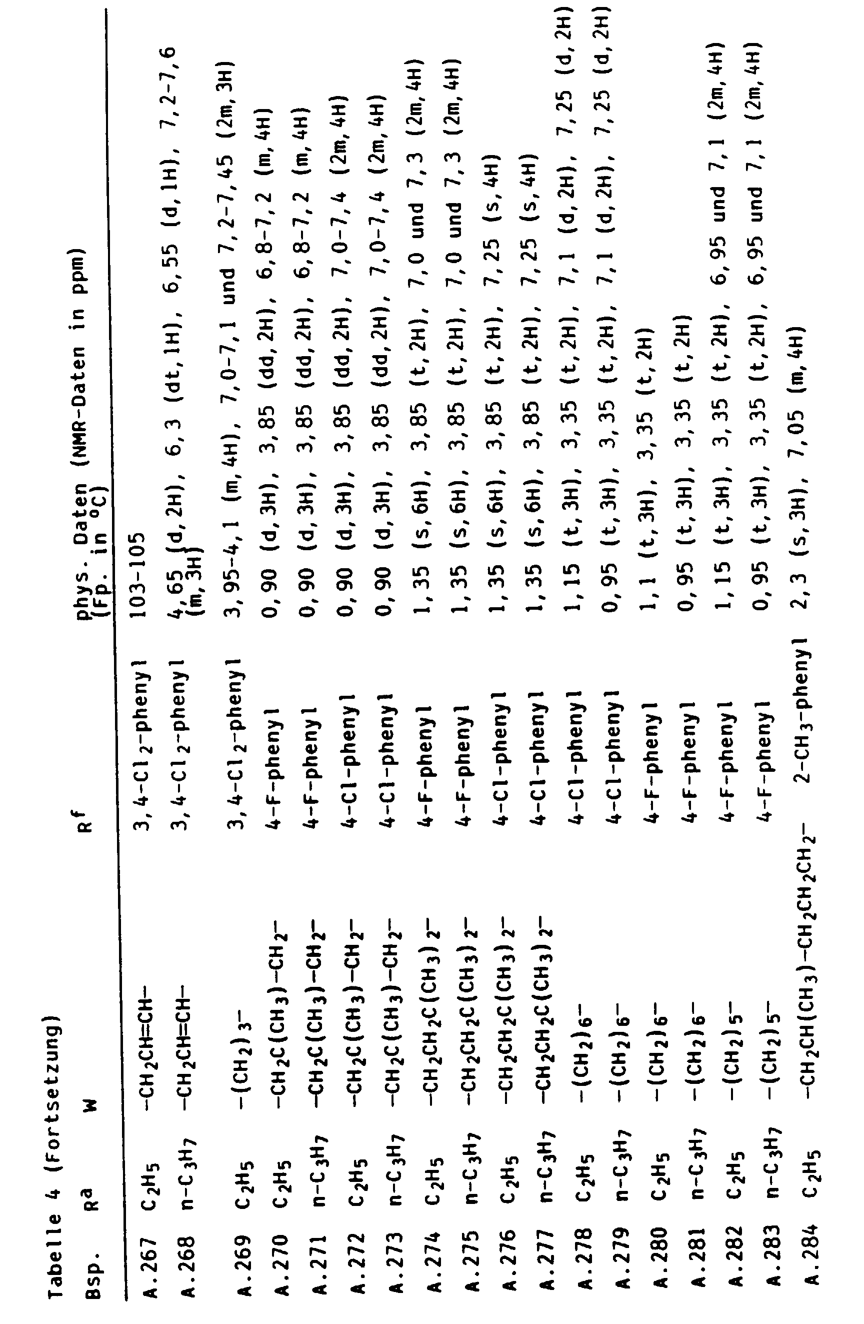

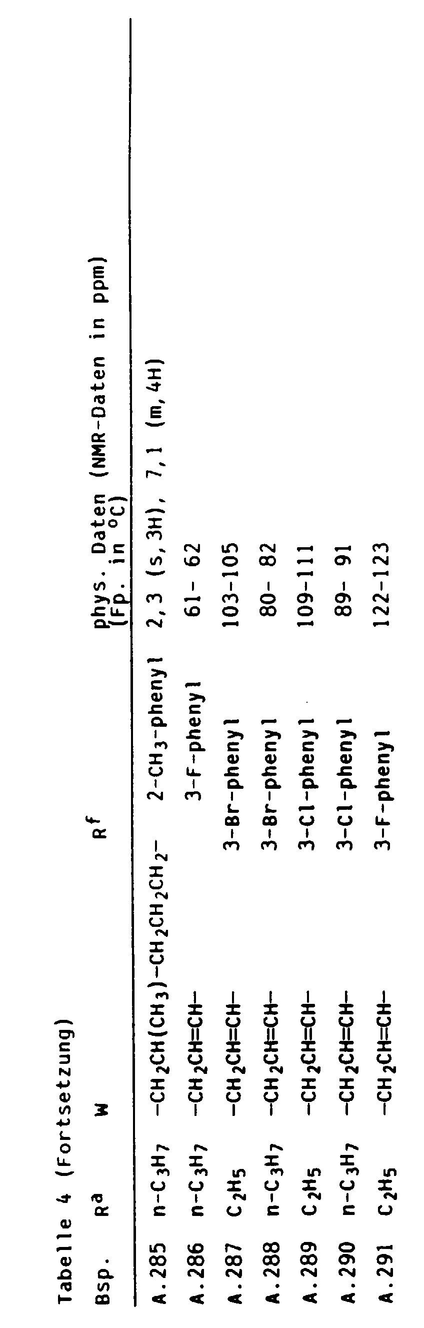

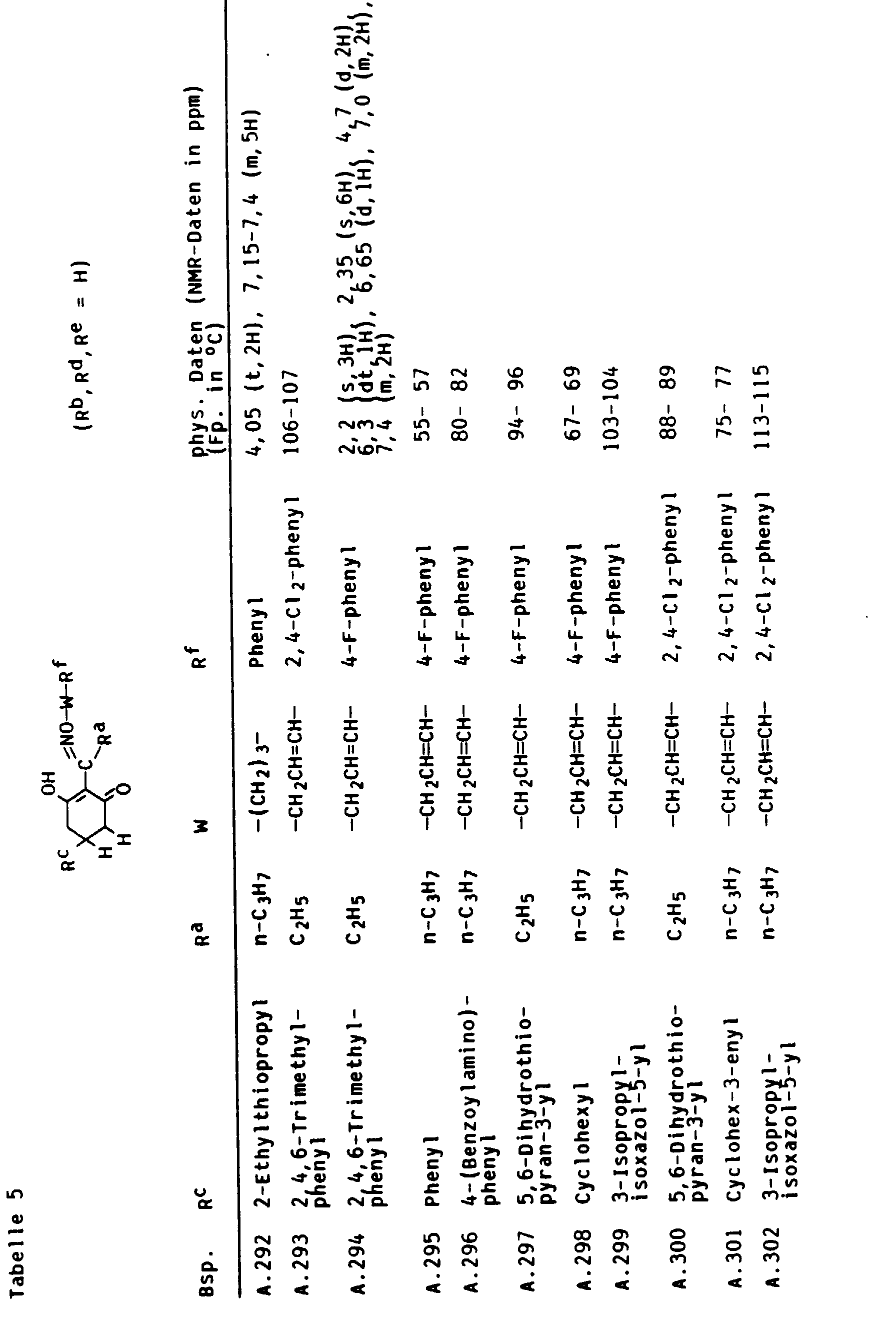

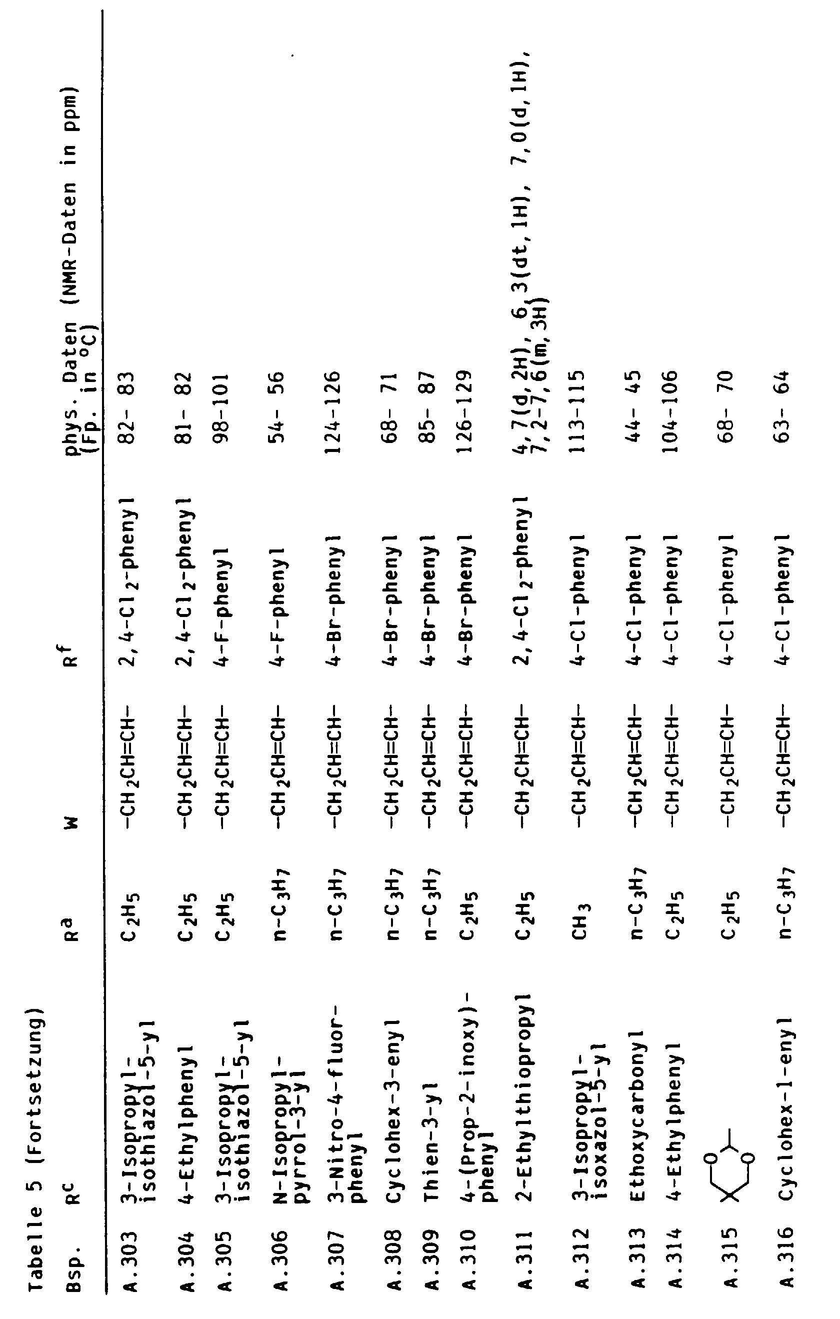

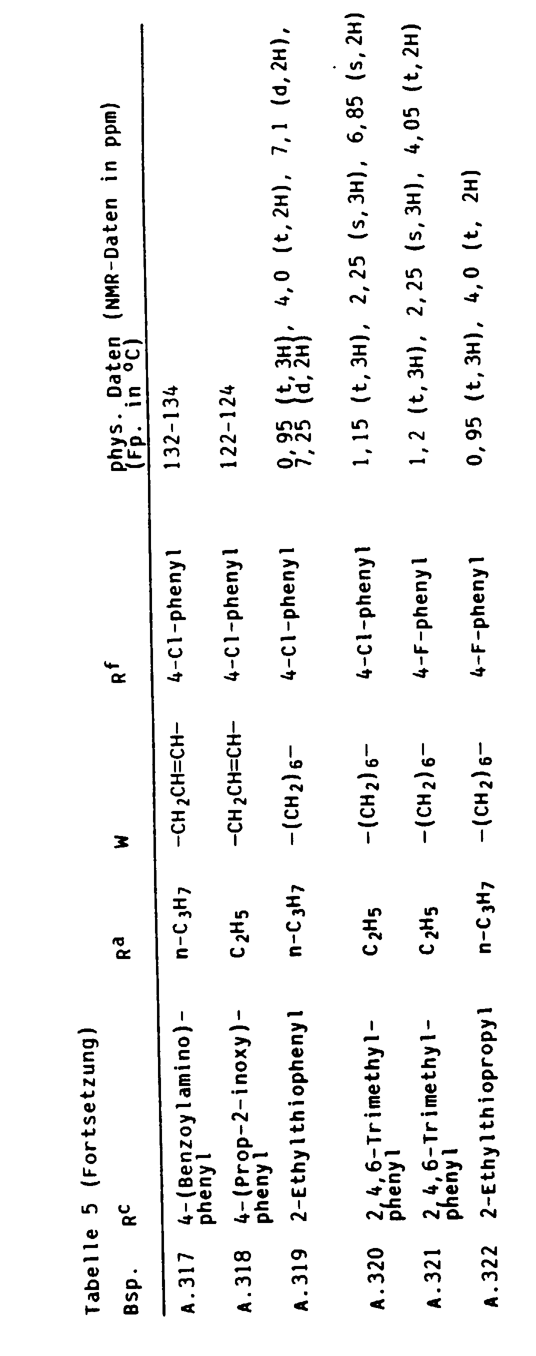

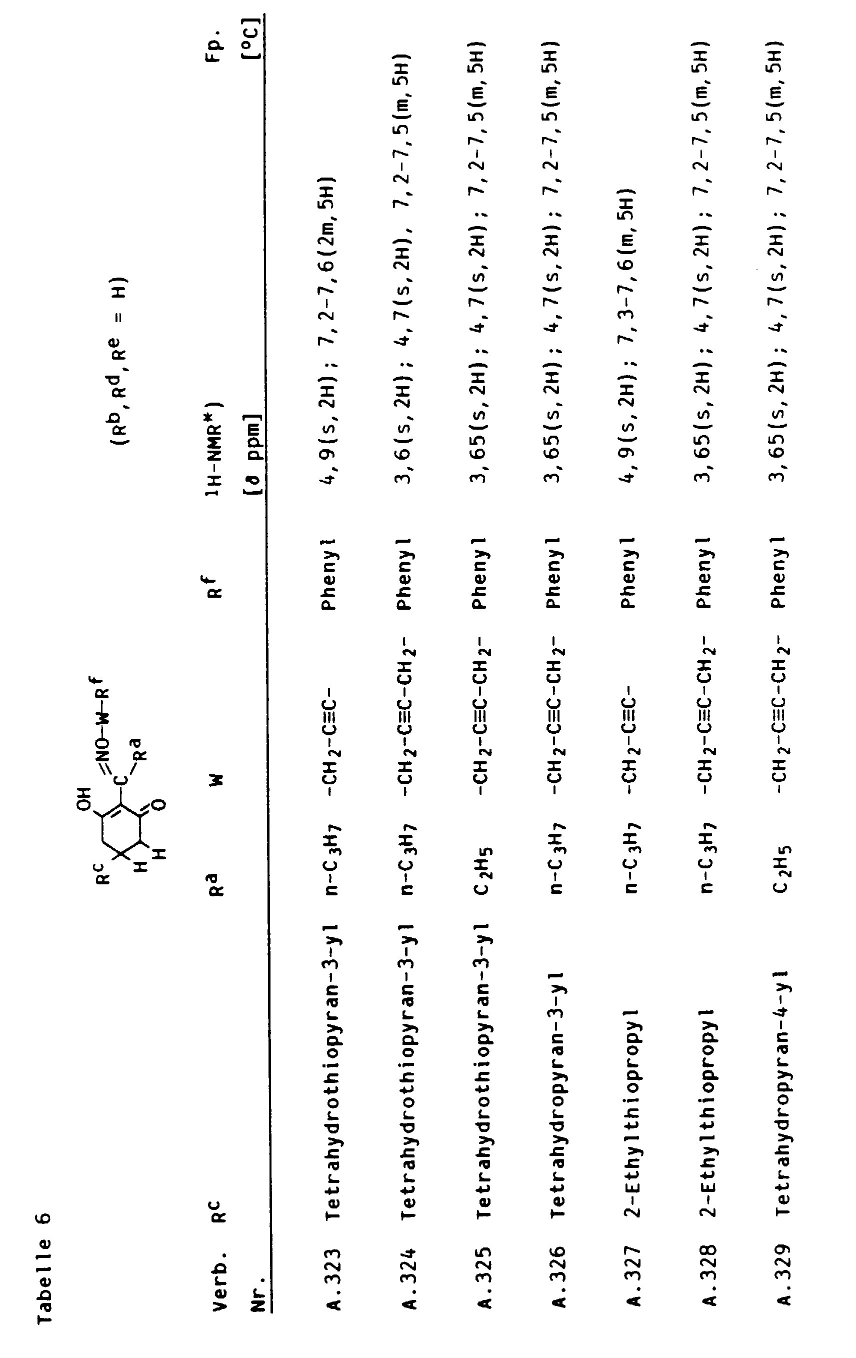

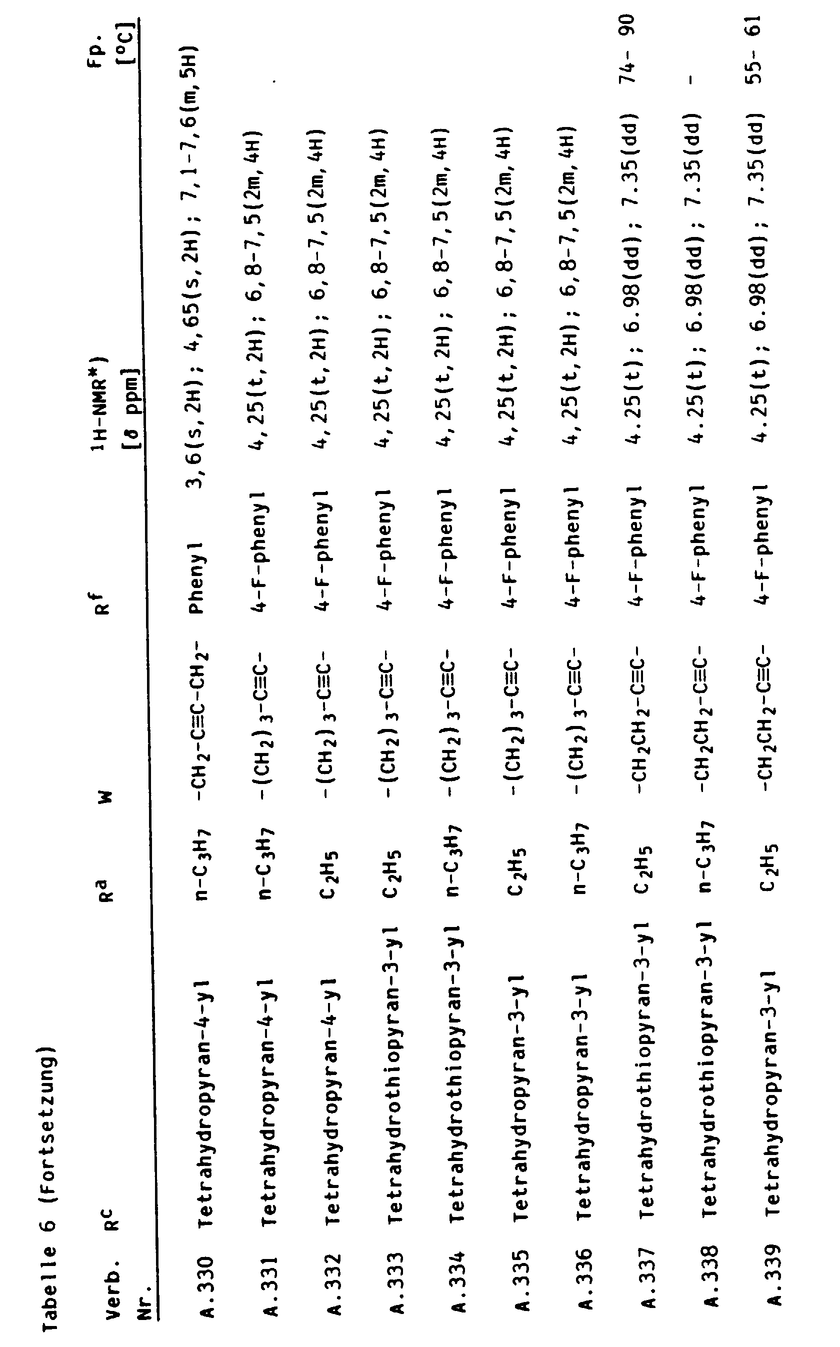

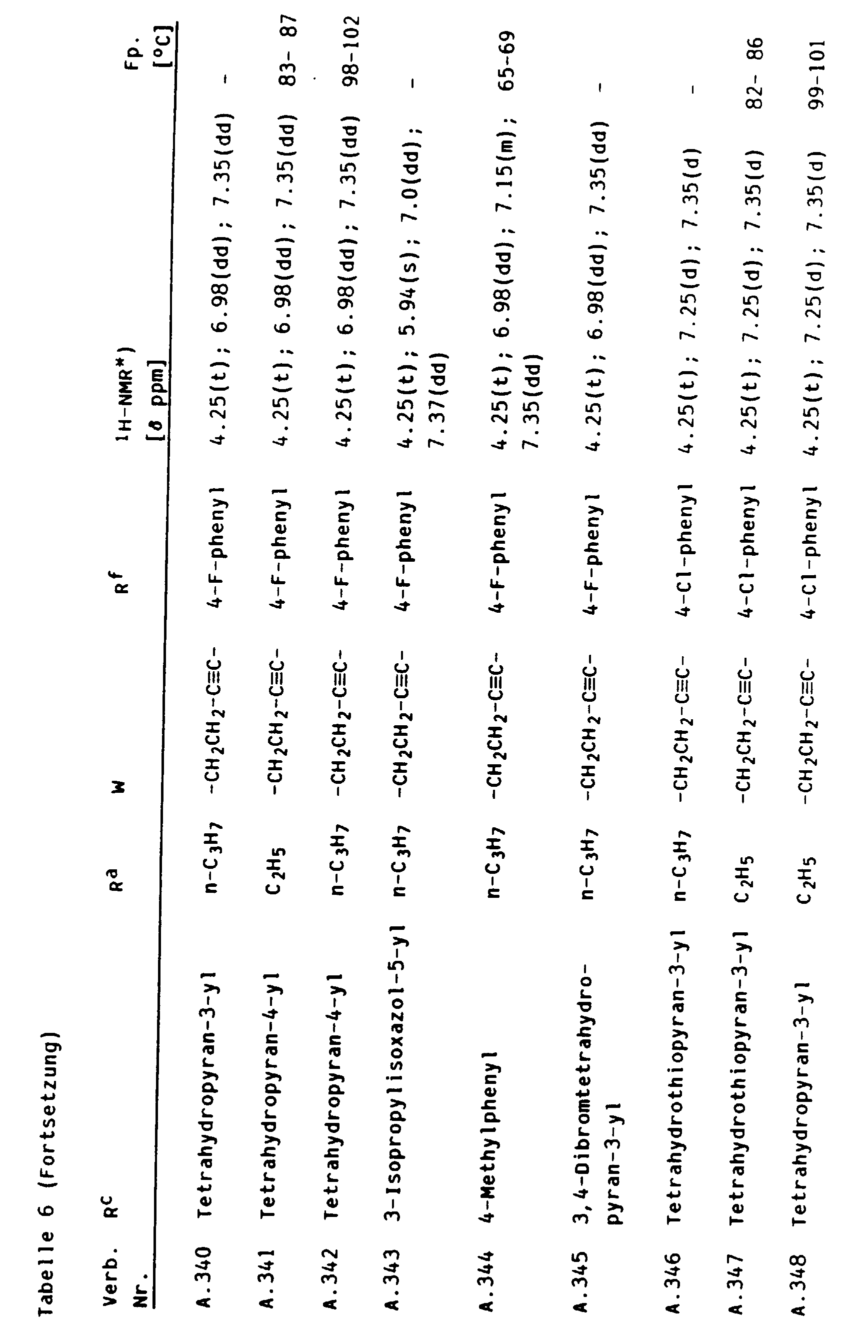

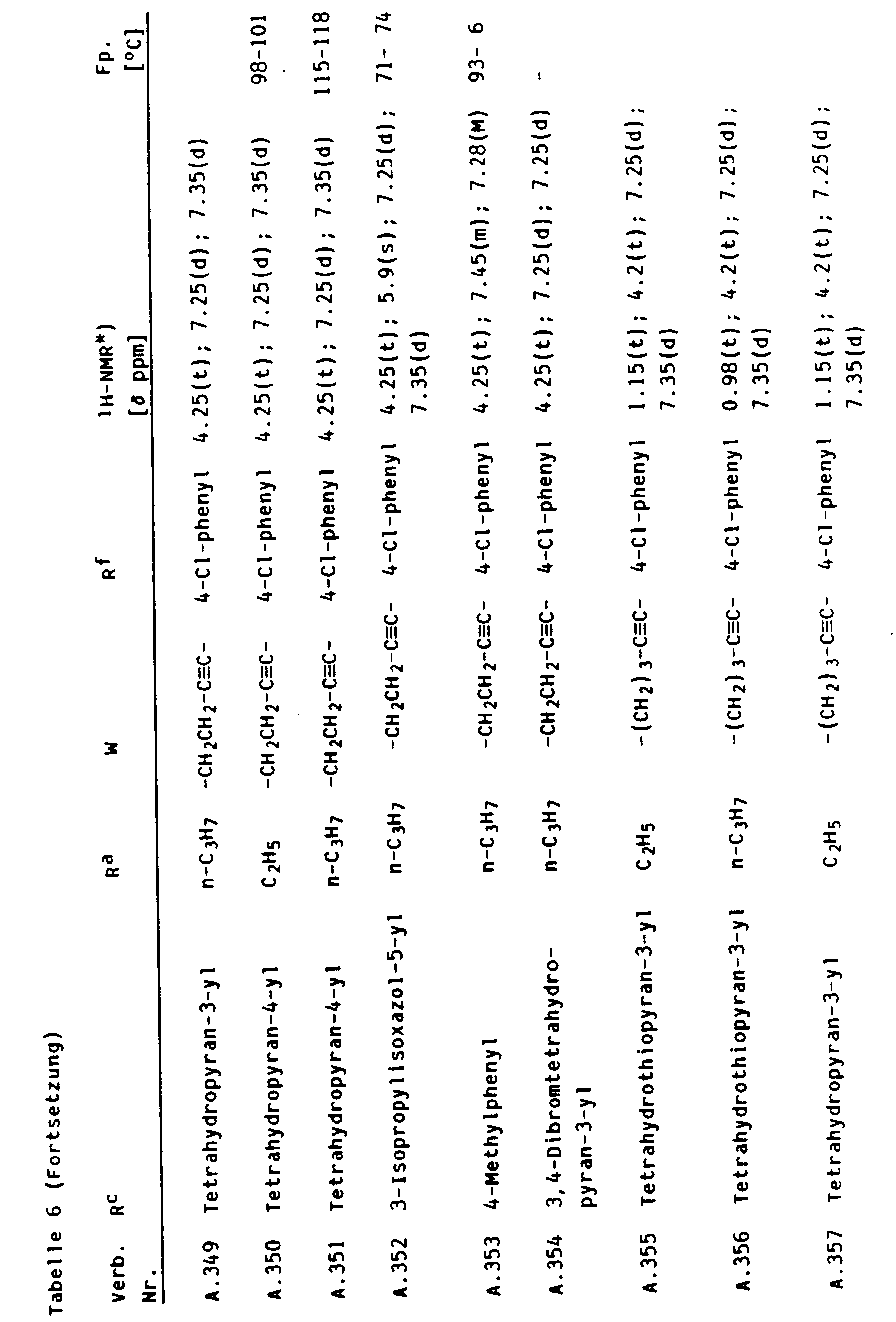

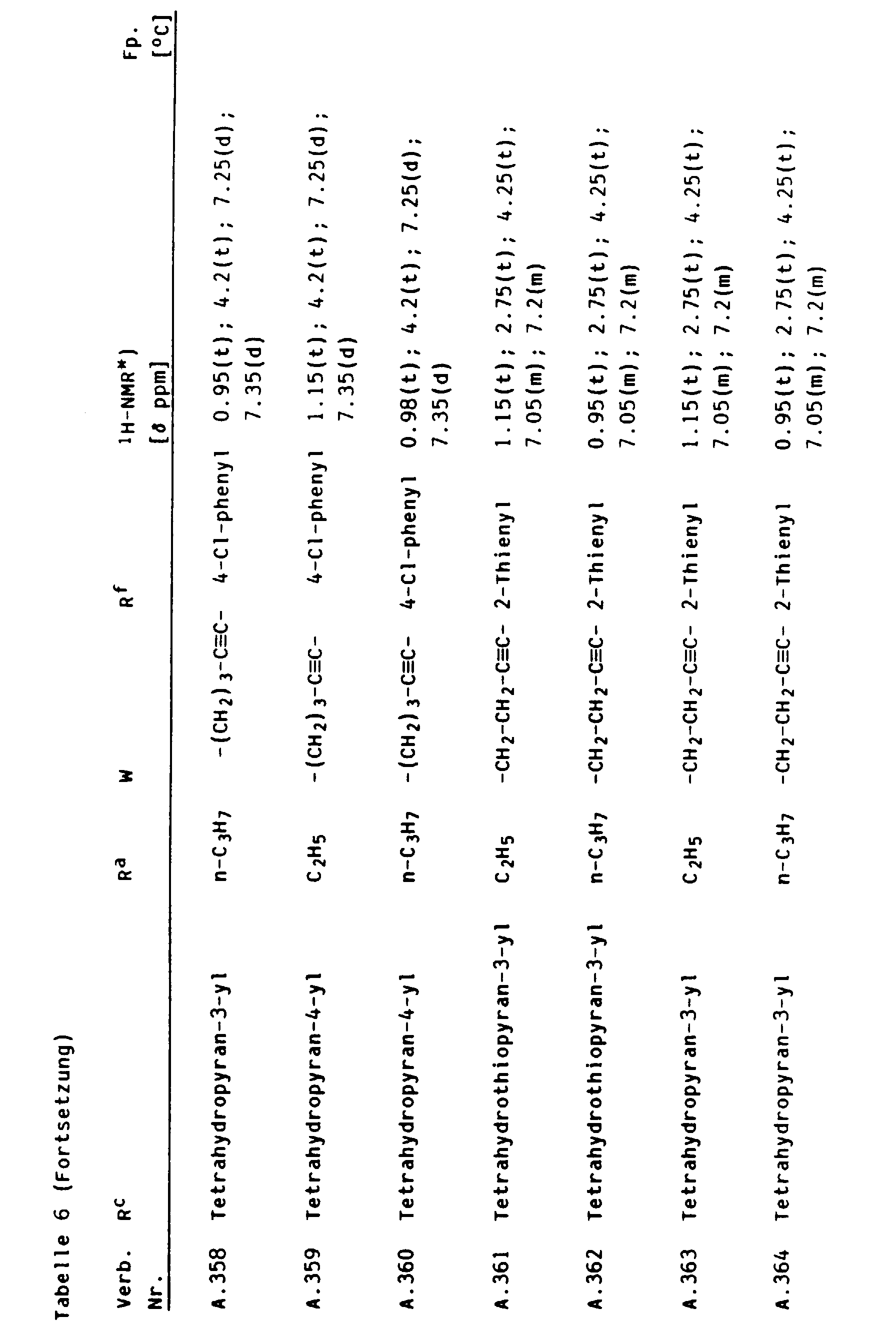

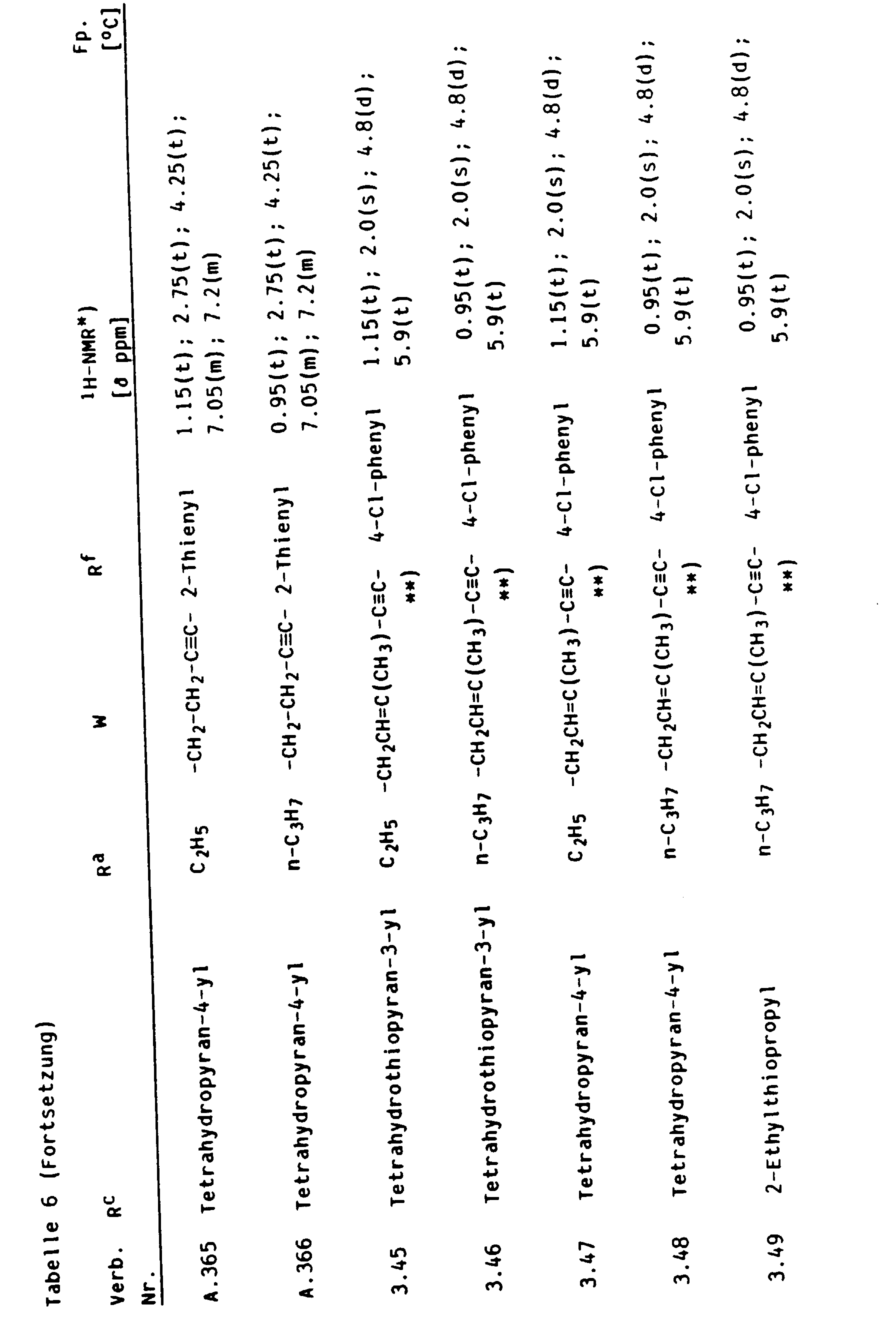

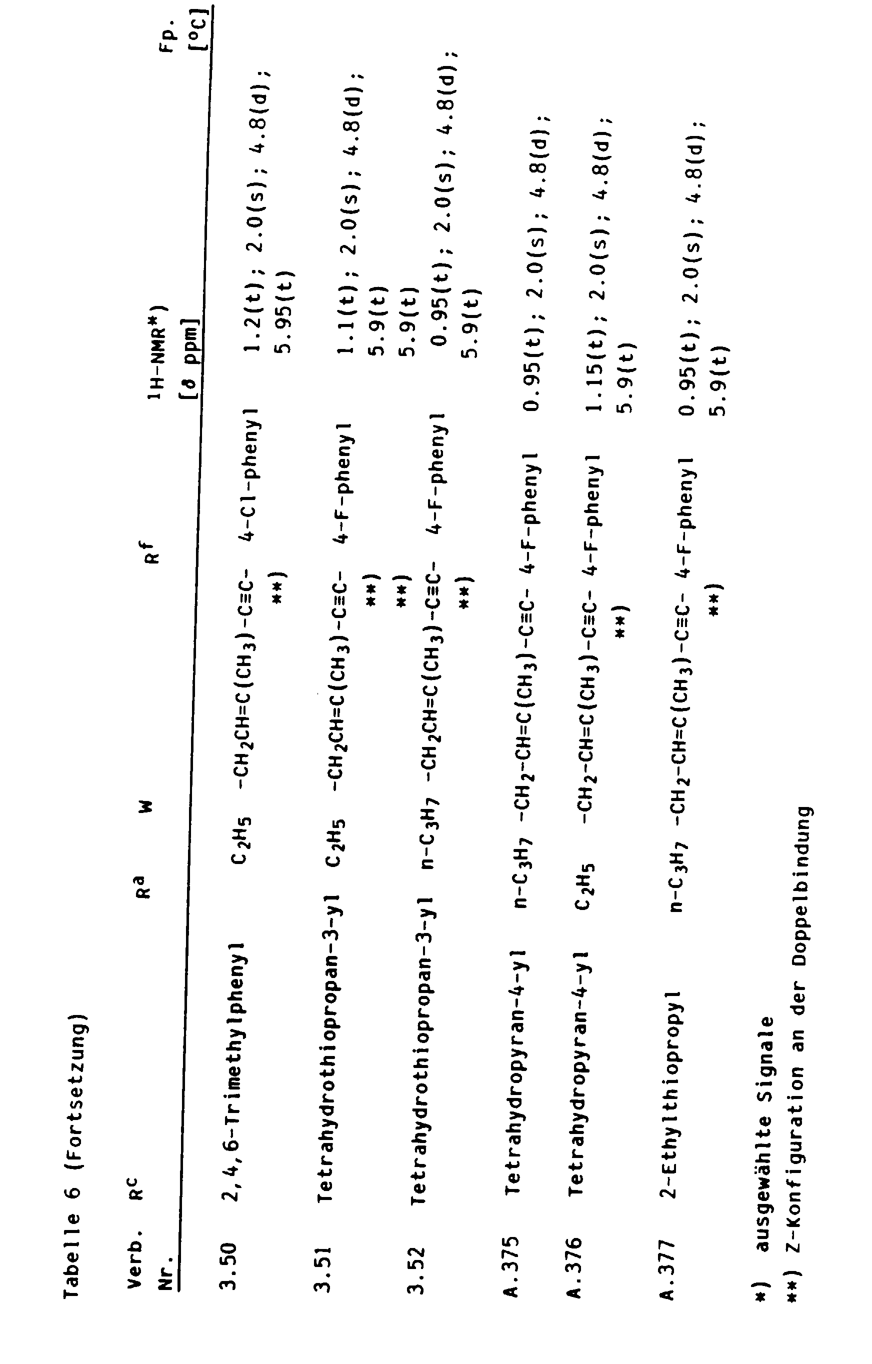

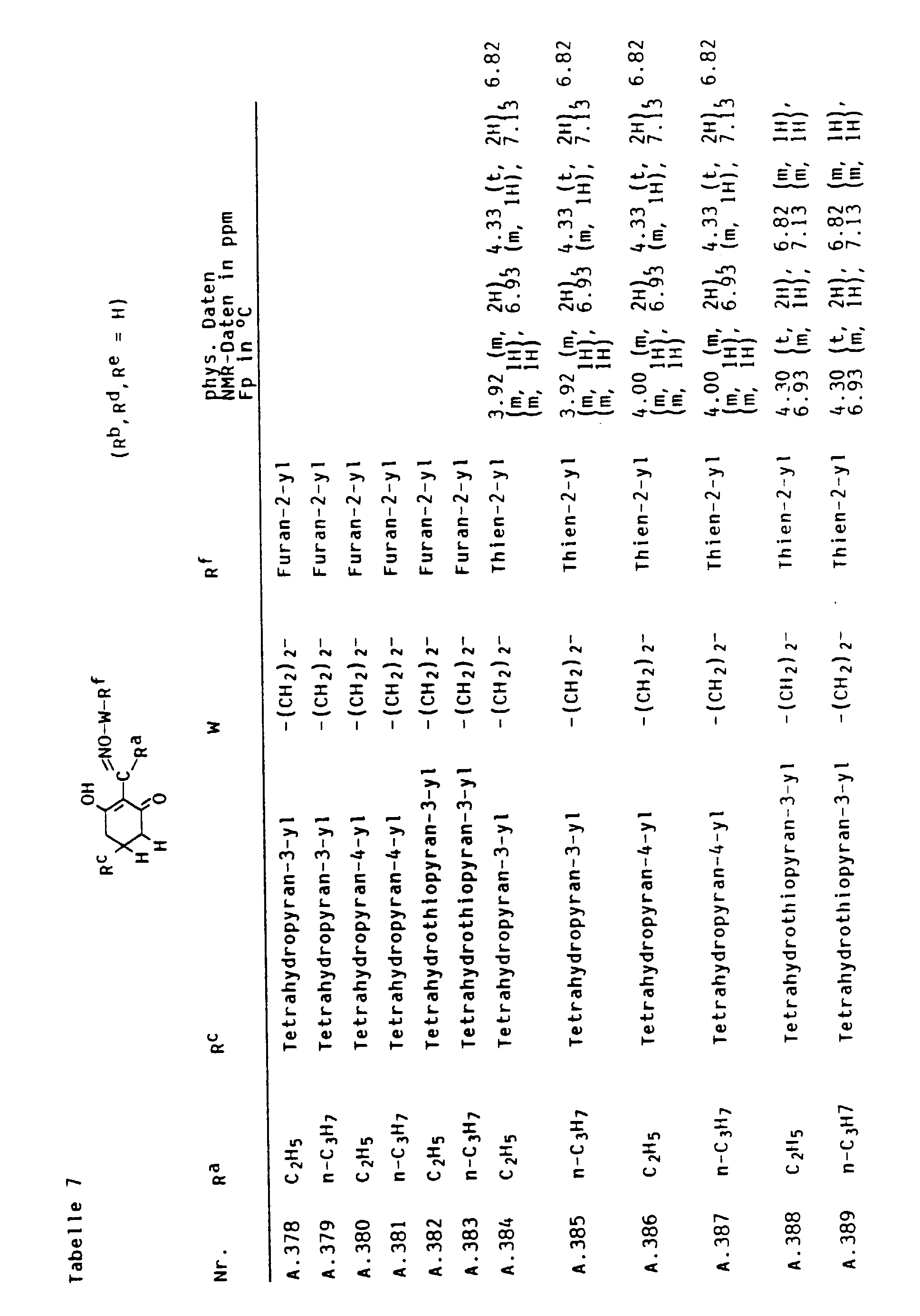

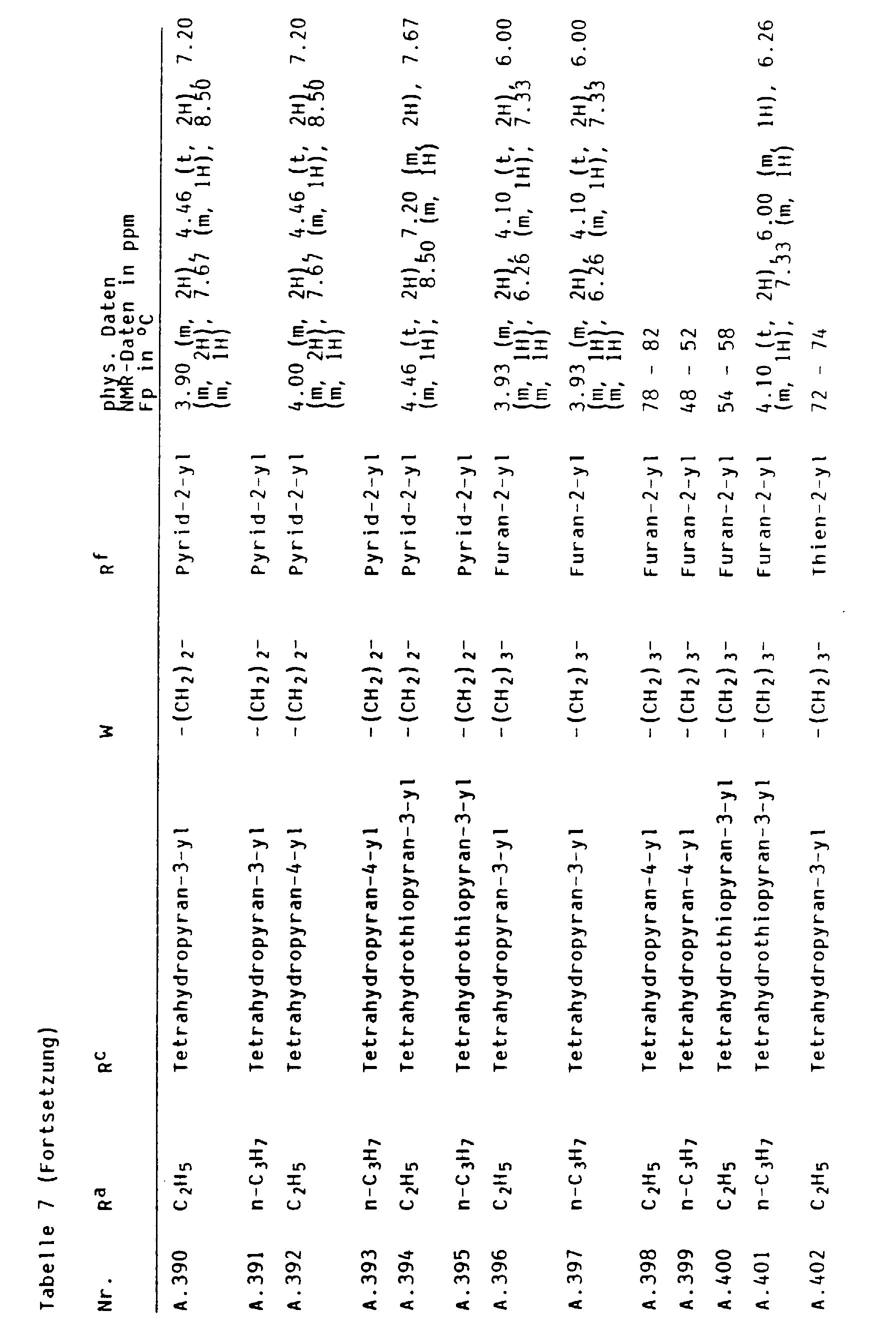

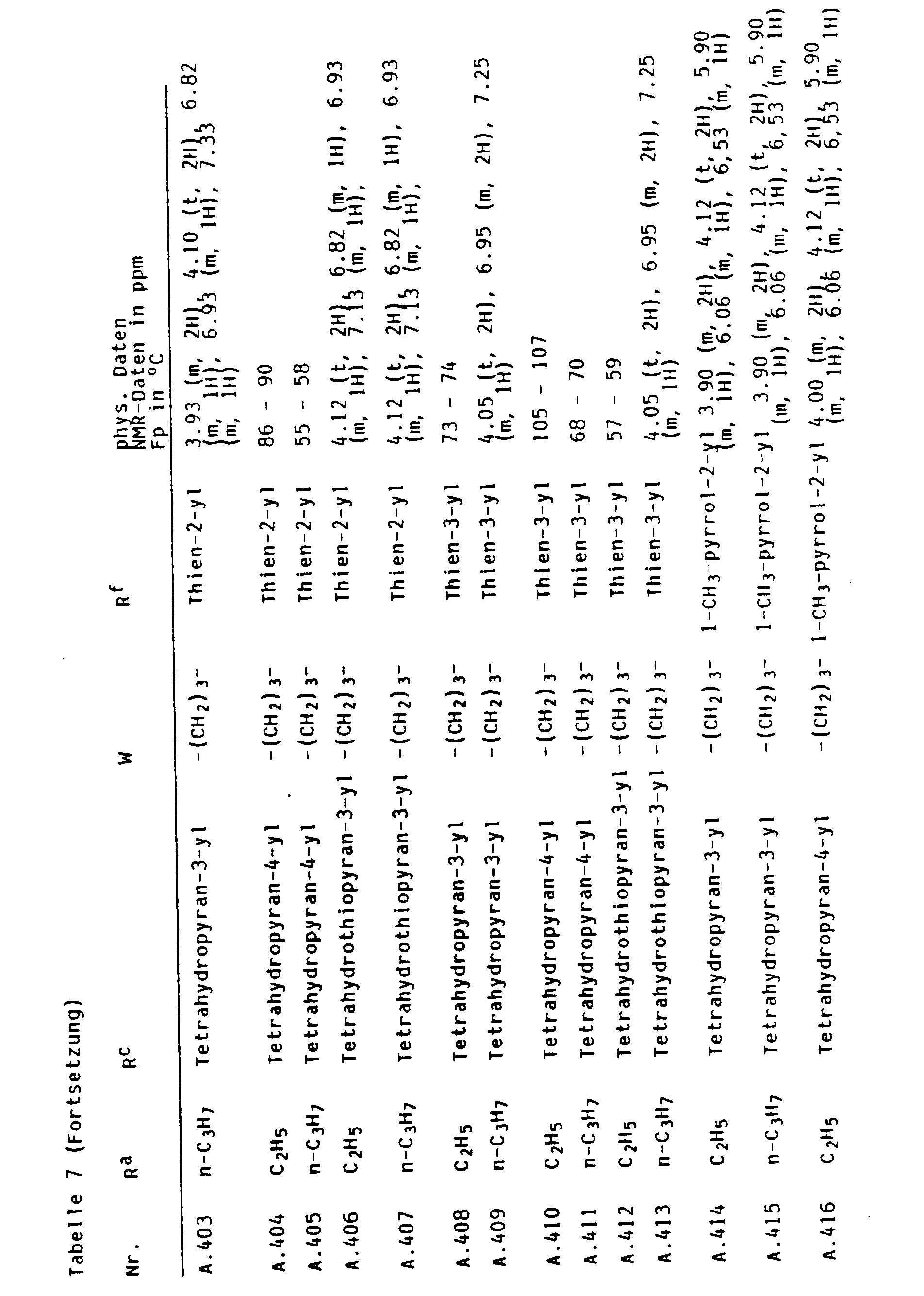

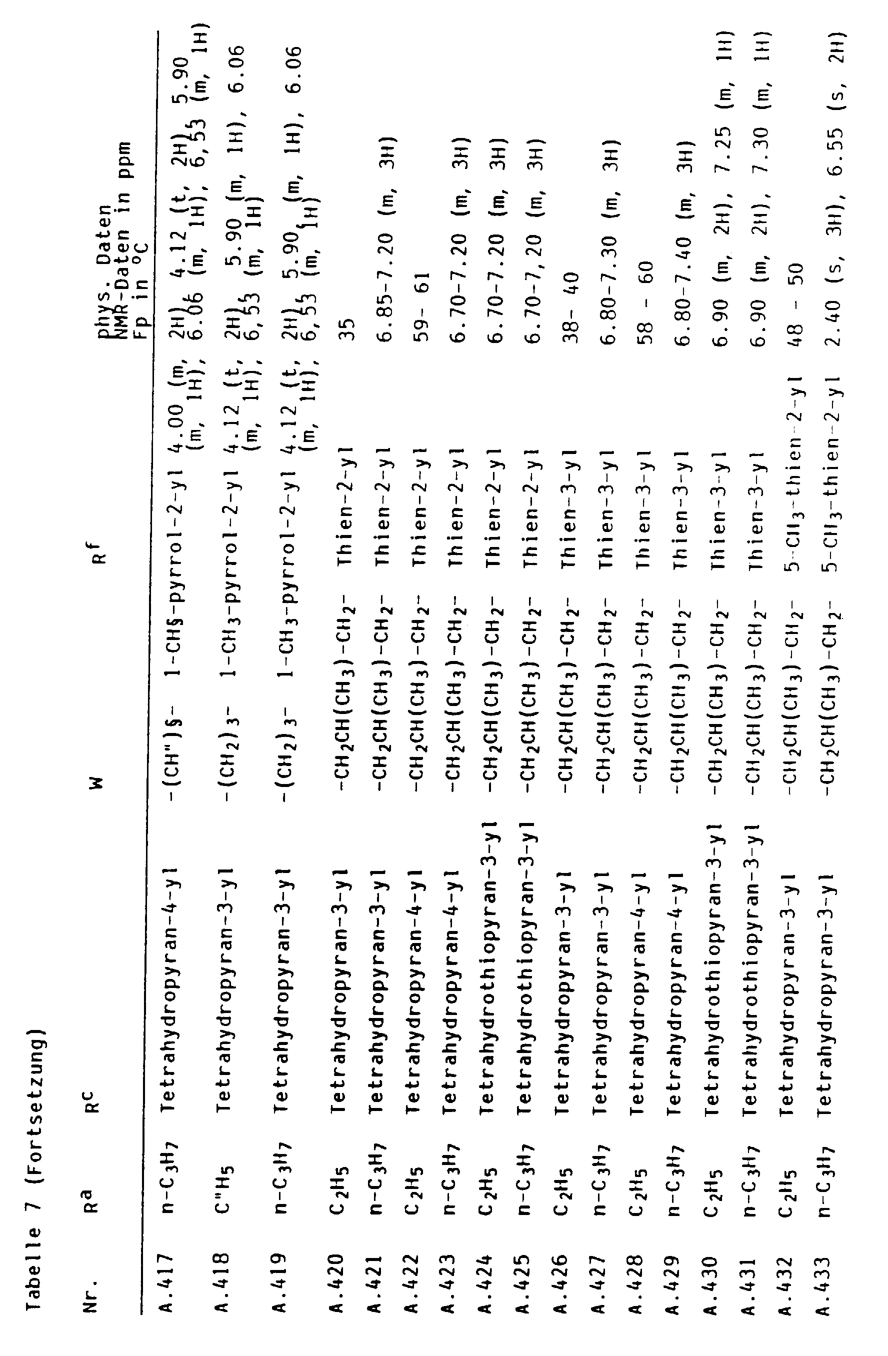

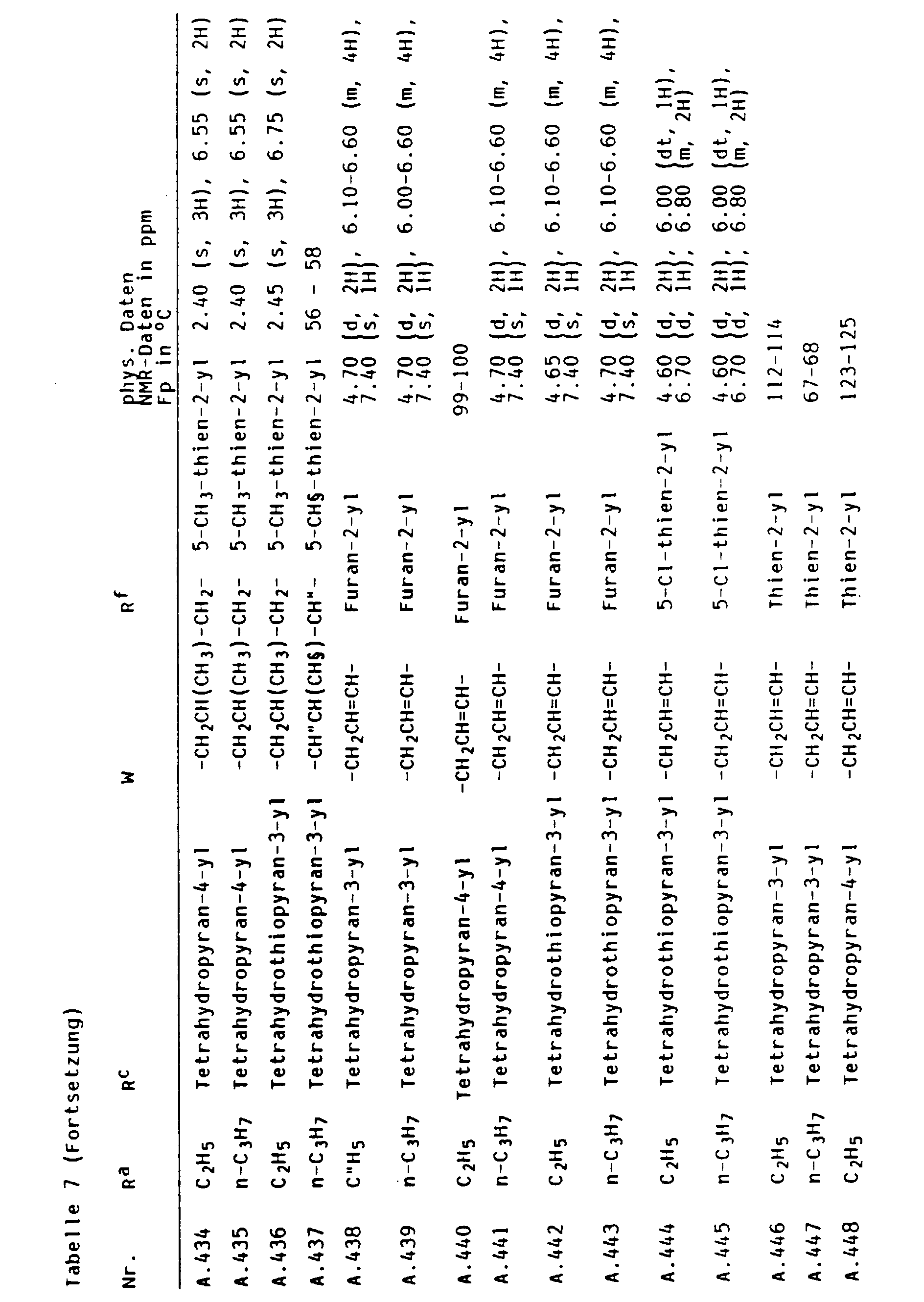

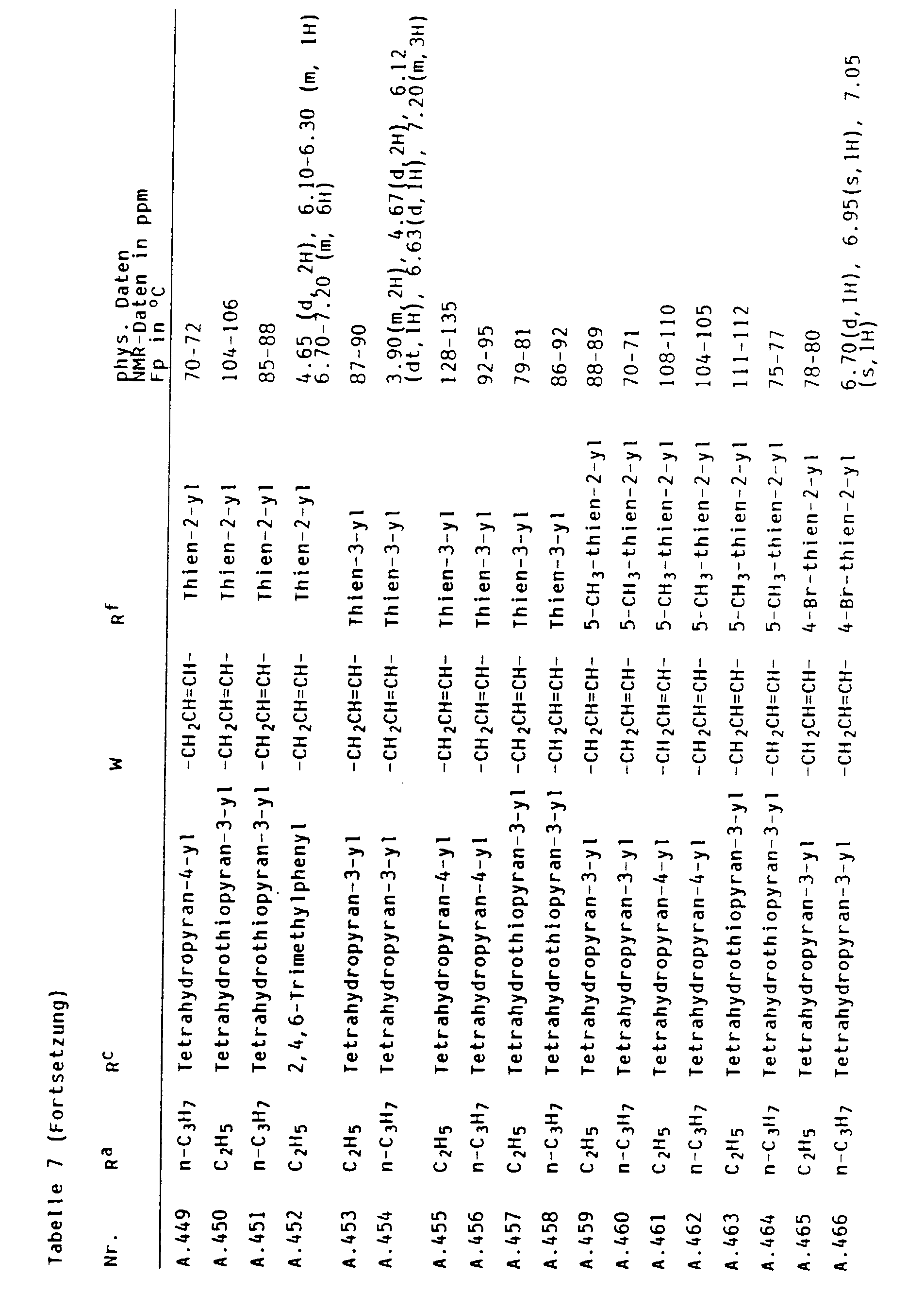

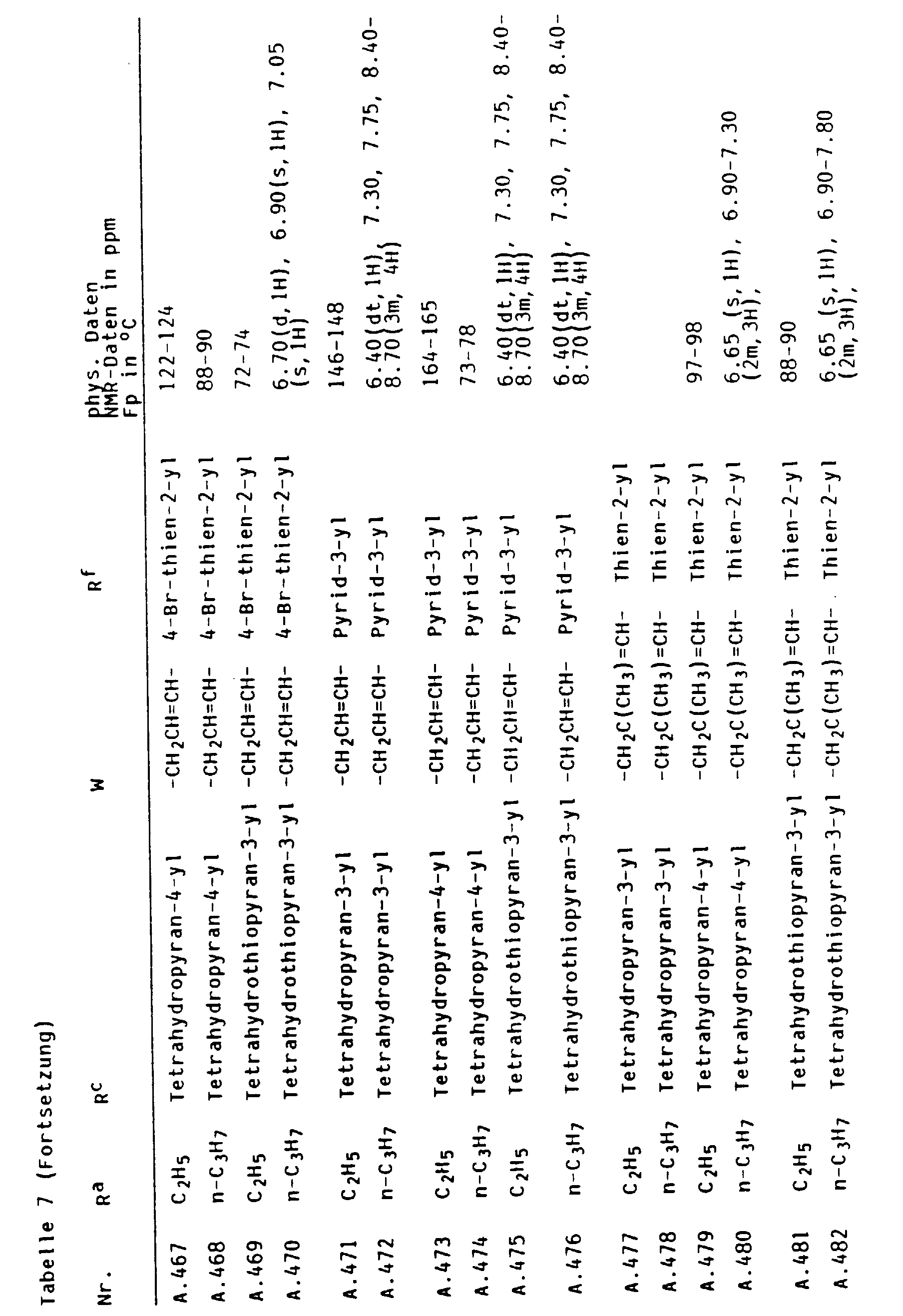

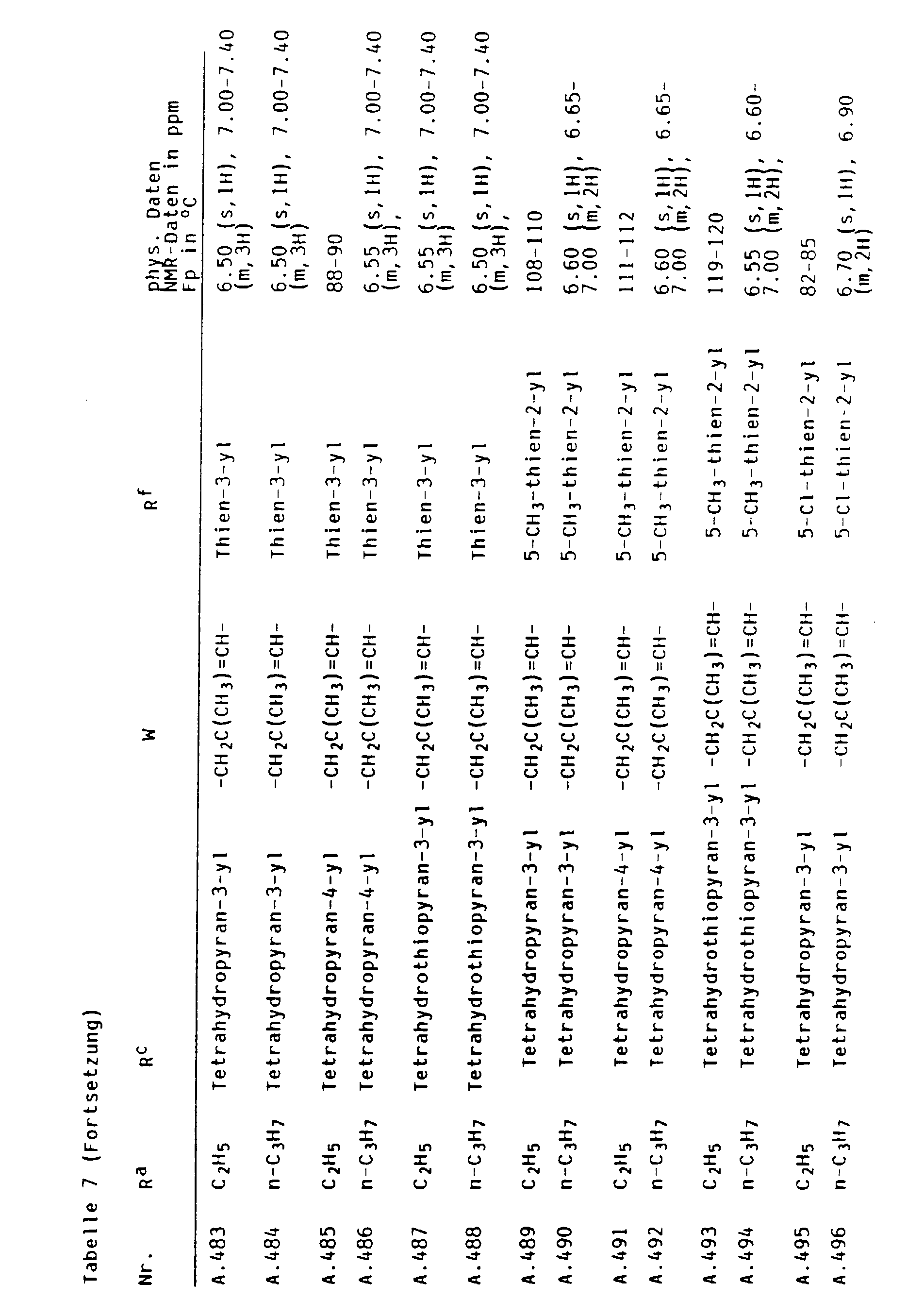

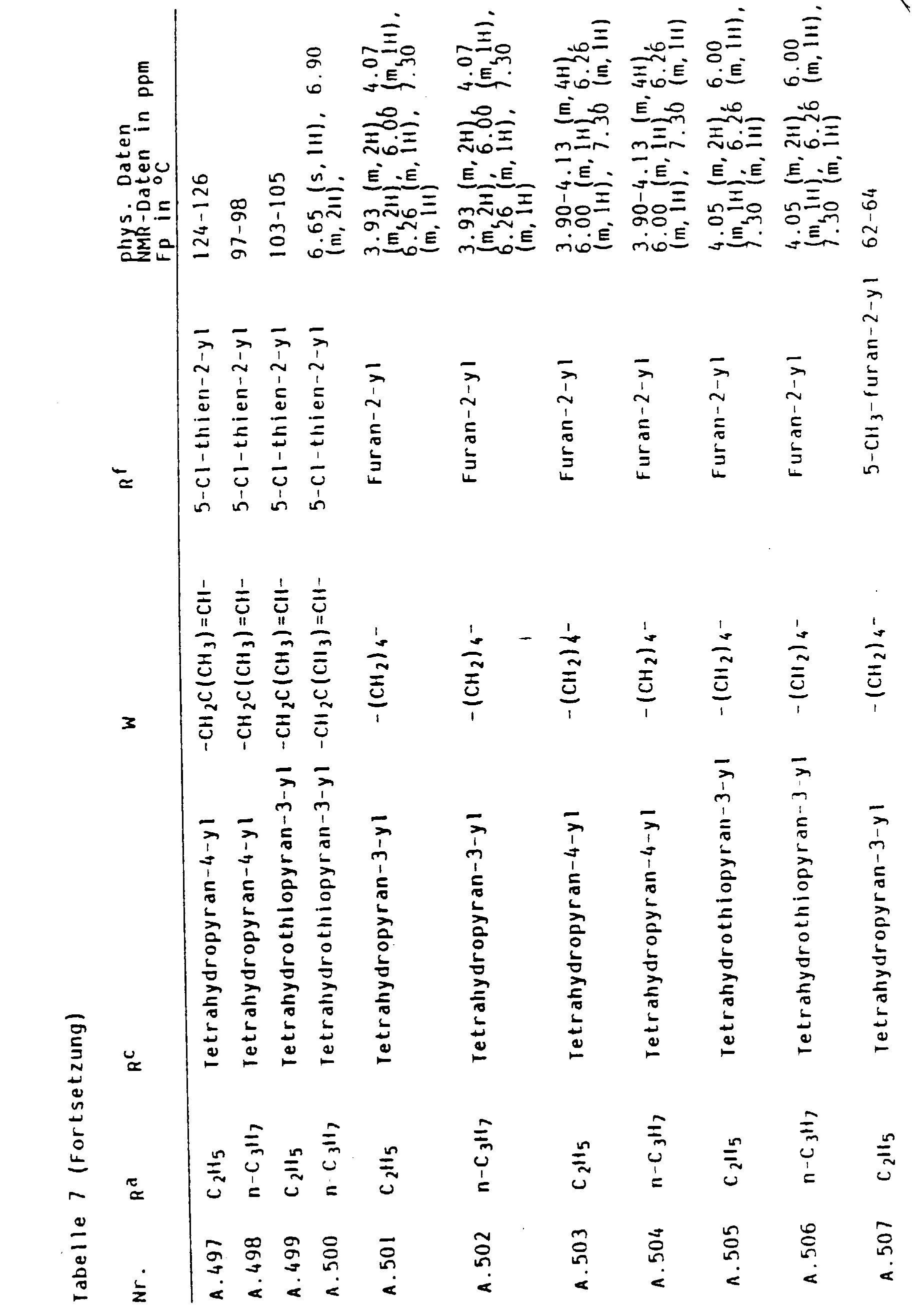

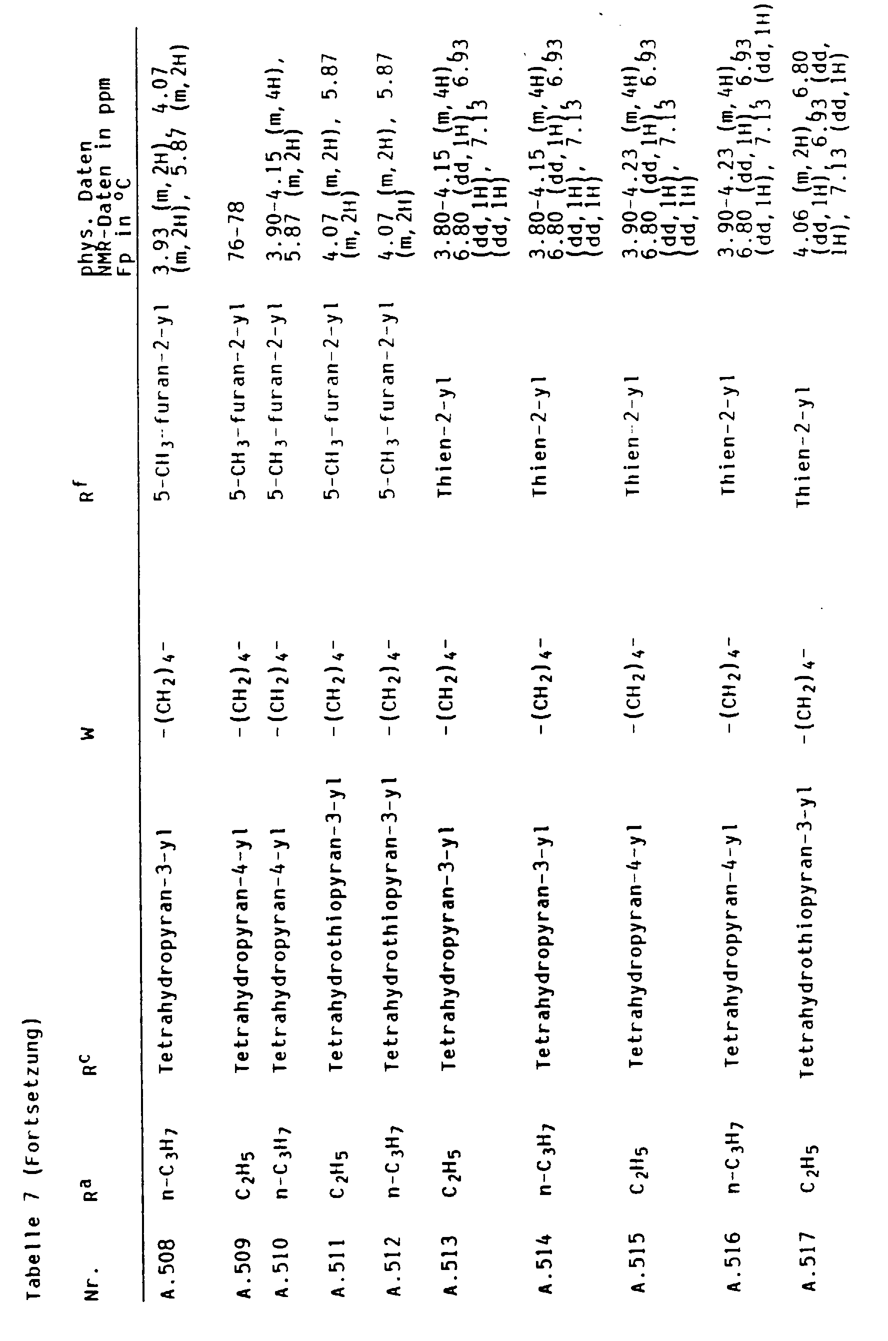

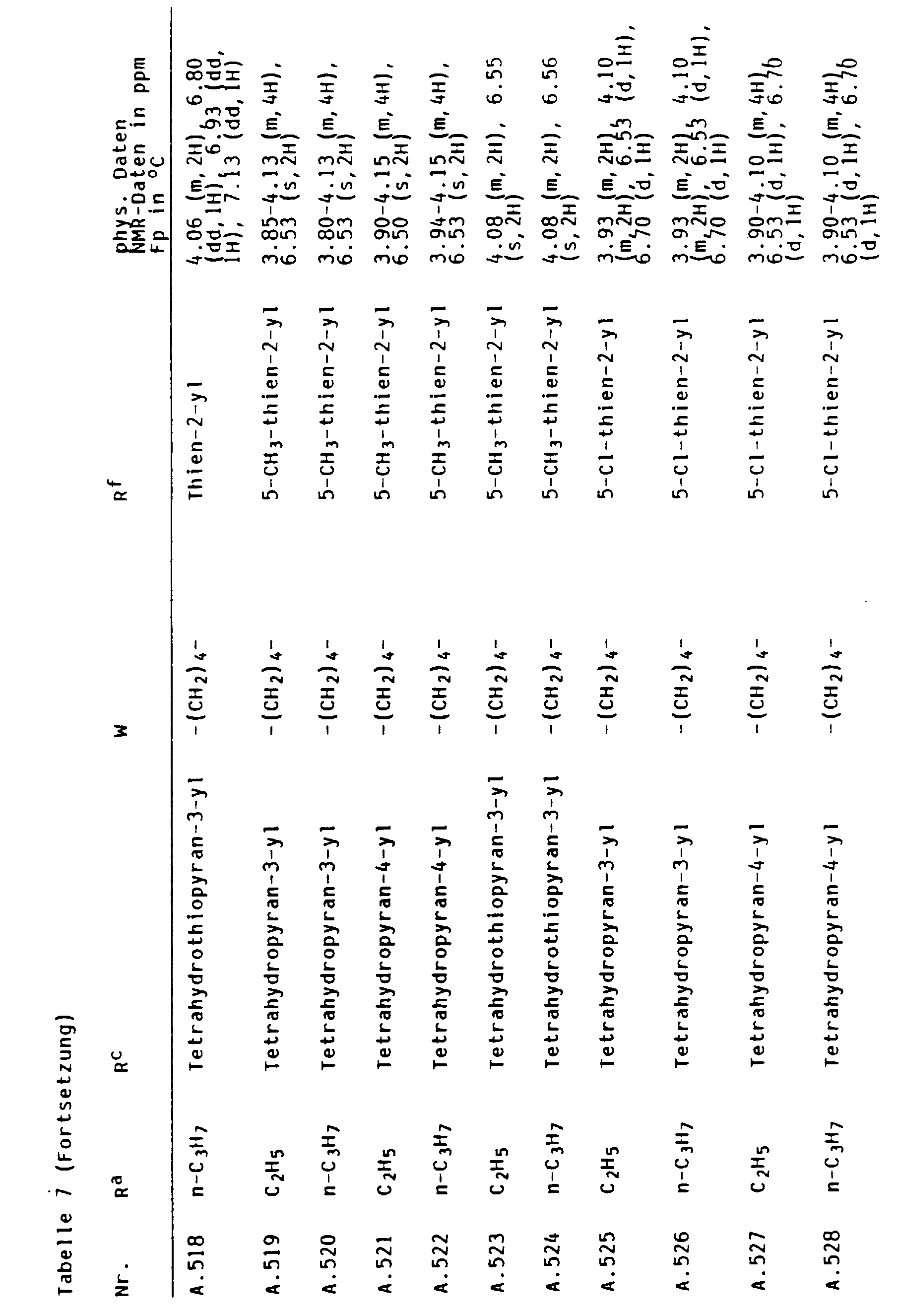

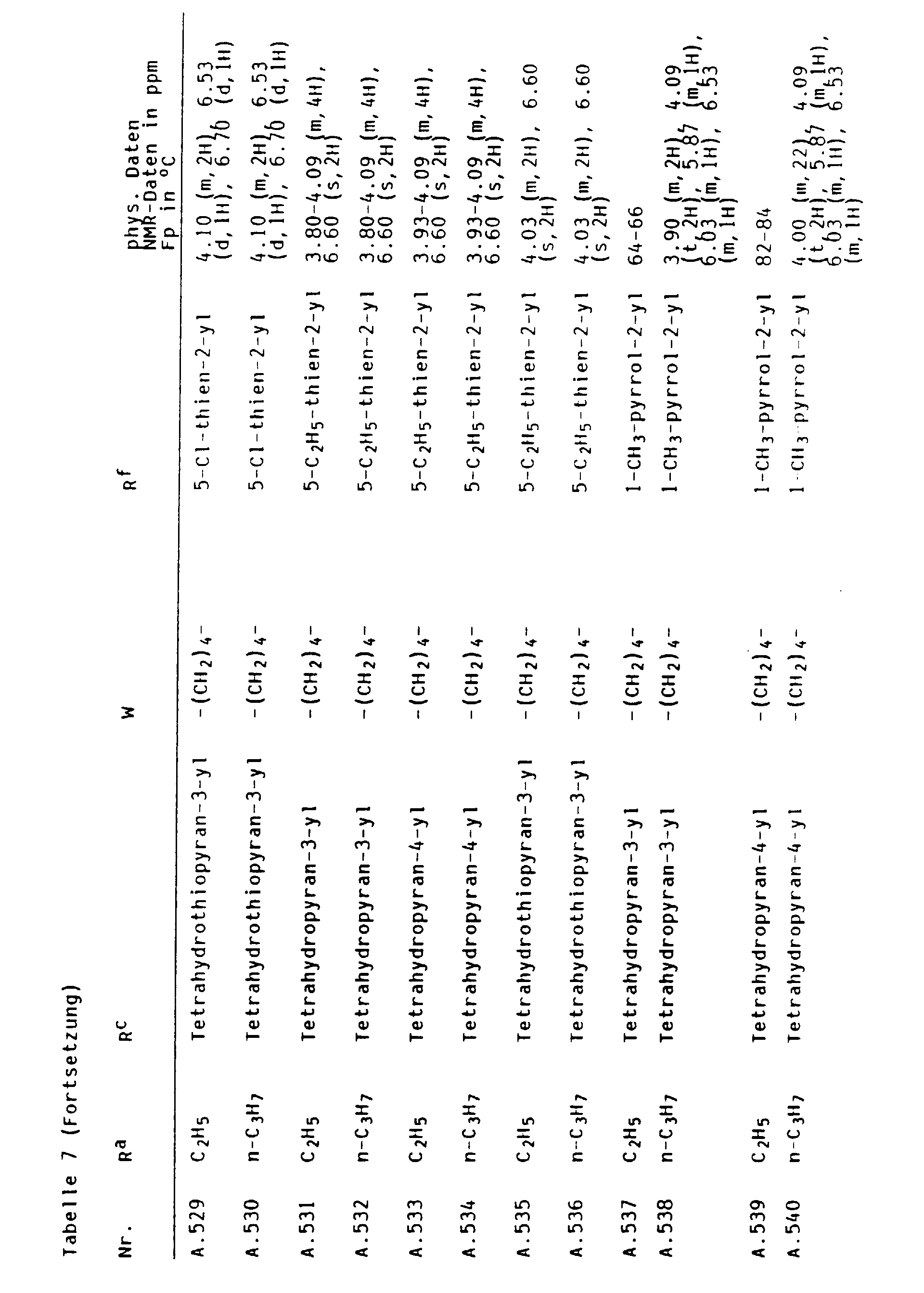

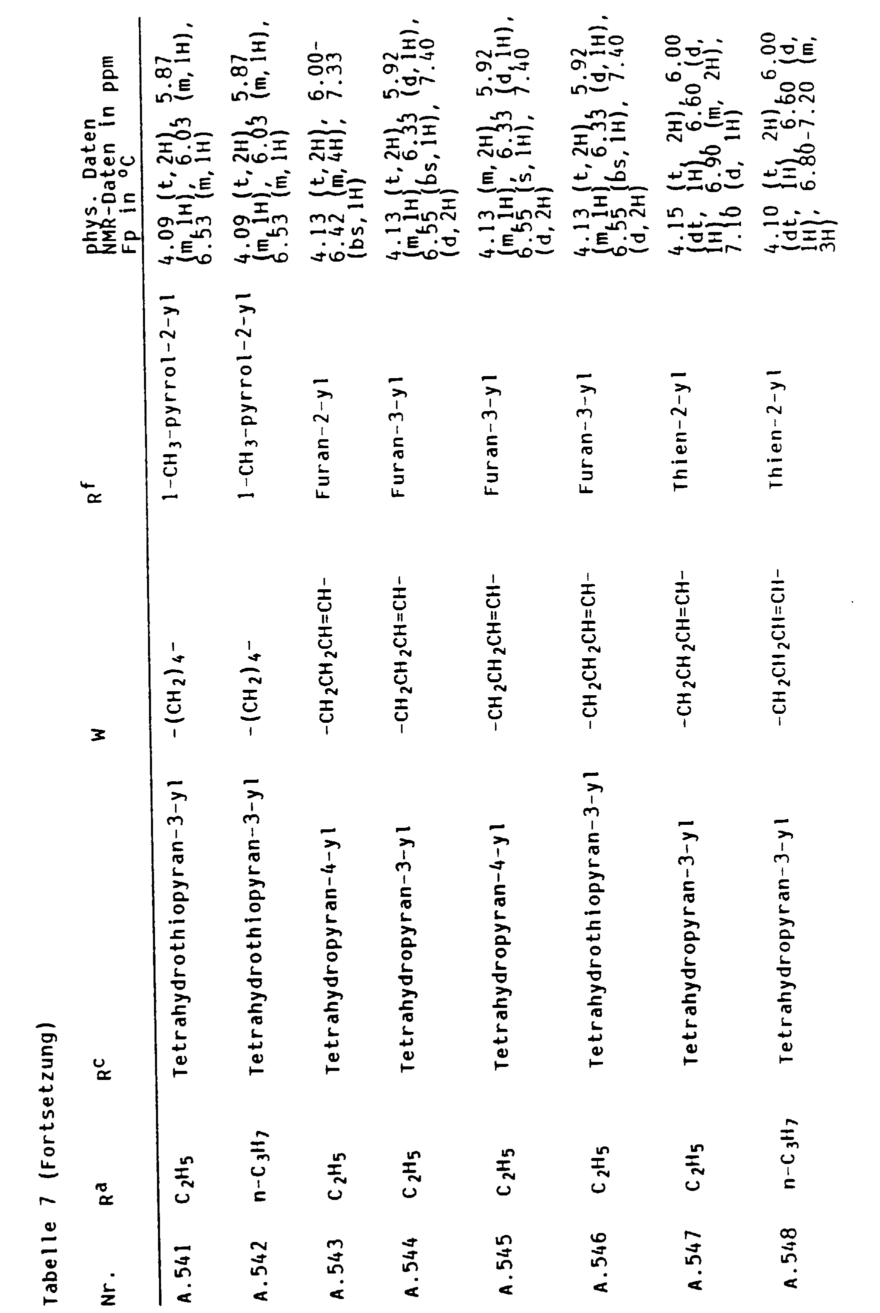

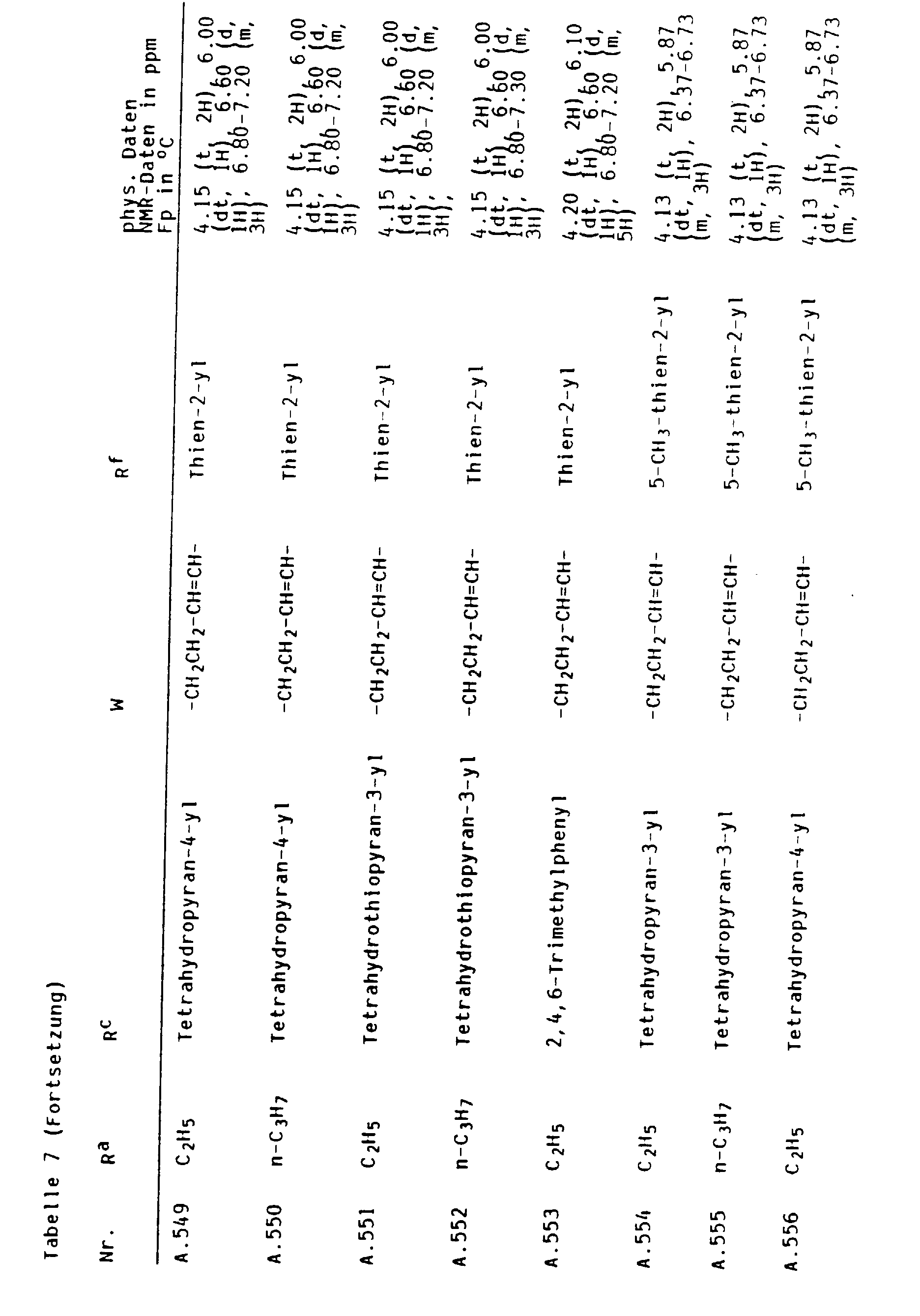

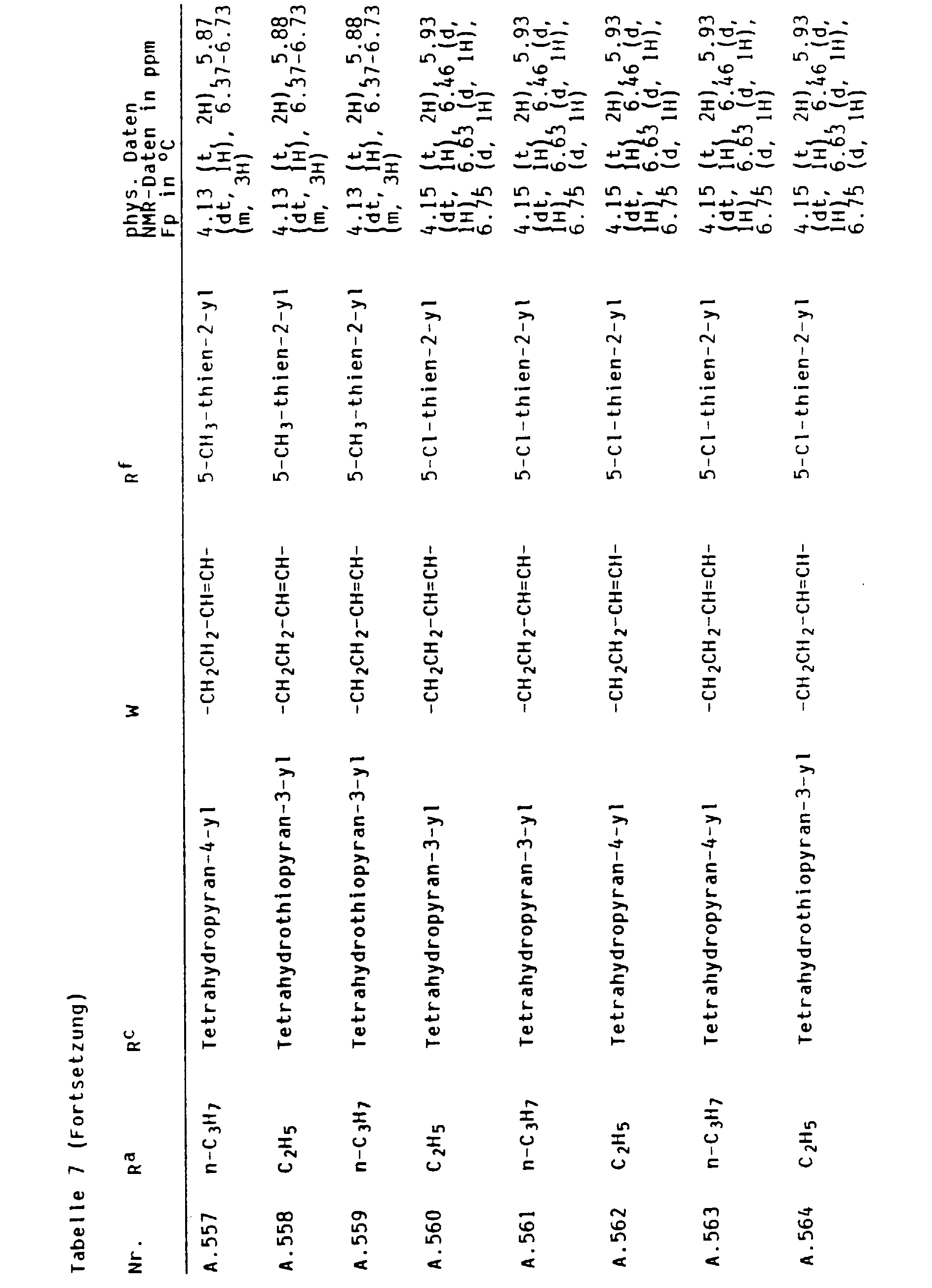

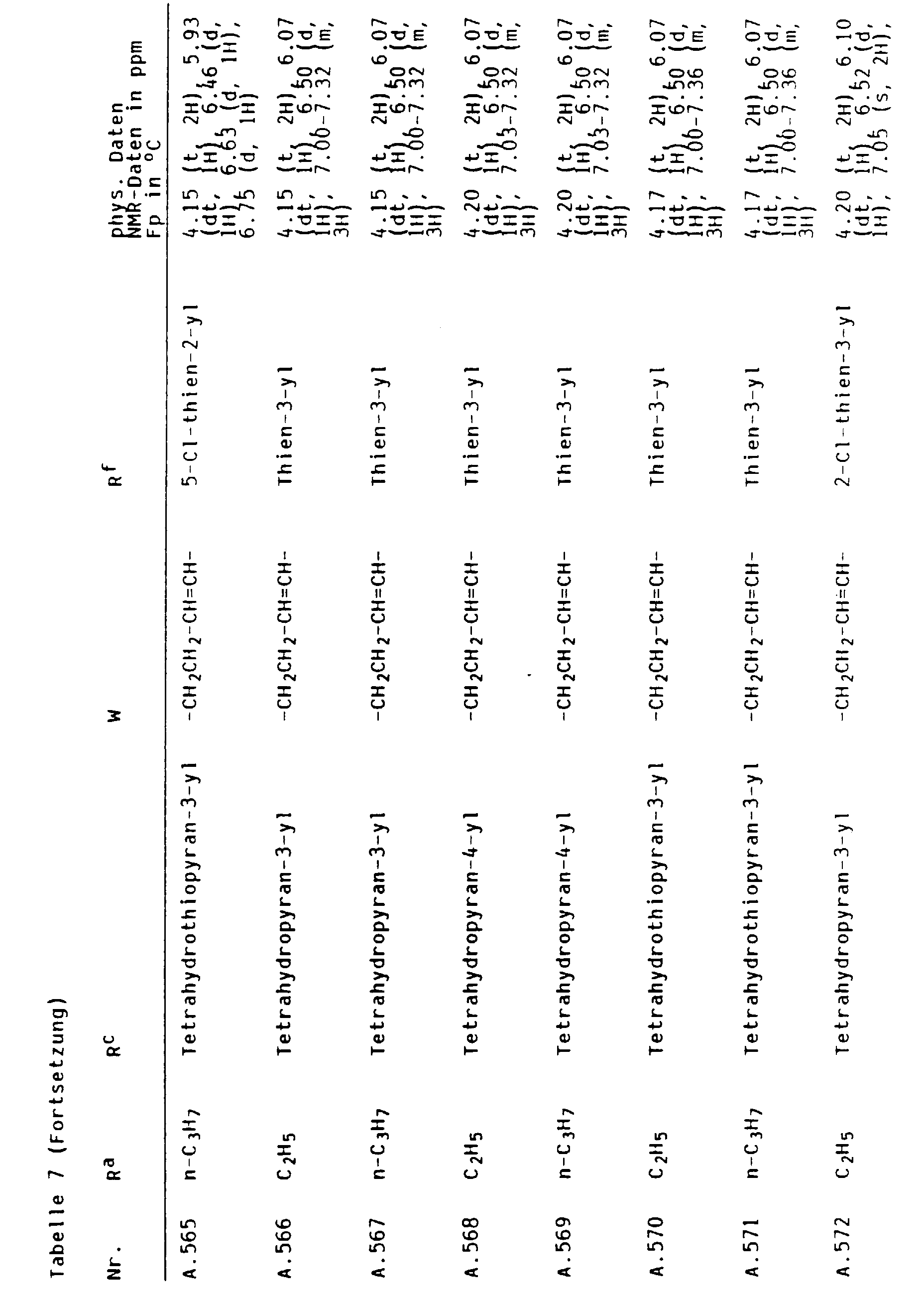

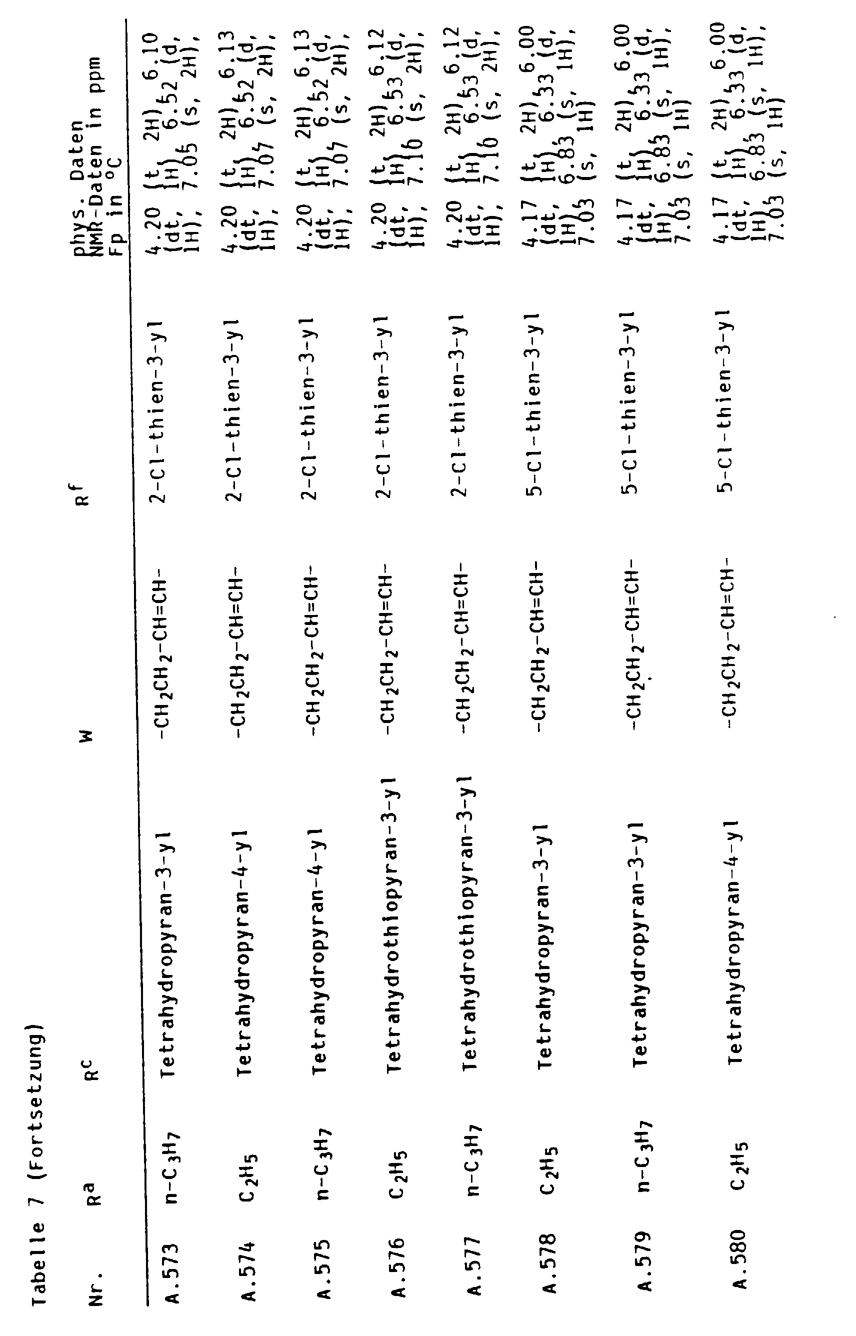

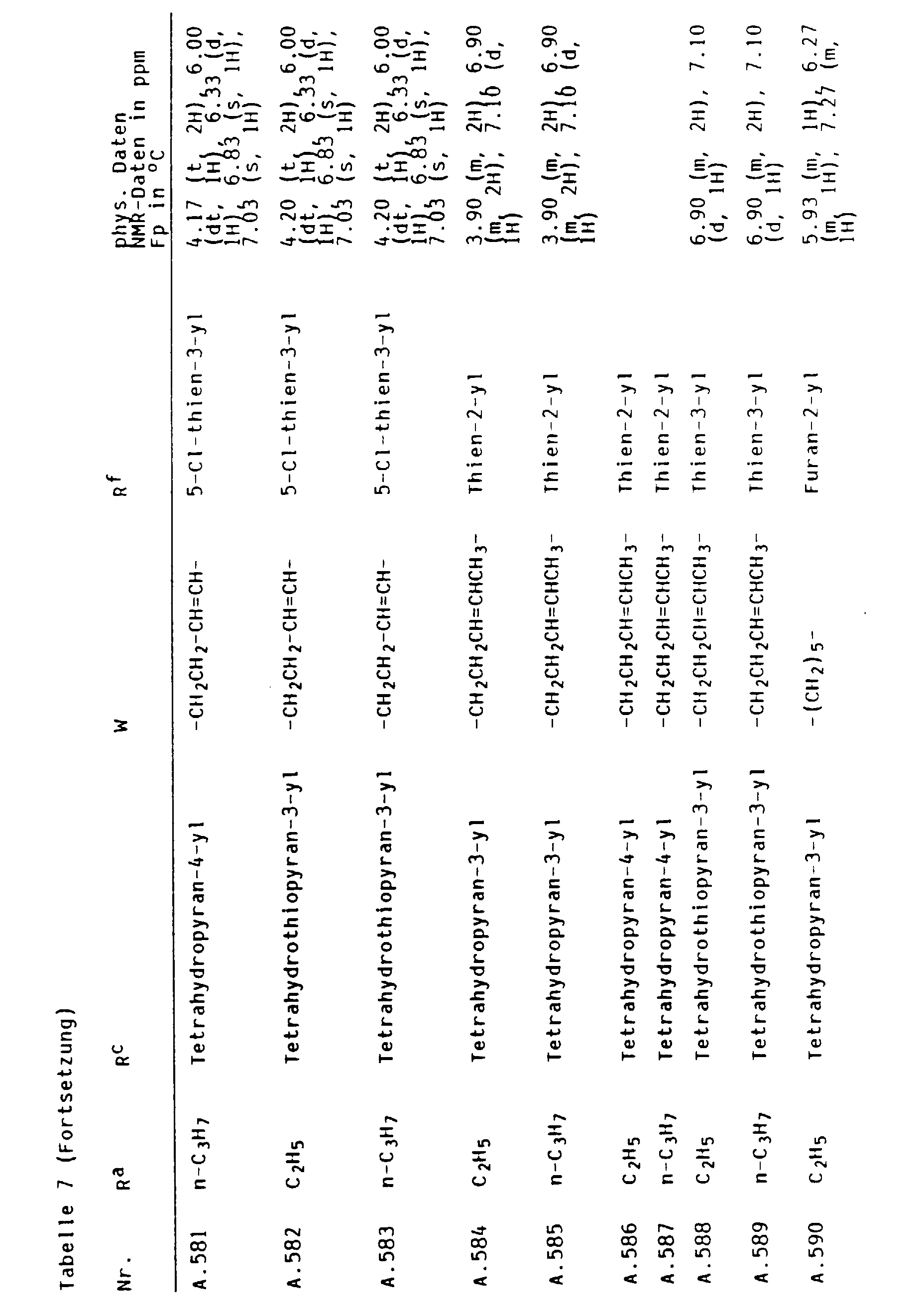

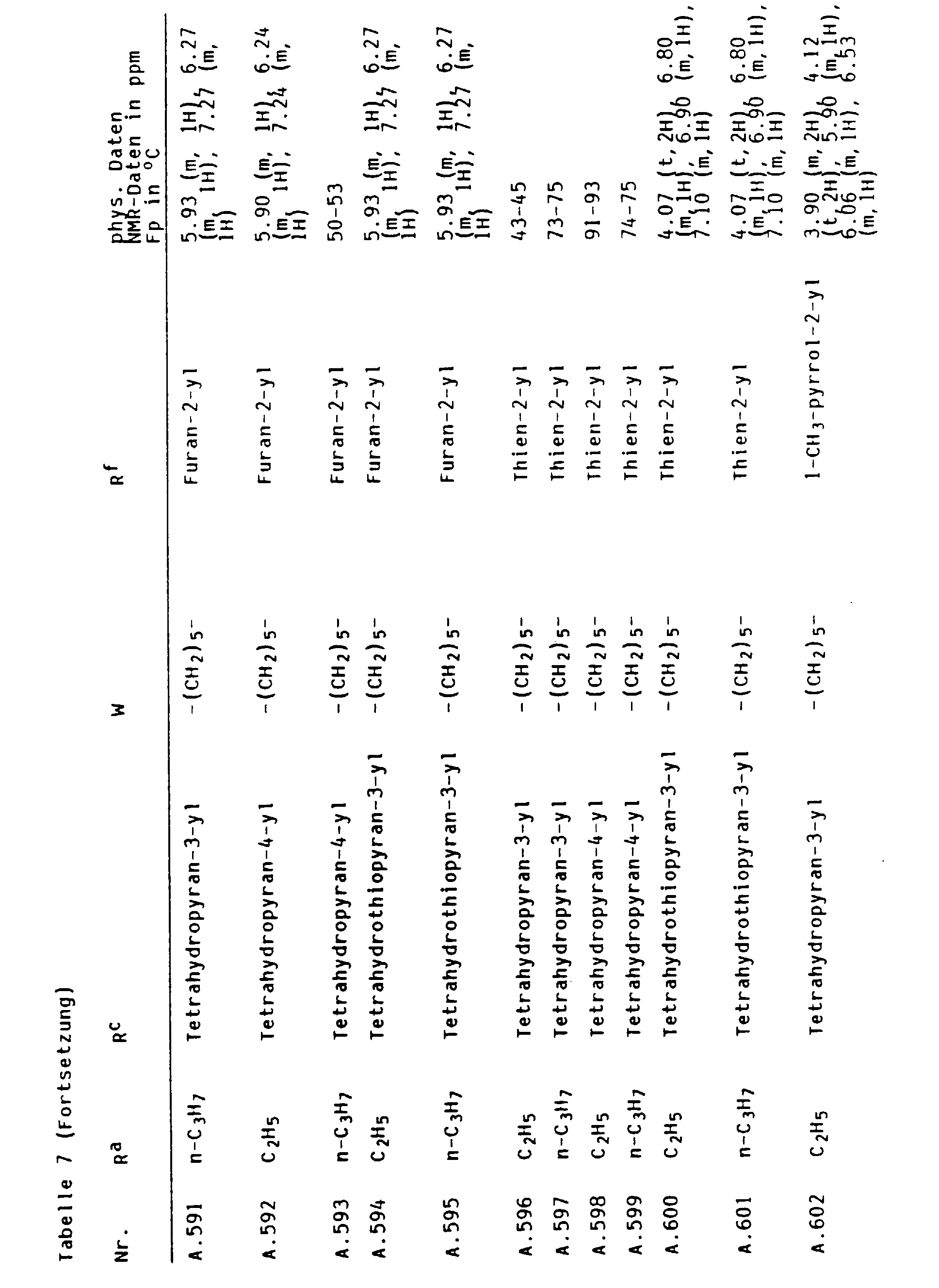

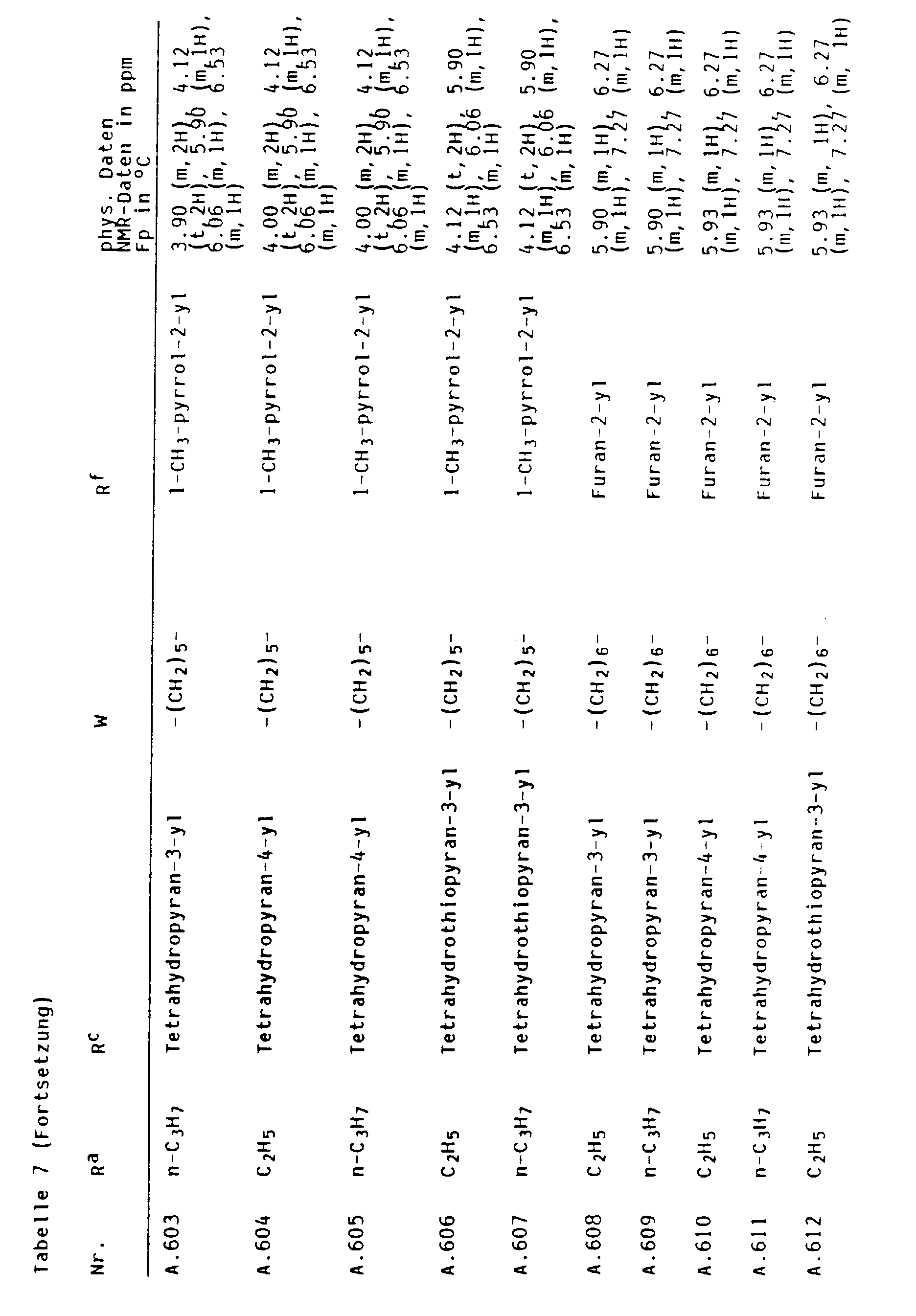

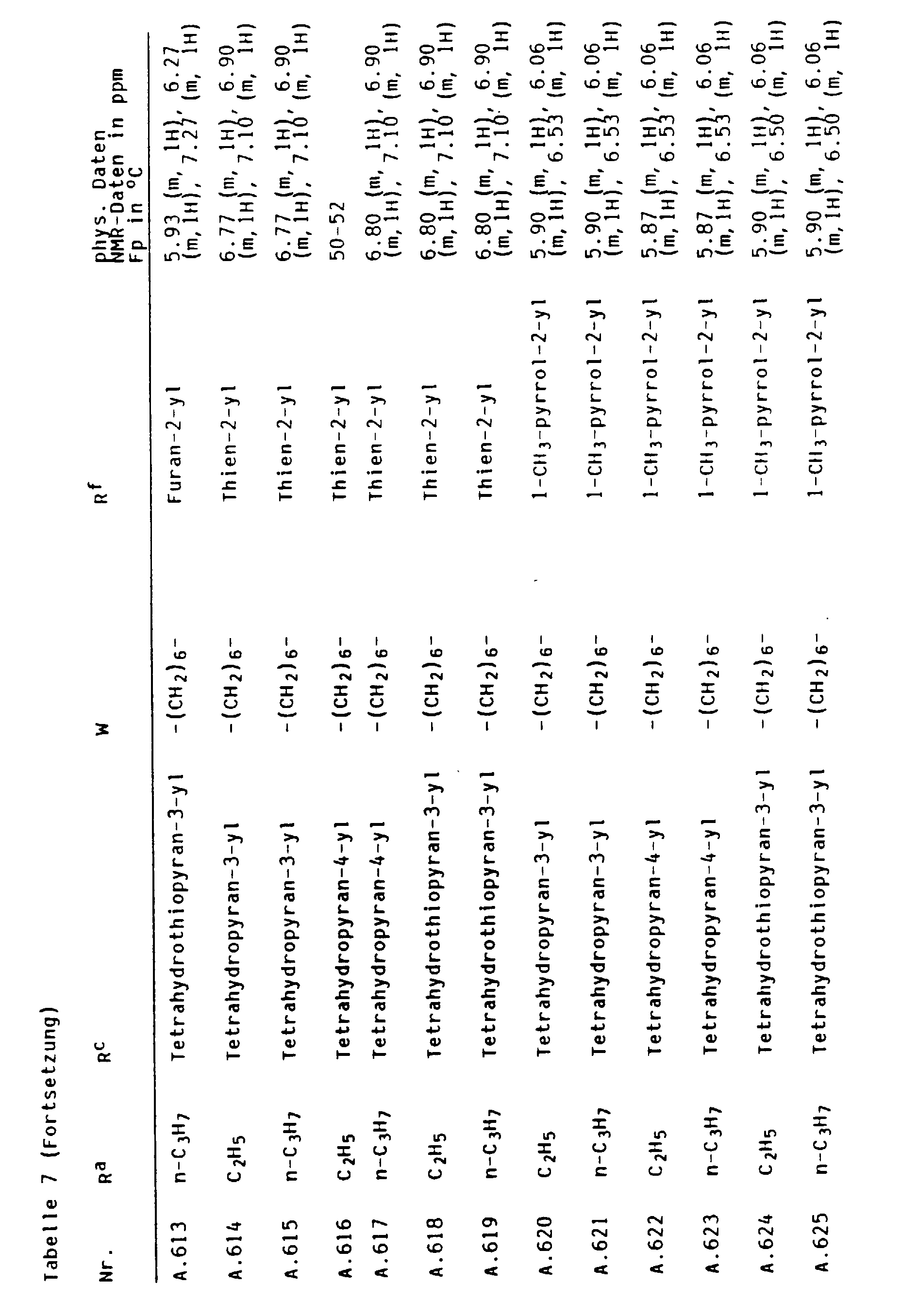

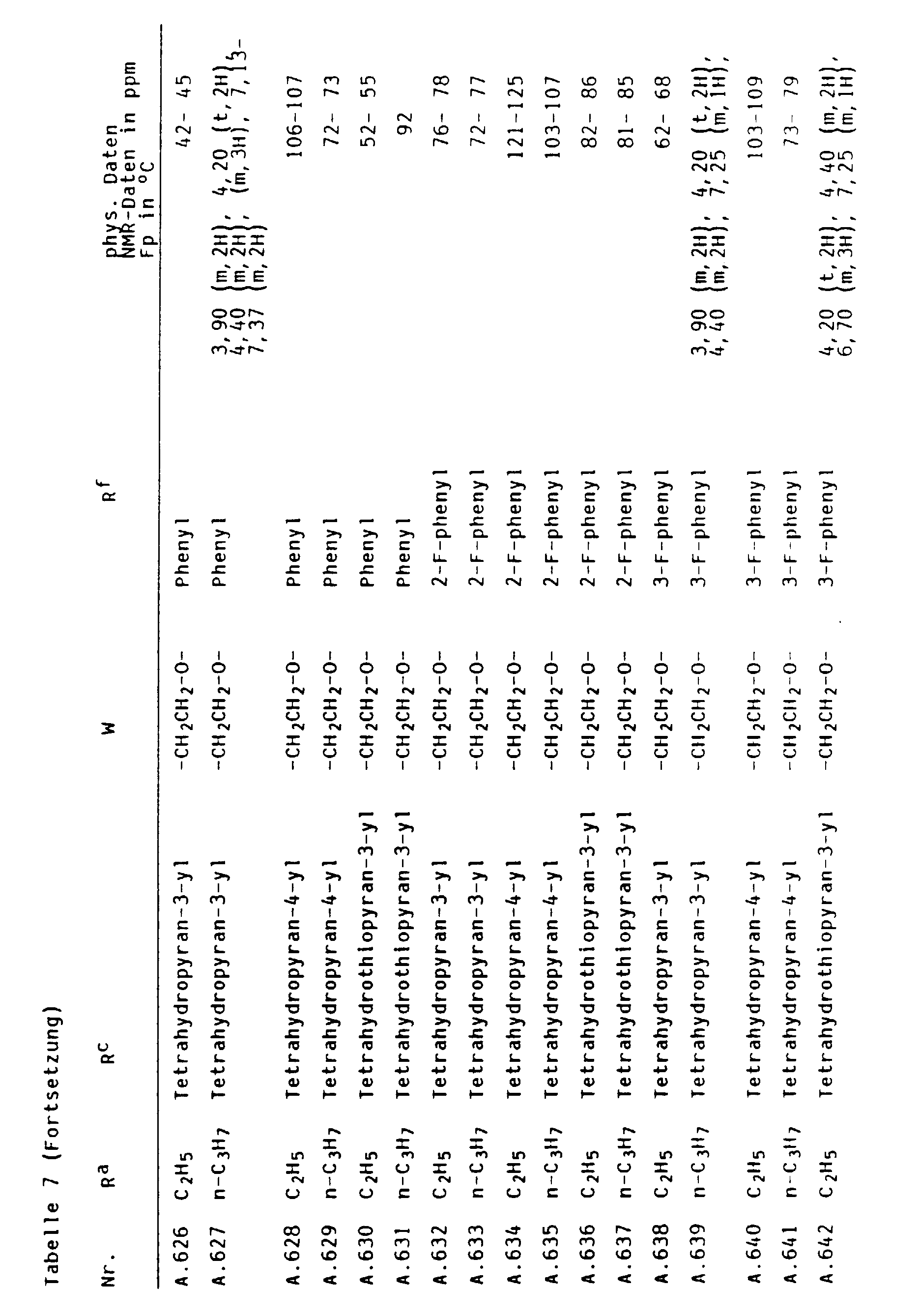

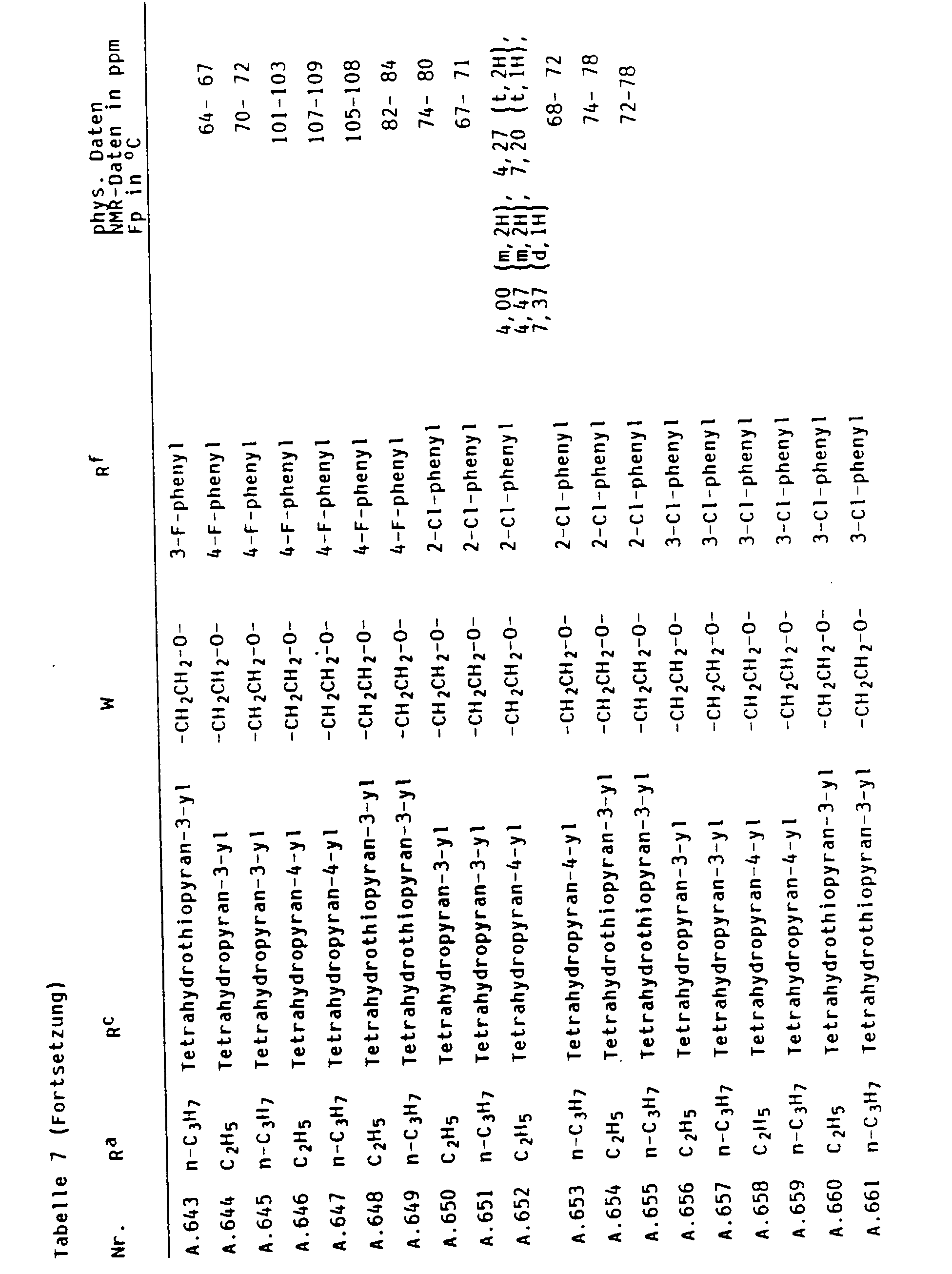

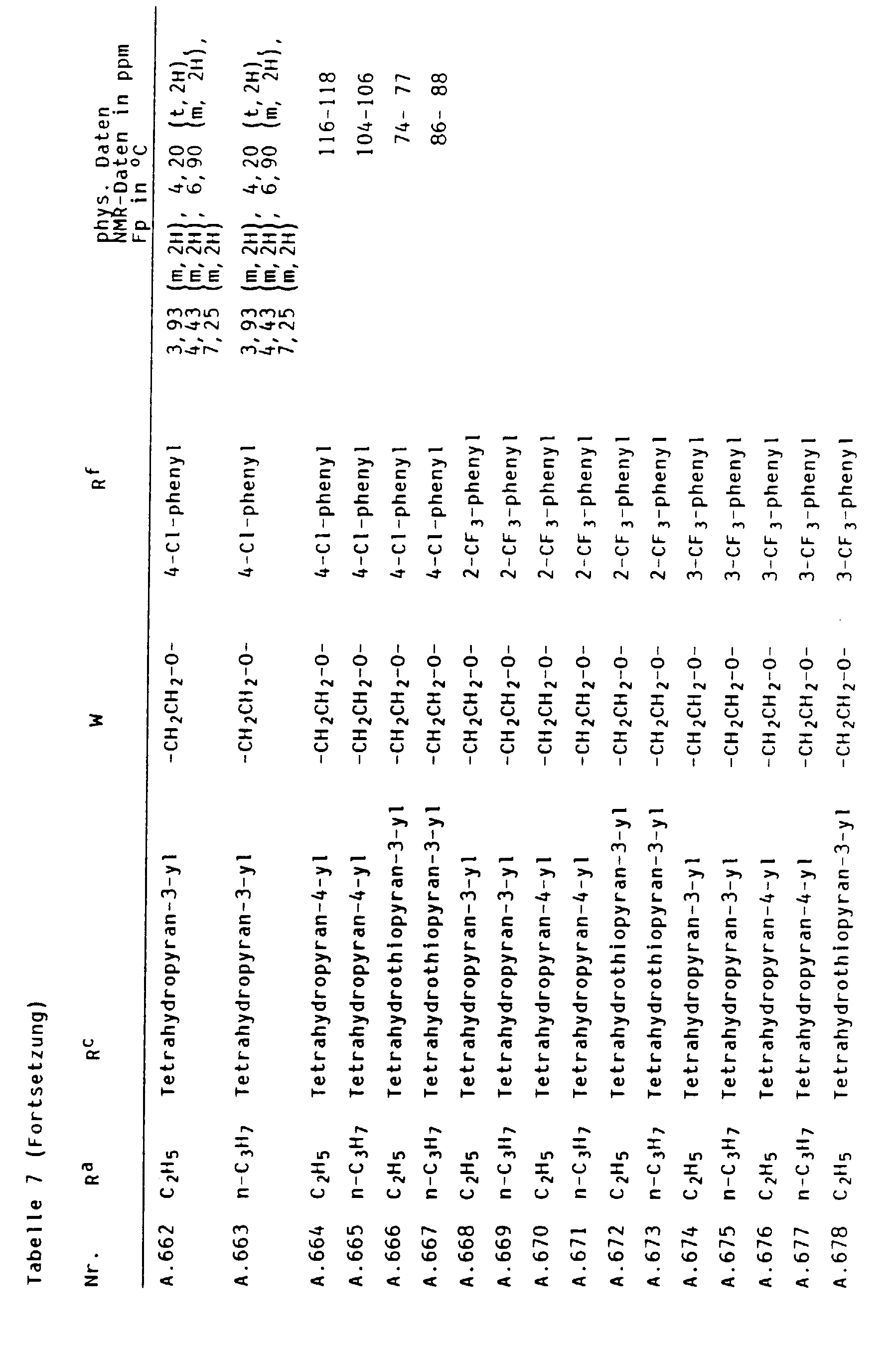

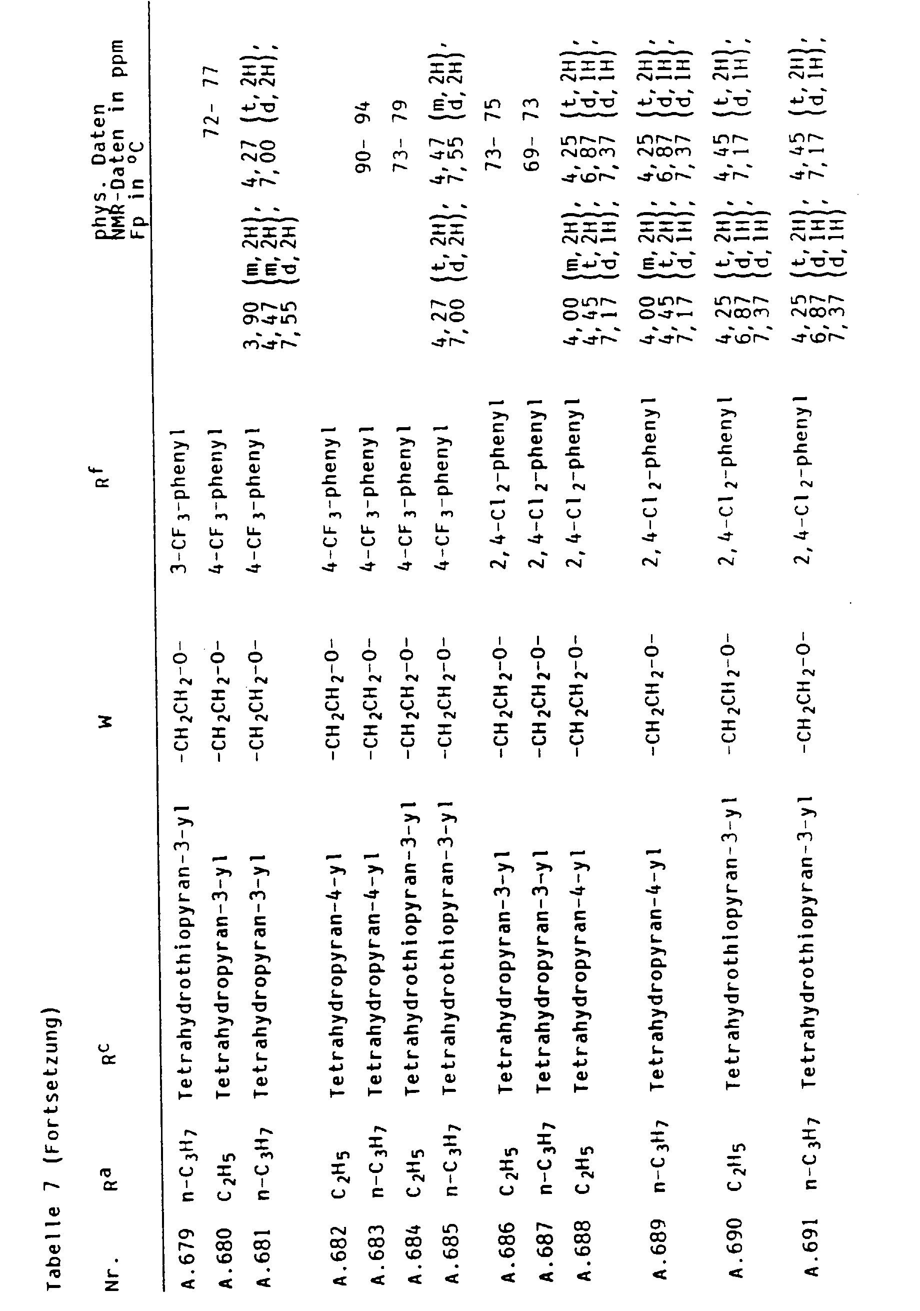

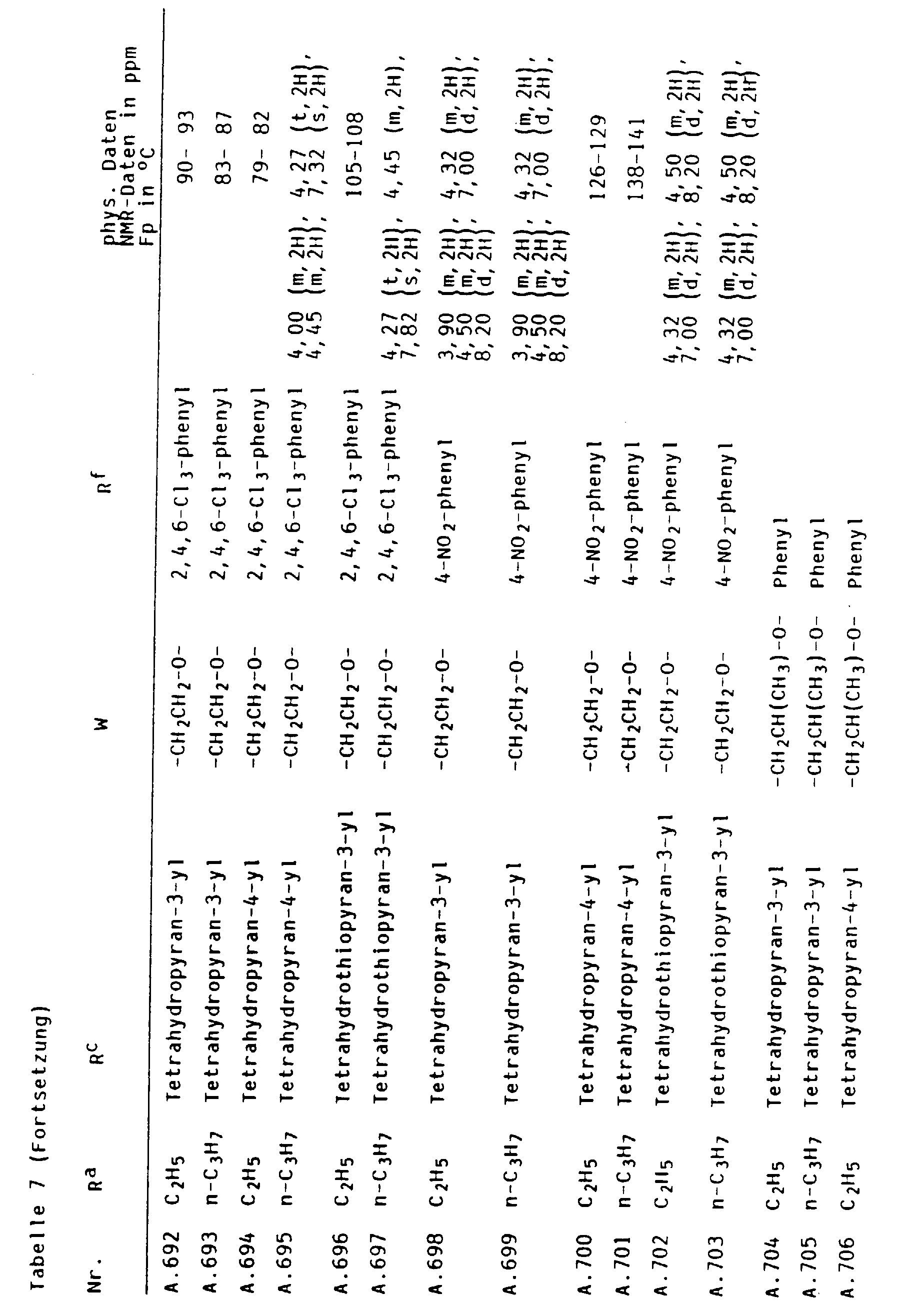

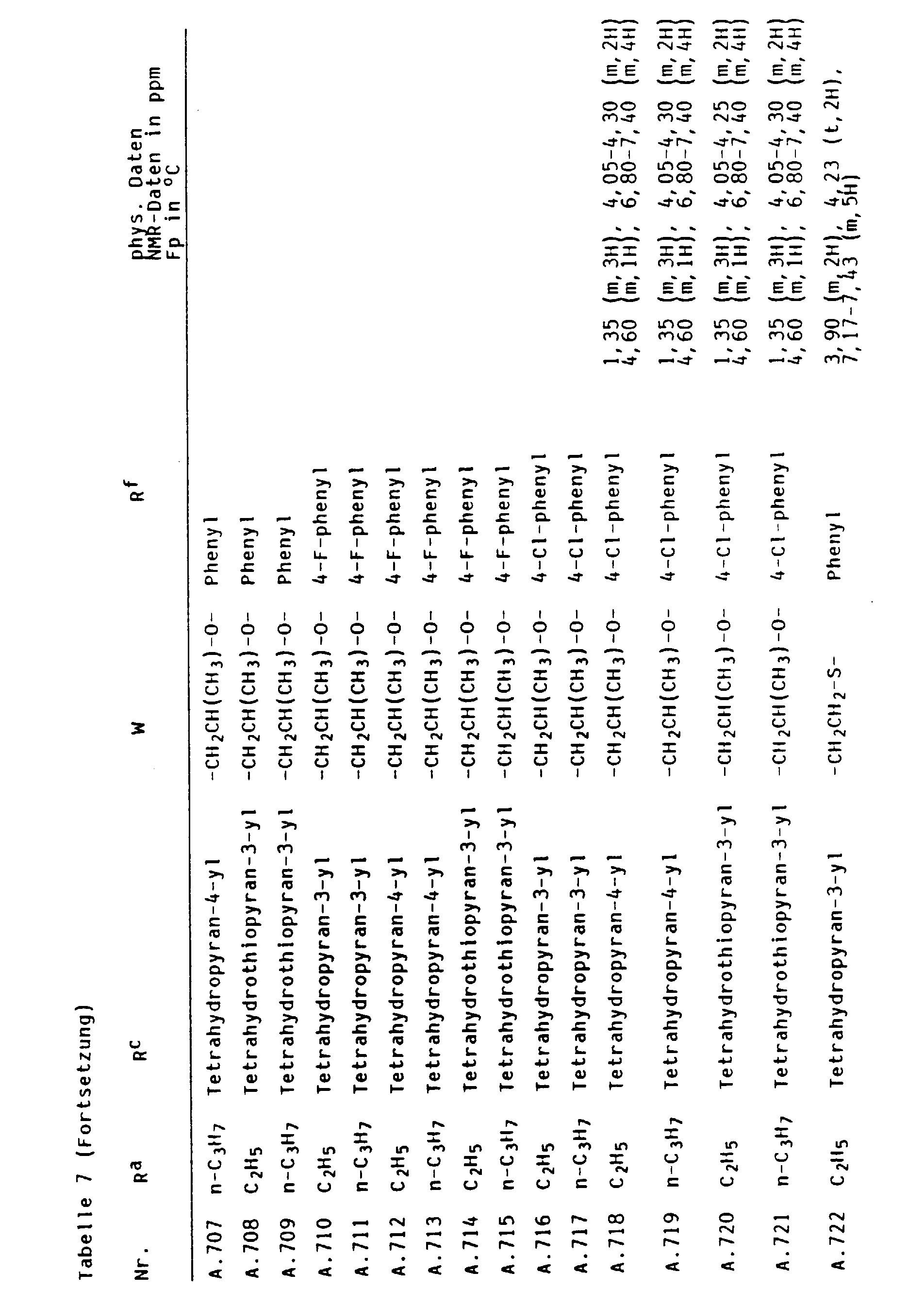

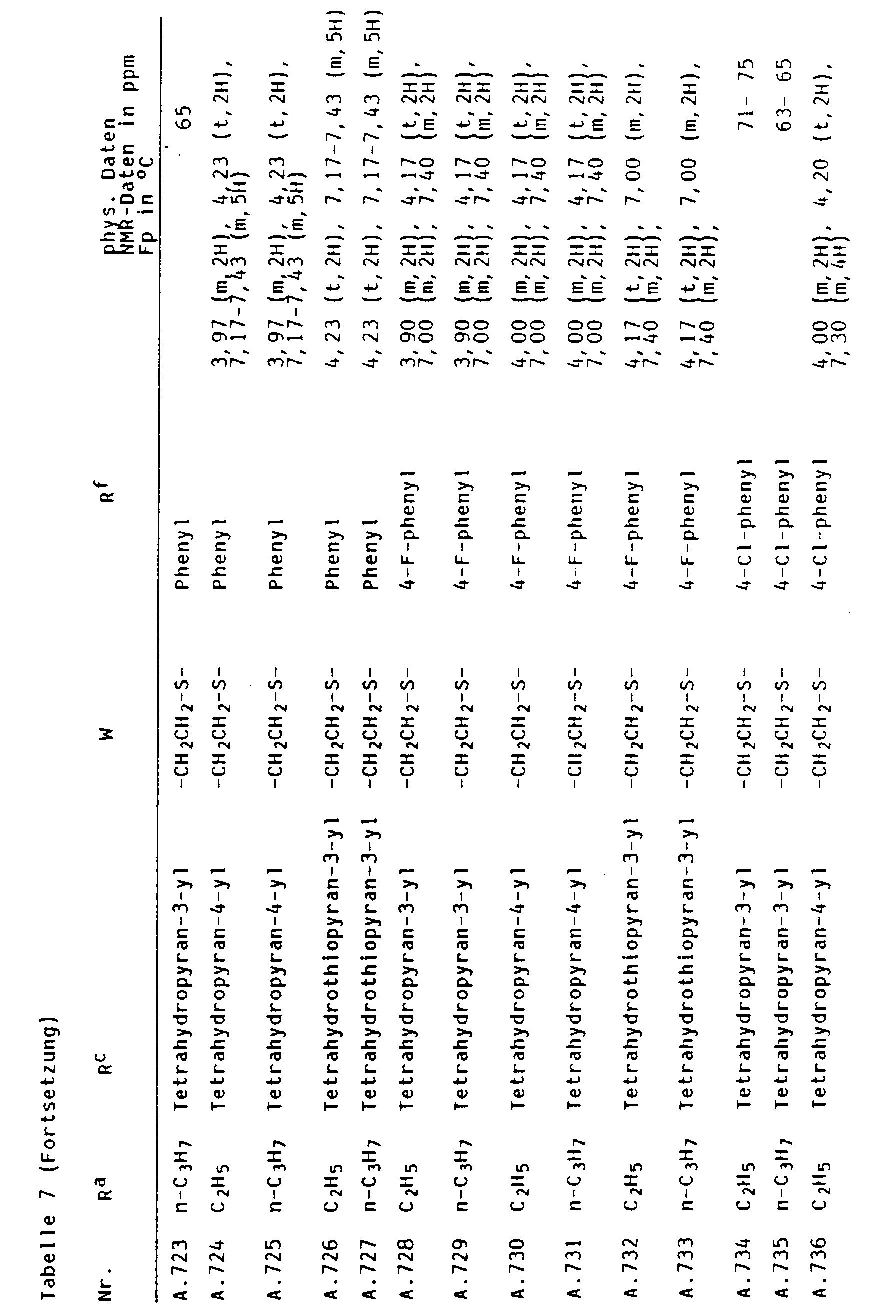

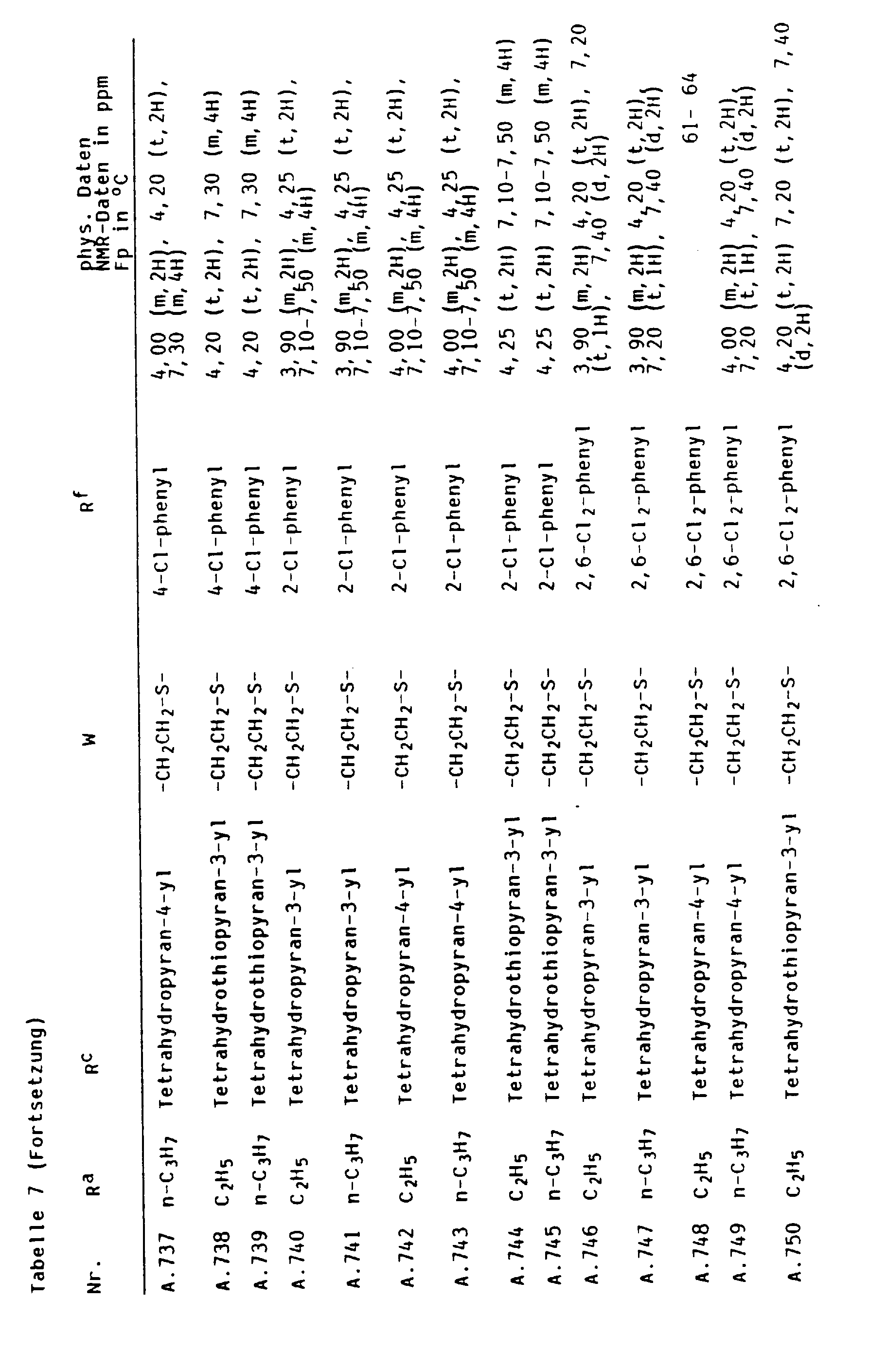

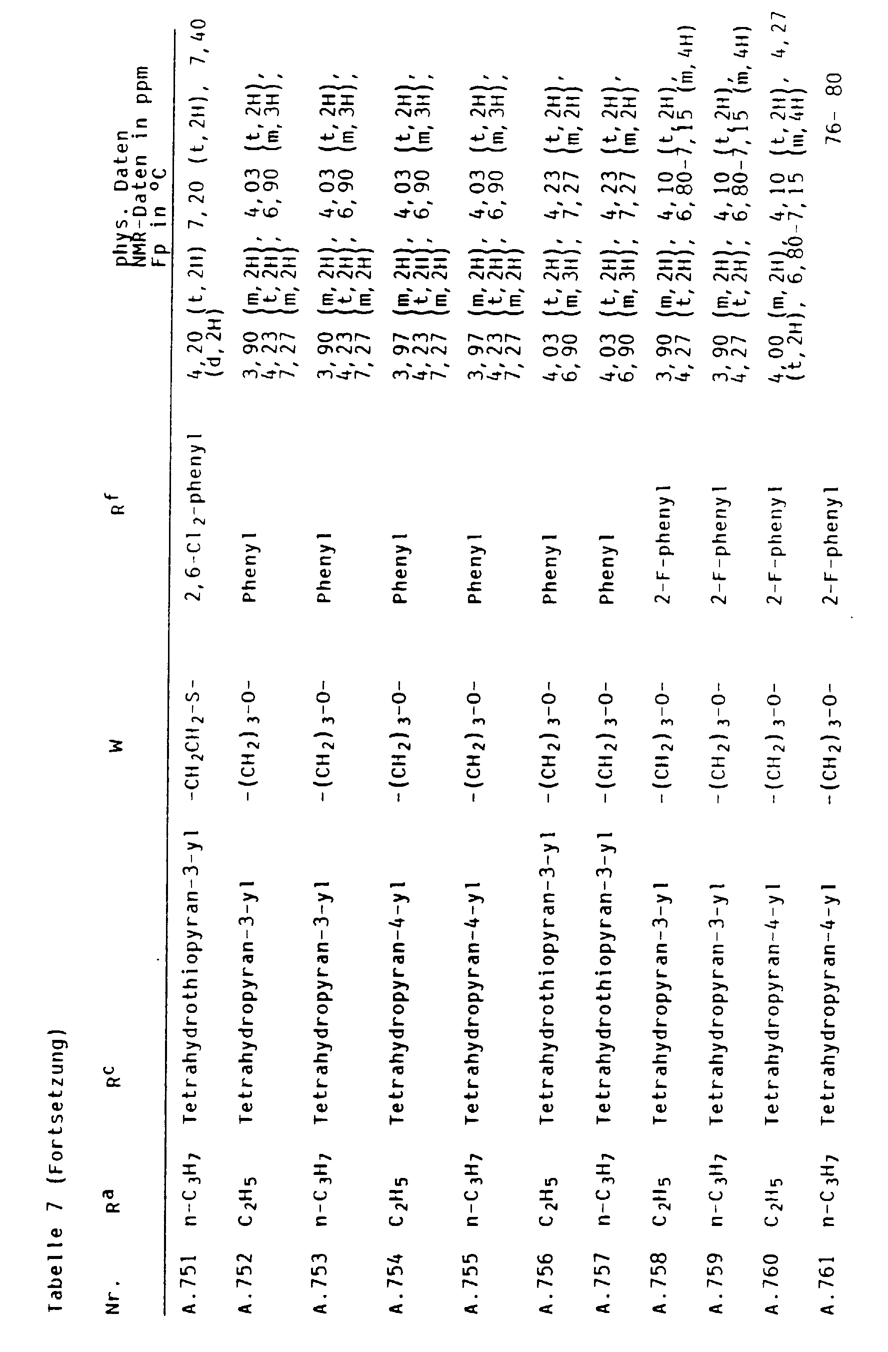

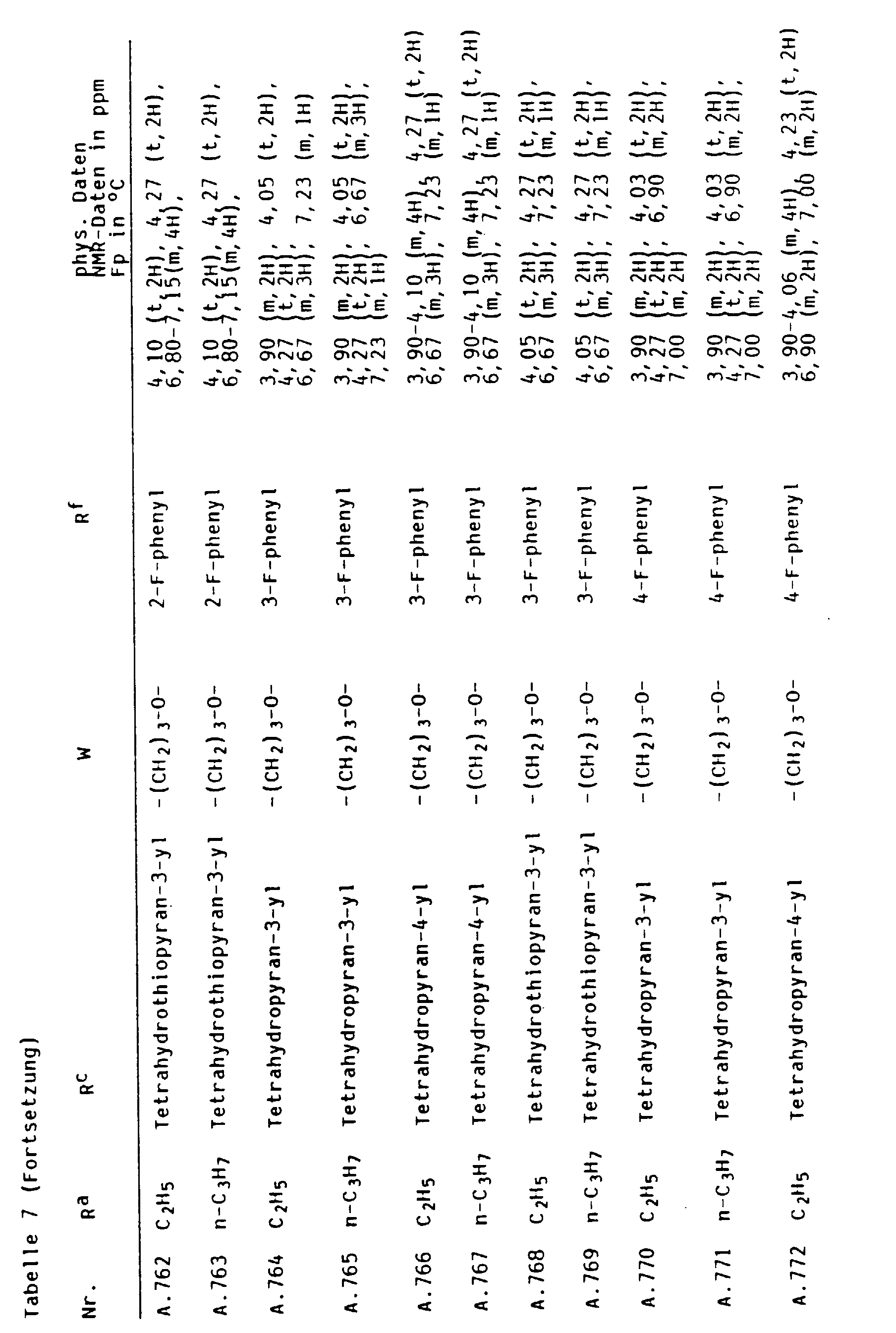

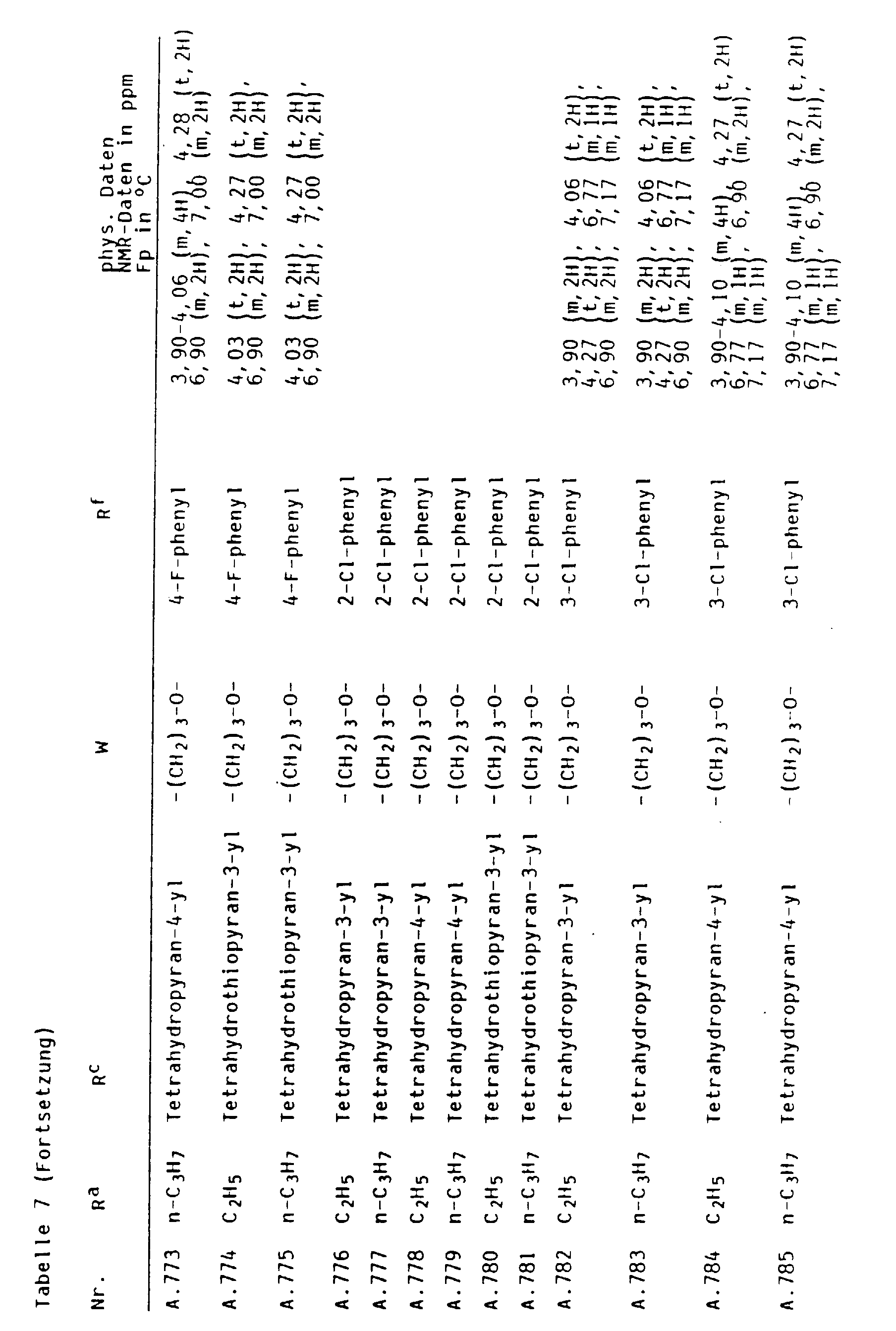

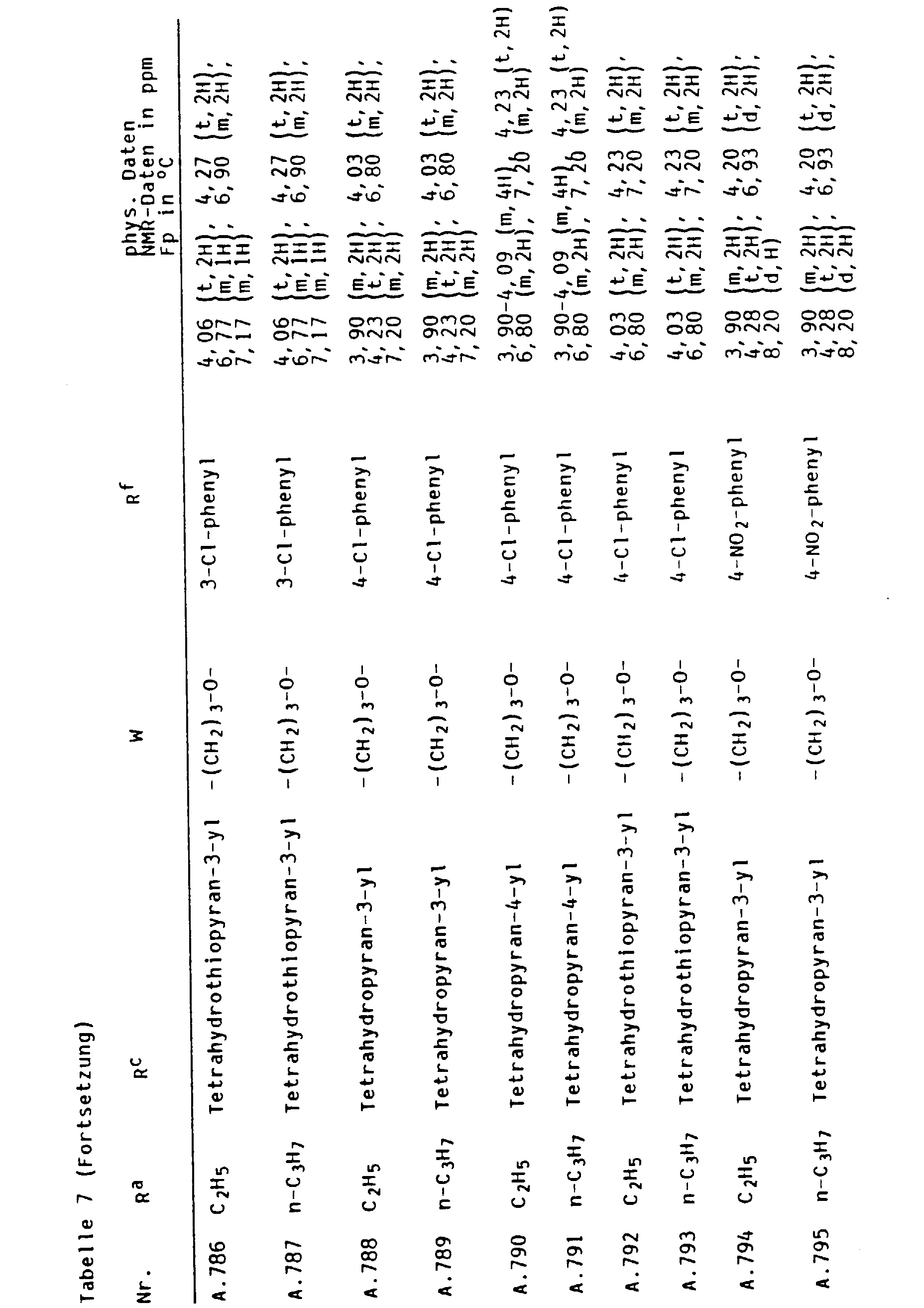

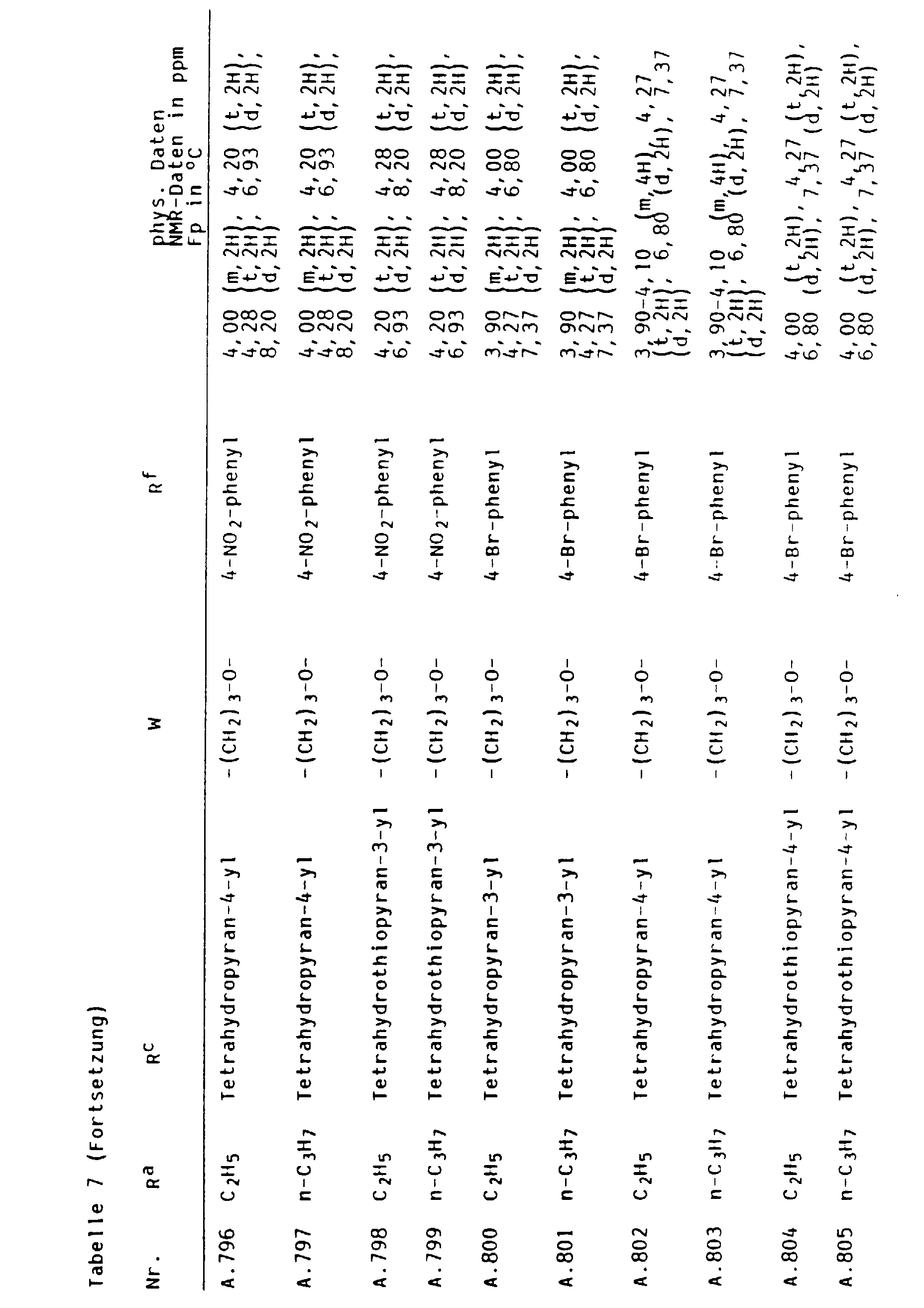

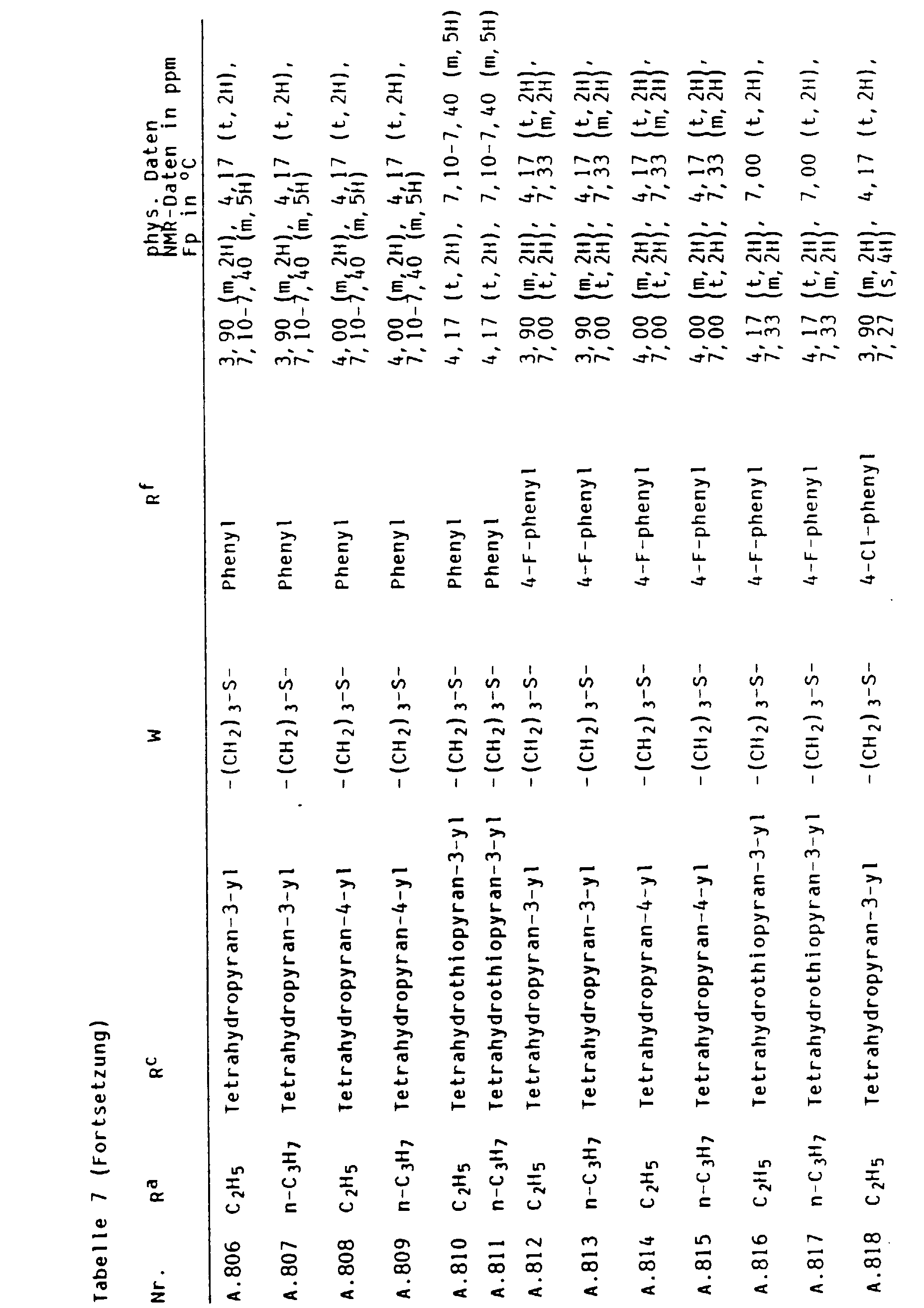

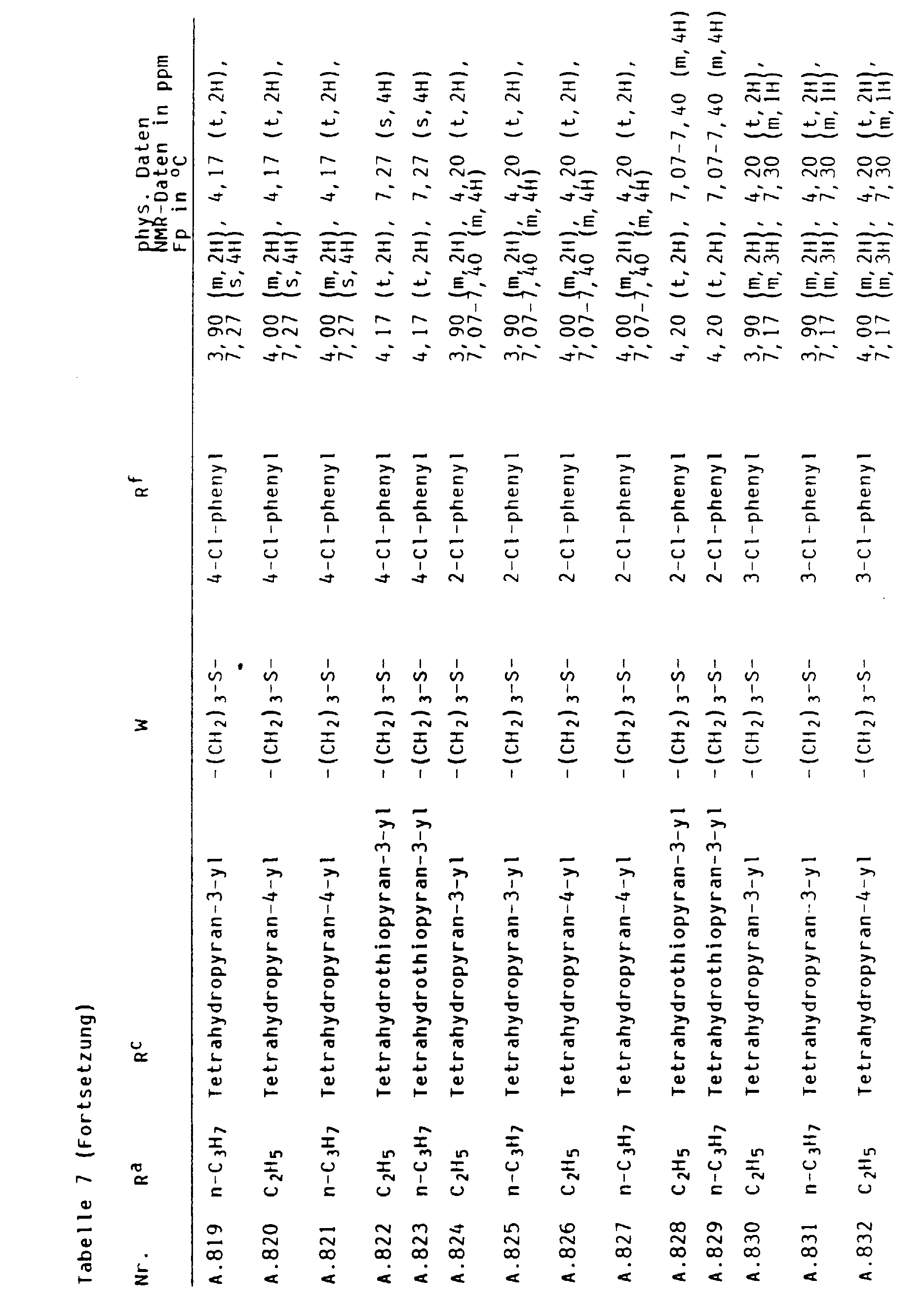

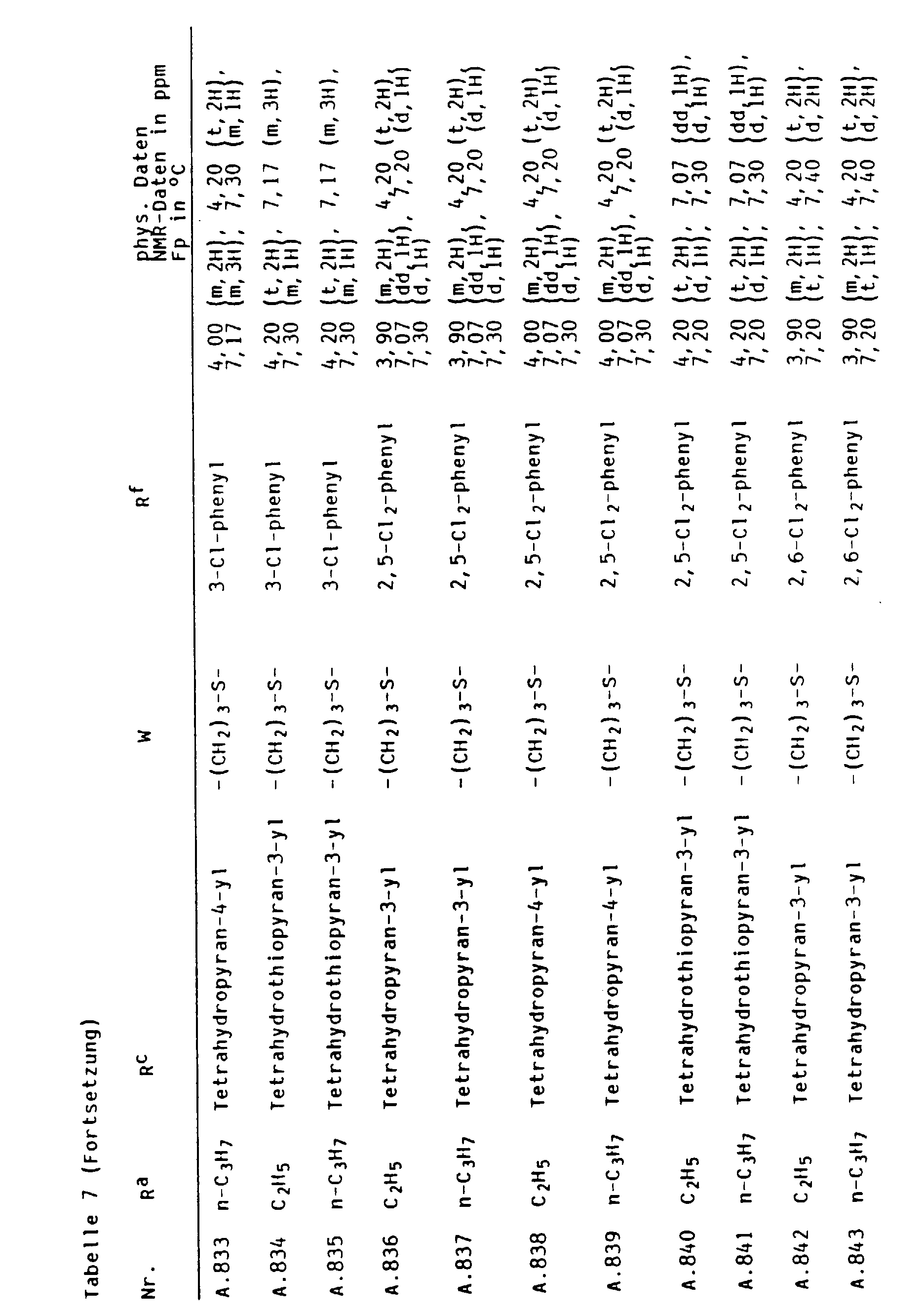

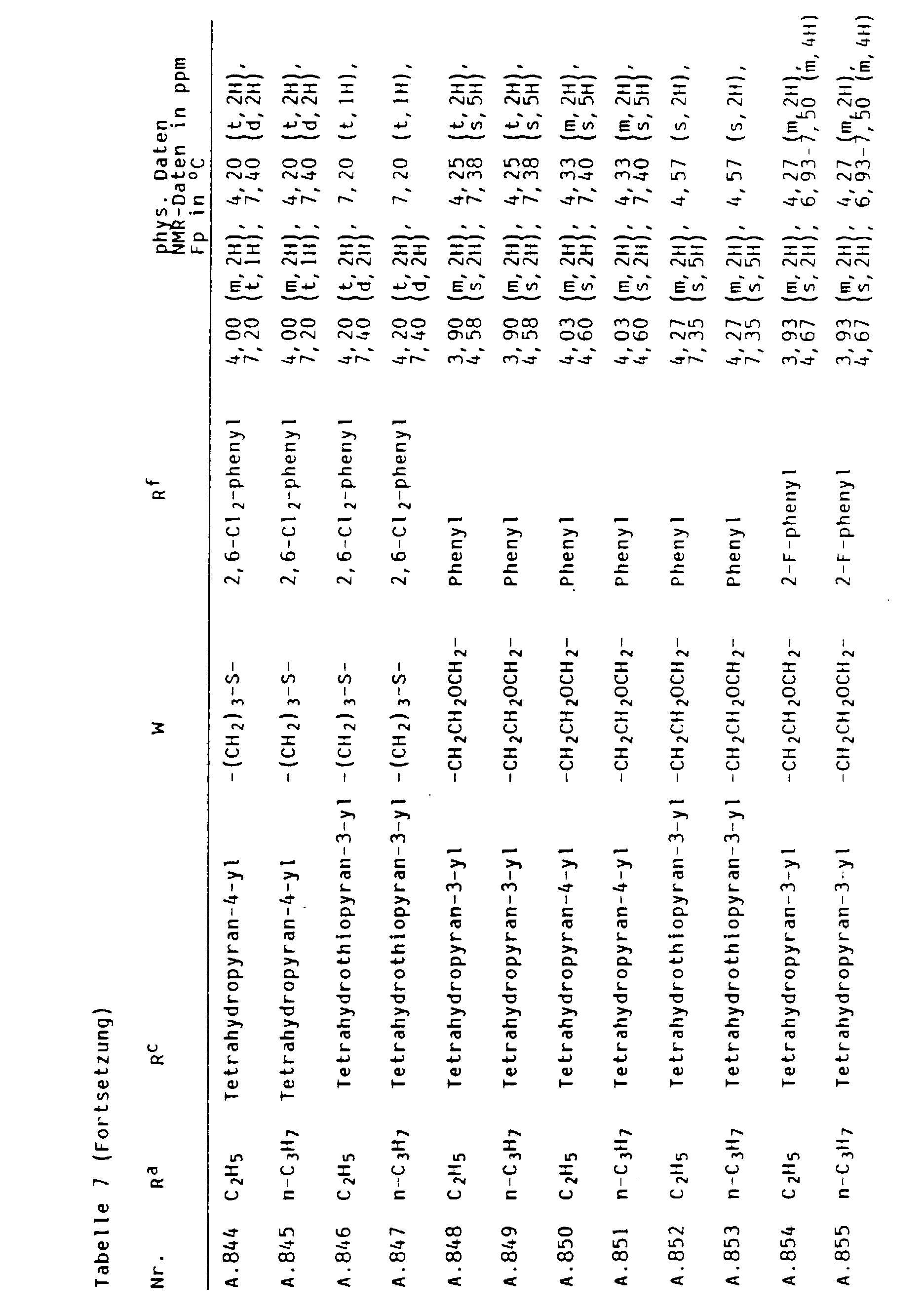

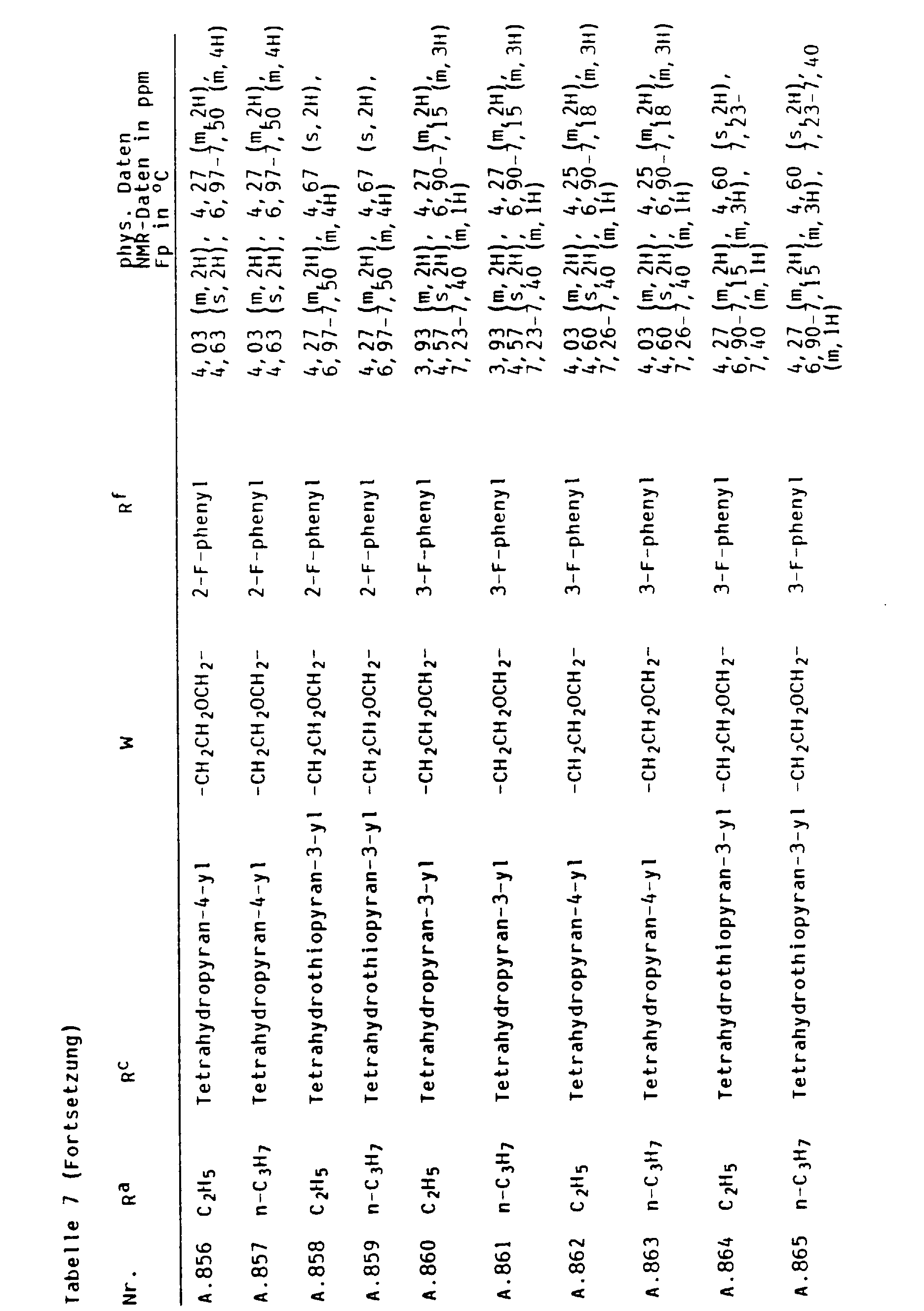

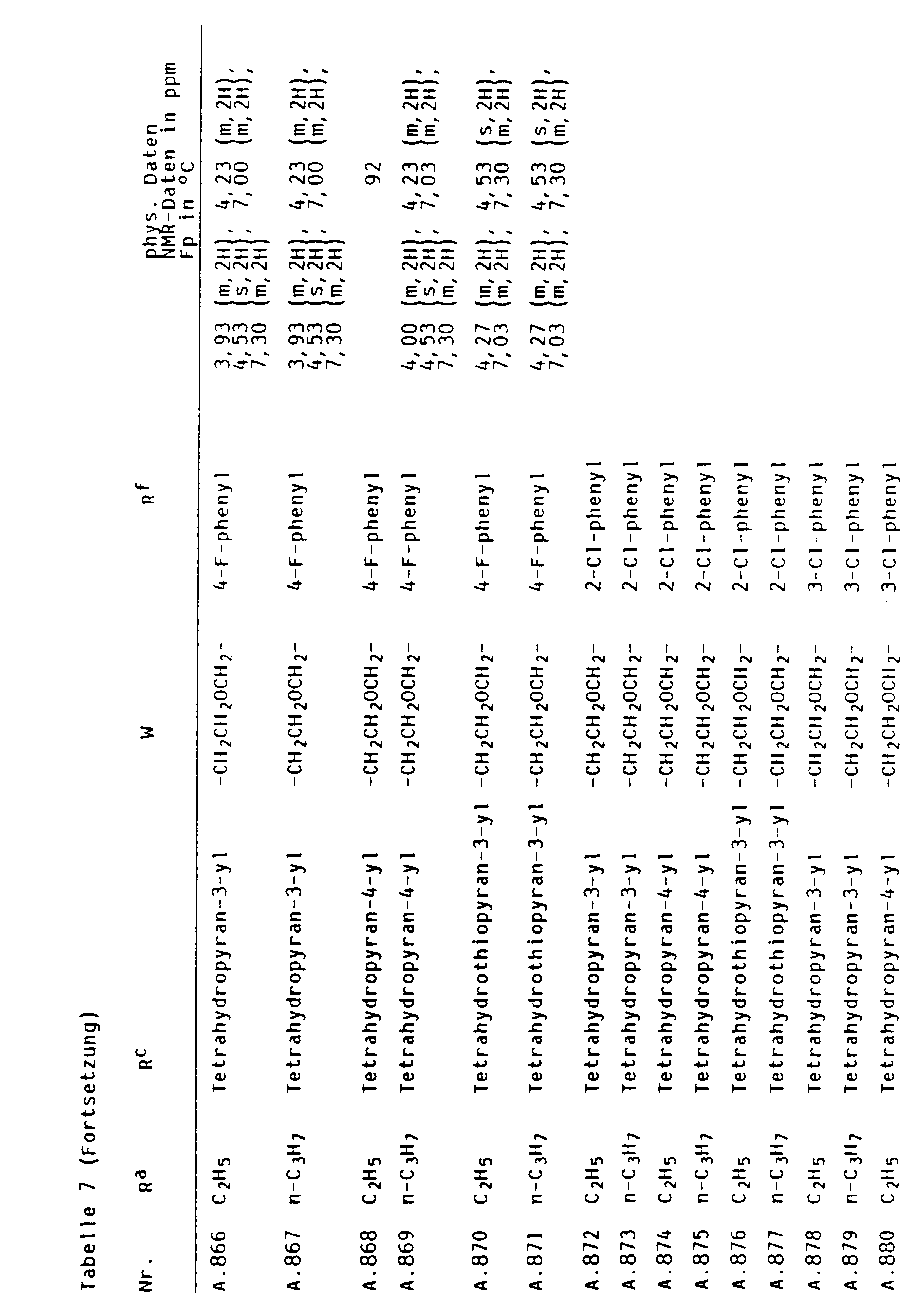

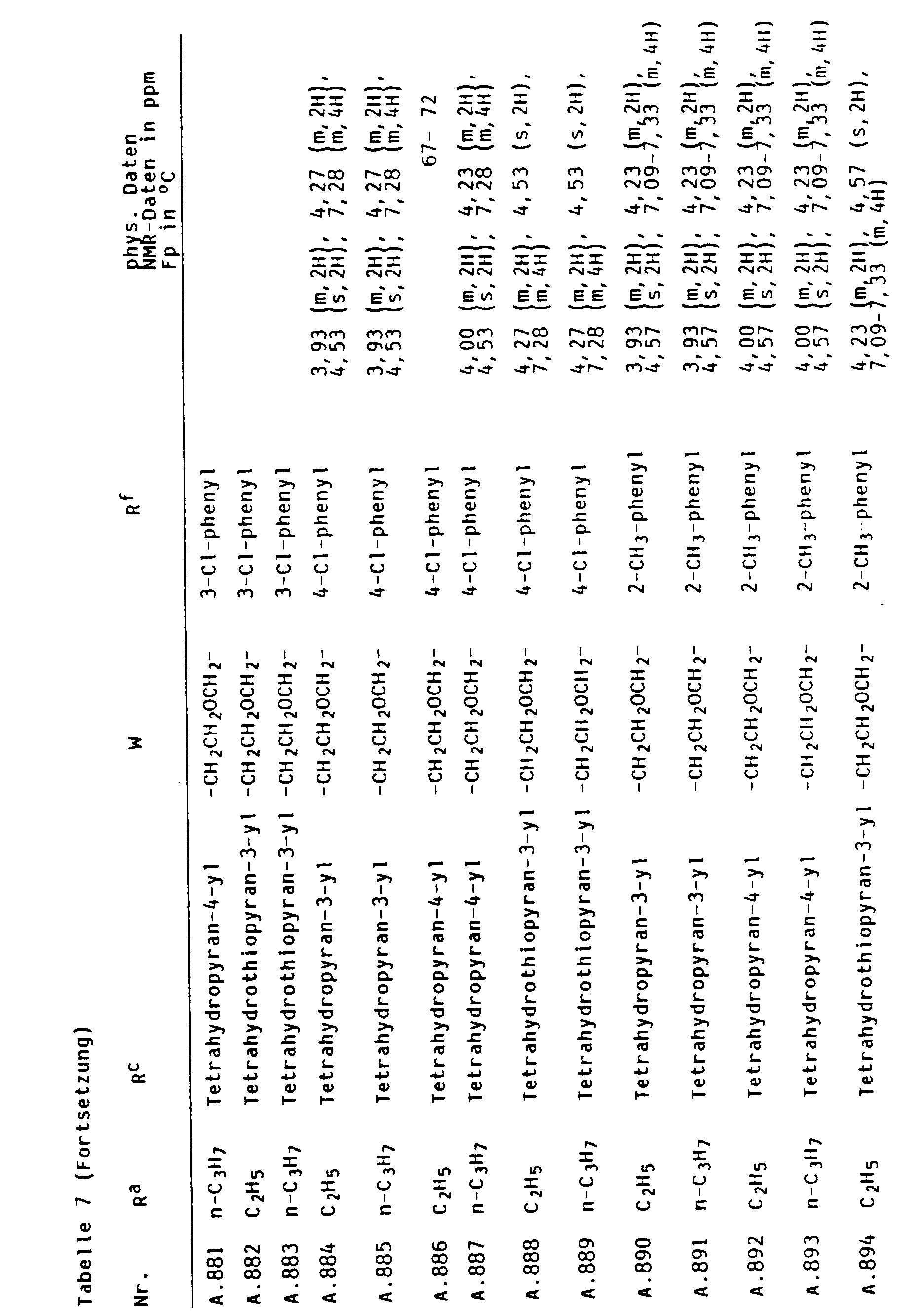

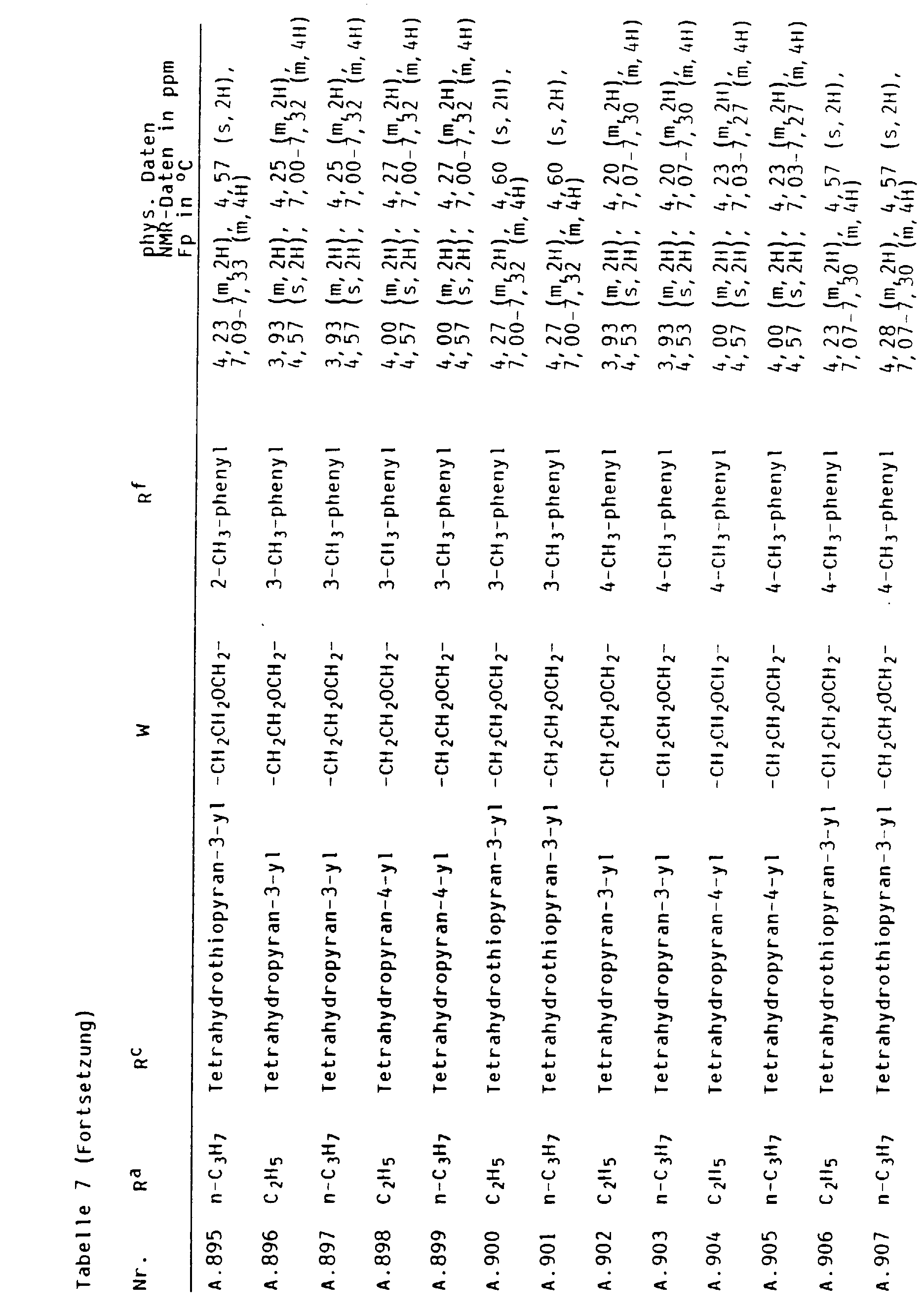

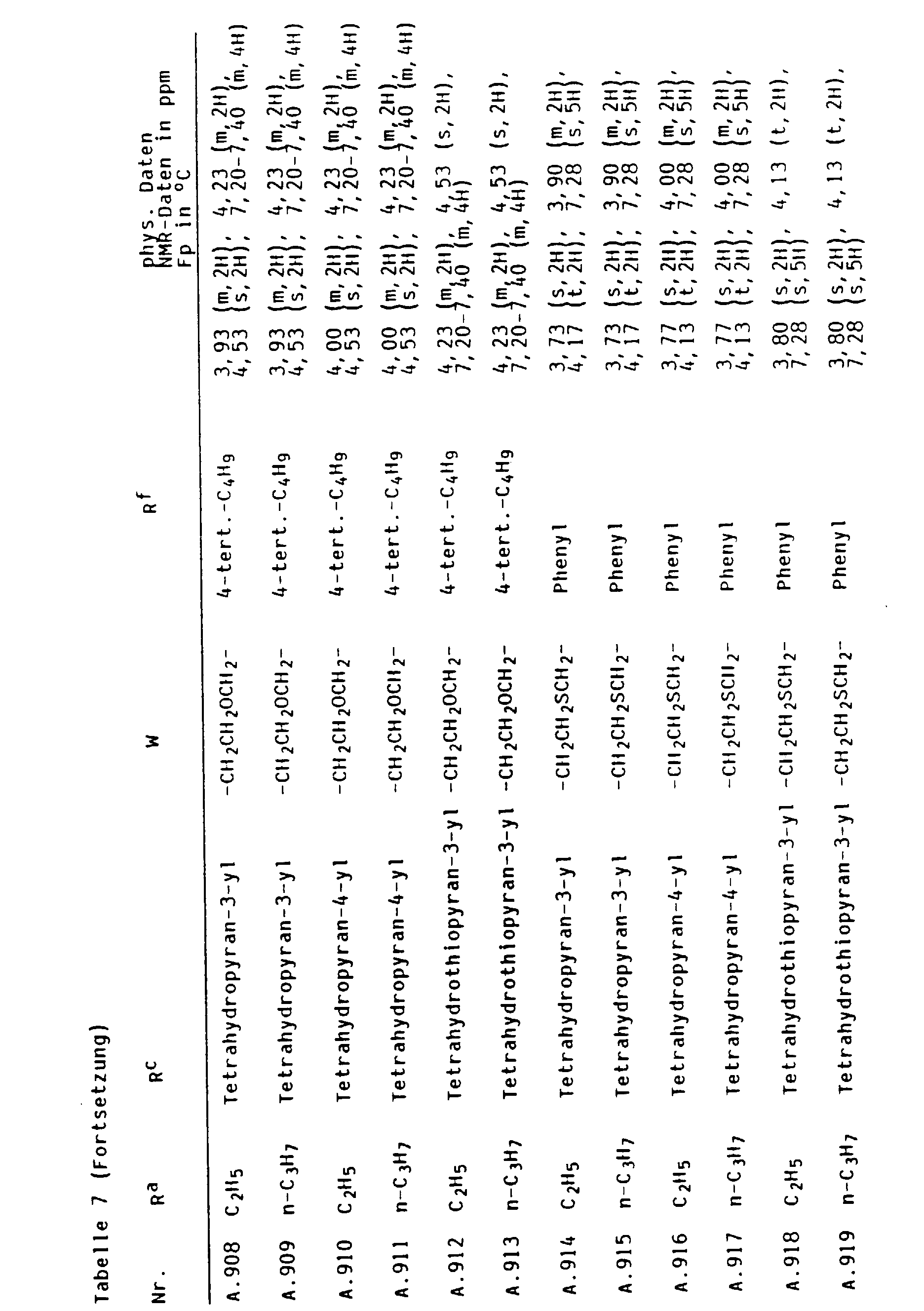

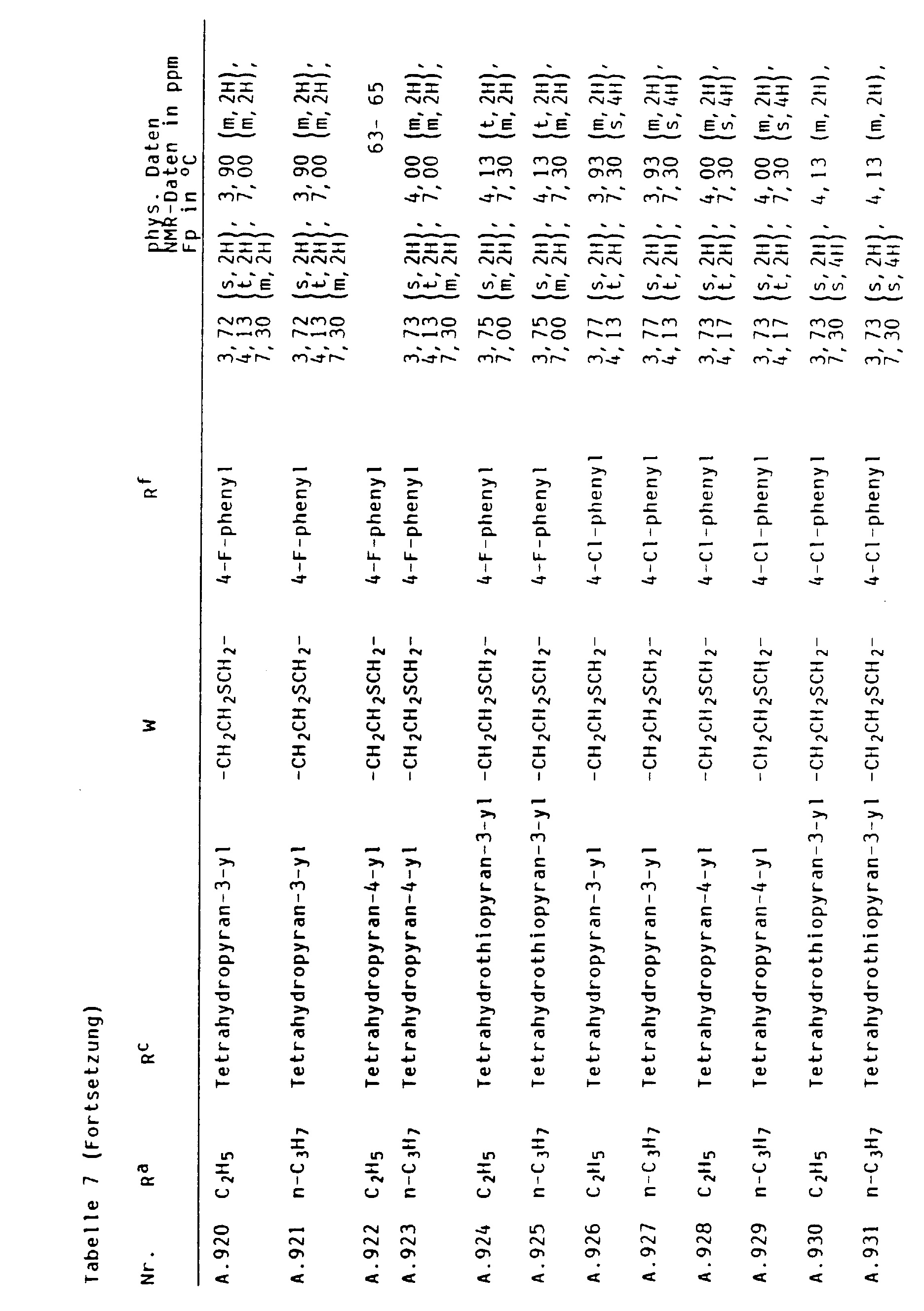

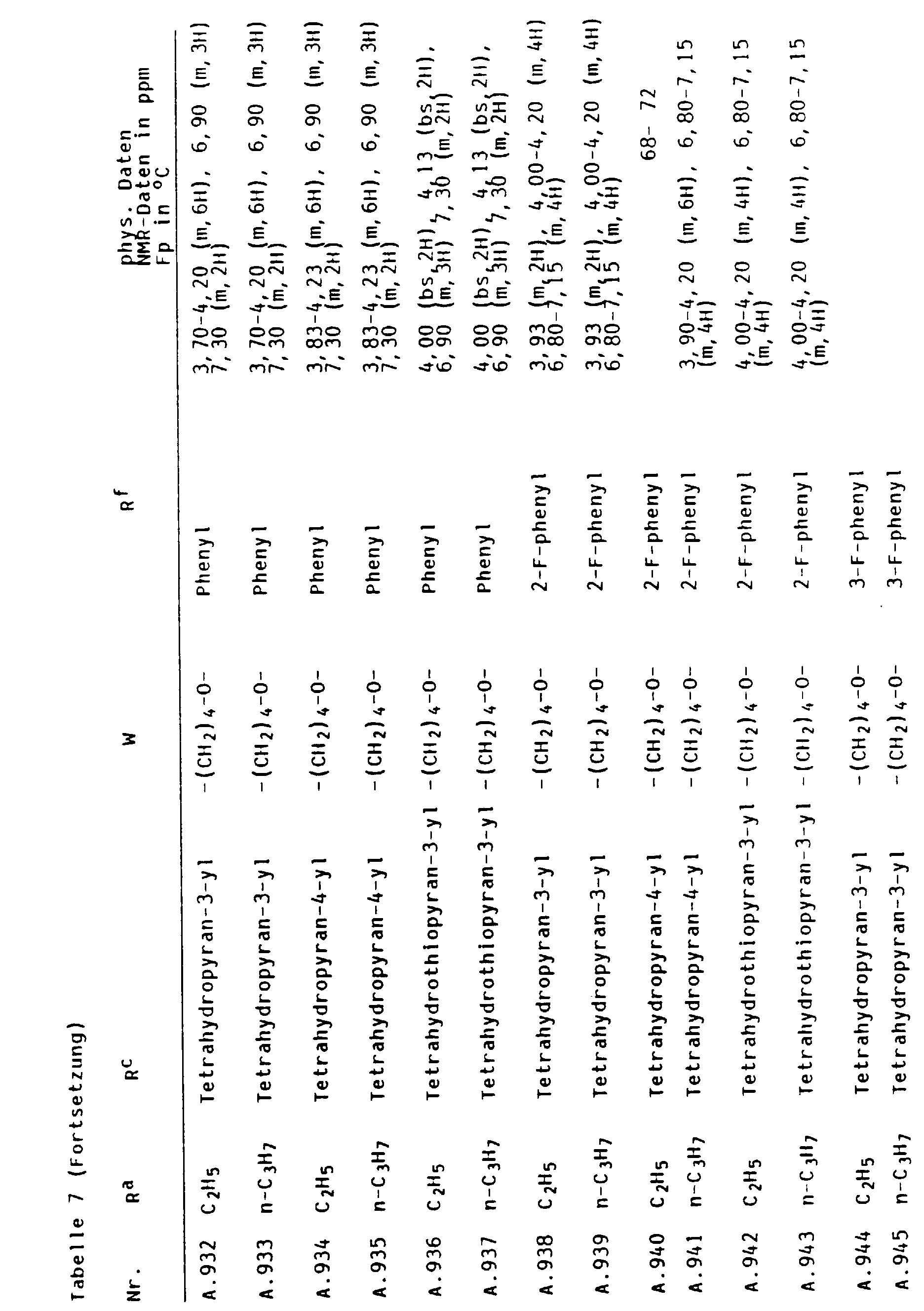

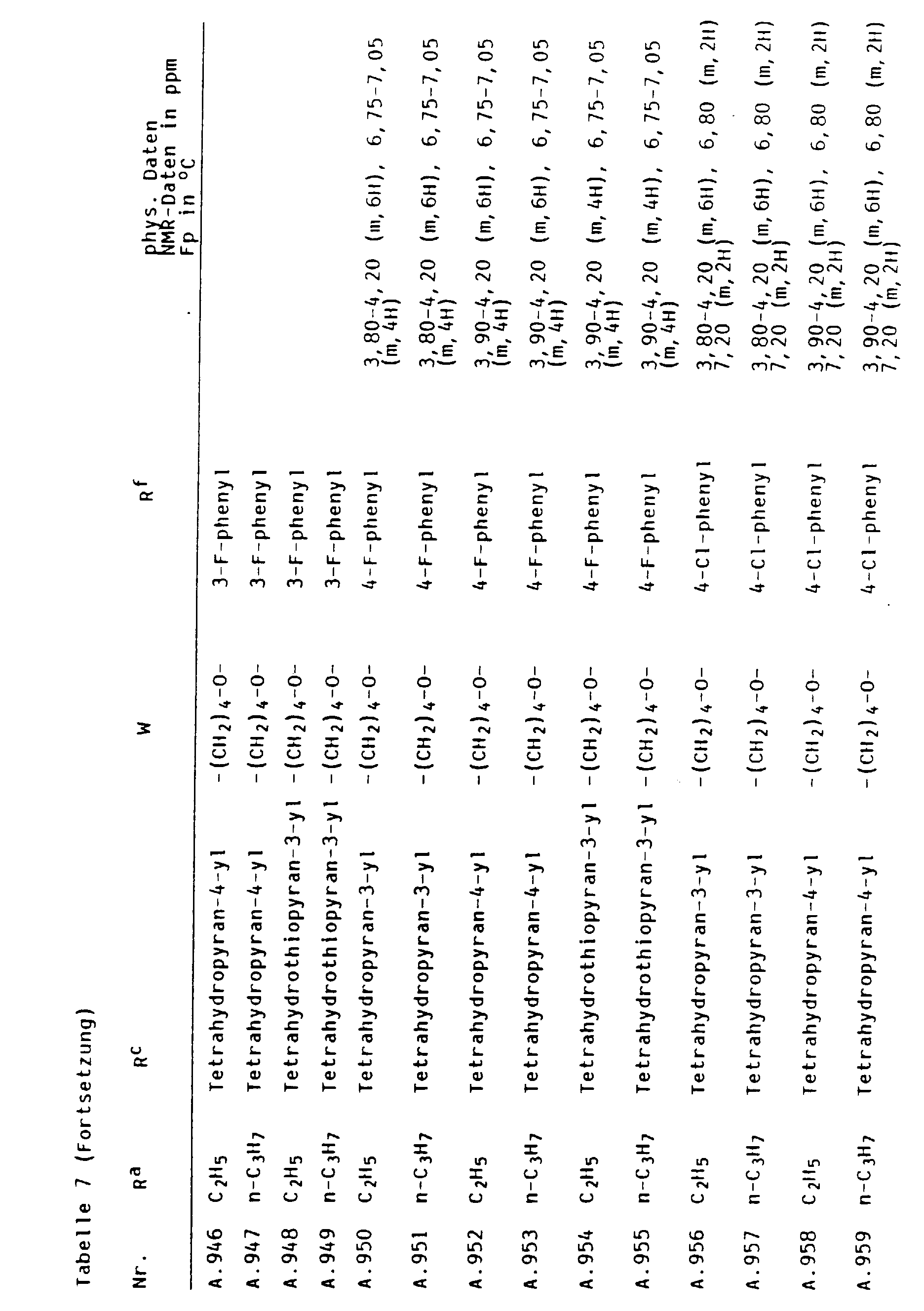

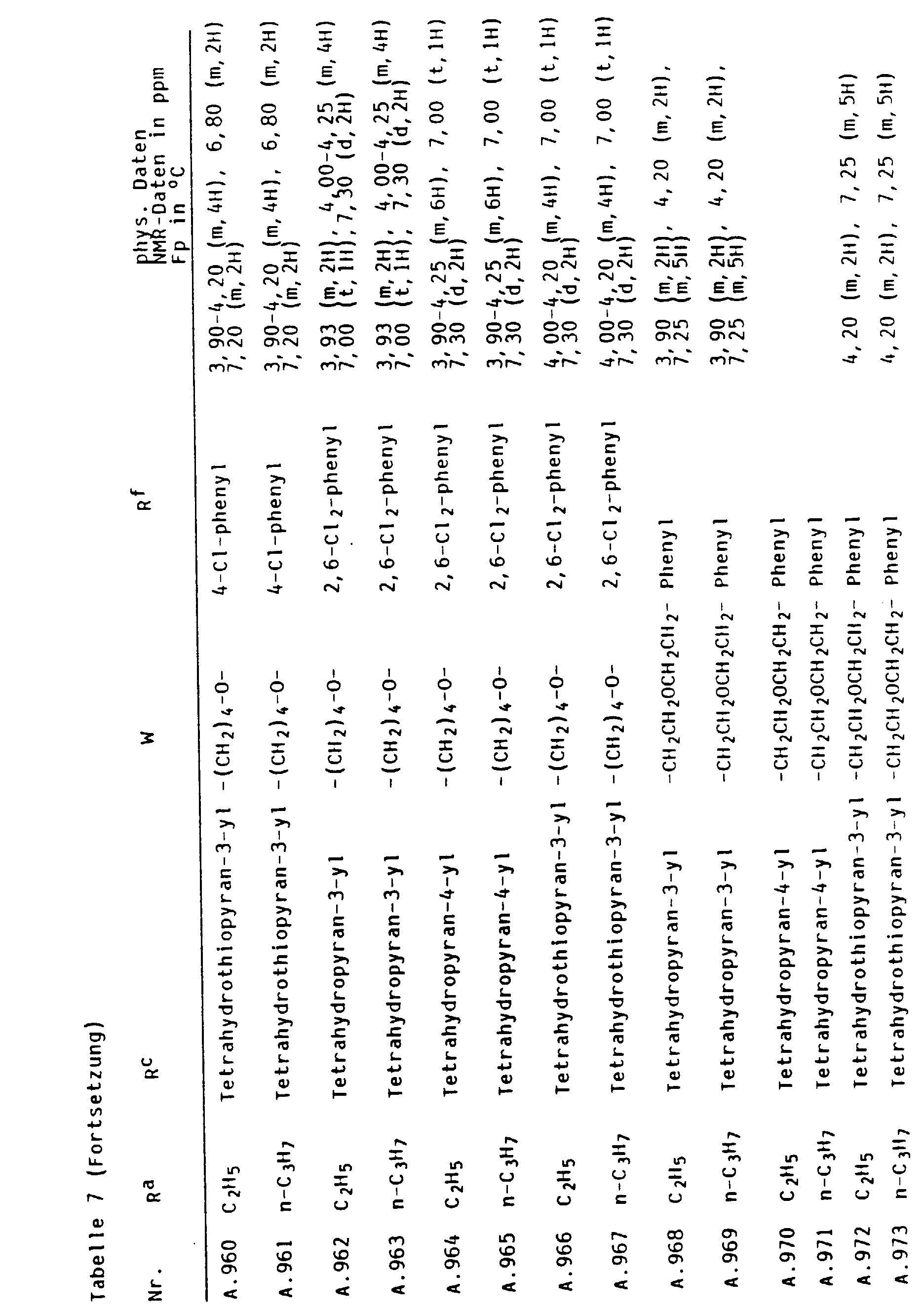

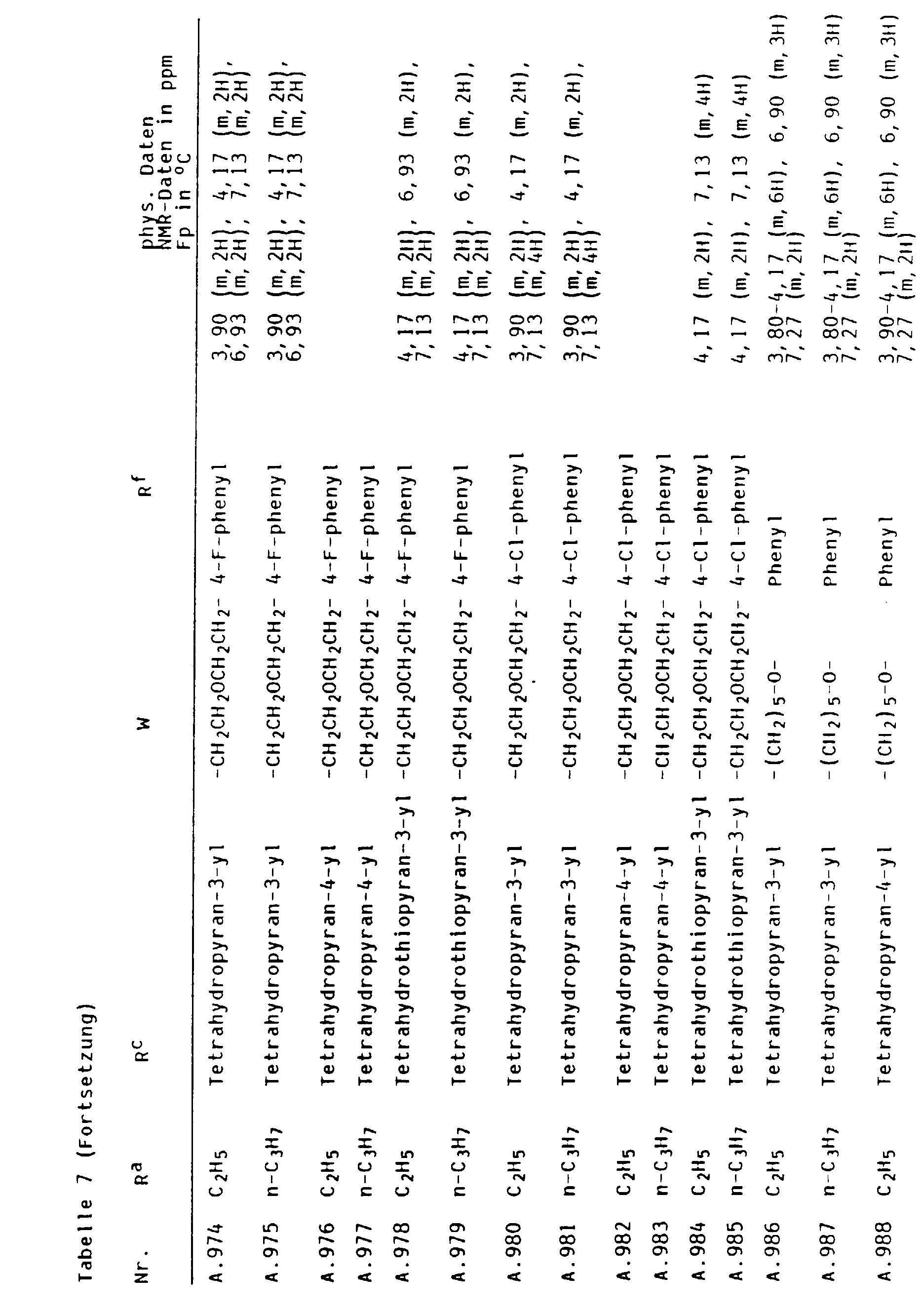

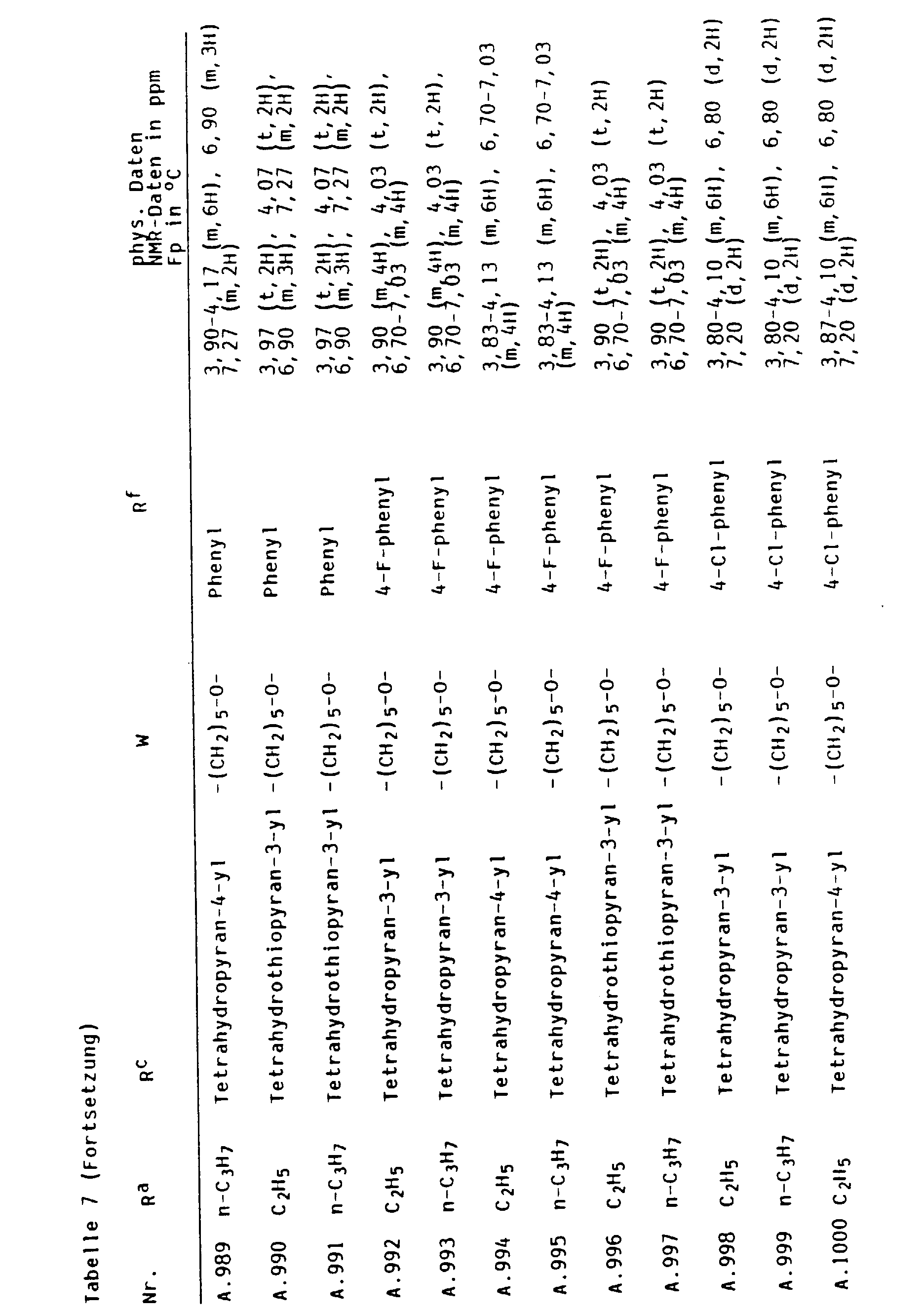

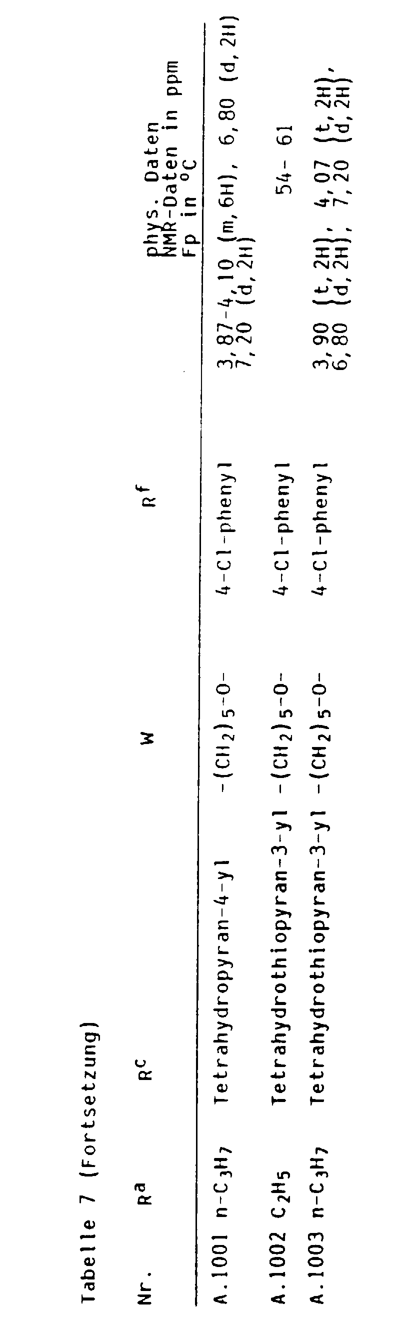

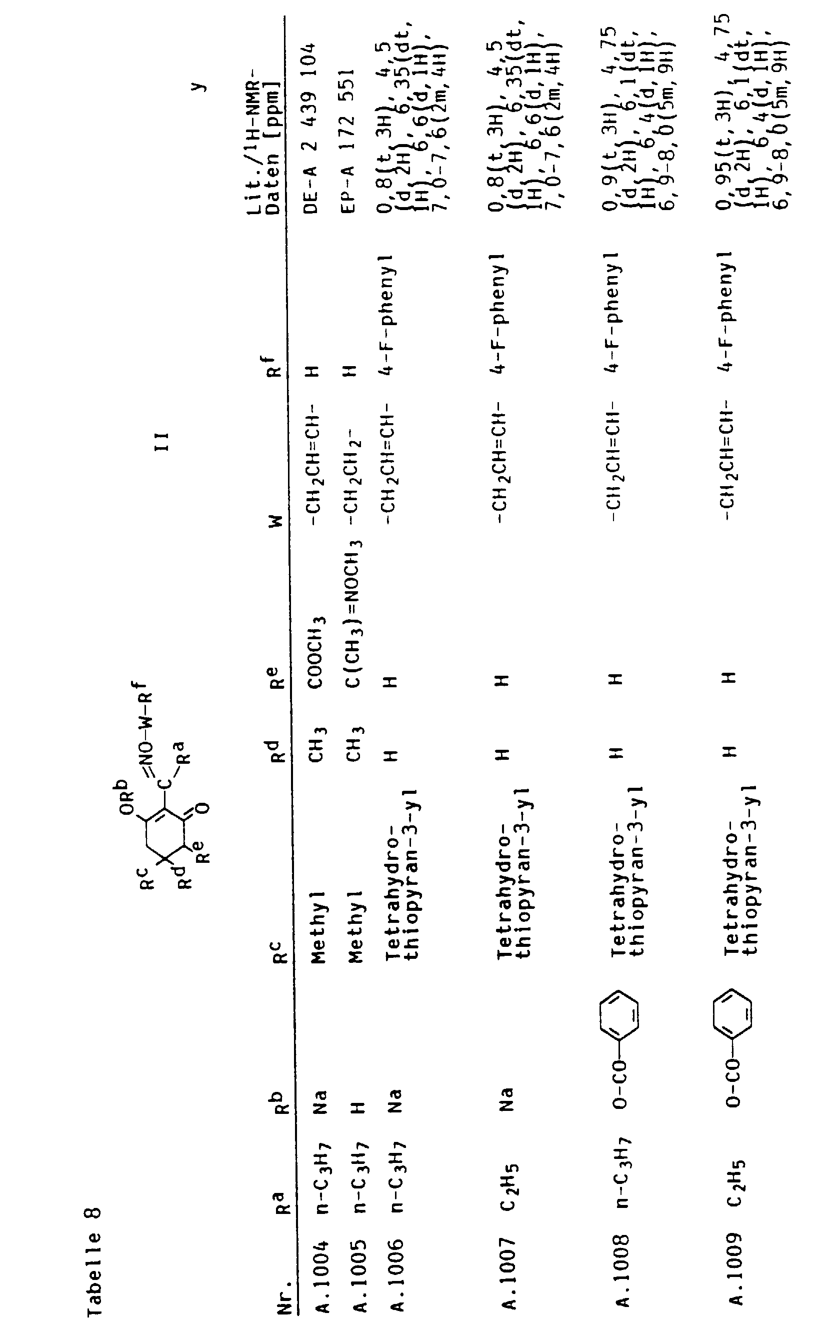

Die herbiziden Wirkstoffe aus der Gruppe der 2-(4-Heteroaryloxy) oder 2-(4-Aryloxy)-phenoxyessigsäurederivate der Formel III 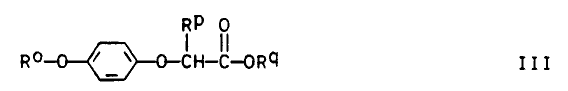 in der die Substituenten im einzelnen die folgende Bedeutung haben:
R^{o}
   die Phenylgruppe, die Pyridylgruppe, eine Benzoxazylgruppe, eine Benzthiazylgruppe oder eine Benzpyrazinylgruppe, wobei diese aromatischen Ringsysteme noch einen bis zwei Reste tragen können, ausgewählt aus einer Gruppe bestehend aus:
   Nitro, Halogen wie Fluor, Chlor, Brom und Jod, insbesondere Fluor und Chlor,
   unverzweigtem oder verzweigtem C₁-C₄-Alkyl wie Methyl, Ethyl, n-Propyl, Isopropyl, n-Butyl und tert.-Butyl, partiell oder vollständig halogeniertem C₁-C₄-Alkyl, insbesondere C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl,
   partiell oder vollständig halogeniertes C₁-C₄-Alkoxy, insbesondere C₁-C₂-Halogenalkoxy wie Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy und Pentafluorethoxy, vorzugsweise Trifluormethoxy;
RP
   Wasserstoff oder Methyl, bevorzugt Methyl,
R^{q}
   Wasserstoff, eine unverzweigte oder verzweigte C₁-C₄-Alkylgruppe wie vorstehend genannt, insbesondere Methyl, Ethyl, n-Propyl und n-Butyl,
   eine C₃-C₄-Alkenylgruppe wie Prop-1-en-1-yl, Prop-2-en-1-yl, But-2-en-1-yl, But-3-en-1-yl, 1-Methyl-prop-2-en-1-yl, 2-Methyl-prop-2-en-1-yl, insbesondere Prop-2-en-1-yl, eine C₃-C₄-Alkinylgruppe wie Prop-1-in-1-yl, Prop-2-in-1-yl, But-2-in-1-yl, But-3-in-1-yl, 1-Methyl-prop-2-in-1-yl, 2-Methyl-prop-2-in-1-yl, insbesondere Prop-2-in-1-yl, eine C₁-C₄-Alkoxy-C₁-C₄-alkylgruppe wie Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, Methoxyethyl, Ethoxyethyl, n-Propoxyethyl, (1-Methylethoxy)ethyl, n-Butoxyethyl, (1-Methylpropoxy)ethyl, (2-Methylpropoxy)ethyl, (1,1-Dimethylethoxy)ethyl, 3-(Methoxy)-propyl, 2-(Methoxy)propyl und 2-(Ethoxy)propyl, vorzugsweise Methoxymethyl und Ethoxyethyl,
   eine C₃-C₄-Alkylideniminooxy-C₂-C₃-alkylgruppe, insbesondere 2-Propylideniminooxy-ethyl,
   eine Tetrahydrofuranylmethylgruppe, eine Isoxazolidingruppe oder das Äquivalent eines pflanzenverträglichen Kations, beispielweise ein Alkalimetallkation wie Natrium oder Kalium, ein Äquivalent eines Erdalkalimetallkations wie Calzium, Magnesium und Barium, Mangan-, Kupfer-, Zink-oder Eisenkationen, Ammoniumkationen mit gewünschtenfalls einem bis drei Substituenten, ausgewählt aus einer Gruppe bestehend aus 3 C₁-C₄-Alkylresten, 3 Hydroxy-C₁-C₄-alkylresten, und einem Phenyl- oder Benzylrest, wie Tetraalkylund Benzyltrialkylammoniumkationen, Phosphoniumkationen, Sulfoniumkationen wie Trialkylsulfoniumkationen oder Sulfoxoniumkationen,

sind aus der Literatur bekannt (vgl. z.B. DE-A 22 23 894, DE-A 24 33 067, DE-A 25 76 251, DE-A 30 04 770, BE-A 868 875 und BE-A 858 618).

Die 2-(4-Heteroaryloxy)- und 2-(4-Aryloxy-phenoxyessigsäurederivate III können ein oder mehrere Asymmetriezentren enthalten. Sie wirken als Racemate, wie sie bei den meisten Herstellungsverfahren anfallen, können gewünschtenfalls aber auch nach den hierfür üblichen Methoden, beispielsweise an einem optisch aktiven Adsorptionsmittel, in die reinen Isomeren aufgetrennt werden.

Sowohl die Racemate als auch die reinen Isomeren dienen zur Bekämpfung von unerwünschten Pflanzen aus der Familie der Gramineen. Die Verträglichkeit dieser Substanzen für Kulturpflanzen variiert jedoch zwischen kommerziell akzeptabel und unverträglich, je nach Substituenten und Aufwandmenge.

Spezielle Beispiele für herbizide 2-(4-Heteroaryloxy)- und 2-(4-Aryloxy)-phenoxyessigsäurederivate der Formel III, deren Kulturpflanzenverträglichkeit durch substituierte 3-Aminobenzo[b]thiophene I verbessert werden kann, sind in der folgenden Tabelle 9 aufgeführt:

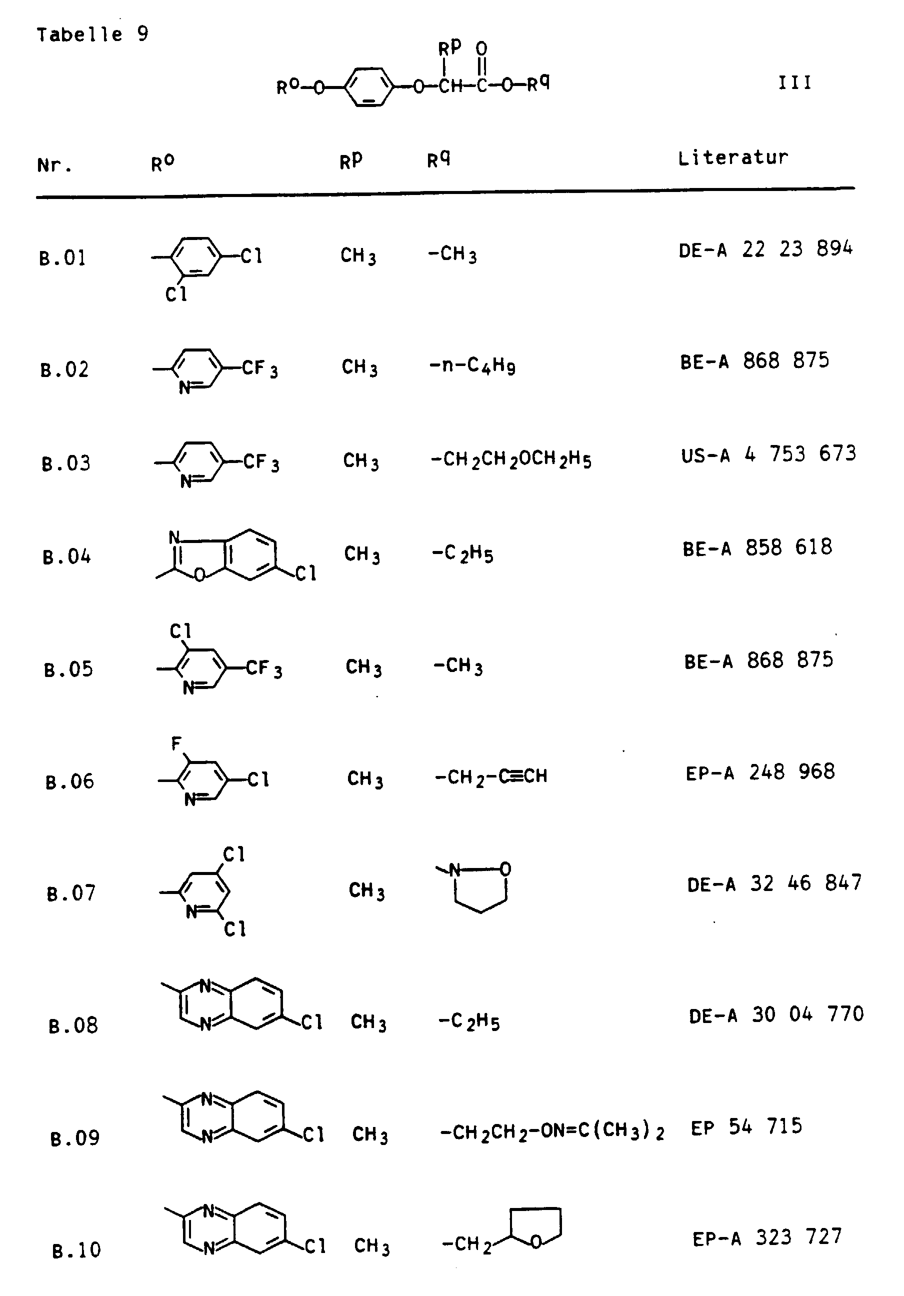

Die herbiziden Wirkstoffe und die antidotisch wirkenden Verbindungen können gemeinsam oder getrennt nach dem Auflaufen auf die Blätter und Sprossen der Kulturpflanzen und unerwünschten Gräser ausgebracht werden. Bevorzugt bringt man jedoch die herbiziden und antidotischen Wirkstoffe gleichzeitig auf das Feld. Bei getrennter Ausbringung von Antidot und herbizidem Wirkstoff wird vorzugsweise das Antidot zuerst ausgebracht.

Der antidotische und der herbizide Wirkstoff können gemeinsam oder getrennt formuliert werden und dann in suspendierbarer, emulgierbarer oder löslicher Form zur Bereitung von Spritzmitteln vorliegen.

Antidotische Effekte werden auch durch Bahandlung der Kulturpflanzensamen oder der Stecklinge mit dem Antidot vor der Aussaat bzw. vor dem Auspflanzen erzielt. Der herbizide Wirkstoff wird dann allein in der üblichen Weise appliziert.

Bei der Saatgutbehandlung werden im allgemeinen Wirkstoffmengen von 0,1 bis 10 g, vorzugsweise 1 bis 2 g, je Kilogramm Saatgut benötigt.

Bei der Applikation des Antidots durch Samenquellung oder bei der Stecklingsbehandlung werden bevorzugt Lösungen eingesetzt, die den antagonistischen Wirkstoff in einer Konzentration von 1 bis 10'000 ppm, insbesondere von 100 bis 10'000 ppm, enthalten.

In den verschiedenen Pflanzenkulturen benötigt man üblicherweise unterschiedliche Mengen an antidotisch wirksamer Verbindung I und herbizider Verbindung II oder III, wobei die Mengenverhältnisse in breiten Bereichen variabel sind. Sie sind abhängig von der Struktur der Cyclohexenon-Derivate II bzw. der Heteroaryloxy- und Aryloxyphenoxyessigsäurederivate III, der substituierten 3-Aminobenzo[b]thiophene I und der jeweiligen Pflanzenkultur, auf die die Verbindungen ausgebracht werden. Geeignete Anteilsverhältnisse von herbizidem Wirkstoff zu antidotisch wirksamen substituierten 3-Aminobenzo[b]thiophen I liegen zwischen 1:10 und 1:0,01, vorzugsweise zwischen 1:4 bis 1:0,1.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentigen wäßrigen, öligen oder sonstigen Suspensionen, Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsform richtet sich hierbei ganz nach dem jeweiligen Verwendungszweck.

Zur Herstellung von direkt versprühbaren Lösungen, Emulsionen, Pasten und Öldispersionen kommen Mineralölfraktionen von mittlerem bis hohem Siedepunkt, wie Kerosin und Dieselöl, ferner Kohlenteeröle, sowie Öle und Fette pflanzlichen oder tierischen Ursprungs, aliphatische, cyclische oder aromatische Kohlenwasserstoffe, beispielsweise Methanol, Ethanol, Isopropanol, Butanol, Chloroform, Tetrachlorkohlenstoff, Cyclohexanol, Cyclohexanon, Chlorbenol, Toluol, Xylole, Paraffin, Tetrahydronaphthalin, alkylierte Naphthaline oder deren Derivate oder Isophoron, sowie stark polare Lösungsmittel wie Dimethylformamid, Dimethylsulfoxid, N-Methylpyrrolidon und vorzugsweise Wasser, in Betracht.

Wäßrige Anwendungsformen können aus Emulsionskonzentraten, Pasten, netzbaren Pulvern (Spritzpulvern) oder Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel mit Wasser homogenisiert werden. Es können aber auch aus herbizidem Wirkstoff und/oder Antidot Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und gewünschtenfalls Lösungsmittel oder Öl bestehende Konzentrate hergestellt werden, die zur Verdünnung mit Wasser geeignet sind.

Als oberflächenaktive Salze kommen Alkalimetall-, Erdalkalimetall-, Ammoniumsalze von Ligninsulfonsäure, Naphthalinsulfonsäure, Phenolsulfonsäure, Alkylarylsulfonate, Alkylsulfate, Alkylsulfonate, Alkalimetall- und Erdalkalimetallsalze der Dibutylnaphthalinsulfonsäure, Laurylethersulfat, Fettalkoholsulfate, fettsaure Alkalimetall- und Erdalkalimetallsalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctyl-phenolether, ethoxylierte Isooctylphenol, Octylphenol oder Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver, Streu- und Stäubemittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogenisierungsgranulate, können durch Bindung der Wirkstoffe an feste Trägerstoffe hergestellt werden. Feste Trägerstoffe sind z.B. Mineralerden wie Kieselsäuren, Kieselgele, Silikate, Talkum, Kaolin, Attaclay, Kalkstein, Kreide, Talkum, Bolus, Lös, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe, und pflanzliche Produkte wie Getreidemehl, Baumrinden-, Holz- und Nußschalenmehl, Cellulosepulver und andere feste Trägerstoffe.

Die Formulierungen enthalten 0,02 bis 95 Gew.%, vorzugsweise 0,5 bis 90 Gew.% an herbizidem Wirkstoff und Antidot. Die Aufwandmengen an herbizidem Wirkstoff betragen 0,05 bis 5 kg/ha.

Die herbiziden Mittel können neben den antagonistisch wirksamen substituierten 3-Aminobenzo[b]thiophenen I und dem Herbizid aus der Gruppe der Cyclohexenone II oder der (Heteroaryloxy)- bzw. Aryloxyphenoxyessigsäuren III weitere herbizide oder wachstumsregulierende Wirkstoffe anderer chemischer Struktur enthalten, wobei der antagonistische Effekt der 3-Aminobenzo[b]thiophene erhalten bleibt.

### Herstellungsbeispiele (3-Aminobenzo[b]thiophene I und I')

### Beispiel 5

### 3,5-Diamino-4-cyan-2-acylbenzo[b]thiophen

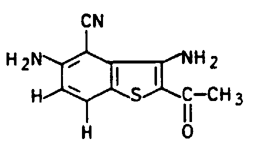

Zu einer Lösung von 0,1 mol 5-Amino-4-cyanobenzisothiazol in 300 ml Methoxypropanol wurden 0,1 mol Natriummethanolat (als 30 gew.-%igen Lösung in Methanol) gegeben. Man rührte 1 Std. bei 100°C und kühlte danach auf 50°C ab. Nach Zugabe von 0,1 mol Chloraceton und weiteren 0,1 mol Natriummethanolat (als 30 gew.-%ige Lösung in Methanol) wurde die Mischung noch 2,5 Std. auf 100°C erhitzt und anschließend in 3000 ml Wasser eingerührt. Der gebildete Niederschlag wurde abgetrennt, mit Wasser gewaschen und getrocknet. Ausbeute: 80%; Fp.: >250°C.
Verwendet man statt Chloraceton eine andere Carbonylverbindung, so erhält man auf die gleiche Weise, ausgehend von 5-Amino-4-cyanobenzisothiazol, weitere 3,5-Diamino-4-cyano-2-benzo[b]-thiophen-Derivate. In Tabelle 10 sind beispielhaft entsprechende Carbonylverbindungen und die daraus erhaltenen neuen Verbindungen I' aufgeführt:

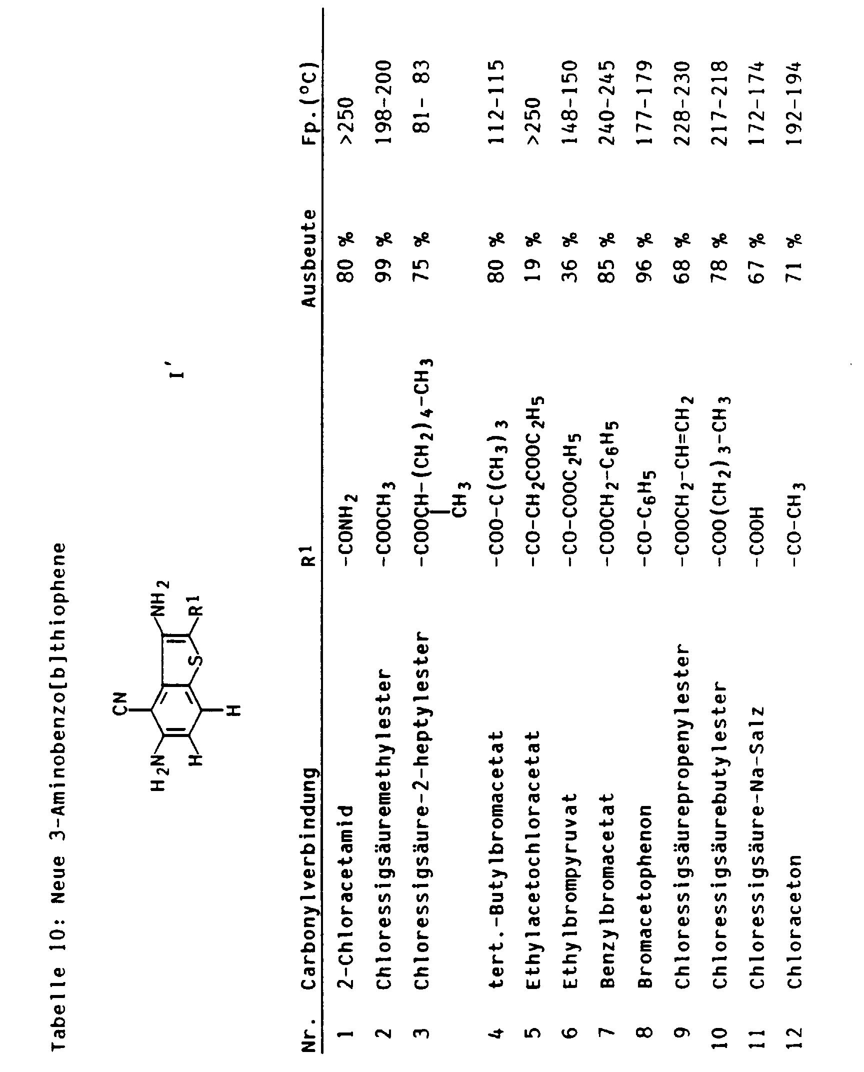

### Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:

Bei Gewächshausversuchen dienten als Kultur gefäße Plastiktöpfe mit rund 300 cm3 Inhalt und lehmigem Sand mit etwa 3,0 Gew.-% Humus als Substrat. Die Samen der Testpflanzen wurden, nach Arten getrennt, flach eingesät und befeuchtet. Danach wurden die Gefäße mit durchsichtigen Plastikhauben abgedeckt, bis die Samen gleichmäßig gekeimt und die Pflanzen angewachsen waren.

| Liste der Testpflanzen | | |
|---|---|---|
| Lateinischer Name | Deutscher Name | Englischer Name |
| Triticum acstivum | Winterweizen | winter wheat |
| Triticum acstivum | Sommerweizen | spring wheat |
| Zea mays | Mais | corn |
| Setaria viridis | Grüne Borstenhirse | green foxtail |

Für die Nachauflaufbehandlung wurden die Testpflanzen je nach Wuchsform erst bis zu einer Wuchshöhe von 3 bis 20 cm angezüchtet und erst dann behandelt. Die herbiziden Mittel wurden hierbei in Wasser als Verteilungsmittel suspendiert oder emulgiert und mittels fein verteilender Düsen gespritzt.

Als Beispielherbizide der Cyclohexenon-Derivate II dienten

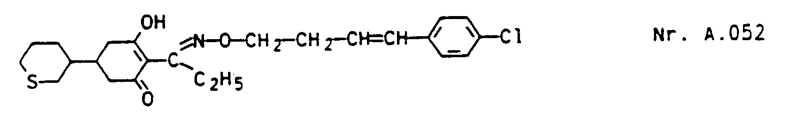

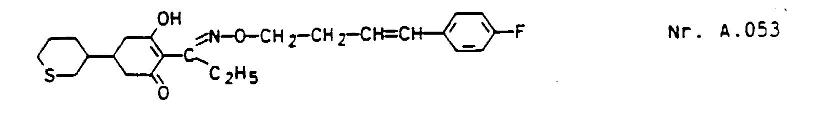

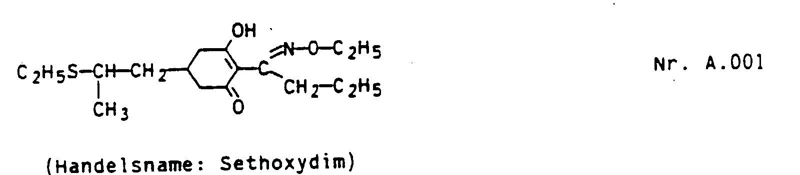

Sämtliche antidotisch wirkenden Verbindungen wurden für die Nachauflaufbehandlung in einem Gemisch, bestehend aus 80 Gew.-% Cyclohexanon als Verdünnungsmittel und 20 Gew.-% Tensid (Emulphor® EL; ethoxyliertes Rizinusöl (castor oil)) mit 10 Gew.-% Wirkstoff aufbereitet.

Zum Vergleich wurde der herbizide Wirkstoff als 10 bis 20 gew.-%iges Emulsionskonzentrat formuliert und jeweils unter Zugabe von derjenigen Menge an Lösungsmittelsystem in die Spritzbrühe eingesetzt, mit welcher die antidotisch wirkende Verbindung in den angegebenen Aufwandmengen ausgebracht wurde. Die Herstellung der Lösung erfolgte durch Einmischen des Wirkstoffs in eine Lösung aus 93 Gew.-% Xylol und 7 Gew.-% Lutensol® AP-8 (nichtionisches oberflächenaktives Mittel auf Basis von Alkylphenolpolyethylenglykolethern).

Nach Applikation der jeweiligen Wirkstoffmischung wurden die Testpflanzen im Gewächshaus kultiviert, und zwar wärmeliebende Arten bei etwa 18 bis 30°C, solche gemäßigterer Klimate bei ca. 10 bis 25°C.

Die Versuchsperiode erstreckte sich über 3 bis 5 Wochen. Während dieser Zeit wurden die Pflanzen gepflegt, wobei ihre Reaktionen auf die wirkstoff-Behandlungen erfaßt wurden.

Bewertet wurde die Schädigung durch die chemischen Mittel anhand einer Skala von 0 bis 100 % im Vergleich zu den unbehandelten Kontrollpflanzen. Dabei bedeutet 0 keine Schädigung und 100 eine völlige Zerstörung der Pflanzen.

Die Verbesserung der Verträglichkeit von herbiziden Cyclohexenon-Derivaten II für Kulturpflanzen aus der Familie der Gramineen (Gräser) wie Weizen und Mais durch die 3-Aminobenzo[b]thiophene I ist den folgenden Tabellen 11 bis 15 zu entnehmen:

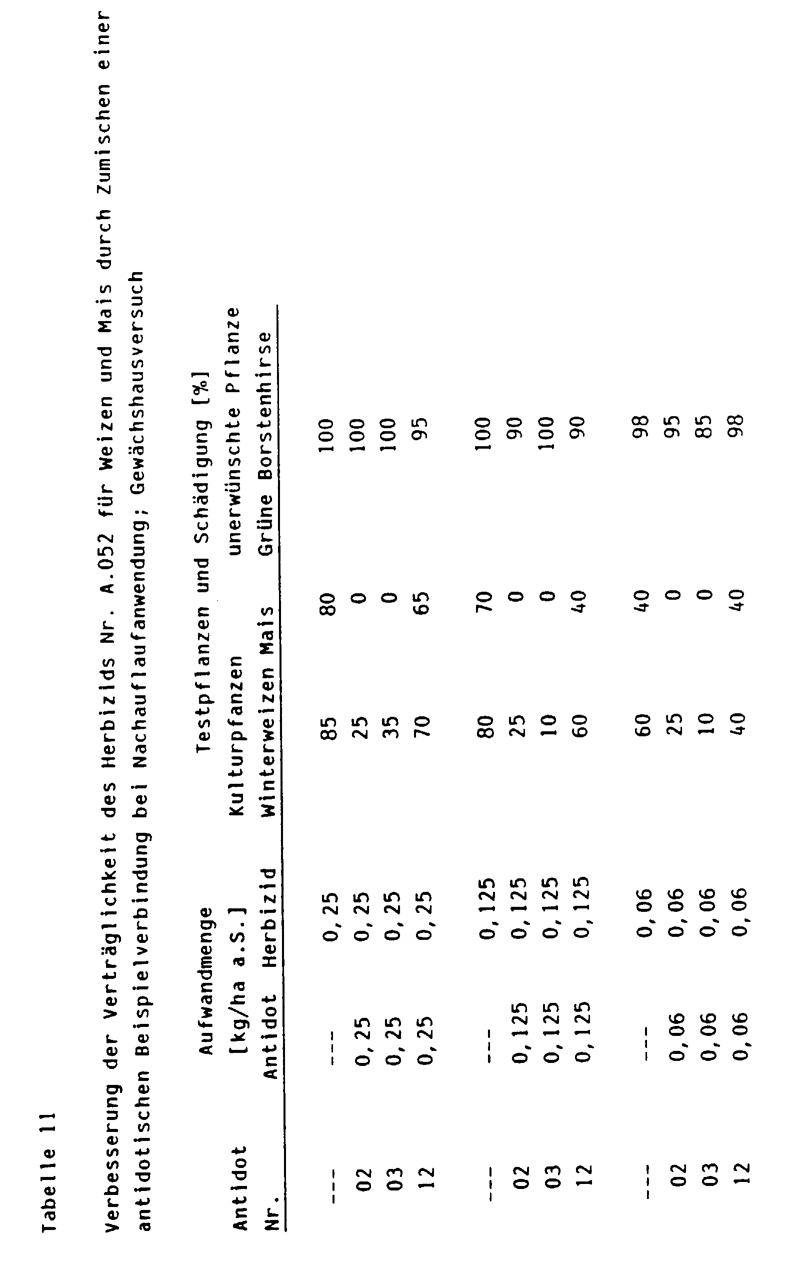

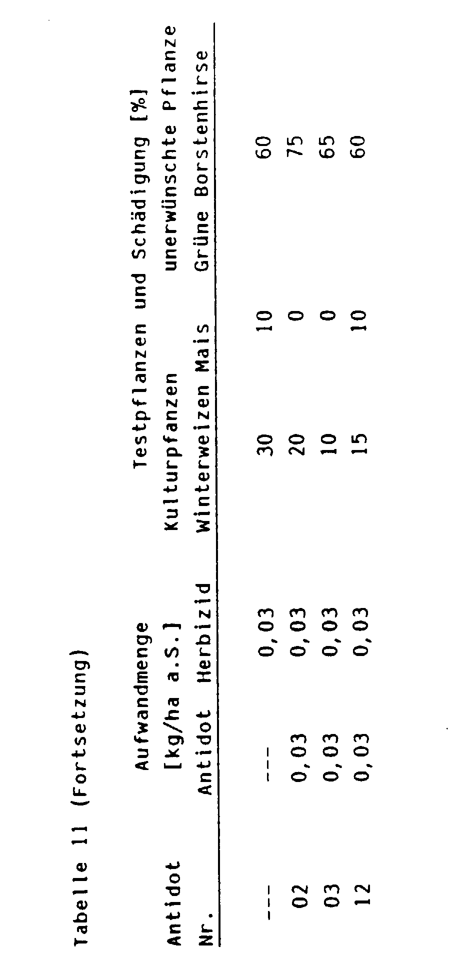

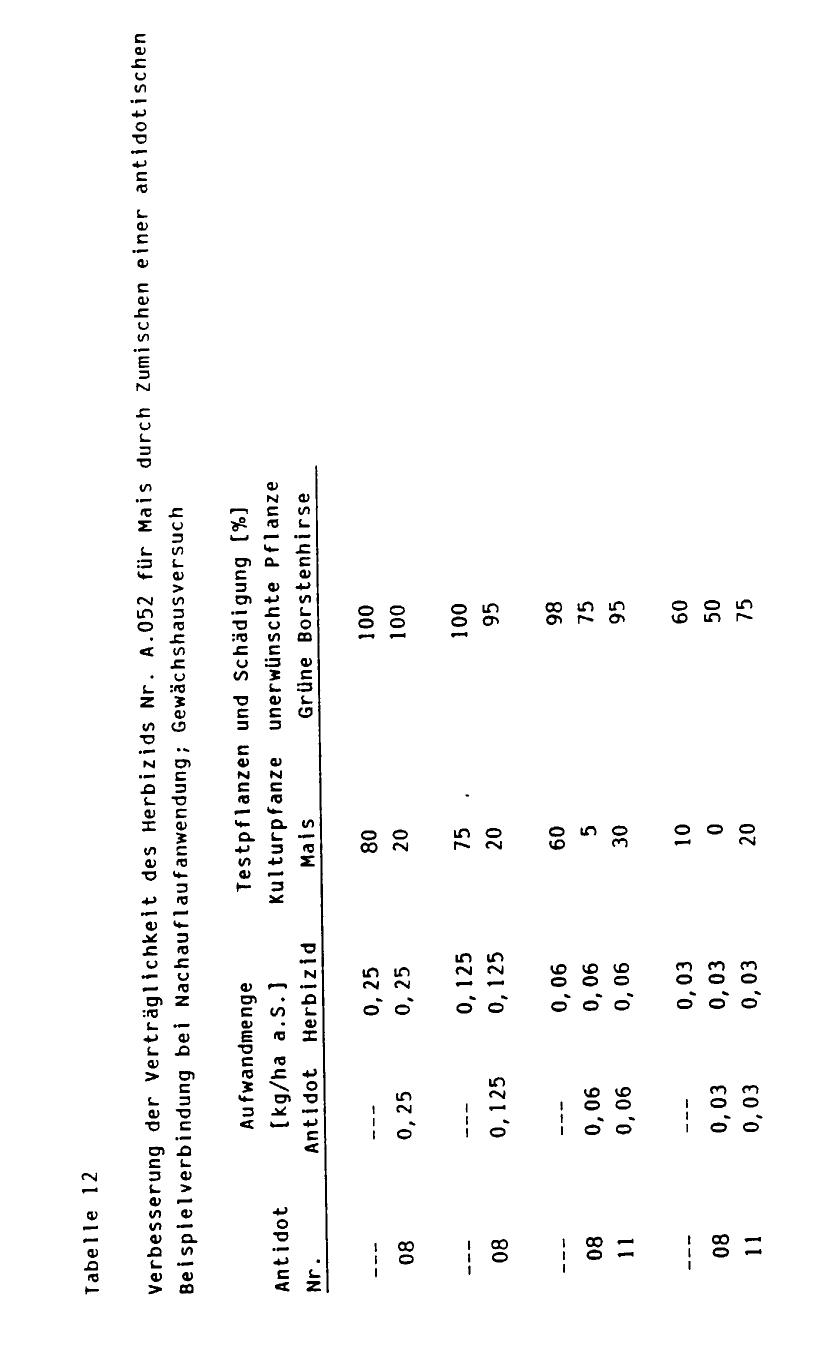

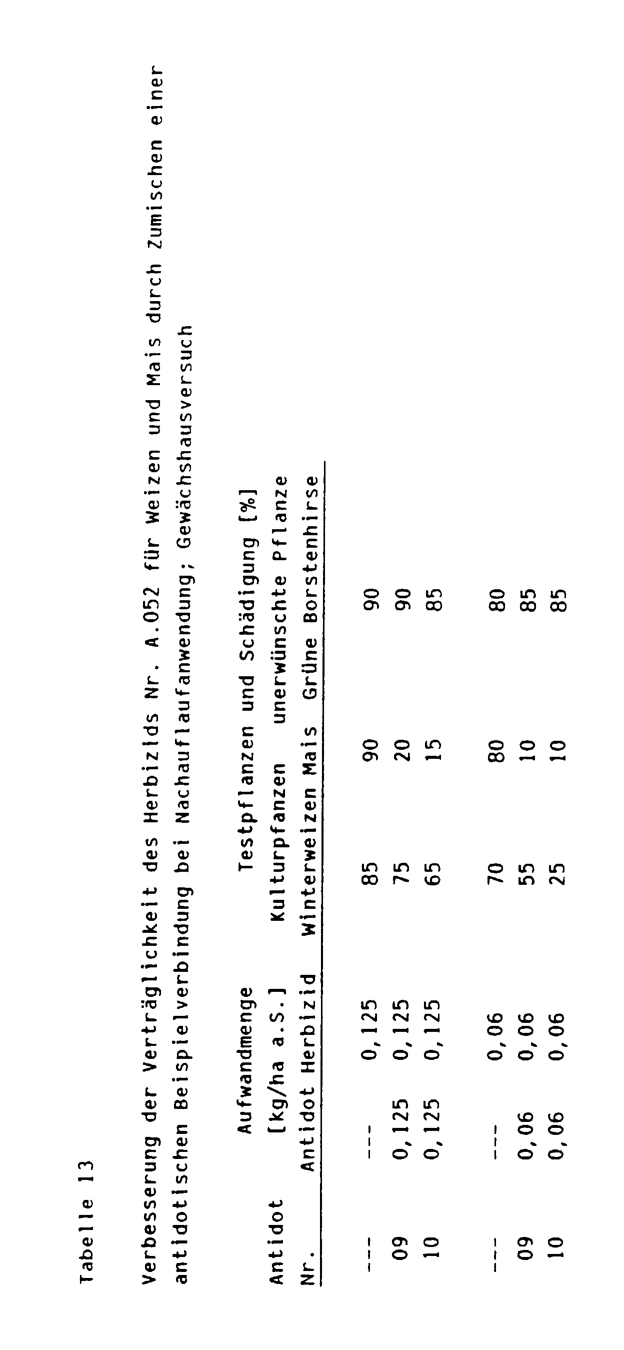

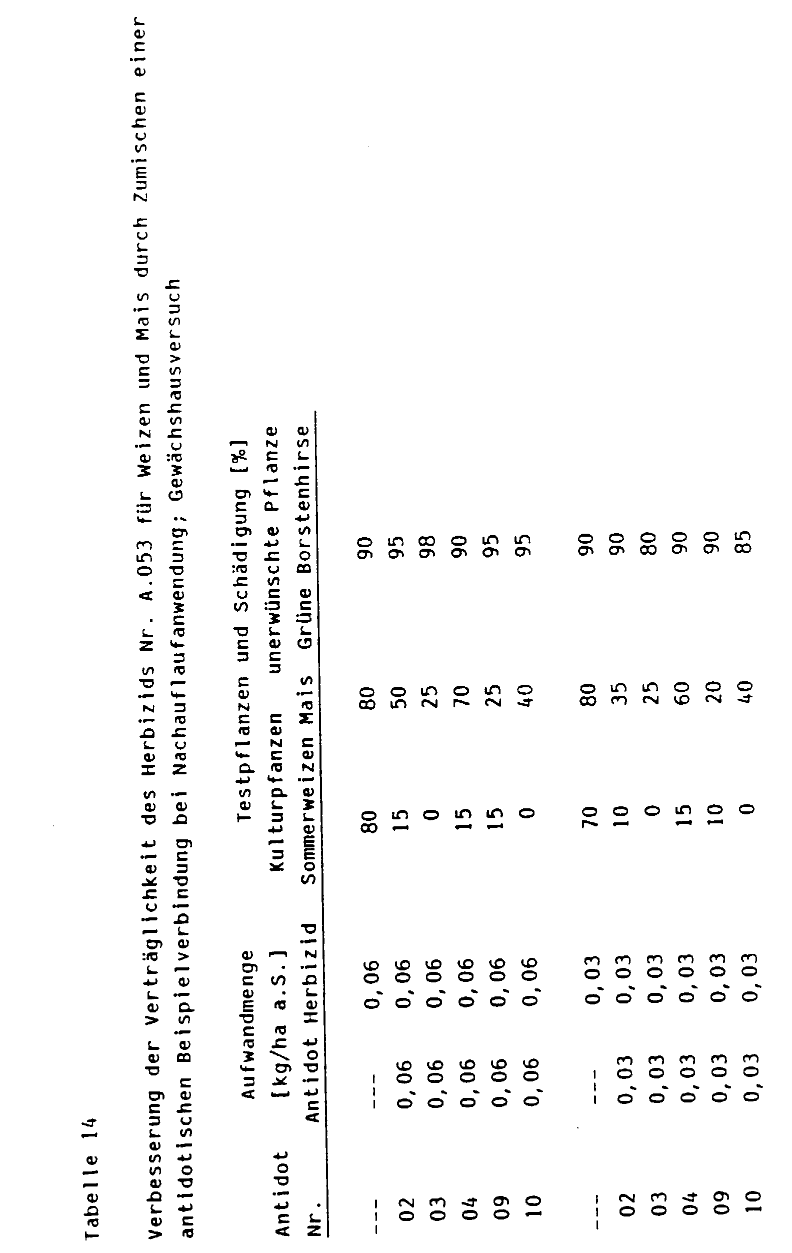

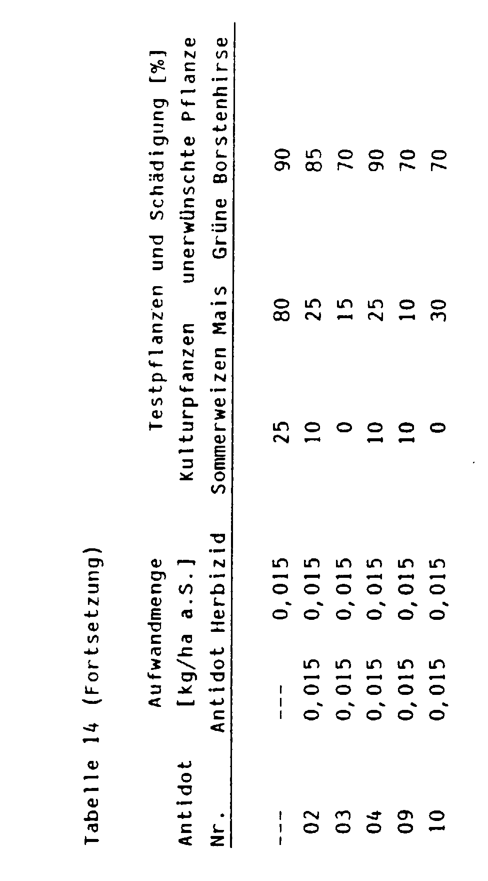

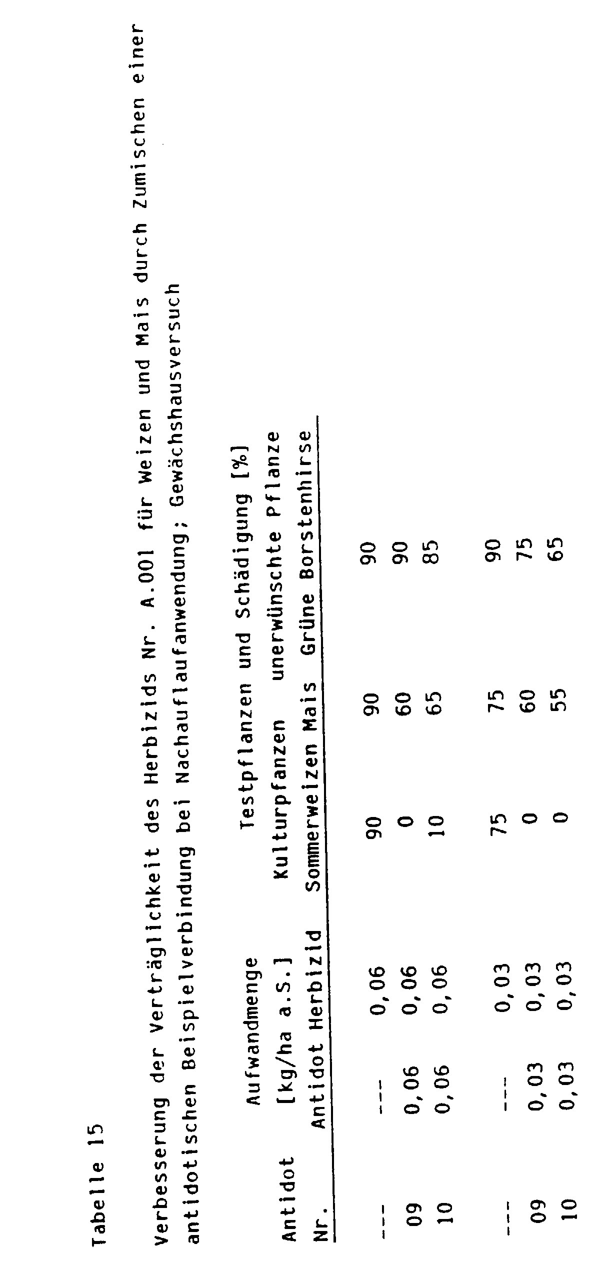

## Patentansprüche

1. Herbizide Mittel, enthaltend mindestens ein antagonistisch wirksames 3-Aminobenzo[b]thiophen der allgemeinen Formel I 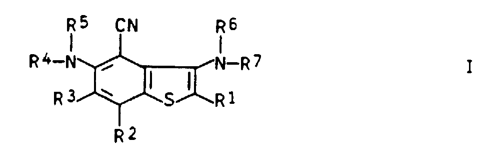 in der die Variablen folgende Bedeutung haben:
R¹ eine Gruppe -COX oder -COOX, wobei
X Wasserstoff, Halogen, die Aminogruppe, die unsubstituiert ist oder ein bis drei Substituenten trägt, ausgewählt aus einer Gruppe bestehend aus C₁-C₄-Alkyl-, Hydroxy-C₁-C₄-alkyl-, Phenyl-, und Benzylsubstituenten,
eine C₁-C₁₀-Alkylgruppe, eine C₂-C₈-Alkenylgruppe, eine C₃-C₁₀-Cycloalkylgruppe oder eine C₃-C₁₀-Cycloalkenylgruppe, wobei die letztgenannten vier Gruppen unsubstituiert oder partiell oder vollständig halogeniert sein können und wobei die letztgenannten vier Gruppen auch eine oder zwei weitere Carboxyl- oder Carbonylgruppen enthalten können,
einen 5- oder 6-gliedrigen aromatischen Heterocyclus mit einem Sauerstoff- und einem Schwefelatom oder mit 1 bis 3 Heteroatomen, ausgewählt aus einer Gruppe bestehend aus 1 bis 3 Stickstoffatomen und einem Sauerstoff- oder Schwefelatom, wobei nicht drei Heteroatome gleichzeitig benachbart sein können,
die Phenyl- oder die Naphthylgruppe, wobei diese Gruppen unsubstituiert sind oder einen bis drei Substituenten tragen, ausgewählt aus der Gruppe bestehend aus Halogen, -COY oder -COOY, und wobei diese Gruppen noch zusätzlich so viele Halogenatome tragen können, wie substituierbare C-Atome vorhanden sind;
Y steht für C₁-C₆-Alkyl, C₃-C₆-Cycloalkyl oder Phenyl;
R², R³ Wasserstoff, Cyano, Nitro, Halogen, Mercapto, die Aminogruppe, deren Stickstoffatom ein bis drei Substituenten tragen kann, ausgewählt aus einer Gruppe bestehend aus C₁-C₄-Alkyl-, Hydroxy-C₁-C₄-alkyl-, Phenyl-, und Benzylsubstituenten,
eine C₁-C₁₀-Alkylgruppe, eine C₁-C₆-Alkoxygruppe, eine C₂-C₈-Alkenylgruppe, eine C₃-C₁₀-Cycloalkylgruppe oder eine C₃-C₁₀-Cycloalkenylgruppe, wobei die letztgenannten vier Gruppen partiell oder vollständig halogeniert sein können, die Phenyl- oder die Naphthylgruppe,
einen 5- oder 6-gliedrigen aromatischen Heterocyclus mit einem Sauerstoff- und einem Schwefelatom oder mit einem bis drei Heteroatomen, ausgewählt aus einer Gruppe bestehend aus drei Stickstoffatomen und einem Sauerstoff- oder Schwefelatom, wobei im Falle von drei Heteroatomen nicht alle gleichzeitig benachbart sein können;
R⁴ bis R⁷ Wasserstoff, die Phenylgruppe, die Naphthylgruppe oder einen 5- oder 6-gliedrigen aromatischen Heterocyclus mit einem Sauerstoff- und einem Schwefelatom oder mit einem bis drei Heteroatomen, ausgewählt aus einer Gruppe bestehend aus drei Stickstoffatomen und einem Sauerstoff- oder Schwefelatom, wobei im Falle von drei Heteroatomen nicht alle gleichzeitig benachbart sein können;
wobei die aromatischen und heteroaromatischen Gruppen noch einen bis drei Substituenten tragen können, ausgewählt aus einer Gruppe bestehend aus Halogen, -COY oder -COOY, und wobei diese Gruppen noch zusätzlich so viele Halogenatome tragen können, wie substituierbare C-Atome vorhanden sind, eine C₁-C₁₀-Alkylgruppe, eine C₂-C₆-Alkenylgruppe, eine C₃-C₁₀-Cycloalkylgruppe, eine C₃-C₁₀-Cycloalkenylgruppe ;
oder R⁴ und R⁵ und/oder R⁶ und R⁷ bilden zusammen mit dem Stickstoffatom, an das sie gebunden sind, einen 5- bis 7-gliedrigen Ring;
sowie die basischen Salze derjenigen Verbindung I, die mindestens eine Carboxyl-, Hydroxythiocarbonyl- oder Sulfonsäuregruppe tragen und die sauren Salze derjenigen Verbindungen I, die ein basisches Stickstoffatom enthalten,
und mindestens einen herbiziden Wirkstoff aus
A) der Gruppe der Cyclohexenon-Derivate der allgemeinen Formel II 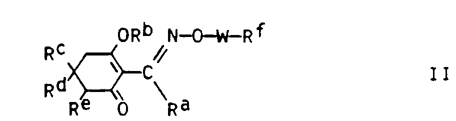 in der die Substituenten folgende Bedeutung haben:
R^{a}
eine C₁-C₆-Alkylgruppe;
R^{b}
Wasserstoff, das Äquivalent eines landwirtschaftlich brauchbaren Kations, eine C₂-C₈-Alkylcarbonylgruppe, eine C₁-C₁₀-Alkylsulfonylgruppe, eine C₁-C₁₀-Alkylphosphonylgruppe oder die Benzoyl-, Benzolsulfonyl- oder Benzolphosphonylgruppe, wobei die drei letztgenannten Gruppen noch je 1 bis 5 Halogenatome tragen können;
R^{c}
Wasserstoff, die Cyanogruppe, die Formylgruppe, eine C₁-C₆-Alkylgruppe, eine C₁-C₄-Alkoxy-C₁-C₆-alkyl- oder C₁-C₄-Alkylthio-C₁-C₆-alkylgruppe, eine Phenoxy-C₁-C₆-alkyl-, Phenylthio-C₁-C₆-alkyl-, Pyridyloxy-C₁-C₆-alkyl- oder Pyridylthio-C₁-C₆-alkylgruppe, wobei die Phenylund Pyridylringe jeweils noch einen bis drei Reste tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Alkenyl, C₃-C₆-Alkenyloxy, C₃-C₆-Alkinyl, C₃-C₆-Alkinyloxy und -NR^{g}R^{h},
wobei
R^{g} Wasserstoff, C₁-C₄-Alkyl, C₃-C₆-Alkenyl, C₃-C₆-Alkinyl, C₁-C₆-Acyl oder Benzoyl, das einen bis drei Reste tragen kann, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy und C₁-C₄-Alkylthio und
R^{h} Wasserstoff, C₁-C₄-Alkyl, C₃-C₆-Alkenyl oder C₃-C₆-Alkinyl
bedeuten;
eine C₃-C₇-Cycloalkyl- oder eine C₅-C₇-Cycloalkenylgruppe, wobei diese Gruppen noch einen bis drei Reste tragen können, ausgewählt aus einer Gruppe bestehend aus Hydroxyl, Halogen, C₁-C₄-Alkyl, partiell oder vollständig halogeniertem C₁-C₄-Alkyl, C₁-C₄-Alkoxy, C₁-C₄-Alkylthio, Benzylthio, C₁-C₄-Alkylsulfonyl, C₁-C₄-Alkylsulfenyl und C₁-C₄-Alkylsulfinyl,
ein 5-gliedriger gesättigter Heterocyclus, der ein oder zwei Sauerstoff- oder Schwefelatome oder ein Sauerstoff- und ein Schwefelatom als Heteroatome enthält, und der noch einen bis drei Reste tragen kann, ausgewählt aus einer Gruppe bestehend aus C₁-C₄-Alkyl, partiell oder vollständig halogeniertem C₁-C₄-Alkyl, C₁-C₄-Alkoxy und C₁-C₄-Alkylthio,
ein 6- oder 7-gliedriger gesättigter oder ein- oder zweifach ungesättigter Heterocyclus, der ein oder zwei Sauerstoffoder Schwefelatome oder ein Sauerstoff und ein Schwefelatom als Heteroatome enthält, und der noch einen bis drei Reste tragen kann, ausgewählt aus einer Gruppe bestehend aus Hydroxyl, Halogen, C₁-C₄-Alkyl, partiell oder vollständig halogeniertem C₁-C₄-Alkyl, C₁-C₄-Alkoxy, C₁-C₄-Alkylthio,
ein 5-gliedriger Heteroaromat, enthaltend ein bis drei Heteroatome, ausgewählt aus einer Gruppe bestehend aus ein oder zwei Stickstoffatomen und einem Sauerstoff- oder Schwefelatom, wobei der Heteroaromat noch einen bis drei Reste tragen kann, ausgewählt aus einer Gruppe bestehend aus Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₂-C₆-Alkenyl, C₂-C₆-Alkenyloxy, C₃-C₆-Alkinyloxy und C₁-C₄-Alkoxy-C₁-C₄-alkyl,
eine Phenyl- oder Pyridylgruppe, die jeweils noch einen bis drei Reste tragen kann, ausgewählt aus einer Gruppe bestehend aus Nitro, Cyano, Formyl, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Alkenyl,
C₃-C₆-Alkenyloxy, C₃-C₆-Alkinyl, C₃-C₆-Alkinyloxy und -NR^{k}R^{l}, wobei R^{k} und R^{l} die oben genannte Bedeutung haben;
R^{d}
Wasserstoff, die Hydroxylgruppe oder, wenn R^{c} für eine C₁-C₆-Alkylgruppe steht, eine C₁-C₆-Alkylgruppe;
R^{e}
Wasserstoff, Halogen, die Cyanogruppe, eine C₁-C₄-Alkoxycarbonylgruppe oder eine C₁-C₄-Alkylketoximgruppe;
W
eine C₁-C₆-Alkylen-, C₃-C₆-Alkenylen- oder C₃-C₆-Alkinylenkette, die jeweils noch ein bis drei Reste tragen können, ausgewählt aus einer Gruppe bestehend aus drei C₁-C₃-Alkylsubstituenten, drei Halogenatomen und einem Methylensubstituenten;
eine C₃-C₆-Alkylen- oder C₄-C₆-Alkenylenkette, die beide noch eine bis drei C₁-C₃-Alkylreste tragen können, wobei jeweils eine Methylengruppe der Ketten durch ein Sauerstoffoder Schwefelatom, eine Sulfoxid-oder Sulfongruppe oder eine Gruppe -N(Rⁱ)- substituiert sein kann, wobei
Rⁱ für Wasserstoff, C₁-C₄-Alkyl, C₃-C₆-Alkenyl oder C₃-C₆-Alkinyl steht;
R^{f}
Wasserstoff; die Vinylgruppe;
eine Gruppe -CH=CH-Z, wobei Z für Cyano, Halogen, einen C₁-C₄-Alkylrest, einen partiell oder vollständig halogenierten C₁-C₄-Alkylrest,
einen C₃-C₆-Cycloalkylrest, der gewünschtenfalls seinerseits noch einen bis drei Substituenten tragen kann, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy;
den Carboxylrest, einen C₁-C₈-Alkoxycarbonylrest, den Benzyloxycarbonylrest, den Phenyl-, Thienyl- oder Pyridylrest, wobei diese drei aromatischen Reste unsubstituiert sein oder jeweils noch einen bis drei Substituenten tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Cycloalkyl wobei der Cycloalkylsubstituent unsubstituiert sein oder seinerseits noch einen bis drei Reste tragen kann, ausgewählt aus einer Gruppe bestehend aus Halogen, C₁-C₄-Alkyl, partiell oder vollständig halogeniertem C₁-C₄-Alkyl und C₁-C₄-Alkoxy, steht;
die Ethinylgruppe, die einen der folgenden Reste tragen kann: C₁-C₄-Alkyl, C₃-C₆-Cycloalkyl, das gewünschtenfalls noch einen bis drei Substituenten tragen kann, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy, oder den Phenyl-, Thienyl- oder Pyridylrest, wobei diese aromatischen Reste unsubstituiert sein oder jeweils noch einen bis drei Substituenten tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy und C₁-C₄-Alkylthio; die Phenylgruppe, eine Halogenphenylgruppe, eine Dihalogenphenylgruppe, eine 5-gliedrige heteroaromatische Gruppe mit ein bis drei Heteroatomen, ausgewählt aus einer Gruppe bestehend aus ein bis drei Stickstoffatomen und einem Sauerstoff- oder Schwefelatom, oder eine 6-gliedrige heteroaromatische Gruppe mit ein bis vier Stickstoffatomen, die nicht alle gleichzeitig benachbart sein können, wobei die Phenyl- und Heteroarylgruppen gewünschtenfalls noch einen bis drei Reste tragen können, ausgewählt aus einer Gruppe bestehend aus Nitro, C₁-C₄-Alkoxyresten, C₁-C₄-Alkylthioresten, partiell oder vollständig halogenierten C₁-C₄-Alkoxyresten, Resten Z und einem Rest -NR^{k}R^{l}, wobei
R^{k} Wasserstoff, C₁-C₄-Alkyl, C₃-C₆-Alkenyl oder C₃-C₆-Alkinyl und
R^{l} Wasserstoff, C₁-C₄-Alkyl, C₃-C₆-Alkenyl, C₃-C₆-Alkinyl, C₁-C₆-Acyl oder Benzoyl, das gewünschtenfalls noch einen bis drei Substituenten tragen kann, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy und C₁-C₄-Alkylthio,
bedeuten,
oder
B) der Gruppe der 2-(4-Heteroaryloxy)- oder 2-(4-Aryloxy)-phenoxyessigsäurederivate der Formel III 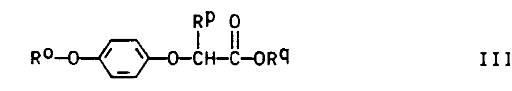 in der die Substituenten folgende Bedeutung haben:
R^{o}
die Phenylgruppe, die Pyridylgruppe, eine Benzoxazylgruppe, eine Benzthiazylgruppe oder eine Benzpyrazinylgruppe, wobei diese aromatischen Ringsysteme unsubstituiert sein oder ein bis zwei der folgenden Reste tragen können: Halogen, Nitro, C₁-C₄-Alkyl, partiell oder vollständig halogeniertes C₁-C₄-Alkyl und/oder partiell oder vollständig halogeniertes C₁-C₄-Alkoxy;
RP
Wasserstoff oder die Methylgruppe und
R^{q}
Wasserstoff, eine C₁-C₄-Alkylgruppe, eine C₃-C₄-Alkenylgruppe, eine C₃-C₄-Alkinylgruppe, eine C₁-C₄-Alkoxy-C₁-C₄-alkylgruppe, eine C₃-C₄-Alkylideniminooxy-C₂-C₃-alkylgruppe, eine Tetrahydrofuranylmethylgruppe, eine Isoxazolidingruppe oder das Äquivalent eines pflanzenverträglichen Kations.

2. Herbizide Mittel nach Anspruch 1, enthaltend eine antagonistisch wirksame Menge mindestens eines 3-Aminobenzo[b]thiophens I, wobei die Substituenten R² bis R⁷ Wasserstoff bedeuten, als antagonistisch wirksame Verbindung.

3. Herbizide Mittel nach Anspruch 1, enthaltend mindestens ein antagonistisch wirksames 3-Aminobenzo[b]thiophen I und mindestens ein Herbizid II und/oder ein Herbizid III im Verhältnis 1:10 bis 10:1.

4. Herbizides Mittel nach Anspruch 2, enthaltend mindestens ein antagonistisch wirksames 3-Aminobenzo[b]thiophen I und mindestens ein Herbizid II und/oder ein Herbizid III im Verhältnis 1:10 bis 10:1.

5. Verfahren zur Herstellung von 3-Aminobenzo[b]thiophenen der Formel I nach Anspruch 1, wobei R⁴ bis R⁷ Wasserstoff bedeuten, dadurch gekennzeichnet, daß man Nitrobenzthiazole der Formel IV 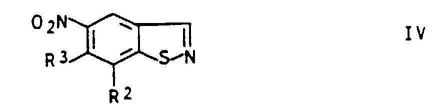 mit einem Cyanessigester in Gegenwart einer Base umsetzt und das erhaltene Cyano-benzthioazo V 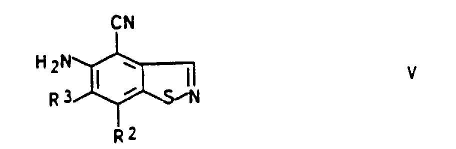 mit ClCH₂-R¹ oder BrCH₂-R¹ zur Reaktion bringt.

6. Verfahren zur selektiven Bekämpfung des Pflanzenwuchses unerwünschter Pflanzen auf Kulturpflanzenanbauflächen, dadurch gekennzeichnet, daß man eine antagonistisch wirksame Menge mindestens eines 3-Aminobenzo[b]thiophen I und
A) eine herbizid wirksame Menge mindestens eines Cyclohexenonderivates der Formel II oder
B) eines 2-(4-Heteroaryloxy)- oder 2-(4-Aryloxy)-phenoxyessigsäurederivates der Formel III
gemäß Anspruch 1 vor, während oder nach der Aussat der Kulturpflanzen oder vor oder während des Auflaufens der Kulturpflanzen gleichzeitig oder nacheinander ausbringt.

7. Verfahren zur selektiven Inhibierung des Pflanzenwuchses unerwünschter Pflanzen auf Kulturpflanzenanbauflächen, dadurch gekennzeichnet, daß man die Blätter der Kulturpflanzen und der unerwünschten Pflanzen im Nachlaufverfahren mit einem herbiziden Mittel gemäß Anspruch 1 behandelt, wobei die Wirkstoff-Komponenten des herbiziden Mittels gleichzeitig oder nacheinander aufgebracht werden können.

8. Verfahren zur selektiven Inhibierung des Pflanzenwuchses unerwünschter Pflanzen auf Kulturpflanzenanbauflächen, dadurch gekennzeichnet, daß man die Blätter der Kulturpflanzen und der unerwünschten Pflanzen im Nachlaufverfahren mit einem herbiziden Mittel gemäß Anspruch 2 behandelt, wobei die Wirkstoff-Komponenten des herbiziden Mittels gleichzeitig oder nacheinander aufgebracht werden können.

9. Verfahren zur selektiven Inhibierung des Pflanzenwuchses unerwünschter Pflanzen auf Kulturpflanzenanbauflächen, dadurch gekennzeichnet, daß man die Blätter der Kulturpflanzen und der unerwünschten Pflanzen im Nachlaufverfahren mit einem herbiziden Mittel gemäß Anspruch 3 behandelt, wobei die Wirkstoff-Komponenten des herbiziden Mittels gleichzeitig oder nacheinander aufgebracht werden können.

10. Verfahren zur Verhinderung von Schädigungen von Kulturpflanzen durch
A) herbizide Cyclohexenonderivate der Formel II oder
B) herbizide 2-(4-Heteroaryloxy)- oder 2-(4-Aryloxy)-phenoxyessigsäurederivate der Formel III,
dadurch gekennzeichnet, daß man das Saatgut der Kulturpflanzen mit einer antagonistisch wirksamen Menge eines 3-Aminobenzo[b]thiophens der Formel I gemäß Anspruch 1 behandelt.

11. Verfahren gemäß Anspruch 6, dadurch gekennzeichnet, daß die Kulturpflanzen Gerste, Weizen, Mais, Kultursorghum und Reis sind.

12. 3-Aminobenzo[b]thiophene der allgemeinen Formel I' 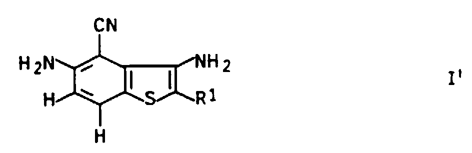 in der die Variablen folgende Bedeutung haben:
R¹ eine Gruppe -COX oder -COOX, wobei
X Wasserstoff, Halogen, die Aminogruppe, die unsubstituiert sein oder ein bis drei Substituenten tragen kann, ausgewählt aus einer Gruppe bestehend aus C₁-C₄-Alkyl-, Hydroxy-C₁-C₄-alkyl-, Phenyl-, und Benzylsubstituenten,
eine C₁-C₁₀-Alkylgruppe, eine C₂-C₈-Alkenylgruppe, eine C₃-C₁₀-Cycloalkylgruppe oder eine C₃-C₁₀-Cycloalkenylgruppe, wobei die letztgenannten vier Gruppen unsubstituiert oder partiell oder vollständig halogeniert sein können und wobei die letztgenannten vier Gruppen auch eine oder zwei weitere Carboxyl- oder Carbonylgruppen enthalten können,
einen 5- oder 6-gliedrigen aromatischen Heterocyclus mit einem Sauerstoff- und einem Schwefelatom oder mit 1 bis 3 Heteroatomen, ausgewählt aus einer Gruppe bestehend aus 3 Stickstoffatomen und einem Sauerstoff- oder Schwefelatom, wobei nicht drei Heteroatome gleichzeitig benachbart sein können,
die Phenyl- oder die Naphthylgruppe, wobei diese Gruppen einen bis drei Substituenten tragen können, ausgewählt aus einer Gruppe bestehend aus Halogen, -COY oder -COOY, und wobei die Phenyl- und Naphthylgruppen noch zusätzlich so viele Halogenatome tragen können, wie substituierbare C-Atome vorhanden sind;
Y steht für C₁-C₆-Alkyl, C₃-C₆-Cycloalkyl oder Phenyl.

13. 3-Aminobenz[b]thiophene der allgemeinen Formel I' gemäß Anspruch 8, wobei R¹ Wasserstoff, Acyl, Methoxycarbonyl, tert.-Butoxycarbonyl, 1-Propen-3-oxycarbonyl, n-Butoxycarbonyl, 2-Heptyloxycarbonyl, Benzyloxycarbonyl, Carbonsäureamid, Carboxyessigsäureethylester, Oxalylsäureethylester oder Benzoyl bedeutet.

14. Cyano-benzthiazole der allgemeinen Formel V 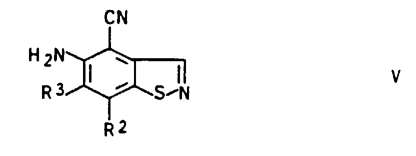 in der R² und R³ folgende Bedeutung haben:
R², R³ Wasserstoff, Cyano, Nitro, Halogen, Mercapto, die Aminogruppe, deren Stickstoffatom ein bis drei Substituenten tragen kann, ausgewählt aus einer Gruppe bestehend aus C₁-C₄-Alkyl-, Hydroxy-C₁-C₄-alkyl-, Phenyl-, und Benzylsubstituenten,
eine C₁-C₁₀-Alkylgruppe, eine C₁-C₆-Alkoxygruppe, eine C₂-C₈-Alkenylgruppe, eine C₃-C₁₀-Cycloalkylgruppe oder eine C₃-C₁₀-Cycloalkenylgruppe, wobei die letztgenannten vier Gruppen partiell oder vollständig halogeniert sein können, die Phenyl- oder die Naphthylgruppe,
einen 5- oder 6-gliedrigen aromatischen Heterocyclus mit einem Sauerstoff- und einem Schwefelatom oder mit einem bis drei Heteroatomen, ausgewählt aus einer Gruppe bestehend aus drei Stickstoffatomen und einem Sauerstoff- oder Schwefelatom, wobei im Falle von drei Heteroatomen nicht alle gleichzeitig benachbart sein können.

15. Verfahren zur Herstellung von Cyano-benzthiazolen der Formel V gemäß Anspruch 14, dadurch gekennzeichnet, daß man Nitrobenzole der Formel IV 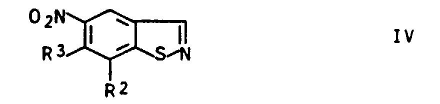 mit einem Cyanessigester in Gegenwart einer Base umsetzt.

## Claims

1. A herbicide containing one or more antagonistic 3-aminobenzo[b]thiophenes of the formula I 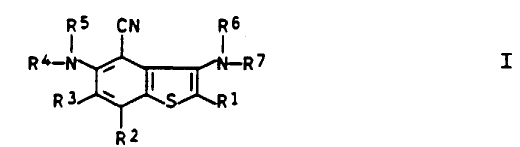 where
R¹ is -COX or -COOX,
X is hydrogen, halogen, amino which may be unsubstituted or may carry from one to three substituents selected from the group consisting of C₁-C₄-alkyl, hydroxy-C₁-C₄-alkyl, phenyl and benzyl substituents, C₁-C₁₀-alkyl, C₂-C₈-alkenyl, C₃-C₁₀-cycloalkyl or C₃-C₁₀-cycloalkenyl, where the four last-mentioned groups may be unsubstituted or partially or completely halogenated and the four last-mentioned groups may furthermore contain one or two further carboxyl or carbonyl groups,
a 5-membered or 6-membered aromatic heterocyclic structure having one oxygen and one sulfur atom or having from 1 to 3 hetero atoms selected from the group consisting of from 1 to 3 nitrogen atoms and one oxygen or sulfur atom, and all three hetero atoms may not be adjacent to one another at the same time,
phenyl or naphthyl, where these groups may carry from one to three substituents selected from the group consisting of halogen, -COY and -COOY, and these groups may additionally carry a number of halogen atoms corresponding to the number of substitutable carbon atoms present;
Y is C₁-C₆-alkyl, C₃-C₆-cycloalkyl or phenyl;
R² and R³ are each hydrogen, cyano, nitro, halogen, mercapto, amino whose nitrogen atom may carry from one to three substituents selected from the group consisting of C₁-C₄-alkyl, hydroxy-C₁-C₄-alkyl, phenyl and benzyl substituents,
C₁-C₁₀-alkyl, C₁-C₆-alkoxy, C₂-C₈-alkenyl, C₃-C₁₀-cycloalkyl or C₃-C₁₀-cycloalkenyl, where the four last-mentioned groups may be partially or completely halogenated, phenyl or naphthyl,
a 5-membered or 6-membered aromatic heterocyclic structure having one oxygen and one sulfur atom or having from one to three hetero atoms selected from the group consisting of three nitrogen atoms and one oxygen or sulfur atom, where in the case of three hetero atoms all may not be adjacent to one another at the same time;
R⁴ to R⁷ are each hydrogen, phenyl, naphthyl or a 5-membered or 6-membered aromatic heterocyclic structure having one oxygen and one sulfur atom or having from one to three hetero atoms selected from the group consisting of three nitrogen atoms and one oxygen or sulfur atom, where in the case of three hetero atoms all may not be adjacent to one another at the same time and where the aromatic and heteroaromatic groups may furthermore carry from one to three substituents selected from the group consisting of halogen, -COY and -COOY, and these groups may additionally carry a number of halogen atoms corresponding to the number of substitutable carbon atoms present,
C₁-C₁₀-alkyl, C₂-C₆-alkenyl, C₃-C₁₀-cycloalkyl or C₃-C₁₀-cycloalkenyl;
or R⁴ and R⁵ and/or R⁶ and R⁷, together with the nitrogen atom to which they are bonded, form a 5-membered to 7-membered ring,
and the basic salts of the compounds I which carry one or more carboxyl, hydroxythiocarbonyl or sulfo groups and the acidic salts of the compounds I which contain a basic nitrogen atom,
and one or more herbicidal active ingredients selected from
A) the group consisting of the cyclohexenone derivatives of the formula II 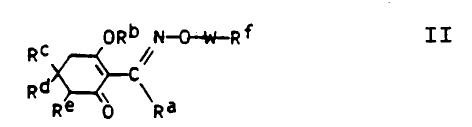 where
R^{a} is C₁-C₆-alkyl;
R^{b} is hydrogen, one equivalent of an agriculturally useful cation, C₂-C₈-alkylcarbonyloxy, C₁-C₁₀-alkylsulfonyl, C₁-C₁₀-alkylphosphonyl or benzoyl, benzenesulfonyl or benzenephosphonyl, where the three last-mentioned groups may furthermore each carry from one to five halogen atoms;
R^{c} is hydrogen, cyano, formyl, C₁-C₆-alkyl, C₁-C₄-alkoxy-C₁-C₆-alkyl or C₁-C₄-alkylthio-C₁-C₆-alkyl, phenoxy-C₁-C₆-alkyl, phenylthio-C₁-C₆-alkyl, pyridyloxy-C₁-C₆-alkyl or pyridylthio-C₁-C₆-alkyl, where the phenyl and pyridyl rings may each furthermore carry from one to three radicals selected from the group consisting of nitro, cyano, halogen, C₁-C₄-alkyl, partially or completely halogenated C₁-C₄-alkyl, C₁-C₄-alkoxy, partially or completely halogenated C₁-C₄-alkoxy, C₁-C₄-alkylthio, C₃-C₆-alkenyl, C₃-C₆-alkenyloxy, C₃-C₆-alkynyl, C₃-C₆-alkynyloxy and -NR^{g}R^{h}, where
R^{g} is hydrogen, C₁-C₄-alkyl, C₃-C₆-alkenyl, C₃-C₆-alkynyl, C₁-C₆-acyl or benzoyl which may carry from one to three radicals selected from the group consisting of nitro, cyano, halogen, C₁-C₄-alkyl, partially or completely halogenated C₁-C₄-alkyl, C₁-C₄-alkoxy and C₁-C₄-alkylthio and
R^{h} is hydrogen, C₁-C₄-alkyl, C₃-C₆-alkenyl or C₃-C₆-alkynyl; C₃-C₇-cycloalkyl or C₅-C₇-cycloalkenyl, where these groups may furthermore carry from one to three radicals selected from the group consisting of hydroxyl, halogen, C₁-C₄-alkyl, partially or completely halogenated C₁-C₄-alkyl, C₁-C₄-alkoxy, C₁-C₄-alkylthio, benzylthio, C₁-C₄-alkylsulfonyl, C₁-C₄-alkylsulfenyl and C₁-C₄-alkylsulfinyl,
a 5-membered saturated heterocyclic structure which contains one or two oxygen or sulfur atoms or one oxygen and one sulfur atom as hetero atoms and which may furthermore carry from one to three radicals selected from the group consisting of C₁-C₄-alkyl, partially or completely halogenated C₁-C₄-alkyl, C₁-C₄-alkoxy and C₁-C₄-alkylthio,
a 6-membered or 7-membered saturated heterocyclic structure or a mono- or diunsaturated heterocyclic structure which contains one or two oxygen or sulfur atoms or one oxygen and one sulfur atom as hetero atoms and which may furthermore carry from one to three radicals selected from the group consisting of hydroxyl, halogen, C₁-C₄-alkyl, partially or completely halogenated C₁-C₄-alkyl, C₁-C₄-alkoxy and C₁-C₄-alkylthio,
a 5-membered heteroaromatic structure containing from one to three hetero atoms selected from the group consisting of one or two nitrogen atoms and one oxygen or sulfur atom, where the heteroaromatic structure may furthermore carry from one to three radicals selected from the group consisting of cyano, halogen, C₁-C₄-alkyl, partially or completely halogenated C₁-C₄-alkyl, C₁-C₄-alkoxy, partially or completely halogenated C₁-C₄-alkoxy, C₁-C₄-alkylthio, C₂-C₆-alkenyl, C₂-C₆-alkenyloxy, C₃-C₆-alkynyloxy and C₁-C₄-alkoxy-C₁-C₄-alkyl,
phenyl or pyridyl, each of which may furthermore carry from one to three radicals selected from the group consisting of nitro, cyano, formyl, halogen, C₁-C₄-alkyl, partially or completely halogenated C₁-C₄-alkyl, C₁-C₄-alkoxy, partially or completely halogenated C₁-C₄-alkoxy, C₁-C₄-alkylthio, C₃-C₆-alkenyl, C₃-C₆-alkenyloxy, C₃-C₆-alkynyl, C₃-C₆-alkynyloxy and -NR^{g}R^{h}, where R^{g} and R^{h} have the abovementioned meanings;
R^{d} is hydrogen or hydroxyl or, when R^{c} is C₁-C₆-alkyl, R^{d} is C₁-C₆-alkyl;
R^{e} is hydrogen, halogen, cyano, a C₁-C₄-alkoxycarbonyl or a C₁-C₄-alkylketoxime group;
W is a C₁-C₆-alkylene, C₃-C₆-alkenylene or C₃-C₆-alkynylene chain, each of which may furthermore carry from one to three radicals selected from the group consisting of three C₁-C₃-alkyl substituents, three halogen atoms and one methylene substituent;
a C₃-C₆-alkylene or C₄-C₆-alkenylene chain, both of which may furthermore carry from one to three C₁-C₃-alkyl radicals, where in each case one methylene group of the chains may be substituted by an oxygen or sulfur atom, a sulfoxyl or sulfonyl group or a group -N(Rⁱ)-, where Rⁱ is hydrogen, C₁-C₄-alkyl, C₃-C₆-alkenyl or C₃-C₆-alkynyl; R^{f} is hydrogen; vinyl;
a group -CH=CH-Z, where Z is cyano, halogen, C₁-C₄-alkyl, partially or completely halogenated C₁-C₄-alkyl, C₃-C₆-cycloalkyl, which, if desired, in turn may carry from one to three substituents selected from the group consisting of hydroxyl, halogen, C₁-C₄-alkyl, partially or completely halogenated C₁-C₄-alkyl and C₁-C₄-alkoxy;
carboxyl, C₁-C₈-alkoxycarbonyl, benzyloxycarbonyl, phenyl, thienyl or pyridyl, where these three aromatic radicals may be unsubstituted or may each furthermore carry from one to three substituents selected from the group consisting of nitro, cyano, halogen, C₁-C₄-alkyl, partially or completely halogenated C₁-C₄-alkyl, C₁-C₄-alkoxy, partially or completely halogenated C₁-C₄-alkoxy, C₁-C₄-alkylthio and C₃-C₆-cycloalkyl, where the cycloalkyl substituent may be unsubstituted or in turn may furthermore carry from one to three radicals selected from the group consisting of halogen, C₁-C₄-alkyl, partially or completely halogenated C₁-C₄-alkyl and C₁-C₄-alkoxy;
ethynyl which may carry one of the following radicals: C₁-C₄-alkyl, C₃-C₆-cycloalkyl, which, if desired, may furthermore carry from one to three substituents selected from the group consisting of hydroxyl, halogen, C₁-C₄-alkyl, partially or completely halogenated C₁-C₄-alkyl and C₁-C₄-alkoxy, or phenyl, thienyl or pyridyl, where these aromatic radicals may be unsubstituted or may each furthermore carry from one to three substituents selected from the group consisting of nitro, cyano, halogen, C₁-C₄-alkyl, partially or completely halogenated C₁-C₄-alkyl, C₁-C₄-alkoxy, partially or completely halogenated C₁-C₄-alkoxy and C₁-C₄-alkylthio;
phenyl, halophenyl, dihalophenyl, a 5-membered heteroaromatic group having from one to three hetero atoms, selected from the group consisting of from one to three nitrogen atoms and one oxygen or sulfur atom, or a 6-membered heteroaromatic group having from one to four nitrogen atoms, all of which may not be adjacent to one another at the same time, where the phenyl and hetaryl groups may, if desired, furthermore carry from one to three radicals selected from the group consisting of nitro, C₁-C₄-alkoxy, C₁-C₄-alkylthio, partially or completely halogenated C₁-C₄-alkoxy, radicals Z and -NR^{k}R^{l}, where
R^{k} is hydrogen, C₁-C₄-alkyl, C₃-C₆-alkenyl or C₃-C₆-alkynyl and
R^{l} is hydrogen, C₁-C₄-alkyl, C₃-C₆-alkenyl, C₃-C₆-alkynyl, C₁-C₆-acyl or benzoyl which, if desired, may furthermore carry from one to three substituents selected from the group consisting of nitro, cyano, halogen, C₁-C₄-alkyl, partially or completely halogenated C₁-C₄-alkyl, C₁-C₄-alkoxy and C₁-C₄-alkylthio,
or
B) the group consisting of the 2-(4-hetaryloxy)- or 2-(4-aryloxy)-phenoxyacetic acid derivatives of the formula III 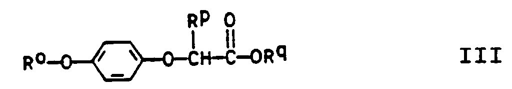 where
R^{o} is phenyl, pyridyl, benzoxazyl, benzothiazyl or benzopyrazinyl, where these aromatic ring systems may be unsubstituted or may carry one or two of the following radicals: halogen, nitro, C₁-C₄-alkyl, partially or completely halogenated C₁-C₄-alkyl and/or partially or completely halogenated C₁-C₄-alkoxy;
R^{p} is hydrogen or methyl and
R^{q} is hydrogen, C₁-C₄-alkyl, C₃- or C₄-alkenyl, C₃- or C₄-alkynyl, C₁-C₄-alkoxy-C₁-C₄-alkyl, C₃- or C₄-alkylideneiminooxy-C₂- or -C₃-alkyl, tetrahydrofuranylmethyl, isoxazolidinyl or one equivalent of a plant-tolerated cation.

2. A herbicide as claimed in claim 1, containing an antagonistic amount of one or more 3-aminobenzo[b]thiophenes I, where R² to R⁷ are each hydrogen, as antagonistic compounds.

3. A herbicide as claimed in claim 1, containing one or more antagonistic 3-aminobenzo[b]thiophenes I and one or more herbicides II and/or one or more herbicides III in a ratio of from 1 : 10 to 10 : 1.

4. A herbicide as claimed in claim 2, containing one or more antagonistic 3-aminobenzo[b]thiophenes I and one or more herbicides II and/or one or more herbicides III in a ratio of from 1 : 10 to 10 : 1.

5. A process for the preparation of a 3-aminobenzo-[b]thiophene of the formula I as claimed in claim 1, where R⁴ to R⁷ are each hydrogen, wherein a nitrobenzothiazole of the formula IV 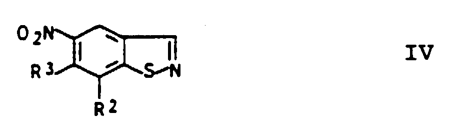 is reacted with cyanoacetate in the presence of a base, and the resulting cyanobenzothiazole V 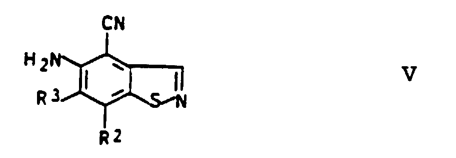 is reacted with ClCH₂-R¹ or BrCH₂-R¹.

6. A method for selectively controlling undesirable plant growth on cultivated areas, wherein an antagonistic amount of one or more 3-aminobenzo[b]thiophenes I and
A) a herbicidal amount of one or more cyclohexenone derivatives of the formula II or
B) of a 2-(4-hetaryloxy)- or 2-(4-aryloxy)-phenoxyacetic acid derivative of the formula III
as claimed in claim 1 are applied, simultaneously or in succession, before, during or after sowing of the crops or before or during emergence of the crops.

7. A method for selectively inhibiting undesirable plant growth on cultivated areas, wherein the leaves of the crops and of the undesirable plants are treated with a herbicide as claimed in claim 1 by the post-emergence method, where the active ingredient components of the herbicide may be applied simultaneously or in succession.

8. A method for selectively inhibiting undesirable plant growth on cultivated areas, wherein the leaves of the crops and of the undesirable plants are treated with a herbicide as claimed in claim 2 by the post-emergence method, where the active ingredient components of the herbicide may be applied simultaneously or in succession.

9. A method for selectively inhibiting undesirable plant growth on cultivated areas, wherein the leaves of the crops and of the undesirable plants are treated with a herbicide as claimed in claim 3 by the post-emergence method, where the active ingredient components of the herbicide may be applied simultaneously or in succession.

10. A method for preventing damage to crops by
A) herbicidal cyclohexenone derivatives of the formula II or
B) herbicidal 2-(4-hetaryloxy)- or 2-(4-aryloxy)-phenoxyacetic acid derivatives of the formula III,
wherein the seed of the crops is treated with an antagonistic amount of a 3-aminobenzo[b]thiophene of the formula I as claimed in claim 1.

11. A method as claimed in claim 6, wherein the crop is barley, wheat, corn, sorghum or rice.

12. A 3-aminobenzo[b]-thiophene of the formula I' 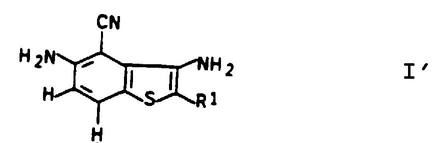 where
R¹ is -COX or -COOX,
X is hydrogen, halogen, amino which may be unsubstituted or may carry from one to three substituents selected from the group consisting of C₁-C₄-alkyl, hydroxy-C₁-C₄-alkyl, phenyl and benzyl substituents,
C₁-C₁₀-alkyl, C₂-C₈-alkenyl, C₃-C₁₀-cycloalkyl or C₃-C₁₀-cycloalkenyl, where the four last-mentioned groups may be unsubstituted or partially or completely halogenated and the four last-mentioned groups may furthermore contain one or two further carboxyl or carbonyl groups,
a 5-membered or 6-membered aromatic heterocyclic structure having one oxygen and one sulfur atom or having from one to three hetero atoms selected from the group consisting of three nitrogen atoms and one oxygen or sulfur atom, where three hetero atoms may not be adjacent to one another simultaneously,
phenyl or naphthyl, where these groups may carry from one to three substituents selected from the group consisting of halogen, -COY and -COOY, and the phenyl and naphthyl groups may additionally carry a number of halogen atoms corresponding to the number of substitutable carbon atoms present, and
Y is C₁-C₆-alkyl, C₃-C₆-cycloalkyl or phenyl.

13. A 3-aminobenzo[b]thiophene of the formula I' as claimed in claim 8, wherein R¹ is hydrogen, acyl, methoxycarbonyl, tert-butoxycarbonyl, 1-propen-3-oxycarbonyl, n-butoxycarbonyl, 2-heptyloxycarbonyl, benzyloxycarbonyl, carboxamido, an ethyl carboxyacetate group, an ethyl oxalate group or benzoyl.

14. A cyanobenzothiazole of the formula V 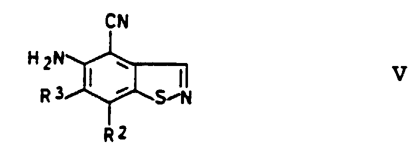 where R² and R³ are each hydrogen, cyano, nitro, halogen, mercapto, amino whose nitrogen atom may carry from one to three substituents selected from the group consisting of C₁-C₄-alkyl, hydroxy-C₁-C₄-alkyl, phenyl and benzyl substituents,
C₁-C₁₀-alkyl, C₁-C₆-alkoxy, C₂-C₈-alkenyl, C₃-C₁₀-cycloalkyl or C₃-C₁₀-cycloalkenyl, where the four last-mentioned groups may be partially or completely halogenated, phenyl or naphthyl,
a 5-membered or 6-membered aromatic heterocyclic structure having one oxygen and one sulfur atom or having from one to three hetero atoms selected from the group consisting of three nitrogen atoms and one oxygen or sulfur atom, where in the case of three hetero atoms all may not be adjacent to one another at the same time.

15. A process for the preparation of a cyanobenzothiazole of the formula V as claimed in claim 14, wherein a nitrobenzene of the formula IV 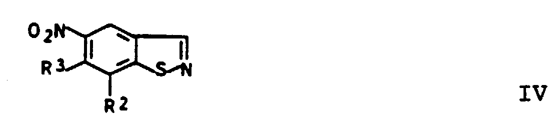 is reacted with a cyanoacetate in the presence of a base.

## Revendications

1. Agent herbicide, contenant au moins un 3-aminobenzo[b]thiophène à activité antagoniste, de formule générale I 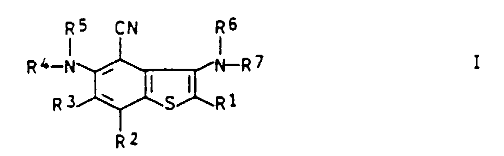 où les variables ont la signification suivante:
R¹ un groupe -COX ou -COOX, tandis que
X l'hydrogène, un halogène, le groupe amino, qui est non substitué ou porte un à trois substituants choisis dans un groupe consistant en les substituants alkyle en C₁-C₄, hydroxyalkyle en C₁-C₄, phényle et benzyle,
un groupe alkyle en C₁-C₁₀, un groupe alcényle en C₂-C₈, un groupe cycloalkyle en C₃-C₁₀ ou un groupe cycloalcényle en C₃-C₁₀, tandis que les quatre groupes nommés en dernier peuvent être non substitués ou partiellement ou totalement halogénés et que les quatre groupes nommés en dernier peuvent contenir également un ou deux autres groupes carboxyle ou carbonyle,
un hétérocycle aromatique à 5 ou 6 chaînons avec un atome d'oxygène et un atome de soufre ou avec 1 à 3 hétéroatomes, choisis dans un groupe consistant en 1 à 3 atomes d'azote et un atome d'oxygène ou de soufre, tandis que trois hétéroatomes ne peuvent pas être simultanément voisins,
le groupe phényle ou le groupe naphtyle, tandis que ces groupes sont non substitués ou portent un à trois substituants, choisis dans le groupe consistant en un halogène, -COY ou -COOY, et tandis que ces groupes peuvent encore porter en plus autant d'atomes d'halogène qu'il y a d'atomes de carbone substituables présents;
Y désigne un groupe alkyle en C₁-C₆, cycloalkyle en C₃-C₆ ou phényle;
R², R³ un reste hydrogène, cyano, nitro, halogène, mercapto, le groupe amino, dont l'atome d'azote peut porter un à trois substituants, choisis dans un groupe consistant en les substituants alkyle en C₁-C₄, hydroxyalkyle en C₁-C₄, phényle et benzyle,
un groupe alkyle en C₁-C₁₀, un groupe alcoxy en C₁-C₆, un groupe alcényle en C₂-C₈, un groupe cycloalkyle en C₃-C₁₀ ou un groupe cycloalcényle en C₃-C₁₀, tandis que les quatre groupes nommés en dernier peuvent être partiellement ou totalement halogénés, le groupe phényle ou le groupe naphtyle,
un hétérocycle à 5 ou 6 chaînons ayant un atome d'oxygène et un atome de soufre ou ayant un à trois hétéroatomes choisis dans le groupe consistant en trois atomes d'azote et un atome d'oxygène ou un atome de soufre, tandis que dans le cas de trois hétéroatomes tous ne peuvent pas être simultanément voisins;
R⁴ à R⁷ l'hydrogène, le groupe phényle, le groupe naphtyle ou un hétérocycle aromatique à 5 ou 6 chaînons ayant un atome d'oxygène et un atome de soufre ou ayant un à trois hétéroatomes, choisis dans le groupe consistant en trois atomes d'azote et un atome d'oxygène ou un atome de soufre, tandis que dans le cas de trois hétéroatomes tous ne peuvent pas être simultanément voisins;
les groupes aromatiques et hétéroaromatiques pouvant encore porter un à trois substituants, choisis dans un groupe consistant en un halogène, -COY ou -COOY, et tandis que ces groupes peuvent encore porter autant d'atomes d'halogène qu'il y a d'atomes de carbone substituables présents,
un groupe alkyle en C₁-C₁₀, un groupe alcényle en C₂-C₆, un groupe cycloalkyle en C₃-C₁₀, un groupe cycloalcényle en C₃-C₁₀;
ou R⁴ et R⁵ et/ou R⁶ et R⁷ forment conjointement avec l'atome d'azote auquel ils sont liés un cycle à 5 à 7 chaînons;
ainsi que les sels basiques des composés I qui portent au moins un groupe acide carboxylique, hydroxythiocarbonylique ou sulfonique et les sels acides des composés I qui possèdent un atome d'azote basique,
et au moins un agent à activité herbicide choisi dans
A) le groupe des dérivés de cyclohexénone de formule générale II 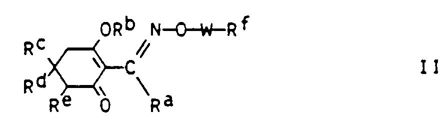 où les substituants ont la signification suivante:
R^{a}
un groupe alkyle en C₁-C₆;
R^{b}
l'hydrogène, l'équivalent d'un cation compatible avec l'environnement, un groupe alkyl(C₂-C₈)carbonyle, un groupe alkyl(C₁-C₁₀)sulfonyle, un groupe alkyl(C₁-C₁₀)-phosphonyle ou le groupe benzoyle, benzènesulfonyle ou benzènephosphonyle, tandis que les trois groupes mentionnés en dernier peuvent encore porter chacun 1 à 5 atomes d'halogène;
R^{c}
l'hydrogène, le groupe cyano, le groupe formyle, un groupe alkyle en C₁-C₆, un groupe alcoxy(C₁-C₄)-alkyle en C₁-C₆ ou alkylthio(C₁-C₄)-alkyle en C₁-C₆, un groupe phénoxyalkyle(C₁-C₆), phénylthio-alkyle(C₁-C₆), pyridyloxyalkyle(C₁-C₆) ou pyridylthio-alkyle(C₁-C₆), tandis que les cycles phényle et pyridyle peuvent encore porter chacun un à trois restes choisis dans un groupe consistant en les restes nitro, cyano, halogène, alkyle en C₁-C₄, alkyle en C₁-C₄ partiellement ou totalement halogéné, alcoxy en C₁-C₄, alcoxy en C₁-C₄ partiellement ou totalement halogéné, alkylthio en C₁-C₄, alcényle en C₃-C₆, alcényloxy en C₃-C₆, alcynyle en C₃-C₆, alcynyloxy en C₃-C₆ et NR^{g}R^{h},
tandis que
R^{g} représente l'hydrogène ou un groupe alkyle en C₁-C₄, alcényle en C₃-C₆, alcynyle en C₃-C₆, acyle en C₁-C₆ ou benzoyle, qui peut porter un à trois restes choisis dans un groupe consistant en les restes nitro, cyano, halogène, alkyle en C₁-C₄, alkyle en C₁-C₄ partiellement ou totalement halogéné, alcoxy en C₁-C₄ et alkylthio en C₁-C₄ et
R^{h} représente l'hydrogène ou un radical alkyle en C₁-C₄, alcényle en C₃-C₆ ou alcynyle en C₃-C₆;
un groupe cycloalkyle en C₃-C₇ ou un groupe cycloalcényle en C₅-C₇, tandis que ces groupes peuvent encore porter un à trois restes choisis dans un groupe consistant en les restes hyroxyle, halogène, alkyle en C₁-C₄, alkyle en C₁-C₄ partiellement ou totalement halogéné, alcoxy en C₁-C₄, alkylthio en C₁-C₄, benzylthio, alkylsulfonyle en C₁-C₄, alkylsulfényle en C₁-C₄ et alkylsulfinyle en C₁-C₄,
un hétérocycle saturé à 5 chaînons, qui contient un ou deux atomes d'oxygène ou de soufre ou un atome d'oxygène et un atome de soufre en tant qu'hétéroatomes, et qui peut encore porter un à trois restes choisis dans un groupe consistant en les restes alkyle en C₁-C₄, alkyle en C₁-C₄ partiellement ou totalement halogéné, alcoxy en C₁-C₄ et alkylthio en C₁-C₄,
un hétérocycle à 6 ou 7 chaînons, saturé ou une ou deux fois insaturé, qui contient un ou deux atomes d'oxygène ou de soufre ou un atome d'oxygène et un atome de soufre en tant qu'hétéroatomes, et qui peut encore porter un à trois restes choisis dans un groupe consistant en les restes hydroxyle, halogène, alkyle en C₁-C₄, alkyle en C₁-C₄ partiellement ou totalement halogéné, alcoxy en C₁-C₄, alkylthio en C₁-C₄,
un groupe hétéroaromatique à 5 chaînons, contenant un à trois hétéroatomes, choisis dans un groupe consistant en un ou deux atomes d'azote et un atome d'oxygène ou de soufre, tandis que le groupe hétéroaromatique peut encore porter un à trois restes choisis dans un groupe consistant en les restes cyano, halogène, alkyle en C₁-C₄, alkyle en C₁-C₄ partiellement ou totalement halogéné, alcoxy en C₁-C₄, alcoxy en C₁-C₄ partiellement ou totalement halogéné, alkylthio en C₁-C₄, alcényle en C₂-C₆, alcényloxy en C₂-C₆, alcynyloxy en C₃-C₆ et alcoxy(C₁-C₄)-alkyle en C₁-C₄, un groupe phényle ou pyridyle, qui peut encore porter dans chaque cas un à trois restes choisis dans un groupe consistant en les restes nitro, cyano, formyle, halogène, alkyle en C₁-C₄, alkyle en C₁-C₄ partiellement ou totalement halogéné, alcoxy en C₁-C₄, alcoxy en C₁-C₄ partiellement ou totalement halogéné, alkylthio en C₁-C₄, alcényle en C₃-C₆, alcényloxy en C₃-C₆, alcynyle en C₃-C₆, alcynyloxy en C₃-C₆ et -NR^{k}R^{l}, tandis que R^{k} et R^{l} ont la signification indiquée ci-dessous;
R^{d}
l'hydrogène, le groupe hydroxyle, ou, lorsque R^{c} désigne un groupe alkyle en C₁-C₆, un groupe alkyle en C₁-C₆;
R^{e}
l'hydrogène, un halogène, le groupe cyano, un groupe alcoxy(C₁-C₄)carbonyle ou un groupe alkyl(C₁-C₄)cétoxime;
W
une chaîne alkylène en C₁-C₆, alcénylène en C₃-C₆ ou alcynylène en C₃-C₆, qui peuvent chacune encore porter un à trois restes choisis dans un groupe consistant en trois substituants alkyle en C₁-C₃, trois atomes d'halogène et un substituant méthylène;
une chaîne alkylène en C₃-C₆ ou alcényle en C₄-C₆, qui peuvent encore toutes deux porter un à trois restes alkyle en C₁-C₃, tandis que dans chaque cas un groupe méthylène des chaînes peut être substitué par un atome d'oxygène ou de soufre, un groupe sulfoxyde ou sulfone ou un groupe -N(Rⁱ)-, tandis que
Rⁱ désigne l'hydrogène ou un groupe alkyle en C₁-C₄, alcényle en C₃-C₆ ou alcynyle en C₃-C₆;
R^{f}
l'hydrogène; le groupe vinyle;
un groupe -CH=CH-Z, où Z désigne un groupe cyano, halogène, un reste alkyle en C₁-C₄, un reste alkyle en C₁-C₄ partiellement ou totalement halogéné,
un reste cycloalkyle en C₃-C₆, qui peut à son tour porter éventuellement encore un à trois substituants choisis dans un groupe consistant en les substituants hydroxyle, halogène, alkyle en C₁-C₄, alkyle en C₁-C₄ partiellement ou totalement halogéné et alcoxy en C₁-C₄;
le reste carboxyle, un reste alcoxy(C₁-C₈)carbonyle, le reste benzyloxycarbonyle, le reste phényle, thiényle ou pyridyle, ces trois restes aromatiques étant non substitués ou pouvant porter chacun encore un à trois substituants choisis dans un groupe consistant en les substituants nitro, cyano, halogène, alkyle en C₁-C₄, alkyle en C₁-C₄ partiellment ou totalement halogéné, alcoxy en C₁-C₄, alcoxy en C₁-C₄ partiellement ou totalement halogéné, alkylthio en C₁-C₄, cycloalkyle en C₃-C₆, tandis que le substituant cycloalkyle peut être non substitué ou encore porter à son tour un à trois restes choisis dans un groupe consistant en des restes halogène, alkyle en C₁-C₄, alkyle en C₁-C₄ partiellement ou totalement halogéné et alcoxy en C₁-C₄;
le groupe éthynyle, qui peut porter l'un des restes suivants: alkyle en C₁-C₄, cycloalkyle en C₃-C₆, qui peut éventuellement porter encore un à trois substituants choisis dans un groupe consistant en des substituants hydroxyle, halogène, alkyle en C₁-C₄, alkyle en C₁-C₄ partiellement ou totalement halogéné et alcoxy en C₁-C₄, ou le reste phényle, thiényle ou pyridyle, ces restes aromatiques étant non substitués ou pouvant porter chacun encore un à trois substituants choisis dans un groupe consistant en des substituants nitro, cyano, halogène, alkyle en C₁-C₄, alkyle en C₁-C₄ partiellement ou totalement halogéné, alcoxy en C₁-C₄, alcoxy en C₁-C₄ partiellement ou totalement halogéné et alkylthio en C₁-C₄; le groupe phényle, un groupe halogénophényle, un groupe dihalogénophényle, un groupe hétéroaromatique à 5 chaînons ayant un à trois hétéroatomes choisis dans le groupe consistant en un à trois atomes d'azote et un atome d'oxygène ou de soufre, ou un groupe hétéroaromatique à 6 chaînons ayant un à quatre atomes d'azote, qui ne peuvent pas être simultanément contigus, tandis que les groupes phényle et hétéroaryle peuvent éventuellement encore porter un à trois restes choisis dans un groupe consistant en des restes nitro, des restes alcoxy en C₁-C₄, des restes alkylthio en C₁-C₄, des restes alcoxy en C₁-C₄ partiellement ou totalement halogénés, des restes Z et un reste -NR^{k}R^{l}, tandis que
R^{k} représente l'hydrogène ou un groupe alkyle en C₁-C₄, alcényle en C₃-C₆ ou alcynyle en C₃-C₆, et
R^{l} représente l'hydrogène ou un groupe alkyle en C₁-C₄, alcényle en C₃-C₆, alcynyle en C₃-C₆, acyle en C₁-C₆ ou benzoyle, qui peut éventuellement porter encore un à trois substituants choisis dans un groupe consistant en des susbtituants nitro, cyano, halogène, alkyle en C₁-C₄, alkyle en C₁-C₄ partiellement ou totalement halogéné, alcoxy en C₁-C₄ et alkylthio en C₁-C₄,
ou
B) le groupe des dérivés d'acide 2-(4-hétéroaryloxy)- ou 2-(4-aryloxy)-phénoxyacétique de formule III 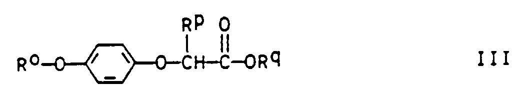 où les substituants ont la signification suivante:
R^{o}
le groupe phényle, le groupe pyridyle, un groupe benzoxazyle, un groupe benzothiazyle ou un groupe benzopyrazinyle, tandis que ces systèmes cycliques aromatiques sont non substitués ou peuvent porter un à deux des restes suivants: halogène, nitro, alkyle en C₁-C₄, alkyle en C₁-C₄ partiellement ou totalement halogéné et/ou alcoxy en C₁-C₄ partiellement ou totalement halogéné;
RP
l'hydrogène ou le groupe méthyle et
R^{q}
l'hydrogène, un groupe alkyle en C₁-C₄, un groupe alcényle en C₃-C₄, un groupe alcynyle en C₃-C₄, un groupe alcoxy(C₁-C₄)-alkyle en C₁-C₄, un groupe alkylidène(C₃-C₄)iminooxy-alkyle en C₂-C₃, un groupe tétrahydrofurannylméthyle, un groupe isoxazolidine ou l'équivalent d'un cation compatible avec les plantes.

2. Agent herbicide selon la revendication 1, contenant une quantité à activité antagoniste d'un 3-amino-benzo[b]thiophène I, tandis que les substituants R² à R⁷ représentent l'hydrogène, comme composé à activité antagoniste.

3. Agent herbicide selon la revendication 1, contenant au moins un 3-aminobenzo[b]thiophène I à activité antagoniste et au moins un herbicide II et/ou un herbicide III dans le rapport 1:10 à 10:1.

4. Agent herbicide selon la revendication 2, contenant au moins un 3-aminobenzo[b]thiophène I à activité antagoniste et au moins un herbicide II et/ou un herbicide III dans le rapport 1:10 à 10:1.

5. Procédé pour la préparation de 3-aminobenzo[b]thiophènes de formule I selon la revendication 1, dans lequel R⁴ à R⁷ représentent l'hydrogène, caractérisé en ce qu'on fait réagir des nitrobenzothiazoles de formule IV 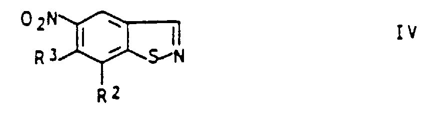 avec un ester cyanoacétique en présence d'une base et on fait réagir le cyano-benzothioazole V 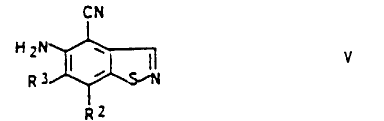 avec ClCH₂-R¹ ou BrCH₂-R¹.

6. Procédé pour la lutte sélective contre la croissance des plantes parasites sur des surfaces de culture de planes cultivées, caractérisé par le fait qu'on applique, simultanément ou successivement, une quantité à activité antagoniste d'un 3-aminobenzo[b]thiophène I et
A) une quantité à activité herbicide d'au moins un dérivé de cyclohexénone de formule II ou
B) d'un dérivé d'acide 2-(4-hétéroaryloxy)- ou 2-(4-aryloxy)-phénoxyacétique de formule III
selon la revendication 1, avant, pendant ou après le semis des plantes cultivées ou avant ou après la levée des plantes cultivées.

7. Procédé pour l'inhibition sélective de la croissance de plantes parasites sur des surfaces de culture de plantes cultivées, caractérisé par le fait qu'on traite les feuilles des plantes cultivées et des plantes parasites dans le procédé en post-levée avec un agent herbicide selon la revendication 1, tandis que les composants actifs de l'agent herbicide peuvent être appliqués simultanément ou successivement.

8. Procédé pour l'inhibition sélective de la croissance de plantes parasites sur des surfaces de culture de plantes cultivées, caractérisé par le fait qu'on traite les feuilles des plantes cultivées et des plantes parasites dans le procédé en post-levée avec un agent herbicide selon la revendication 2, tandis que les composants actifs de l'agent herbicide peuvent être appliqués simultanément ou successivement.

9. Procédé pour l'inhibition sélective de la croissance de plantes parasites sur des surfaces de culture de plantes cultivées, caractérisé par le fait qu'on traite les feuilles des plantes cultivées et des plantes parasites dans le procédé en post-levée avec un agent herbicide selon la revendication 3, tandis que les composants actifs de l'agent herbicide peuvent être appliqués simultanément ou successivement.

10. Procédé pour empêcher que soient endommagées des plantes cultivées par
A) des dérivés herbicides de cyclohexénone de formule II ou
B) des dérivés herbicides d'acide 2-(4-hétéroaryloxy)- ou 2-(4-aryloxy)-phénoxyacétique de formule III,
caractérisé par le fait qu'on traite les semences des plantes cultivées avec une quantité à activité antagoniste d'un 3-aminobenzo[b]thiophène de formule I selon la revendication 1.

11. Procédé selon la revendication 6, caractérisé par le fait que les plantes cultivées sont l'orge, le blé, le maïs, le sorgho de culture et le riz.

12. 3-Aminobenzo[b]thiophènes de formule générale I' 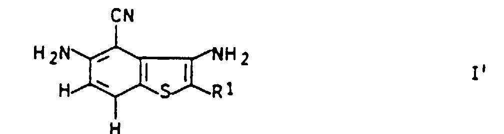 où les variables ont la signification suivante:
R¹ un groupe -COX ou -COOX, tandis que
X représente l'hydrogène, un halogène, le groupe amino, qui est non substitué ou peut porter un à trois substituants choisis dans un groupe consistant en des substituants alkyle en C₁-C₄, hydroxyalkyle en C₁-C₄, phényle et benzyle,
un groupe alkyle en C₁-C₁₀, un groupe alcényle en C₂-C₈, un groupe cycloalkyle en C₃-C₁₀ ou un groupe cycloalcényle en C₃-C₁₀, tandis que les quatre groupes mentionnés en dernier peuvent être non substitués ou partiellement ou totalement halogénés et tandis que les quatre groupes mentionnés en dernier peuvent également porter un ou deux autres groupes carboxyle ou carbonyle,
un hétérocycle aromatique à 5 ou 6 chaînons ayant un atome d'oxygène ou un atome de soufre ou ayant 1 à 3 hétéroatomes choisis dans un groupe consistant en 3 atomes d'azote et un atome d'oxygène ou de soufre, tandis que trois hétéroatomes ne peuvent pas être simultanément contigus,
le groupe phényle ou le groupe naphtyle, tandis que ces groupes peuvent porter un à trois substituants choisis dans un groupe consistant en des substituants halogène, -COY ou -COOY, et tandis que les groupes phényle et naphtyle peuvent encore porter autant d'atomes d'halogène qu'il y a d'atomes de carbone substituables présents;
Y désigne un reste alkyle en C₁-C₆, cycloalkyle en C₃-C₆ ou phényle.

13. 3-Aminobenzo[b]thiophènes de formule générale I' selon la revendication 12, tandis que R¹ représente un reste hydrogène, acyle, méthoxycarbonyle, tert-butoxycarbonyle, 1-propène-3-oxycarbonyle, n-butoxycarbonyle, 2-heptyloxycarbonyle, benzyloxycarbonyle, amide d'acide carboxylique, ester éthylique d'acide carboxyacétique, ester éthylique d'acide oxalique ou benzoyle.

14. Cyano-benzothiazoles de formule générale V 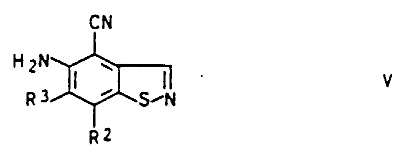 où R² et R³ ont la signification suivante:
R², R³ un reste hydrogène, cyano, nitro, halogène, mercapto, le groupe amino, dont l'atome d'azote peut porter un à trois substituants choisis dans un groupe consistant en des substituants alkyle en C₁-C₄, hydroxyalkyle en C₁-C₄, phényle et benzyle,
un groupe alkyle en C₁-C₁₀, un groupe alcoxy en C₁-C₆, un groupe alcényle en C₂-C₈, un groupe cycloalkyle en C₃-C₁₀ ou un groupe cycloalcényle en C₃-C₁₀, tandis que les quatre groupes mentionnés en dernier peuvent être partiellement ou totalement halogénés,
le groupe phényle ou le groupe naphtyle,
un hétérocycle aromatique à 5 ou 6 chaînons ayant un atome d'oxygène et un atome de soufre ou ayant un à trois hétéroatomes choisis dans un groupe consistant en trois atomes d'azote et un atome d'oxygène ou de soufre, tandis que, dans le cas de trois hétéroatomes, tous ne peuvent pas être simultanément contigus.

15. Procédé pour la préparation de cyano-benzothiazoles de formule V selon la revendication 14, caractérisé par le fait qu'on fait réagir des nitrobenzènes de formule IV 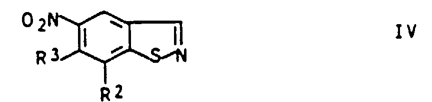 avec un ester cyanoacétique en présence d'une base.
